# EUROPEAN PATENT APPLICATION

(11) **EP 1 882 698 A1**
(43) Date of publication of application: **30.01.2008**
(21) Application number: 07004471.4
(22) Date of filing: 05.03.2007
(51) Int. Cl.: C07K 14/52, A61K 38/00, C07K 16/24

(54) **Design of interferon-inducible protein-10 (IP10 or CXCL10) chemokine analogs for the treatment of human diseases**

(30) Priority: 26.07.2006 US 494232; 30.10.2006 US 590210; 18.01.2007 EP 07001040
(71) Applicant: Chemokine Therapeutics Corp., Irvine, CA 92612 (US)
(72) Inventor: Merzouk, Ahmed, Richmond British Columbia V6V 2A4 (CA); Wong, Donald, Vancouver British Columbia V5R 1C7 (CA); Salari, Hassan, Vancouver British Columbia V6P 1W6 (CA)
(74) Representative: Vossius & Partner

(57) **Abstract**

This invention is directed to peptide analogs of interferon-inducible protein-10 (IP-10 or CXCL10) chemokine that bind to the CXCR3 receptor or any other receptor in which IP-10 analogs can bind to as a ligand, such that the analogs can be designed to serve as agonists or antagonists of IP-10 chemokine. The analogs can be used to prevent, treat, or ameliorate the symptoms of, a disease.

SEQUENCE LISTING

<110> MERZOUK, AHMED
      WONG, DONALD
      SALARI, HASSAN

<120> DESIGN OF INTERFERON-INDUCIBLE PROTEIN-10 (IP-10 OR
      CXCL10) CHEMOKINE ANALOGS FOR THE TREATMENT OF HUMAN
      DISEASES

<130> N1388 EP S3

<150> 11/494,232
<151> 2006-07-26

<150> 11/590,210
<151> 2006-10-30

<150> 07001040.0
<151> 2007-01-18

<160> 1647

<170> PatentIn version 3.3

<210> 1
<211> 77
<212> PRT
<213> Artificial Sequence

<220>
<223> Description of Artificial Sequence: Synthetic peptide

<400> 1
Ala Val Ile Pro Arg Ser Ala Lys Glu Leu Arg Cys Gln Cys Ile Lys
1               5                   10                  15

Thr Tyr Ser Lys Pro Phe His Pro Lys Phe Ile Lys Glu Leu Arg Val
            20                  25                  30

Ile Glu Ser Gly Pro His Cys Ala Asn Thr Glu Ile Ile Val Lys Leu
        35                  40                  45

Ser Asp Gly Arg Glu Leu Cys Leu Asp Pro Lys Glu Asn Trp Val Gln
    50                  55                  60

Arg Val Val Glu Lys Phe Leu Lys Arg Ala Glu Asn Ser
65                  70                  75

<210> 2
<211> 78
<212> PRT
<213> Artificial Sequence

<220>
<223> Description of Artificial Sequence: Synthetic peptide

<400> 2
Val Pro Leu Ser Arg Thr Val Arg Cys Thr Cys Ile Ser Ile Ser Asn
1               5                   10                  15

Gln Pro Val Asn Pro Pro Arg Ser Leu Glu Lys Leu Glu Ile Ile Pro

20                          25                          30

Ala Ser Gln Phe Cys Pro Arg Val Glu Ile Ile Ala Thr Met Lys Lys
            35                          40                          45

Lys Gly Glu Lys Arg Cys Leu Asn Pro Glu Ser Lys Ala Ile Lys Asn
    50                          55                          60

Leu Leu Lys Ala Val Ser Lys Glu Met Ser Lys Arg Ser Pro
65                          70                          75

<210> 3
<211> 69
<212> PRT
<213> Artificial Sequence

<220>
<223> Description of Artificial Sequence: Synthetic peptide

<400> 3
Ser Leu Ala Ala Asp Thr Pro Thr Ala Cys Cys Phe Ser Tyr Thr Ser
1               5                   10                  15

Arg Gln Ile Pro Gln Asn Phe Ile Ala Asp Tyr Phe Glu Thr Ser Ser
            20                  25                  30

Gln Cys Ser Lys Pro Gly Val Ile Phe Leu Thr Lys Arg Ser Arg Gln
        35                  40                  45

Val Cys Ala Asp Pro Ser Glu Glu Trp Val Gln Lys Tyr Val Ser Asp
    50                  55                  60

Leu Glu Leu Ser Ala
65

<210> 4
<211> 68
<212> PRT
<213> Artificial Sequence

<220>
<223> Description of Artificial Sequence: Synthetic peptide

<400> 4
Ser Pro Tyr Ser Ser Asp Thr Thr Pro Cys Cys Phe Ala Tyr Ile Ala
1               5                   10                  15

Arg Pro Leu Pro Arg Ala His Ile Lys Glu Tyr Phe Tyr Thr Ser Gly
            20                  25                  30

Lys Cys Ser Asn Pro Ala Val Val Phe Val Thr Arg Lys Asn Arg Gln
        35                  40                  45

Val Cys Ala Asn Pro Glu Lys Lys Trp Val Arg Glu Tyr Ile Asn Ser
    50                  55                  60

Leu Glu Met Ser
65

<210> 5
<211> 73
<212> PRT
<213> Artificial Sequence

<220>
<223> Description of Artificial Sequence: Synthetic peptide

<400> 5
Lys Ser Met Gln Val Pro Phe Ser Arg Cys Cys Phe Ser Phe Ala Glu
1               5                   10                  15

Gln Glu Ile Pro Leu Arg Ala Ile Leu Cys Tyr Arg Asn Thr Ser Ser
            20                  25                  30

Ile Cys Ser Asn Glu Gly Leu Ile Phe Lys Leu Lys Arg Gly Lys Glu
        35                  40                  45

Ala Cys Ala Leu Asp Thr Val Gly Trp Val Gln Arg His Arg Lys Met
    50                  55                  60

Leu Arg His Cys Pro Ser Lys Arg Lys
65                  70

<210> 6

<400> 6
000

<210> 7
<211> 76
<212> PRT
<213> Artificial Sequence

<220>
<223> Description of Artificial Sequence: Synthetic peptide

<400> 7
Gln Pro Asp Ala Ile Asn Ala Pro Val Thr Cys Cys Tyr Asn Phe Thr
1               5                   10                  15

Asn Arg Lys Ile Ser Val Gln Arg Leu Ala Ser Tyr Arg Arg Ile Thr
            20                  25                  30

Ser Ser Lys Cys Pro Lys Glu Ala Val Ile Phe Lys Thr Ile Val Ala
        35                  40                  45

Lys Glu Ile Cys Ala Asp Pro Lys Gln Lys Trp Val Gln Asp Ser Met
    50                  55                  60

Asp His Leu Asp Lys Gln Thr Gln Thr Pro Lys Thr
65                  70                  75

<210> 8
<211> 105
<212> PRT
<213> Artificial Sequence

<220>
<223> Description of Artificial Sequence: Synthetic peptide

<400> 8
Ile Leu Pro Ile Ala Ser Ser Cys Cys Thr Glu Val Ser His His Ile
1               5                   10                  15

Ser Arg Arg Leu Leu Glu Arg Val Asn Met Cys Arg Ile Gln Arg Ala
            20                  25                  30

Asp Gly Asp Cys Asp Leu Ala Ala Val Ile Leu His Val Lys Arg Arg
        35                  40                  45

Arg Ile Cys Val Ser Pro His Asn His Thr Val Lys Gln Trp Met Lys
    50                  55                  60

Val Gln Ala Ala Lys Lys Asn Gly Lys Gly Asn Val Cys His Arg Lys
65                  70                  75                  80

Lys His His Gly Lys Arg Asn Ser Asn Arg Ala His Gln Gly Lys His
                85                  90                  95

Glu Thr Tyr Gly His Lys Thr Pro Tyr
            100                 105


<210> 9
<211> 15
<212> PRT
<213> Artificial Sequence

<220>
<223> Description of Artificial Sequence: Synthetic peptide

<220>
<223> c-term may be amidated

<400> 9
Ser Ala Lys Glu Leu Arg Cys Gln Cys Ile Lys Thr Tyr Ser Lys
1               5                   10                  15


<210> 10
<211> 15
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups

and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<223> c-term may be amidated

<400> 10
Ser Ala Lys Glu Leu Arg Xaa Gln Xaa Ile Lys Thr Tyr Ser Lys
1               5                   10                  15


<210> 11
<211> 15
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<223> c-term may be amidated

<400> 11
Xaa Ala Lys Glu Leu Arg Xaa Gln Xaa Ile Lys Thr Tyr Ser Lys
1               5                   10                  15


<210> 12
<211> 15
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see

specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<223> c-term may be amidated

<400> 12
Ser Xaa Lys Glu Leu Arg Xaa Gln Xaa Ile Lys Thr Tyr Ser Lys
1               5                   10                  15


<210> 13
<211> 15
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<223> c-term may be amidated

<400> 13
Ser Ala Xaa Glu Leu Arg Xaa Gln Xaa Ile Lys Thr Tyr Ser Lys
1               5                   10                  15


<210> 14
<211> 13
<212> PRT
<213> Artificial Sequence

<220>
<223> Description of Artificial Sequence: Synthetic peptide

<220>
<223> c-term may be amidated

<400> 14
Ser Ala Lys Glu Leu Arg Cys Gln Cys Ile Lys Thr Tyr
1               5                   10


<210> 15
<211> 13
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<223> c-term may be amidated

<400> 15
Ser Ala Lys Glu Leu Arg Xaa Gln Xaa Ile Lys Thr Tyr
1               5                   10


<210> 16
<211> 13
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)

<223> Any L- or D-natural amino acid and any non-natural amino acid; see
specification as filed for detailed description of substitutions, R-groups
and preferred embodiments

<220>
<223> C-term may be amidated

<400> 16
Xaa Ala Lys Glu Leu Arg Xaa Gln Xaa Ile Lys Thr Tyr
1               5                   10


<210> 17
<211> 13
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<223> C-term may be amidated

<400> 17
Ser Xaa Lys Glu Leu Arg Xaa Gln Xaa Ile Lys Thr Tyr
1               5                   10


<210> 18
<211> 13
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES

<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<223> c-term may be amidated

<400> 18
Ser Ala Xaa Glu Leu Arg Xaa Gln Xaa Ile Lys Thr Tyr
1               5                   10


<210> 19
<211> 11
<212> PRT
<213> Artificial Sequence

<220>
<223> Description of Artificial Sequence: Synthetic peptide

<220>
<223> c-term may be amidated

<400> 19
Ser Ala Lys Glu Leu Arg Cys Gln Cys Ile Lys
1               5                   10


<210> 20
<211> 11
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<223> c-term may be amidated

<400> 20
Ser Ala Lys Glu Leu Arg Xaa Gln Xaa Ile Lys
1               5                   10


<210> 21

<211> 11
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<223> c-term may be amidated

<400> 21
Xaa Ala Lys Glu Leu Arg Xaa Gln Xaa Ile Lys
1               5                   10


<210> 22
<211> 11
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>

<223> c-term may be amidated

<400> 22
Ser Xaa Lys Glu Leu Arg Xaa Gln Xaa Ile Lys
1               5                   10


<210> 23
<211> 11
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<223> c-term may be amidated

<400> 23
Ser Ala Xaa Glu Leu Arg Xaa Gln Xaa Ile Lys
1               5                   10


<210> 24
<211> 25
<212> PRT
<213> Artificial Sequence

<220>
<223> Description of Artificial Sequence: Synthetic peptide

<220>
<223> c-term may be amidated

<400> 24
Ala Ile Lys Thr Tyr Ser Lys Pro Phe His Pro Lys Phe Ile Lys Glu
1               5                   10                  15


Leu Arg Val Ile Glu Ser Gly Pro His
            20                  25


<210> 25
<211> 16
<212> PRT

<213> Artificial Sequence

<220>
<223> Description of Artificial Sequence: Synthetic peptide

<220>
<223> c-term may be amidated

<400> 25
Ala Ala Asn Thr Glu Ile Ile Val Lys Leu Ser Asp Gly Arg Glu Leu
1               5                   10                  15


<210> 26
<211> 22
<212> PRT
<213> Artificial Sequence

<220>
<223> Description of Artificial Sequence: Synthetic peptide

<220>
<223> c-term may be amidated

<400> 26
Leu Asp Pro Lys Glu Asn Trp Val Gln Arg Val Val Glu Lys Phe Leu
1               5                   10                  15


Lys Arg Ala Glu Asn Ser
            20


<210> 27
<211> 35
<212> PRT
<213> Artificial Sequence

<220>
<223> Description of Artificial Sequence: Synthetic peptide


<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 27
Ser Ala Lys Glu Leu Arg Cys Gln Cys Ile Lys Thr Tyr Ser Lys Xaa
1               5                   10                  15


Xaa Xaa Xaa Asn Trp Val Gln Arg Val Val Glu Lys Phe Leu Lys Arg
            20                  25                  30


Ala Glu Asn
        35


<210> 28
<211> 35
<212> PRT

<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 28
Ser Ala Lys Glu Leu Arg Xaa Gln Xaa Ile Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 29
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES

<222> (9)..(9)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 29
Xaa Ala Lys Glu Leu Arg Xaa Gln Xaa Ile Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 30
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 30
Ser Xaa Lys Glu Leu Arg Xaa Gln Xaa Ile Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 31
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 31
Ser Ala Xaa Glu Leu Arg Xaa Gln Xaa Ile Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 32
<211> 35

<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 32
Ser Ala Lys Glu Leu Arg Xaa Gln Xaa Xaa Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 33
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>

<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 33
Xaa Ala Lys Glu Leu Arg Xaa Gln Xaa Xaa Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 34
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 34
Ser Xaa Lys Glu Leu Arg Xaa Gln Xaa Xaa Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 35
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 35
Ser Ala Xaa Glu Leu Arg Xaa Gln Xaa Xaa Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 36

<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 36
Ser Ala Lys Glu Leu Arg Xaa Gln Xaa Ile Xaa Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 37
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 37
Xaa Ala Lys Glu Leu Arg Xaa Gln Xaa Ile Xaa Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 38
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 38
Ser Xaa Lys Glu Leu Arg Xaa Gln Xaa Ile Xaa Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 39
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 39
Ser Ala Xaa Glu Leu Arg Xaa Gln Xaa Ile Xaa Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 40
<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 40
Ser Ala Lys Glu Leu Arg Xaa Gln Xaa Ile Lys Xaa Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 41
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 41
Xaa Ala Lys Glu Leu Arg Xaa Gln Xaa Ile Lys Xaa Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 42
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 42
Ser Xaa Lys Glu Leu Arg Xaa Gln Xaa Ile Lys Xaa Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 43
<211> 35
<212> PRT

<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 43
Ser Ala Xaa Glu Leu Arg Xaa Gln Xaa Ile Lys Xaa Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 44
<211> 35
<212> PRT
<213> Artificial Sequence

<220>
<223> Description of Artificial Sequence: Synthetic peptide

<220>
<221> MOD_RES

<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 44
Ser Ala Lys Glu Leu Arg Xaa Gln Xaa Ile Lys Thr Xaa Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 45
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)

<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 45
Xaa Ala Lys Glu Leu Arg Xaa Gln Xaa Ile Lys Thr Xaa Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 46
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see

specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 46
Ser Xaa Lys Glu Leu Arg Xaa Gln Xaa Ile Lys Thr Xaa Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 47
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 47
Ser Ala Xaa Glu Leu Arg Xaa Gln Xaa Ile Lys Thr Xaa Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 48
<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 48
Ser Ala Lys Glu Leu Arg Xaa Gln Xaa Ile Lys Thr Tyr Xaa Lys Xaa
1               5                   10                  15

Xaa Xaa Xaa Asn Trp Val Gln Arg Val Val Glu Lys Phe Leu Lys Arg
            20                  25                  30

Ala Glu Asn

        35

<210> 49
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 49
Xaa Ala Lys Glu Leu Arg Xaa Gln Xaa Ile Lys Thr Tyr Xaa Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 50
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 50
Ser Xaa Lys Glu Leu Arg Xaa Gln Xaa Ile Lys Thr Tyr Xaa Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 51
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see

specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 51
Ser Ala Xaa Glu Leu Arg Xaa Gln Xaa Ile Lys Thr Tyr Xaa Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 52
<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 52
Ser Ala Lys Glu Leu Arg Xaa Gln Xaa Ile Lys Thr Tyr Ser Xaa Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 53
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 53
Xaa Ala Lys Glu Leu Arg Xaa Gln Xaa Ile Lys Thr Tyr Ser Xaa Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 54
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 54
Ser Xaa Lys Glu Leu Arg Xaa Gln Xaa Ile Lys Thr Tyr Ser Xaa Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 55
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 55
Ser Ala Xaa Glu Leu Arg Xaa Gln Xaa Ile Lys Thr Tyr Ser Xaa Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 56
<211> 33
<212> PRT
<213> Artificial Sequence

<220>
<223> Description of Artificial Sequence: Synthetic peptide

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 56
Ser Ala Lys Glu Leu Arg Cys Gln Cys Ile Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 57
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 57
Ser Ala Lys Glu Leu Arg Xaa Gln Xaa Ile Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 58
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 58
Xaa Ala Lys Glu Leu Arg Xaa Gln Xaa Ile Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 59
<211> 33
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments
<220>
<223> c-term may be amidated

<400> 59
Ser Xaa Lys Glu Leu Arg Xaa Gln Xaa Ile Lys Thr Tyr Xaa Xaa Xaa
1               5                   10                  15


Xaa Asn Trp Val Gln Arg Val Val Glu Lys Phe Leu Lys Arg Ala Glu
            20                  25                  30


Asn




<210> 60
<211> 33
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 60
Ser Ala Xaa Glu Leu Arg Xaa Gln Xaa Ile Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 61
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 61
Ser Ala Lys Glu Leu Arg Xaa Gln Xaa Xaa Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 62
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 62
Xaa Ala Lys Glu Leu Arg Xaa Gln Xaa Xaa Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 63
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 63
Ser Xaa Lys Glu Leu Arg Xaa Gln Xaa Xaa Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 64
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 64
Ser Ala Xaa Glu Leu Arg Xaa Gln Xaa Xaa Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 65
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 65
Ser Ala Lys Glu Leu Arg Xaa Gln Xaa Ile Xaa Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 66
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 66
Xaa Ala Lys Glu Leu Arg Xaa Gln Xaa Ile Xaa Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 67
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 67
Ser Xaa Lys Glu Leu Arg Xaa Gln Xaa Ile Xaa Thr Tyr Xaa Xaa Xaa
1               5                   10                  15

Xaa Asn Trp Val Gln Arg Val Val Glu Lys Phe Leu Lys Arg Ala Glu

20                  25                  30

Asn

<210> 68
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 68
Ser Ala Xaa Glu Leu Arg Xaa Gln Xaa Ile Xaa Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 69

<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 69
Ser Ala Lys Glu Leu Arg Xaa Gln Xaa Ile Lys Xaa Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 70
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 70
Xaa Ala Lys Glu Leu Arg Xaa Gln Xaa Ile Lys Xaa Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 71
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 71
Ser Xaa Lys Glu Leu Arg Xaa Gln Xaa Ile Lys Xaa Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 72
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (12)..(12)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 72
Ser Ala Xaa Glu Leu Arg Xaa Gln Xaa Ile Lys Xaa Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 73
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 73
Ser Ala Lys Glu Leu Arg Xaa Gln Xaa Ile Lys Thr Xaa Xaa Xaa Xaa
1               5                   10                  15

Xaa Asn Trp Val Gln Arg Val Val Glu Lys Phe Leu Lys Arg Ala Glu
            20                  25                  30

Asn

<210> 74
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 74
Xaa Ala Lys Glu Leu Arg Xaa Gln Xaa Ile Lys Thr Xaa Xaa Xaa Xaa
1               5                   10                  15

Xaa Asn Trp Val Gln Arg Val Val Glu Lys Phe Leu Lys Arg Ala Glu
            20                  25                  30

Asn

<210> 75
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 75
Ser Xaa Lys Glu Leu Arg Xaa Gln Xaa Ile Lys Thr Xaa Xaa Xaa Xaa
1               5                   10                  15

Xaa Asn Trp Val Gln Arg Val Val Glu Lys Phe Leu Lys Arg Ala Glu
            20                  25                  30

Asn

<210> 76
<211> 33
<212> PRT

<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 76
Ser Ala Xaa Glu Leu Arg Xaa Gln Xaa Ile Lys Thr Xaa Xaa Xaa Xaa
1               5                   10                  15

Xaa Asn Trp Val Gln Arg Val Val Glu Lys Phe Leu Lys Arg Ala Glu
            20                  25                  30

Asn

<210> 77
<211> 31
<212> PRT
<213> Artificial Sequence

<220>
<223> Description of Artificial Sequence: Synthetic peptide

<220>
<221> MOD_RES

<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 77
Ser Ala Lys Glu Leu Arg Cys Gln Cys Ile Lys Xaa Xaa Xaa Xaa Asn
1               5                   10                  15

Trp Val Gln Arg Val Val Glu Lys Phe Leu Lys Arg Ala Glu Asn
            20                  25                  30

<210> 78
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 78
Ser Ala Lys Glu Leu Arg Xaa Gln Xaa Ile Lys Xaa Xaa Xaa Xaa Asn
1               5                   10                  15

Trp Val Gln Arg Val Val Glu Lys Phe Leu Lys Arg Ala Glu Asn
            20                  25                  30

<210> 79
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 79
Xaa Ala Lys Glu Leu Arg Xaa Gln Xaa Ile Lys Xaa Xaa Xaa Xaa Asn
1               5                   10                  15

Trp Val Gln Arg Val Val Glu Lys Phe Leu Lys Arg Ala Glu Asn
            20                  25                  30


<210> 80
<211> 31
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 80
Ser Xaa Lys Glu Leu Arg Xaa Gln Xaa Ile Lys Xaa Xaa Xaa Xaa Asn
1               5                   10                  15

Trp Val Gln Arg Val Val Glu Lys Phe Leu Lys Arg Ala Glu Asn
            20                  25                  30

<210> 81
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 81
Ser Ala Xaa Glu Leu Arg Xaa Gln Xaa Ile Lys Xaa Xaa Xaa Xaa Asn
1               5                   10                  15

Trp Val Gln Arg Val Val Glu Lys Phe Leu Lys Arg Ala Glu Asn
            20                  25                  30

<210> 82
<211> 31
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 82
Ser Ala Lys Glu Leu Arg Xaa Gln Xaa Xaa Lys Xaa Xaa Xaa Xaa Asn
1               5                   10                  15


Trp Val Gln Arg Val Val Glu Lys Phe Leu Lys Arg Ala Glu Asn
            20                  25                  30


<210> 83
<211> 31
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>

<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 83
Xaa Ala Lys Glu Leu Arg Xaa Gln Xaa Xaa Lys Xaa Xaa Xaa Xaa Asn
1               5                   10                  15

Trp Val Gln Arg Val Val Glu Lys Phe Leu Lys Arg Ala Glu Asn
            20                  25                  30

<210> 84
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 84
Ser Xaa Lys Glu Leu Arg Xaa Gln Xaa Xaa Lys Xaa Xaa Xaa Xaa Asn
1               5                   10                  15

Trp Val Gln Arg Val Val Glu Lys Phe Leu Lys Arg Ala Glu Asn
            20                  25                  30

<210> 85
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 85
Ser Ala Xaa Glu Leu Arg Xaa Gln Xaa Xaa Lys Xaa Xaa Xaa Xaa Asn
1               5                   10                  15

Trp Val Gln Arg Val Val Glu Lys Phe Leu Lys Arg Ala Glu Asn
            20                  25                  30

<210> 86
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 86
Ser Ala Lys Glu Leu Arg Xaa Gln Xaa Ile Xaa Xaa Xaa Xaa Xaa Asn
1               5                   10                  15

Trp Val Gln Arg Val Val Glu Lys Phe Leu Lys Arg Ala Glu Asn
            20                  25                  30

<210> 87
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 87
Xaa Ala Lys Glu Leu Arg Xaa Gln Xaa Ile Xaa Xaa Xaa Xaa Xaa Asn
1               5                   10                  15

Trp Val Gln Arg Val Val Glu Lys Phe Leu Lys Arg Ala Glu Asn
            20                  25                  30

<210> 88
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 88
Ser Xaa Lys Glu Leu Arg Xaa Gln Xaa Ile Xaa Xaa Xaa Xaa Xaa Asn
1               5                   10                  15

Trp Val Gln Arg Val Val Glu Lys Phe Leu Lys Arg Ala Glu Asn
            20                  25                  30

<210> 89
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 89
Ser Ala Xaa Glu Leu Arg Xaa Gln Xaa Ile Xaa Xaa Xaa Xaa Xaa Asn
1               5                   10                  15

Trp Val Gln Arg Val Val Glu Lys Phe Leu Lys Arg Ala Glu Asn
            20                  25                  30

<210> 90
<211> 13
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<223> c-term may be amidated

<400> 90
Xaa Ala Lys Glu Leu Arg Xaa Gln Xaa Ile Lys Thr Xaa
1               5                   10


<210> 91
<211> 13
<212> PRT
<213> Artificial Sequence

<220>
<223> Description of Artificial Sequence: Synthetic peptide


<220>
<221> MOD_RES
<222> (1)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<223> c-term may be amidated

<400> 91
Xaa Xaa Lys Glu Leu Arg Xaa Gln Xaa Ile Lys Thr Xaa
1               5                   10


<210> 92
<211> 13
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<223> c-term may be amidated

<400> 92
Xaa Ala Xaa Glu Leu Arg Xaa Gln Xaa Ile Lys Thr Xaa
1               5                   10

<210> 93
<211> 26
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (26)..(26)
<223> Any L- or D-natural amino acid and any non-natural amino acid; see
specification as filed for detailed description of substitutions, R-groups
and preferred embodiments

<220>
<223> c-term may be amidated

<400> 93
Xaa Ile Lys Thr Tyr Ser Lys Pro Phe His Pro Lys Phe Ile Lys Glu
1               5                   10                  15

Leu Arg Val Ile Glu Ser Gly Pro His Xaa
            20                  25

<210> 94
<211> 17
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<223> c-term may be amidated

<400> 94

Xaa Ala Asn Thr Glu Ile Ile Val Lys Leu Ser Asp Gly Arg Glu Leu
1               5                   10                  15

Xaa

<210> 95
<211> 23
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (23)..(23)
<223> Any L- or D-natural amino acid and any non-natural amino acid; see
specification as filed for detailed description of substitutions, R-groups
and preferred embodiments

<220>
<223> C-term may be amidated

<400> 95
Xaa Leu Asp Pro Lys Glu Asn Trp Val Gln Arg Val Val Glu Lys Phe
1               5                   10                  15

Leu Lys Arg Ala Glu Asn Xaa
            20

<210> 96
<211> 23
<212> PRT
<213> Artificial Sequence

<220>
<223> Description of Artificial Sequence: Synthetic peptide

<220>
<223> C-term may be amidated

<400> 96
Ala Leu Asp Pro Lys Glu Asn Trp Val Gln Arg Val Val Glu Lys Phe
1               5                   10                  15

Leu Lys Arg Ala Glu Asn Ser
            20

<210> 97
<211> 23
<212> PRT
<213> Artificial Sequence

<220>
<223> Description of Artificial Sequence: Synthetic peptide

<220>
<223> c-term may be amidated

<400> 97
Ala Leu Asp Pro Lys Glu Asn Trp Val Gln Arg Val Val Glu Lys Phe
1               5                   10                  15

Leu Lys Arg Ala Glu Asn Ser
            20

<210> 98

<400> 98
000

<210> 99
<211> 23
<212> PRT
<213> Artificial Sequence

<220>
<223> Description of Artificial Sequence: Synthetic peptide

<220>
<223> c-term may be amidated

<400> 99
Ala Leu Asp Pro Lys Glu Asn Trp Val Gln Arg Val Val Glu Lys Phe
1               5                   10                  15

Leu Lys Arg Ala Glu Asn Ser
            20

<210> 100
<211> 35
<212> PRT
<213> Artificial Sequence

<220>
<223> Description of Artificial Sequence: Synthetic peptide

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 100
Ser Ala Lys Glu Leu Arg Cys Gln Cys Ile Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 101
<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 101
Ser Ala Lys Glu Leu Arg Xaa Gln Xaa Ile Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 102
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>

<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 102
Xaa Ala Lys Glu Leu Arg Xaa Gln Xaa Ile Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 103
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 103
Ser Xaa Lys Glu Leu Arg Xaa Gln Xaa Ile Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 104
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 104
Ser Ala Xaa Glu Leu Arg Xaa Gln Xaa Ile Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 105
<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 105
Ser Ala Lys Glu Leu Arg Xaa Gln Xaa Xaa Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 106
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 106
Xaa Ala Lys Glu Leu Arg Xaa Gln Xaa Xaa Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 107
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>

<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 107
Ser Xaa Lys Glu Leu Arg Xaa Gln Xaa Xaa Lys Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 108
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 108
Ser Ala Xaa Glu Leu Arg Xaa Gln Xaa Xaa Lys Thr Tyr Ser Lys Xaa
1               5                   10                  15

Xaa Xaa Xaa Asn Trp Val Gln Arg Val Val Glu Lys Phe Leu Lys Arg

20                  25                  30

Ala Glu Asn
        35

<210> 109
<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 109
Ser Ala Lys Glu Leu Arg Xaa Gln Xaa Ile Xaa Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 110
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 110
Xaa Ala Lys Glu Leu Arg Xaa Gln Xaa Ile Xaa Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 111
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 111
Ser Xaa Lys Glu Leu Arg Xaa Gln Xaa Ile Xaa Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 112
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 112
Ser Ala Xaa Glu Leu Arg Xaa Gln Xaa Ile Xaa Thr Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 113
<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES

<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 113
Ser Ala Lys Glu Leu Arg Xaa Gln Xaa Ile Lys Xaa Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 114
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 114
Xaa Ala Lys Glu Leu Arg Xaa Gln Xaa Ile Lys Xaa Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 115
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 115
Ser Xaa Lys Glu Leu Arg Xaa Gln Xaa Ile Lys Xaa Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 116
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 116
Ser Ala Xaa Glu Leu Arg Xaa Gln Xaa Ile Lys Xaa Tyr Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 117
<211> 35
<212> PRT

<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 117
Ser Ala Lys Glu Leu Arg Xaa Gln Xaa Ile Lys Thr Xaa Ser Lys Xaa
1               5                   10                  15


Xaa Xaa Xaa Asn Trp Val Gln Arg Val Val Glu Lys Phe Leu Lys Arg
            20                  25                  30


Ala Glu Asn
        35


<210> 118
<211> 35
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES

<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 118
Xaa Ala Lys Glu Leu Arg Xaa Gln Xaa Ile Lys Thr Xaa Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 119
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)

<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 119
Ser Xaa Lys Glu Leu Arg Xaa Gln Xaa Ile Lys Thr Xaa Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 120
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see

specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 120
Ser Ala Xaa Glu Leu Arg Xaa Gln Xaa Ile Lys Thr Xaa Ser Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 121
<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 121
Ser Ala Lys Glu Leu Arg Xaa Gln Xaa Ile Lys Thr Tyr Xaa Lys Xaa

1                   5                   10                  15

Xaa Xaa Xaa Asn Trp Val Gln Arg Val Val Glu Lys Phe Leu Lys Arg
            20                  25                  30

Ala Glu Asn
        35

<210> 122
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 122
Xaa Ala Lys Glu Leu Arg Xaa Gln Xaa Ile Lys Thr Tyr Xaa Lys Xaa
1               5                   10                  15

Xaa Xaa Xaa Asn Trp Val Gln Arg Val Val Glu Lys Phe Leu Lys Arg
            20                  25                  30

Ala Glu Asn

        35

<210> 123
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 123
Ser Xaa Lys Glu Leu Arg Xaa Gln Xaa Ile Lys Thr Tyr Xaa Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 124
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 124
Ser Ala Xaa Glu Leu Arg Xaa Gln Xaa Ile Lys Thr Tyr Xaa Lys Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 125
<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 125
Ser Ala Lys Glu Leu Arg Xaa Gln Xaa Ile Lys Thr Tyr Ser Xaa Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 126
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 126
Xaa Ala Lys Glu Leu Arg Xaa Gln Xaa Ile Lys Thr Tyr Ser Xaa Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 127
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 127
Ser Xaa Lys Glu Leu Arg Xaa Gln Xaa Ile Lys Thr Tyr Ser Xaa Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 128
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (16)..(19)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 128
Ser Ala Xaa Glu Leu Arg Xaa Gln Xaa Ile Lys Thr Tyr Ser Xaa Xaa
1               5                   10                  15

Xaa Xaa Xaa Asn Trp Val Gln Arg Val Val Glu Lys Phe Leu Lys Arg
            20                  25                  30

Ala Glu Asn
        35

<210> 129
<211> 33
<212> PRT
<213> Artificial Sequence

<220>
<223> Description of Artificial Sequence: Synthetic peptide

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 129
Ser Ala Lys Glu Leu Arg Cys Gln Cys Ile Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 130
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see

specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 130
Ser Ala Lys Glu Leu Arg Xaa Gln Xaa Ile Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 131
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 131
Xaa Ala Lys Glu Leu Arg Xaa Gln Xaa Ile Lys Thr Tyr Xaa Xaa Xaa

1                   5                   10                  15

Xaa Asn Trp Val Gln Arg Val Val Glu Lys Phe Leu Lys Arg Ala Glu
            20                  25                  30

Asn

<210> 132
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 132
Ser Xaa Lys Glu Leu Arg Xaa Gln Xaa Ile Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 133
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 133
Ser Ala Xaa Glu Leu Arg Xaa Gln Xaa Ile Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 134
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)

<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 134
Ser Ala Lys Glu Leu Arg Xaa Gln Xaa Xaa Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 135
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 135

Xaa Ala Lys Glu Leu Arg Xaa Gln Xaa Xaa Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 136
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 136
Ser Xaa Lys Glu Leu Arg Xaa Gln Xaa Xaa Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 137
<211> 33
<212> PRT

<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 137
Ser Ala Xaa Glu Leu Arg Xaa Gln Xaa Xaa Lys Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 138
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES

<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 138
Ser Ala Lys Glu Leu Arg Xaa Gln Xaa Ile Xaa Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 139
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)

<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 139
Xaa Ala Lys Glu Leu Arg Xaa Gln Xaa Ile Xaa Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 140
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 140
Ser Xaa Lys Glu Leu Arg Xaa Gln Xaa Ile Xaa Thr Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 141
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 141
Ser Ala Xaa Glu Leu Arg Xaa Gln Xaa Ile Xaa Thr Tyr Xaa Xaa Xaa

1                5                   10                  15

Xaa Asn Trp Val Gln Arg Val Val Glu Lys Phe Leu Lys Arg Ala Glu
            20                  25                  30

Asn

<210> 142
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 142
Ser Ala Lys Glu Leu Arg Xaa Gln Xaa Ile Lys Xaa Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 143
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 143
Xaa Ala Lys Glu Leu Arg Xaa Gln Xaa Ile Lys Xaa Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 144
<211> 33
<212> PRT
<213> Artificial Sequence

<220>
<223> Description of Artificial Sequence: Synthetic peptide

<220>
<221> MOD_RES
<222> (2)..(2)

<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 144
Ser Xaa Lys Glu Leu Arg Xaa Gln Xaa Ile Lys Xaa Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 145
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 145
Ser Ala Xaa Glu Leu Arg Xaa Gln Xaa Ile Lys Xaa Tyr Xaa Xaa Xaa
1               5                   10                  15

Xaa Asn Trp Val Gln Arg Val Val Glu Lys Phe Leu Lys Arg Ala Glu
            20                  25                  30

Asn

<210> 146
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups

and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 146
Ser Ala Lys Glu Leu Arg Xaa Gln Xaa Ile Lys Thr Xaa Xaa Xaa Xaa
1               5                   10                  15

Xaa Asn Trp Val Gln Arg Val Val Glu Lys Phe Leu Lys Arg Ala Glu
            20                  25                  30

Asn

<210> 147
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 147
Xaa Ala Lys Glu Leu Arg Xaa Gln Xaa Ile Lys Thr Xaa Xaa Xaa Xaa
1               5                   10                  15

Xaa Asn Trp Val Gln Arg Val Val Glu Lys Phe Leu Lys Arg Ala Glu
            20                  25                  30

Asn

<210> 148
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 148
Ser Xaa Lys Glu Leu Arg Xaa Gln Xaa Ile Lys Thr Xaa Xaa Xaa Xaa
1               5                   10                  15

Xaa Asn Trp Val Gln Arg Val Val Glu Lys Phe Leu Lys Arg Ala Glu
            20                  25                  30

Asn

<210> 149
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (14)..(17)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 149
Ser Ala Xaa Glu Leu Arg Xaa Gln Xaa Ile Lys Thr Xaa Xaa Xaa Xaa
1               5                   10                  15

Xaa Asn Trp Val Gln Arg Val Val Glu Lys Phe Leu Lys Arg Ala Glu
            20                  25                  30

Asn

<210> 150
<211> 31
<212> PRT
<213> Artificial Sequence

<220>
<223> Description of Artificial Sequence: Synthetic peptide

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 150
Ser Ala Lys Glu Leu Arg Cys Gln Cys Ile Lys Xaa Xaa Xaa Xaa Asn
1               5                   10                  15

Trp Val Gln Arg Val Val Glu Lys Phe Leu Lys Arg Ala Glu Asn
            20                  25                  30

<210> 151
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 151
Ser Ala Lys Glu Leu Arg Xaa Gln Xaa Ile Lys Xaa Xaa Xaa Xaa Asn
1               5                   10                  15

Trp Val Gln Arg Val Val Glu Lys Phe Leu Lys Arg Ala Glu Asn

20                  25                  30

<210> 152
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 152
Xaa Ala Lys Glu Leu Arg Xaa Gln Xaa Ile Lys Xaa Xaa Xaa Xaa Asn
1               5                   10                  15

Trp Val Gln Arg Val Val Glu Lys Phe Leu Lys Arg Ala Glu Asn
            20                  25                  30

<210> 153
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 153
Ser Xaa Lys Glu Leu Arg Xaa Gln Xaa Ile Lys Xaa Xaa Xaa Xaa Asn
1               5                   10                  15

Trp Val Gln Arg Val Val Glu Lys Phe Leu Lys Arg Ala Glu Asn
            20                  25                  30

<210> 154
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 154
Ser Ala Xaa Glu Leu Arg Xaa Gln Xaa Ile Lys Xaa Xaa Xaa Xaa Asn
1               5                   10                  15

Trp Val Gln Arg Val Val Glu Lys Phe Leu Lys Arg Ala Glu Asn
            20                  25                  30

<210> 155
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 155
Ser Ala Lys Glu Leu Arg Xaa Gln Xaa Xaa Lys Xaa Xaa Xaa Xaa Asn
1               5                   10                  15

Trp Val Gln Arg Val Val Glu Lys Phe Leu Lys Arg Ala Glu Asn
            20                  25                  30

<210> 156
<211> 31
<212> PRT
<213> Artificial Sequence

<220>
<223> Description of Artificial Sequence: Synthetic peptide

<220>
<221> MOD_RES
<222> (1)..(1)

<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 156
Xaa Ala Lys Glu Leu Arg Xaa Gln Xaa Xaa Lys Xaa Xaa Xaa Xaa Asn
1               5                   10                  15

Trp Val Gln Arg Val Val Glu Lys Phe Leu Lys Arg Ala Glu Asn
            20                  25                  30

<210> 157
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>

<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 157
Ser Xaa Lys Glu Leu Arg Xaa Gln Xaa Xaa Lys Xaa Xaa Xaa Xaa Asn
1               5                   10                  15

Trp Val Gln Arg Val Val Glu Lys Phe Leu Lys Arg Ala Glu Asn
            20                  25                  30

<210> 158
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 158
Ser Ala Xaa Glu Leu Arg Xaa Gln Xaa Xaa Lys Xaa Xaa Xaa Xaa Asn
1               5                   10                  15

Trp Val Gln Arg Val Val Glu Lys Phe Leu Lys Arg Ala Glu Asn
            20                  25                  30

<210> 159

<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 159
Ser Ala Lys Glu Leu Arg Xaa Gln Xaa Ile Xaa Xaa Xaa Xaa Xaa Asn
1               5                   10                  15

Trp Val Gln Arg Val Val Glu Lys Phe Leu Lys Arg Ala Glu Asn
            20                  25                  30

<210> 160
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 160
Xaa Ala Lys Glu Leu Arg Xaa Gln Xaa Ile Xaa Xaa Xaa Xaa Xaa Asn
1               5                   10                  15

Trp Val Gln Arg Val Val Glu Lys Phe Leu Lys Arg Ala Glu Asn
            20                  25                  30

<210> 161
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 161
Ser Xaa Lys Glu Leu Arg Xaa Gln Xaa Ile Xaa Xaa Xaa Xaa Xaa Asn
1               5                   10                  15

Trp Val Gln Arg Val Val Glu Lys Phe Leu Lys Arg Ala Glu Asn
            20                  25                  30

<210> 162
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (12)..(15)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 162
Ser Ala Xaa Glu Leu Arg Xaa Gln Xaa Ile Xaa Xaa Xaa Xaa Xaa Asn
1               5                   10                  15

Trp Val Gln Arg Val Val Glu Lys Phe Leu Lys Arg Ala Glu Asn
            20                  25                  30

<210> 163
<211> 14
<212> PRT
<213> Artificial Sequence

<220>
<223> Description of Artificial Sequence: Synthetic peptide

<220>
<223> c-term may be amidated

<400> 163
Val Pro Leu Ser Arg Thr Val Arg Cys Thr Cys Ile Ser Ile
1               5                   10

<210> 164
<211> 14
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 164
Val Pro Xaa Ser Arg Thr Val Arg Xaa Thr Xaa Ile Ser Ile
1               5                   10

<210> 165
<211> 14

<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 165
Val Pro Leu Xaa Arg Thr Val Arg Xaa Thr Xaa Ile Ser Ile
1               5                   10


<210> 166
<211> 14
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 166
Val Pro Leu Ser Xaa Thr Val Arg Xaa Thr Xaa Ile Ser Ile
1               5                   10


<210> 167
<211> 14
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 167
Val Pro Leu Ser Arg Xaa Val Arg Xaa Thr Xaa Ile Ser Ile
1               5                   10


<210> 168
<211> 14
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>

<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 168
Val Pro Leu Ser Arg Thr Xaa Arg Xaa Thr Xaa Ile Ser Ile
1               5                   10


<210> 169
<211> 15
<212> PRT
<213> Artificial Sequence

<220>
<223> Description of Artificial Sequence: Synthetic peptide


<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<223> c-term may be amidated

<400> 169
Val Pro Leu Ser Arg Thr Val Arg Xaa Xaa Thr Xaa Ile Ser Ile
1               5                   10                  15


<210> 170
<211> 17
<212> PRT
<213> Artificial Sequence

<220>
<223> Description of Artificial Sequence: Synthetic peptide

<220>
<223> c-term may be amidated

<400> 170
Val Pro Leu Ser Arg Thr Val Arg Cys Thr Cys Ile Ser Ile Ser Asn
1               5                   10                  15


Gln



<210> 171
<211> 17
<212> PRT

<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> C-term may be amidated

<400> 171
Val Pro Xaa Ser Arg Thr Val Arg Xaa Thr Xaa Ile Ser Ile Ser Asn
1               5                   10                  15

Gln

<210> 172
<211> 17
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 172
Val Pro Leu Xaa Arg Thr Val Arg Xaa Thr Xaa Ile Ser Ile Ser Asn
1               5                   10                  15

Gln

<210> 173
<211> 17
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 173
Val Pro Leu Ser Xaa Thr Val Arg Xaa Thr Xaa Ile Ser Ile Ser Asn
1               5                   10                  15

Gln

<210> 174
<211> 17
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 174
Val Pro Leu Ser Arg Xaa Val Arg Xaa Thr Xaa Ile Ser Ile Ser Asn
1               5                   10                  15

Gln

<210> 175
<211> 17
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 175
Val Pro Leu Ser Arg Thr Xaa Arg Xaa Thr Xaa Ile Ser Ile Ser Asn
1               5                   10                  15

Gln

<210> 176
<211> 18
<212> PRT
<213> Artificial Sequence

<220>
<223> Description of Artificial Sequence: Synthetic peptide


<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<223> c-term may be amidated

<400> 176
Val Pro Leu Ser Arg Thr Val Arg Xaa Xaa Thr Xaa Ile Ser Ile Ser
1               5                   10                  15


Asn Gln



<210> 177
<211> 25
<212> PRT
<213> Artificial Sequence

<220>
<223> Description of Artificial Sequence: Synthetic peptide

<220>
<223> c-term may be amidated

<400> 177
Ala Ile Ser Ile Ser Asn Gln Pro Val Asn Pro Arg Ser Leu Glu Lys
1               5                   10                  15


Leu Glu Ile Ile Pro Ala Ser Gln Phe
            20                  25


<210> 178
<211> 35
<212> PRT
<213> Artificial Sequence

<220>
<223> Description of Artificial Sequence: Synthetic peptide

<220>
<223> c-term may be amidated

<400> 178
Val Pro Leu Ser Arg Thr Val Arg Cys Thr Cys Ile Ser Ile Ser Asn
1               5                   10                  15

Gln Pro Val Asn Pro Arg Ser Leu Glu Lys Leu Glu Ile Ile Pro Ala
            20                  25                  30

Ser Gln Phe
        35

<210> 179
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> C-term may be amidated

<400> 179
Xaa Pro Leu Ser Arg Thr Val Arg Xaa Thr Xaa Ile Ser Ile Ser Asn
1               5                   10                  15

Gln Pro Val Asn Pro Arg Ser Leu Glu Lys Leu Glu Ile Ile Pro Ala
            20                  25                  30

Ser Gln Phe
        35

<210> 180
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 180
Val Xaa Leu Ser Arg Thr Val Arg Xaa Thr Xaa Ile Ser Ile Ser Asn
1               5                   10                  15

Gln Pro Val Asn Pro Arg Ser Leu Glu Lys Leu Glu Ile Ile Pro Ala
            20                  25                  30

Ser Gln Phe
        35

<210> 181
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 181
Val Pro Xaa Ser Arg Thr Val Arg Xaa Thr Xaa Ile Ser Ile Ser Asn
1               5                   10                  15

Gln Pro Val Asn Pro Arg Ser Leu Glu Lys Leu Glu Ile Ile Pro Ala
            20                  25                  30

Ser Gln Phe
        35

<210> 182
<211> 35
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 182
Val Pro Leu Xaa Arg Thr Val Arg Xaa Thr Xaa Ile Ser Ile Ser Asn
1               5                   10                  15

Gln Pro Val Asn Pro Arg Ser Leu Glu Lys Leu Glu Ile Ile Pro Ala
            20                  25                  30

Ser Gln Phe
        35

<210> 183
<211> 35
<212> PRT
<213> Artificial Sequence

<220>

<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 183
Val Pro Leu Ser Xaa Thr Val Arg Xaa Thr Xaa Ile Ser Ile Ser Asn
1               5                   10                  15

Gln Pro Val Asn Pro Arg Ser Leu Glu Lys Leu Glu Ile Ile Pro Ala
            20                  25                  30

Ser Gln Phe
        35

<210> 184
<211> 35
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups

and preferred embodiments

<220>
<223> c-term may be amidated

<400> 184
Val Pro Leu Ser Arg Xaa Val Arg Xaa Thr Xaa Ile Ser Ile Ser Asn
1               5                   10                  15

Gln Pro Val Asn Pro Arg Ser Leu Glu Lys Leu Glu Ile Ile Pro Ala
            20                  25                  30

Ser Gln Phe
        35

<210> 185
<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 185
Val Pro Leu Ser Arg Thr Xaa Arg Xaa Thr Xaa Ile Ser Ile Ser Asn
1               5                   10                  15

Gln Pro Val Asn Pro Arg Ser Leu Glu Lys Leu Glu Ile Ile Pro Ala
            20                  25                  30

Ser Gln Phe
        35

<210> 186
<211> 35
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<223> c-term may be amidated

<400> 186
Val Pro Leu Ser Arg Thr Val Xaa Xaa Thr Xaa Ile Ser Ile Ser Asn
1               5                   10                  15

Gln Pro Val Asn Pro Arg Ser Leu Glu Lys Leu Glu Ile Ile Pro Ala
            20                  25                  30

Ser Gln Phe
        35

<210> 187
<211> 24
<212> PRT
<213> Artificial Sequence

<220>
<223> Description of Artificial Sequence: Synthetic peptide

<220>
<223> c-term may be amidated

<400> 187
Leu Asn Pro Glu Ser Lys Ala Ile Lys Asn Leu Leu Lys Ala Val Ser
1               5                   10                  15

Lys Glu Met Ser Lys Arg Ser Pro
            20

<210> 188
<211> 31
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 188
Val Pro Leu Ser Arg Thr Val Arg Cys Thr Cys Ile Ser Ile Xaa Xaa
1               5                   10                  15

Xaa Xaa Leu Lys Ala Val Ser Lys Glu Met Ser Lys Arg Ser Pro
            20                  25                  30

<210> 189
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 189
Xaa Pro Leu Ser Arg Thr Val Arg Xaa Thr Xaa Ile Ser Ile Xaa Xaa
1               5                   10                  15

Xaa Xaa Leu Lys Ala Val Ser Lys Glu Met Ser Lys Arg Ser Pro
            20                  25                  30

<210> 190
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 190
Val Xaa Leu Ser Arg Thr Val Arg Xaa Thr Xaa Ile Ser Ile Xaa Xaa
1               5                   10                  15

Xaa Xaa Leu Lys Ala Val Ser Lys Glu Met Ser Lys Arg Ser Pro
            20                  25                  30

<210> 191
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 191
Val Pro Xaa Ser Arg Thr Val Arg Xaa Thr Xaa Ile Ser Ile Xaa Xaa
1               5                   10                  15


Xaa Xaa Leu Lys Ala Val Ser Lys Glu Met Ser Lys Arg Ser Pro
            20                  25                  30


<210> 192
<211> 31
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 192
Val Pro Leu Xaa Arg Thr Val Arg Xaa Thr Xaa Ile Ser Ile Xaa Xaa

1                5                   10                  15

Xaa Xaa Leu Lys Ala Val Ser Lys Glu Met Ser Lys Arg Ser Pro
            20                  25                  30

<210> 193
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 193
Val Pro Leu Ser Xaa Thr Val Arg Xaa Thr Xaa Ile Ser Ile Xaa Xaa
1               5                   10                  15

Xaa Xaa Leu Lys Ala Val Ser Lys Glu Met Ser Lys Arg Ser Pro
            20                  25                  30

<210> 194
<211> 31
<212> PRT
<213> Artificial Sequence

<220>
<223> Description of Artificial Sequence: Synthetic peptide

<220>
<221> MOD_RES
<222> (6)..(6)

<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 194
Val Pro Leu Ser Arg Xaa Val Arg Xaa Thr Xaa Ile Ser Ile Xaa Xaa
1               5                   10                  15

Xaa Xaa Leu Lys Ala Val Ser Lys Glu Met Ser Lys Arg Ser Pro
            20                  25                  30

<210> 195
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>

<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 195
Val Pro Leu Ser Arg Thr Xaa Arg Xaa Thr Xaa Ile Ser Ile Xaa Xaa
1               5                   10                  15

Xaa Xaa Leu Lys Ala Val Ser Lys Glu Met Ser Lys Arg Ser Pro
            20                  25                  30

<210> 196
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 196
Val Pro Leu Ser Arg Thr Val Xaa Xaa Thr Xaa Ile Ser Ile Xaa Xaa
1               5                   10                  15

Xaa Xaa Leu Lys Ala Val Ser Lys Glu Met Ser Lys Arg Ser Pro
            20                  25                  30

<210> 197
<211> 31
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 197
Val Pro Leu Ser Arg Thr Val Arg Cys Thr Cys Xaa Ser Ile Xaa Xaa
1               5                   10                  15

Xaa Xaa Leu Lys Ala Val Ser Lys Glu Met Ser Lys Arg Ser Pro
            20                  25                  30

<210> 198
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 198

Xaa Pro Leu Ser Arg Thr Val Arg Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 199
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 199
Val Xaa Leu Ser Arg Thr Val Arg Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 200
<211> 31
<212> PRT
<213> Artificial Sequence

<220>
<223> Description of Artificial Sequence: Synthetic peptide

<220>
<221> MOD_RES

<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 200
Val Pro Xaa Ser Arg Thr Val Arg Xaa Thr Xaa Xaa Ser Ile Xaa Xaa
1               5                   10                  15


Xaa Xaa Leu Lys Ala Val Ser Lys Glu Met Ser Lys Arg Ser Pro
            20                  25                  30


<210> 201
<211> 31
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 201
Val Pro Leu Xaa Arg Thr Val Arg Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 202
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 202
Val Pro Leu Ser Xaa Thr Val Arg Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 203
<211> 31
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 203
Val Pro Leu Ser Arg Xaa Val Arg Xaa Thr Xaa Xaa Ser Ile Xaa Xaa
1               5                   10                  15


Xaa Xaa Leu Lys Ala Val Ser Lys Glu Met Ser Lys Arg Ser Pro
            20                  25                  30


<210> 204
<211> 31
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)

<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 204
Val Pro Leu Ser Arg Thr Xaa Arg Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 205
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 205
Val Pro Leu Ser Arg Thr Val Xaa Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 206
<211> 31
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 206
Val Pro Leu Ser Arg Thr Val Arg Cys Thr Cys Ile Xaa Ile Xaa Xaa
1               5                   10                  15

Xaa Xaa Leu Lys Ala Val Ser Lys Glu Met Ser Lys Arg Ser Pro
            20                  25                  30

<210> 207
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 207
Xaa Pro Leu Ser Arg Thr Val Arg Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 208
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 208
Val Xaa Leu Ser Arg Thr Val Arg Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 209
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 209
Val Pro Xaa Ser Arg Thr Val Arg Xaa Thr Xaa Ile Xaa Ile Xaa Xaa
1               5                   10                  15

Xaa Xaa Leu Lys Ala Val Ser Lys Glu Met Ser Lys Arg Ser Pro

20                    25                    30

<210> 210
<211> 31
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 210
Val Pro Leu Xaa Arg Thr Val Arg Xaa Thr Xaa Ile Xaa Ile Xaa Xaa
1               5                   10                  15


Xaa Xaa Leu Lys Ala Val Ser Lys Glu Met Ser Lys Arg Ser Pro
            20                  25                  30


<210> 211
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 211
Val Pro Leu Ser Xaa Thr Val Arg Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 212
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 212
Val Pro Leu Ser Arg Xaa Val Arg Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 213
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)

<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 213
Val Pro Leu Ser Arg Thr Xaa Arg Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 214
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 214
Val Pro Leu Ser Arg Thr Val Xaa Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 215
<211> 31
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 215
Val Pro Leu Ser Arg Thr Val Arg Cys Thr Cys Ile Ser Xaa Xaa Xaa
1               5                   10                  15

Xaa Xaa Leu Lys Ala Val Ser Lys Glu Met Ser Lys Arg Ser Pro
            20                  25                  30

<210> 216
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 216
Xaa Pro Leu Ser Arg Thr Val Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 217
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 217
Val Xaa Leu Ser Arg Thr Val Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 218
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 218
Val Pro Xaa Ser Arg Thr Val Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 219
<211> 31
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 219
Val Pro Leu Xaa Arg Thr Val Arg Xaa Thr Xaa Ile Ser Xaa Xaa Xaa
1               5                   10                  15


Xaa Xaa Leu Lys Ala Val Ser Lys Glu Met Ser Lys Arg Ser Pro
            20                  25                  30


<210> 220
<211> 31
<212> PRT
<213> Artificial Sequence

<220>
<223> Description of Artificial Sequence: Synthetic peptide


<220>

<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 220
Val Pro Leu Ser Xaa Thr Val Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 221
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 221
Val Pro Leu Ser Arg Xaa Val Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 222
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 222
Val Pro Leu Ser Arg Thr Xaa Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 223
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 223
Val Pro Leu Ser Arg Thr Val Xaa Xaa Thr Xaa Ile Ser Xaa Xaa Xaa
1               5                   10                  15

Xaa Xaa Leu Lys Ala Val Ser Lys Glu Met Ser Lys Arg Ser Pro

20                  25                  30

<210> 224
<211> 31
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 224
Val Pro Leu Ser Arg Thr Val Arg Cys Thr Cys Ile Ser Ile Xaa Xaa
1               5                   10                  15

Xaa Xaa Leu Asn Pro Glu Ser Lys Ala Ile Lys Asn Leu Leu Lys
            20                  25                  30

<210> 225
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 225
Xaa Pro Leu Ser Arg Thr Val Arg Xaa Thr Xaa Ile Ser Ile Xaa Xaa
1               5                   10                  15

Xaa Xaa Leu Asn Pro Glu Ser Lys Ala Ile Lys Asn Leu Leu Lys
            20                  25                  30

<210> 226
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 226
Val Xaa Leu Ser Arg Thr Val Arg Xaa Thr Xaa Ile Ser Ile Xaa Xaa
1               5                   10                  15

Xaa Xaa Leu Asn Pro Glu Ser Lys Ala Ile Lys Asn Leu Leu Lys
            20                  25                  30

<210> 227
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 227
Val Pro Xaa Ser Arg Thr Val Arg Xaa Thr Xaa Ile Ser Ile Xaa Xaa
1               5                   10                  15


Xaa Xaa Leu Asn Pro Glu Ser Lys Ala Ile Lys Asn Leu Leu Lys
            20                  25                  30


<210> 228
<211> 31
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)

<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 228
Val Pro Leu Xaa Arg Thr Val Arg Xaa Thr Xaa Ile Ser Ile Xaa Xaa
1               5                   10                  15

Xaa Xaa Leu Asn Pro Glu Ser Lys Ala Ile Lys Asn Leu Leu Lys
            20                  25                  30

<210> 229
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 229
Val Pro Leu Ser Xaa Thr Val Arg Xaa Thr Xaa Ile Ser Ile Xaa Xaa
1               5                   10                  15

Xaa Xaa Leu Asn Pro Glu Ser Lys Ala Ile Lys Asn Leu Leu Lys
            20                  25                  30

<210> 230
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 230
Val Pro Leu Ser Arg Xaa Val Arg Xaa Thr Xaa Ile Ser Ile Xaa Xaa
1               5                   10                  15

Xaa Xaa Leu Asn Pro Glu Ser Lys Ala Ile Lys Asn Leu Leu Lys
            20                  25                  30

<210> 231
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 231
Val Pro Leu Ser Arg Thr Xaa Arg Xaa Thr Xaa Ile Ser Ile Xaa Xaa
1               5                   10                  15


Xaa Xaa Leu Asn Pro Glu Ser Lys Ala Ile Lys Asn Leu Leu Lys
            20                  25                  30


<210> 232
<211> 31
<212> PRT
<213> Artificial Sequence

<220>
<223> Description of Artificial Sequence: Synthetic peptide


<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 232

Val Pro Leu Ser Arg Thr Val Xaa Xaa Thr Xaa Ile Ser Ile Xaa Xaa
1               5                   10                  15

Xaa Xaa Leu Asn Pro Glu Ser Lys Ala Ile Lys Asn Leu Leu Lys
            20                  25                  30

<210> 233
<211> 31
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 233
Val Pro Leu Ser Arg Thr Val Arg Cys Thr Cys Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 234
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 234
Xaa Pro Leu Ser Arg Thr Val Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 235
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 235
Val Xaa Leu Ser Arg Thr Val Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 236
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 236
Val Pro Xaa Ser Arg Thr Val Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 237
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups

and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 237
Val Pro Leu Xaa Arg Thr Val Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 238
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 238
Val Pro Leu Ser Xaa Thr Val Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 239
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 239
Val Pro Leu Ser Arg Xaa Val Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 240
<211> 31
<212> PRT

<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 240
Val Pro Leu Ser Arg Thr Xaa Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 241
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 241
Val Pro Leu Ser Arg Thr Val Xaa Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 242
<211> 31
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 242
Val Pro Leu Ser Arg Thr Val Arg Cys Thr Cys Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 243
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 243
Xaa Pro Leu Ser Arg Thr Val Arg Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 244
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 244
Val Xaa Leu Ser Arg Thr Val Arg Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 245
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 245
Val Pro Xaa Ser Arg Thr Val Arg Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 246
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 246
Val Pro Leu Xaa Arg Thr Val Arg Xaa Thr Xaa Ile Xaa Ile Xaa Xaa

1                   5                   10                  15

Xaa Xaa Leu Asn Pro Glu Ser Lys Ala Ile Lys Asn Leu Leu Lys
            20                  25                  30

<210> 247
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 247
Val Pro Leu Ser Xaa Thr Val Arg Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 248
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 248
Val Pro Leu Ser Arg Xaa Val Arg Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 249
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 249
Val Pro Leu Ser Arg Thr Xaa Arg Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 250
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 250
Val Pro Leu Ser Arg Thr Val Xaa Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 251
<211> 31
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 251
Val Pro Leu Ser Arg Thr Val Arg Cys Thr Cys Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 252
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 252
Xaa Pro Leu Ser Arg Thr Val Arg Xaa Thr Xaa Ile Ser Xaa Xaa Xaa
1               5                   10                  15


Xaa Xaa Leu Asn Pro Glu Ser Lys Ala Ile Lys Asn Leu Leu Lys
            20                  25                  30


<210> 253
<211> 31
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 253
Val Xaa Leu Ser Arg Thr Val Arg Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 254
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 254
Val Pro Xaa Ser Arg Thr Val Arg Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 255
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 255
Val Pro Leu Xaa Arg Thr Val Arg Xaa Thr Xaa Ile Ser Xaa Xaa Xaa

1                 5                   10                  15

Xaa Xaa Leu Asn Pro Glu Ser Lys Ala Ile Lys Asn Leu Leu Lys
            20                  25                  30

<210> 256
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 256
Val Pro Leu Ser Xaa Thr Val Arg Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 257
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 257
Val Pro Leu Ser Arg Xaa Val Arg Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 258
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 258
Val Pro Leu Ser Arg Thr Xaa Arg Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 259
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 259
Val Pro Leu Ser Arg Thr Val Xaa Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 260
<211> 31
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 260
Val Pro Leu Ser Arg Thr Val Arg Cys Thr Cys Ile Ser Ile Xaa Xaa
1               5                   10                  15

Xaa Xaa Lys Ala Ile Lys Asn Leu Leu Lys Ala Val Ser Lys Glu
            20                  25                  30

<210> 261
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 261
Xaa Pro Leu Ser Arg Thr Val Arg Xaa Thr Xaa Ile Ser Ile Xaa Xaa
1               5                   10                  15


Xaa Xaa Lys Ala Ile Lys Asn Leu Leu Lys Ala Val Ser Lys Glu
            20                  25                  30


<210> 262
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 262
Val Xaa Leu Ser Arg Thr Val Arg Xaa Thr Xaa Ile Ser Ile Xaa Xaa

1                 5                   10                  15

Xaa Xaa Lys Ala Ile Lys Asn Leu Leu Lys Ala Val Ser Lys Glu
            20                  25                  30

<210> 263
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 263
Val Pro Xaa Ser Arg Thr Val Arg Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 264
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 264
Val Pro Leu Xaa Arg Thr Val Arg Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 265
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES

<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 265
Val Pro Leu Ser Xaa Thr Val Arg Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 266
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 266
Val Pro Leu Ser Arg Xaa Val Arg Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 267
<211> 31

<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 267
Val Pro Leu Ser Arg Thr Xaa Arg Xaa Thr Xaa Ile Ser Ile Xaa Xaa
1               5                   10                  15


Xaa Xaa Lys Ala Ile Lys Asn Leu Leu Lys Ala Val Ser Lys Glu
            20                  25                  30


<210> 268
<211> 31
<212> PRT
<213> Artificial Sequence

<220>
<223> Description of Artificial Sequence: Synthetic peptide


<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups

and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 268
Val Pro Leu Ser Arg Thr Val Xaa Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 269
<211> 31
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 269
Val Pro Leu Ser Arg Thr Val Arg Cys Thr Cys Xaa Ser Ile Xaa Xaa
1               5                   10                  15

Xaa Xaa Lys Ala Ile Lys Asn Leu Leu Lys Ala Val Ser Lys Glu
            20                  25                  30

<210> 270
<211> 31
<212> PRT
<213> Artificial Sequence

<220>
<223> Description of Artificial Sequence: Synthetic peptide

<220>
<221> MOD_RES
<222> (1)..(1)

<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 270
Xaa Pro Leu Ser Arg Thr Val Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 271
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>

<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 271
Val Xaa Leu Ser Arg Thr Val Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 272
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 272
Val Pro Xaa Ser Arg Thr Val Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 273

<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 273
Val Pro Leu Xaa Arg Thr Val Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 274
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see

specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 274
Val Pro Leu Ser Xaa Thr Val Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 275
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 275
Val Pro Leu Ser Arg Xaa Val Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 276
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 276
Val Pro Leu Ser Arg Thr Xaa Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 277
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 277
Val Pro Leu Ser Arg Thr Val Xaa Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 278
<211> 31
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 278
Val Pro Leu Ser Arg Thr Val Arg Cys Thr Cys Ile Xaa Ile Xaa Xaa
1               5                   10                  15

Xaa Xaa Lys Ala Ile Lys Asn Leu Leu Lys Ala Val Ser Lys Glu
            20                  25                  30

<210> 279
<211> 31
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 279
Xaa Pro Leu Ser Arg Thr Val Arg Xaa Thr Xaa Ile Xaa Ile Xaa Xaa
1               5                   10                  15


Xaa Xaa Lys Ala Ile Lys Asn Leu Leu Lys Ala Val Ser Lys Glu
            20                  25                  30


<210> 280
<211> 31
<212> PRT
<213> Artificial Sequence

<220>
<223> Description of Artificial Sequence: Synthetic peptide


<220>
<221> MOD_RES
<222> (2)..(2)

<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 280
Val Xaa Leu Ser Arg Thr Val Arg Xaa Thr Xaa Ile Xaa Ile Xaa Xaa
1               5                   10                  15


Xaa Xaa Lys Ala Ile Lys Asn Leu Leu Lys Ala Val Ser Lys Glu
            20                  25                  30


<210> 281
<211> 31
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>

<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 281
Val Pro Xaa Ser Arg Thr Val Arg Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 282
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 282
Val Pro Leu Xaa Arg Thr Val Arg Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 283
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 283

Val Pro Leu Ser Xaa Thr Val Arg Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 284
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 284
Val Pro Leu Ser Arg Xaa Val Arg Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 285
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 285
Val Pro Leu Ser Arg Thr Xaa Arg Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 286
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 286
Val Pro Leu Ser Arg Thr Val Xaa Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 287
<211> 31
<212> PRT
<213> Artificial Sequence

<220>
<223> Description of Artificial Sequence: Synthetic peptide

<220>
<221> MOD_RES
<222> (14)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 287
Val Pro Leu Ser Arg Thr Val Arg Cys Thr Cys Ile Ser Xaa Xaa Xaa
1               5                   10                  15

Xaa Xaa Lys Ala Ile Lys Asn Leu Leu Lys Ala Val Ser Lys Glu
            20                  25                  30

<210> 288
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 288
Xaa Pro Leu Ser Arg Thr Val Arg Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 289
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES

<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 289
Val Xaa Leu Ser Arg Thr Val Arg Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 290
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 290
Val Pro Xaa Ser Arg Thr Val Arg Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 291
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 291
Val Pro Leu Xaa Arg Thr Val Arg Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 292
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 292
Val Pro Leu Ser Xaa Thr Val Arg Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 293
<211> 31
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 293
Val Pro Leu Ser Arg Xaa Val Arg Xaa Thr Xaa Ile Ser Xaa Xaa Xaa
1               5                   10                  15


Xaa Xaa Lys Ala Ile Lys Asn Leu Leu Lys Ala Val Ser Lys Glu
            20                  25                  30


<210> 294
<211> 31
<212> PRT
<213> Artificial Sequence

<220>
<223> Description of Artificial Sequence: Synthetic peptide


<220>

<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 294
Val Pro Leu Ser Arg Thr Xaa Arg Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 295
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 295
Val Pro Leu Ser Arg Thr Val Xaa Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 296
<211> 34
<212> PRT
<213> Artificial Sequence

<220>
<223> Description of Artificial Sequence: Synthetic peptide

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 296
Val Pro Leu Ser Arg Thr Val Arg Cys Thr Cys Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 297
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 297
Xaa Pro Leu Ser Arg Thr Val Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 298
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>

<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 298
Val Xaa Leu Ser Arg Thr Val Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 299
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 299
Val Pro Xaa Ser Arg Thr Val Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 300
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 300
Val Pro Leu Xaa Arg Thr Val Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 301

<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 301
Val Pro Leu Ser Xaa Thr Val Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 302
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 302
Val Pro Leu Ser Arg Xaa Val Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 303
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>

<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 303
Val Pro Leu Ser Arg Thr Xaa Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 304
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 304
Val Pro Leu Ser Arg Thr Val Xaa Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 305
<211> 34
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 305
Val Pro Leu Ser Arg Thr Val Arg Cys Thr Cys Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 306
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 306
Xaa Pro Leu Ser Arg Thr Val Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 307
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 307
Val Xaa Leu Ser Arg Thr Val Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 308
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 308
Val Pro Xaa Ser Arg Thr Val Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 309
<211> 34
<212> PRT
<213> Artificial Sequence

<220>

<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 309
Val Pro Leu Xaa Arg Thr Val Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 310
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 310
Val Pro Leu Ser Xaa Thr Val Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 311
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 311
Val Pro Leu Ser Arg Xaa Val Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 312
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 312
Val Pro Leu Ser Arg Thr Xaa Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 313
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 313
Val Pro Leu Ser Arg Thr Val Xaa Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 314
<211> 34
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 314
Val Pro Leu Ser Arg Thr Val Arg Cys Thr Cys Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 315
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 315
Xaa Pro Leu Ser Arg Thr Val Arg Xaa Thr Xaa Ile Xaa Ile Ser Asn

1                   5                   10                  15

Gln Xaa Xaa Xaa Xaa Leu Lys Ala Val Ser Lys Glu Met Ser Lys Arg
            20                  25                  30

Ser Pro

<210> 316
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 316
Val Xaa Leu Ser Arg Thr Val Arg Xaa Thr Xaa Ile Xaa Ile Ser Asn
1                   5                   10                  15

Gln Xaa Xaa Xaa Xaa Leu Lys Ala Val Ser Lys Glu Met Ser Lys Arg
            20                  25                  30

Ser Pro

<210> 317
<211> 34
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 317
Val Pro Xaa Ser Arg Thr Val Arg Xaa Thr Xaa Ile Xaa Ile Ser Asn
1               5                   10                  15


Gln Xaa Xaa Xaa Xaa Leu Lys Ala Val Ser Lys Glu Met Ser Lys Arg
            20                  25                  30


Ser Pro



<210> 318
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 318
Val Pro Leu Xaa Arg Thr Val Arg Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 319
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 319
Val Pro Leu Ser Xaa Thr Val Arg Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 320
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 320
Val Pro Leu Ser Arg Xaa Val Arg Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 321
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 321
Val Pro Leu Ser Arg Thr Xaa Arg Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 322
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 322
Val Pro Leu Ser Arg Thr Val Xaa Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 323
<211> 34
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 323
Val Pro Leu Ser Arg Thr Val Arg Cys Thr Cys Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 324
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 324
Xaa Pro Leu Ser Arg Thr Val Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 325
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 325
Val Xaa Leu Ser Arg Thr Val Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 326
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 326
Val Pro Xaa Ser Arg Thr Val Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 327
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 327
Val Pro Leu Xaa Arg Thr Val Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 328
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 328
Val Pro Leu Ser Xaa Thr Val Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 329
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 329
Val Pro Leu Ser Arg Xaa Val Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg

20                  25          .          ˘  30

Ser Pro

<210> 330
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 330
Val Pro Leu Ser Arg Thr Xaa Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 331

<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 331
Val Pro Leu Ser Arg Thr Val Xaa Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 332
<211> 34
<212> PRT
<213> Artificial Sequence

<220>
<223> Description of Artificial Sequence: Synthetic peptide

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 332
Val Pro Leu Ser Arg Thr Val Arg Cys Thr Cys Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 333
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 333
Xaa Pro Leu Ser Arg Thr Val Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 334
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 334
Val Xaa Leu Ser Arg Thr Val Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg

20                  25                  30

Ser Pro

<210> 335
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 335
Val Pro Xaa Ser Arg Thr Val Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 336

<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 336
Val Pro Leu Xaa Arg Thr Val Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 337
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 337
Val Pro Leu Ser Xaa Thr Val Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 338
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 338
Val Pro Leu Ser Arg Xaa Val Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 339
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 339
Val Pro Leu Ser Arg Thr Xaa Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 340
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 340
Val Pro Leu Ser Arg Thr Val Xaa Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 341
<211> 34
<212> PRT
<213> Artificial Sequence

<220>
<223> Description of Artificial Sequence: Synthetic peptide

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 341
Val Pro Leu Ser Arg Thr Val Arg Cys Thr Cys Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 342
<211> 34
<212> PRT
<213> Artificial Sequence

<220>
<223> Description of Artificial Sequence: Synthetic peptide

<220>

<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 342
Xaa Pro Leu Ser Arg Thr Val Arg Xaa Thr Xaa Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 343
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES

<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 343
Val Xaa Leu Ser Arg Thr Val Arg Xaa Thr Xaa Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 344
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)

<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 344
Val Pro Xaa Ser Arg Thr Val Arg Xaa Thr Xaa Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 345
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see

specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 345
Val Pro Leu Xaa Arg Thr Val Arg Xaa Thr Xaa Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 346
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 346
Val Pro Leu Ser Xaa Thr Val Arg Xaa Thr Xaa Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 347
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 347
Val Pro Leu Ser Arg Xaa Val Arg Xaa Thr Xaa Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 348
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 348
Val Pro Leu Ser Arg Thr Xaa Arg Xaa Thr Xaa Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 349
<211> 34
<212> PRT
<213> Artificial Sequence

<220>
<223> Description of Artificial Sequence: Synthetic peptide


<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 349
Val Pro Leu Ser Arg Thr Val Xaa Xaa Thr Xaa Ile Ser Ile Ser Gln
1               5                   10                  15


Xaa Xaa Xaa Xaa Xaa Leu Lys Ala Val Ser Lys Glu Met Ser Lys Arg
            20                  25                  30


Ser Pro




<210> 350
<211> 34
<212> PRT
<213> Artificial Sequence

<220>
<223> Description of Artificial Sequence: Synthetic peptide


<220>
<221> MOD_RES
<222> (18)..(21)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 350
Val Pro Leu Ser Arg Thr Val Arg Cys Thr Cys Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 351
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 351
Xaa Pro Leu Ser Arg Thr Val Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 352
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 352
Val Xaa Leu Ser Arg Thr Val Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 353
<211> 34
<212> PRT
<213> Artificial Sequence

<220>
<223> Description of Artificial Sequence: Synthetic peptide

<220>
<221> MOD_RES

<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 353
Val Pro Xaa Ser Arg Thr Val Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 354
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)

<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 354
Val Pro Leu Xaa Arg Thr Val Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 355
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 355

Val Pro Leu Ser Xaa Thr Val Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 356
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 356
Val Pro Leu Ser Arg Xaa Val Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 357
<211> 34
<212> PRT

<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 357
Val Pro Leu Ser Arg Thr Xaa Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 358
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 358
Val Pro Leu Ser Arg Thr Val Xaa Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 359
<211> 34
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 359
Val Pro Leu Ser Arg Thr Val Arg Cys Thr Cys Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 360

<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 360
Xaa Pro Leu Ser Arg Thr Val Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 361
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 361
Val Xaa Leu Ser Arg Thr Val Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 362
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>

<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 362
Val Pro Xaa Ser Arg Thr Val Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 363
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 363
Val Pro Leu Xaa Arg Thr Val Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu

20                  25                  30

Leu Lys

<210> 364
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 364
Val Pro Leu Ser Xaa Thr Val Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 365
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 365
Val Pro Leu Ser Arg Xaa Val Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 366
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 366
Val Pro Leu Ser Arg Thr Xaa Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 367
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 367
Val Pro Leu Ser Arg Thr Val Xaa Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 368
<211> 34
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 368
Val Pro Leu Ser Arg Thr Val Arg Cys Thr Cys Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 369
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 369
Xaa Pro Leu Ser Arg Thr Val Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 370
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 370
Val Xaa Leu Ser Arg Thr Val Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 371
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 371
Val Pro Xaa Ser Arg Thr Val Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 372
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 372
Val Pro Leu Xaa Arg Thr Val Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 373
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 373
Val Pro Leu Ser Xaa Thr Val Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu

20                  25                  30

Leu Lys

<210> 374
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 374
Val Pro Leu Ser Arg Xaa Val Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 375

<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 375
Val Pro Leu Ser Arg Thr Xaa Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 376
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 376
Val Pro Leu Ser Arg Thr Val Xaa Xaa Thr Xaa Ile Xaa Ile Ser Asn
1               5                   10                  15


Gln Xaa Xaa Xaa Xaa Leu Asn Pro Glu Ser Lys Ala Ile Lys Asn Leu
            20                  25                  30


Leu Lys




<210> 377
<211> 34
<212> PRT
<213> Artificial Sequence

<220>
<223> Description of Artificial Sequence: Synthetic peptide


<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 377
Val Pro Leu Ser Arg Thr Val Arg Cys Thr Cys Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 378
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 378
Xaa Pro Leu Ser Arg Thr Val Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu

20                  25                  30

Leu Lys

<210> 379
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 379
Val Xaa Leu Ser Arg Thr Val Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 380

<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodimentss

<220>
<223> c-term may be amidated

<400> 380
Val Pro Xaa Ser Arg Thr Val Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 381
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 381
Val Pro Leu Xaa Arg Thr Val Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 382
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 382
Val Pro Leu Ser Xaa Thr Val Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 383
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 383
Val Pro Leu Ser Arg Xaa Val Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 384
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)

<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 384
Val Pro Leu Ser Arg Thr Xaa Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 385
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 385

Val Pro Leu Ser Arg Thr Val Xaa Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 386
<211> 34
<212> PRT
<213> Artificial Sequence

<220>
<223> Description of Artificial Sequence: Synthetic peptide

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 386
Val Pro Leu Ser Arg Thr Val Arg Cys Thr Cys Ile Ser Ile Ser Xaa
1               5                   10                  15

Gln Xaa Xaa Xaa Xaa Leu Asn Pro Glu Ser Lys Ala Ile Lys Asn Leu
            20                  25                  30

Leu Lys

<210> 387
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 387
Xaa Pro Leu Ser Arg Thr Val Arg Xaa Thr Xaa Ile Ser Ile Ser Xaa
1               5                   10                  15

Gln Xaa Xaa Xaa Xaa Leu Asn Pro Glu Ser Lys Ala Ile Lys Asn Leu
            20                  25                  30

Leu Lys

<210> 388
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 388
Val Xaa Leu Ser Arg Thr Val Arg Xaa Thr Xaa Ile Ser Ile Ser Xaa
1               5                   10                  15

Gln Xaa Xaa Xaa Xaa Leu Asn Pro Glu Ser Lys Ala Ile Lys Asn Leu
            20                  25                  30

Leu Lys

<210> 389
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)

<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 389
Val Pro Xaa Ser Arg Thr Val Arg Xaa Thr Xaa Ile Ser Ile Ser Xaa
1               5                   10                  15

Gln Xaa Xaa Xaa Xaa Leu Asn Pro Glu Ser Lys Ala Ile Lys Asn Leu
            20                  25                  30

Leu Lys

<210> 390
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may

encompass 1 or 4 residues; see specification as filed for detailed
description of substitutions, R-groups and preferred embodiments

<220>
<223> C-term may be amidated

<400> 390
Val Pro Leu Xaa Arg Thr Val Arg Xaa Thr Xaa Ile Ser Ile Ser Xaa
1               5                   10                  15

Gln Xaa Xaa Xaa Xaa Leu Asn Pro Glu Ser Lys Ala Ile Lys Asn Leu
            20                  25                  30

Leu Lys

<210> 391
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 391
Val Pro Leu Ser Xaa Thr Val Arg Xaa Thr Xaa Ile Ser Ile Ser Xaa

1                   5                   10                  15

Gln Xaa Xaa Xaa Xaa Leu Asn Pro Glu Ser Lys Ala Ile Lys Asn Leu
            20                  25                  30

Leu Lys

<210> 392
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 392
Val Pro Leu Ser Arg Xaa Val Arg Xaa Thr Xaa Ile Ser Ile Ser Xaa
1                   5                   10                  15

Gln Xaa Xaa Xaa Xaa Leu Asn Pro Glu Ser Lys Ala Ile Lys Asn Leu
            20                  25                  30

Leu Lys

<210> 393
<211> 34
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 393
Val Pro Leu Ser Arg Thr Xaa Arg Xaa Thr Xaa Ile Ser Ile Ser Xaa
1               5                   10                  15


Gln Xaa Xaa Xaa Xaa Leu Asn Pro Glu Ser Lys Ala Ile Lys Asn Leu
            20                  25                  30


Leu Lys



<210> 394
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 394
Val Pro Leu Ser Arg Thr Val Xaa Xaa Thr Xaa Ile Ser Ile Ser Xaa
1               5                   10                  15

Gln Xaa Xaa Xaa Xaa Leu Asn Pro Glu Ser Lys Ala Ile Lys Asn Leu
            20                  25                  30

Leu Lys

<210> 395
<211> 34
<212> PRT
<213> Artificial Sequence

<220>
<223> Description of Artificial Sequence: Synthetic peptide

<220>
<221> MOD_RES
<222> (17)..(17)
<223> Any L- or D-natural amino acid and any non-natural amino acid; see
specification as filed for detailed description of substitutions, R-groups
and preferred embodiments

220>
<221> MOD_RES
<222> (18)..(21)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 395
Val Pro Leu Ser Arg Thr Val Arg Cys Thr Cys Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 396
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 396
Xaa Pro Leu Ser Arg Thr Val Arg Xaa Thr Xaa Ile Ser Ile Ser Gln

1                   5                   10                  15

Xaa Xaa Xaa Xaa Xaa Leu Asn Pro Glu Ser Lys Ala Ile Lys Asn Leu
            20                  25                  30

Leu Lys

<210> 397
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 397
Val Xaa Leu Ser Arg Thr Val Arg Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 398
<211> 34
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 398
Val Pro Xaa Ser Arg Thr Val Arg Xaa Thr Xaa Ile Ser Ile Ser Gln
1               5                   10                  15


Xaa Xaa Xaa Xaa Xaa Leu Asn Pro Glu Ser Lys Ala Ile Lys Asn Leu
            20                  25                  30


Leu Lys




<210> 399
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 399
Val Pro Leu Xaa Arg Thr Val Arg Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 400
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 400
Val Pro Leu Ser Xaa Thr Val Arg Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 401
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 401
Val Pro Leu Ser Arg Xaa Val Arg Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 402
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 402
Val Pro Leu Ser Arg Thr Xaa Arg Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 403
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 403
Val Pro Leu Ser Arg Thr Val Xaa Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 404
<211> 34
<212> PRT
<213> Artificial Sequence

<220>
<223> Description of Artificial Sequence: Synthetic peptide

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 404
Val Pro Leu Ser Arg Thr Val Arg Cys Thr Cys Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 405
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see

specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 405
Xaa Pro Leu Ser Arg Thr Val Arg Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 406
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 406
Val Xaa Leu Ser Arg Thr Val Arg Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 407
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 407
Val Pro Xaa Ser Arg Thr Val Arg Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 408
<211> 34
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 408
Val Pro Leu Xaa Arg Thr Val Arg Xaa Thr Xaa Ile Ser Ile Ser Asn
1               5                   10                  15


Gln Xaa Xaa Xaa Xaa Lys Ala Ile Lys Asn Leu Leu Lys Ala Val Ser
            20                  25                  30


Lys Glu




<210> 409
<211> 34
<212> PRT
<213> Artificial Sequence

<220>
<223> Description of Artificial Sequence: Synthetic peptide


<220>
<221> MOD_RES
<222> (5)..(5)

<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 409
Val Pro Leu Ser Xaa Thr Val Arg Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 410
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see

specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 410
Val Pro Leu Ser Arg Xaa Val Arg Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 411
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 411
Val Pro Leu Ser Arg Thr Xaa Arg Xaa Thr Xaa Ile Ser Ile Ser Asn

1                   5                   10                  15

Gln Xaa Xaa Xaa Xaa Lys Ala Ile Lys Asn Leu Leu Lys Ala Val Ser
            20                  25                  30

Lys Glu

<210> 412
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 412
Val Pro Leu Ser Arg Thr Val Xaa Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 413
<211> 34
<212> PRT
<213> Artificial Sequence

<220>
<223> Description of Artificial Sequence: Synthetic peptide

<220>
<221> MOD_RES

<222> (12)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 413
Val Pro Leu Ser Arg Thr Val Arg Cys Thr Cys Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 414
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 414
Xaa Pro Leu Ser Arg Thr Val Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 415
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 415
Val Xaa Leu Ser Arg Thr Val Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 416
<211> 34

<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 416
Val Pro Xaa Ser Arg Thr Val Arg Xaa Thr Xaa Xaa Ser Ile Ser Asn
1               5                   10                  15


Gln Xaa Xaa Xaa Xaa Lys Ala Ile Lys Asn Leu Leu Lys Ala Val Ser
            20                  25                  30


Lys Glu




<210> 417
<211> 34
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 417
Val Pro Leu Xaa Arg Thr Val Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 418
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES

<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 418
Val Pro Leu Ser Xaa Thr Val Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 419
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 419
Val Pro Leu Ser Arg Xaa Val Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 420
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 420
Val Pro Leu Ser Arg Thr Xaa Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 421
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 421
Val Pro Leu Ser Arg Thr Val Xaa Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 422
<211> 34
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 422
Val Pro Leu Ser Arg Thr Val Arg Cys Thr Cys Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 423
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 423
Xaa Pro Leu Ser Arg Thr Val Arg Xaa Thr Xaa Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 424
<211> 34
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 424
Val Xaa Leu Ser Arg Thr Val Arg Xaa Thr Xaa Ile Xaa Ile Ser Asn
1               5                   10                  15


Gln Xaa Xaa Xaa Xaa Lys Ala Ile Lys Asn Leu Leu Lys Ala Val Ser
            20                  25                  30


Lys Glu




<210> 425
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 425
Val Pro Xaa Ser Arg Thr Val Arg Xaa Thr Xaa Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 426
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 426
Val Pro Leu Xaa Arg Thr Val Arg Xaa Thr Xaa Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 427
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>

<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 427
Val Pro Leu Ser Xaa Thr Val Arg Xaa Thr Xaa Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 428
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES

<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 428
Val Pro Leu Ser Arg Xaa Val Arg Xaa Thr Xaa Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 429
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 429
Val Pro Leu Ser Arg Thr Xaa Arg Xaa Thr Xaa Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 430
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 430
Val Pro Leu Ser Arg Thr Val Xaa Xaa Thr Xaa Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 431
<211> 34
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 431
Val Pro Leu Ser Arg Thr Val Arg Cys Thr Cys Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 432
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>

<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 432
Xaa Pro Leu Ser Arg Thr Val Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 433
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES

<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 433
Val Xaa Leu Ser Arg Thr Val Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 434
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 434
Val Pro Xaa Ser Arg Thr Val Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 435
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 435

Val Pro Leu Xaa Arg Thr Val Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 436
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments
<220>
<223> c-term may be amidated

<400> 436
Val Pro Leu Ser Xaa Thr Val Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 437
<211> 34
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 437
Val Pro Leu Ser Arg Xaa Val Arg Xaa Thr Xaa Ile Ser Xaa Ser Asn
1               5                   10                  15


Gln Xaa Xaa Xaa Xaa Lys Ala Ile Lys Asn Leu Leu Lys Ala Val Ser
            20                  25                  30


Lys Glu




<210> 438
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 438
Val Pro Leu Ser Arg Thr Xaa Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 439
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see

specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 439
Val Pro Leu Ser Arg Thr Val Xaa Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 440
<211> 34
<212> PRT
<213> Artificial Sequence

<220>
<223> Description of Artificial Sequence: Synthetic peptide

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 440
Val Pro Leu Ser Arg Thr Val Arg Cys Thr Cys Ile Ser Ile Ser Xaa

1                   5                   10                  15

Gln Xaa Xaa Xaa Xaa Lys Ala Ile Lys Asn Leu Leu Lys Ala Val Ser
            20                  25                  30

Lys Glu

<210> 441
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 441
Xaa Pro Leu Ser Arg Thr Val Arg Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 442
<211> 34
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 442
Val Xaa Leu Ser Arg Thr Val Arg Xaa Thr Xaa Ile Ser Ile Ser Xaa
1               5                   10                  15


Gln Xaa Xaa Xaa Xaa Lys Ala Ile Lys Asn Leu Leu Lys Ala Val Ser
            20                  25                  30


Lys Glu




<210> 443
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 443
Val Pro Xaa Ser Arg Thr Val Arg Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 444
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 444
Val Pro Leu Xaa Arg Thr Val Arg Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 445
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 445
Val Pro Leu Ser Xaa Thr Val Arg Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 446
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 446
Val Pro Leu Ser Arg Xaa Val Arg Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 447
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 447
Val Pro Leu Ser Arg Thr Xaa Arg Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 448
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 448
Val Pro Leu Ser Arg Thr Val Xaa Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 449
<211> 34
<212> PRT
<213> Artificial Sequence

<220>
<223> Description of Artificial Sequence: Synthetic peptide

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 449
Val Pro Leu Ser Arg Thr Val Arg Cys Thr Cys Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 450
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 450
Xaa Pro Leu Ser Arg Thr Val Arg Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 451
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 451
Val Xaa Leu Ser Arg Thr Val Arg Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 452
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 452
Val Pro Xaa Ser Arg Thr Val Arg Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 453
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 453
Val Pro Leu Xaa Arg Thr Val Arg Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 454
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 454
Val Pro Leu Ser Xaa Thr Val Arg Xaa Thr Xaa Ile Ser Ile Ser Gln
1               5                   10                  15

Xaa Xaa Xaa Xaa Xaa Lys Ala Ile Lys Asn Leu Leu Lys Ala Val Ser

20                  25                  30

Lys Glu

<210> 455
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 455
Val Pro Leu Ser Arg Xaa Val Arg Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 456

<211> 34
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 456
Val Pro Leu Ser Arg Thr Xaa Arg Xaa Thr Xaa Ile Ser Ile Ser Gln
1               5                   10                  15


Xaa Xaa Xaa Xaa Xaa Lys Ala Ile Lys Asn Leu Leu Lys Ala Val Ser
            20                  25                  30


Lys Glu



<210> 457
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 457
Val Pro Leu Ser Arg Thr Val Xaa Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 458
<211> 31
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 458
Val Pro Leu Ser Arg Thr Val Arg Cys Thr Cys Ile Ser Ile Xaa Xaa
1               5                   10                  15

Xaa Xaa Leu Lys Ala Val Ser Lys Glu Met Ser Lys Arg Ser Pro
            20                  25                  30

<210> 459
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 459
Xaa Pro Leu Ser Arg Thr Val Arg Xaa Thr Xaa Ile Ser Ile Xaa Xaa
1               5                   10                  15

Xaa Xaa Leu Lys Ala Val Ser Lys Glu Met Ser Lys Arg Ser Pro
            20                  25                  30

<210> 460
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 460
Val Xaa Leu Ser Arg Thr Val Arg Xaa Thr Xaa Ile Ser Ile Xaa Xaa
1               5                   10                  15

Xaa Xaa Leu Lys Ala Val Ser Lys Glu Met Ser Lys Arg Ser Pro
            20                  25                  30

<210> 461
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 461
Val Pro Xaa Ser Arg Thr Val Arg Xaa Thr Xaa Ile Ser Ile Xaa Xaa
1               5                   10                  15

Xaa Xaa Leu Lys Ala Val Ser Lys Glu Met Ser Lys Arg Ser Pro
            20                  25                  30

<210> 462
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 462
Val Pro Leu Xaa Arg Thr Val Arg Xaa Thr Xaa Ile Ser Ile Xaa Xaa
1               5                   10                  15

Xaa Xaa Leu Lys Ala Val Ser Lys Glu Met Ser Lys Arg Ser Pro
            20                  25                  30

<210> 463
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 463
Val Pro Leu Ser Xaa Thr Val Arg Xaa Thr Xaa Ile Ser Ile Xaa Xaa
1               5                   10                  15

Xaa Xaa Leu Lys Ala Val Ser Lys Glu Met Ser Lys Arg Ser Pro
            20                  25                  30

<210> 464
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 464
Val Pro Leu Ser Arg Xaa Val Arg Xaa Thr Xaa Ile Ser Ile Xaa Xaa
1               5                   10                  15


Xaa Xaa Leu Lys Ala Val Ser Lys Glu Met Ser Lys Arg Ser Pro
            20                  25                  30


<210> 465
<211> 31
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 465
Val Pro Leu Ser Arg Thr Xaa Arg Xaa Thr Xaa Ile Ser Ile Xaa Xaa

1                   5                   10                  15

Xaa Xaa Leu Lys Ala Val Ser Lys Glu Met Ser Lys Arg Ser Pro
            20                  25                  30

<210> 466
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 466
Val Pro Leu Ser Arg Thr Val Xaa Xaa Thr Xaa Ile Ser Ile Xaa Xaa
1               5                   10                  15

Xaa Xaa Leu Lys Ala Val Ser Lys Glu Met Ser Lys Arg Ser Pro
            20                  25                  30

<210> 467
<211> 31
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 467
Val Pro Leu Ser Arg Thr Val Arg Cys Thr Cys Xaa Ser Ile Xaa Xaa
1               5                   10                  15

Xaa Xaa Leu Lys Ala Val Ser Lys Glu Met Ser Lys Arg Ser Pro
            20                  25                  30

<210> 468
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 468
Xaa Pro Leu Ser Arg Thr Val Arg Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 469
<211> 31
<212> PRT

<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 469
Val Xaa Leu Ser Arg Thr Val Arg Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 470
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 470
Val Pro Xaa Ser Arg Thr Val Arg Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 471
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 471

Val Pro Leu Xaa Arg Thr Val Arg Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 472
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 472
Val Pro Leu Ser Xaa Thr Val Arg Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 473
<211> 31
<212> PRT
<213> Artificial Sequence

<220>
<223> Description of Artificial Sequence: Synthetic peptide

<220>
<221> MOD_RES

<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 473
Val Pro Leu Ser Arg Xaa Val Arg Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 474
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 474
Val Pro Leu Ser Arg Thr Xaa Arg Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 475
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 475
Val Pro Leu Ser Arg Thr Val Xaa Xaa Thr Xaa Xaa Ser Ile Xaa Xaa
1               5                   10                  15

Xaa Xaa Leu Lys Ala Val Ser Lys Glu Met Ser Lys Arg Ser Pro
            20                  25                  30

<210> 476
<211> 31
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 476
Val Pro Leu Ser Arg Thr Val Arg Cys Thr Cys Ile Xaa Ile Xaa Xaa
1               5                   10                  15

Xaa Xaa Leu Lys Ala Val Ser Lys Glu Met Ser Lys Arg Ser Pro
            20                  25                  30

<210> 477
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 477
Xaa Pro Leu Ser Arg Thr Val Arg Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 478
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 478
Val Xaa Leu Ser Arg Thr Val Arg Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 479
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 479
Val Pro Xaa Ser Arg Thr Val Arg Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 480
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 480
Val Pro Leu Xaa Arg Thr Val Arg Xaa Thr Xaa Ile Xaa Ile Xaa Xaa
1               5                   10                  15


Xaa Xaa Leu Lys Ala Val Ser Lys Glu Met Ser Lys Arg Ser Pro
            20                  25                  30


<210> 481
<211> 31
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see

specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 481
Val Pro Leu Ser Xaa Thr Val Arg Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 482
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES

<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 482
Val Pro Leu Ser Arg Xaa Val Arg Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 483
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 483
Val Pro Leu Ser Arg Thr Xaa Arg Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 484
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 484
Val Pro Leu Ser Arg Thr Val Xaa Xaa Thr Xaa Ile Xaa Ile Xaa Xaa
1               5                   10                  15

Xaa Xaa Leu Lys Ala Val Ser Lys Glu Met Ser Lys Arg Ser Pro
            20                  25                  30

<210> 485
<211> 31
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 485
Val Pro Leu Ser Arg Thr Val Arg Cys Thr Cys Ile Ser Xaa Xaa Xaa
1               5                   10                  15

Xaa Xaa Leu Lys Ala Val Ser Lys Glu Met Ser Lys Arg Ser Pro
            20                  25                  30

<210> 486
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 486
Xaa Pro Leu Ser Arg Thr Val Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 487
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 487
Val Xaa Leu Ser Arg Thr Val Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 488
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 488
Val Pro Xaa Ser Arg Thr Val Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 489
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 489
Val Pro Leu Xaa Arg Thr Val Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 490
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see

specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 490
Val Pro Leu Ser Xaa Thr Val Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 491
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES

<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 491
Val Pro Leu Ser Arg Xaa Val Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 492
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 492
Val Pro Leu Ser Arg Thr Xaa Arg Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 493
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 493
Val Pro Leu Ser Arg Thr Val Xaa Xaa Thr Xaa Ile Ser Xaa Xaa Xaa
1               5                   10                  15

Xaa Xaa Leu Lys Ala Val Ser Lys Glu Met Ser Lys Arg Ser Pro
            20                  25                  30

<210> 494
<211> 34
<212> PRT
<213> Artificial Sequence

<220>
<223> Description of Artificial Sequence: Synthetic peptide

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 494
Val Pro Leu Ser Arg Thr Val Arg Cys Thr Cys Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 495
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 495
Xaa Pro Leu Ser Arg Thr Val Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 496
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 496
Val Xaa Leu Ser Arg Thr Val Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 497
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 497
Val Pro Xaa Ser Arg Thr Val Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 498
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see

specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 498
Val Pro Leu Xaa Arg Thr Val Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 499
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 499
Val Pro Leu Ser Xaa Thr Val Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 500
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 500
Val Pro Leu Ser Arg Xaa Val Arg Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 501
<211> 34
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 501
Val Pro Leu Ser Arg Thr Xaa Arg Xaa Thr Xaa Ile Ser Ile Ser Asn
1               5                   10                  15


Gln Xaa Xaa Xaa Xaa Leu Lys Ala Val Ser Lys Glu Met Ser Lys Arg
            20                  25                  30


Ser Pro




<210> 502
<211> 34
<212> PRT
<213> Artificial Sequence

<220>
<223> Description of Artificial Sequence: Synthetic peptide


<220>
<221> MOD_RES
<222> (8)..(9)

<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 502
Val Pro Leu Ser Arg Thr Val Xaa Xaa Thr Xaa Ile Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 503
<211> 34
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 503
Val Pro Leu Ser Arg Thr Val Arg Cys Thr Cys Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 504
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 504
Xaa Pro Leu Ser Arg Thr Val Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 505
<211> 34
<212> PRT
<213> Artificial Sequence

<220>
<223> Description of Artificial Sequence: Synthetic peptide

<220>

<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 505
Val Xaa Leu Ser Arg Thr Val Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 506
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 506
Val Pro Xaa Ser Arg Thr Val Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 507
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 507
Val Pro Leu Xaa Arg Thr Val Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 508
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 508
Val Pro Leu Ser Xaa Thr Val Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 509
<211> 34

<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 509
Val Pro Leu Ser Arg Xaa Val Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 510
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 510
Val Pro Leu Ser Arg Thr Xaa Arg Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 511
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 511
Val Pro Leu Ser Arg Thr Val Xaa Xaa Thr Xaa Xaa Ser Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 512
<211> 34
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 512
Val Pro Leu Ser Arg Thr Val Arg Cys Thr Cys Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 513
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 513
Xaa Pro Leu Ser Arg Thr Val Arg Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 514
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 514
Val Xaa Leu Ser Arg Thr Val Arg Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 515
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>

<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 515
Val Pro Xaa Ser Arg Thr Val Arg Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 516
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES

<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 516
Val Pro Leu Xaa Arg Thr Val Arg Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 517
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 517
Val Pro Leu Ser Xaa Thr Val Arg Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 518
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 518
Val Pro Leu Ser Arg Xaa Val Arg Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 519
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 519
Val Pro Leu Ser Arg Thr Xaa Arg Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 520
<211> 34
<212> PRT

<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 520
Val Pro Leu Ser Arg Thr Val Xaa Xaa Thr Xaa Ile Xaa Ile Ser Asn
1               5                   10                  15

Gln Xaa Xaa Xaa Xaa Leu Lys Ala Val Ser Lys Glu Met Ser Lys Arg
            20                  25                  30

Ser Pro

<210> 521
<211> 34
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES

<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 521
Val Pro Leu Ser Arg Thr Val Arg Cys Thr Cys Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 522
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 522
Xaa Pro Leu Ser Arg Thr Val Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 523
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 523
Val Xaa Leu Ser Arg Thr Val Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 524
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 524
Val Pro Xaa Ser Arg Thr Val Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 525
<211> 34
<212> PRT

<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 525
Val Pro Leu Xaa Arg Thr Val Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 526
<211> 34
<212> PRT
<213> Artificial Sequence

<220>
<223> Description of Artificial Sequence: Synthetic peptide

<220>
<221> MOD_RES

<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 526
Val Pro Leu Ser Xaa Thr Val Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 527
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)

<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 527
Val Pro Leu Ser Arg Xaa Val Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 528
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see

specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 528
Val Pro Leu Ser Arg Thr Xaa Arg Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 529
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 529
Val Pro Leu Ser Arg Thr Val Xaa Xaa Thr Xaa Ile Ser Xaa Ser Asn
1               5                   10                  15

Gln Xaa Xaa Xaa Xaa Leu Lys Ala Val Ser Lys Glu Met Ser Lys Arg
            20                  25                  30

Ser Pro

<210> 530
<211> 34
<212> PRT
<213> Artificial Sequence

<220>
<223> Description of Artificial Sequence: Synthetic peptide

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 530
Val Pro Leu Ser Arg Thr Val Arg Cys Thr Cys Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 531
<211> 34
<212> PRT
<213> Artificial Sequence

<220>
<223> Description of Artificial Sequence: Synthetic peptide

<220>
<221> MOD_RES

<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 531
Xaa Pro Leu Ser Arg Thr Val Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 532
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)

<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 532
Val Xaa Leu Ser Arg Thr Val Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 533
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see

specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 533
Val Pro Xaa Ser Arg Thr Val Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 534
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups

and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 534
Val Pro Leu Xaa Arg Thr Val Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 535
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 535
Val Pro Leu Ser Xaa Thr Val Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 536
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 536
Val Pro Leu Ser Arg Xaa Val Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 537
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 537
Val Pro Leu Ser Arg Thr Xaa Arg Xaa Thr Xaa Ile Ser Ile Ser Xaa
1               5                   10                  15

Gln Xaa Xaa Xaa Xaa Leu Lys Ala Val Ser Lys Glu Met Ser Lys Arg
            20                  25                  30

Ser Pro

<210> 538
<211> 34
<212> PRT
<213> Artificial Sequence

<220>
<223> Description of Artificial Sequence: Synthetic peptide


<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 538
Val Pro Leu Ser Arg Thr Val Xaa Xaa Thr Xaa Ile Ser Ile Ser Xaa
1               5                   10                  15


Gln Xaa Xaa Xaa Xaa Leu Lys Ala Val Ser Lys Glu Met Ser Lys Arg
            20                  25                  30


Ser Pro




<210> 539
<211> 34
<212> PRT
<213> Artificial Sequence

<220>
<223> Description of Artificial Sequence: Synthetic peptide


<220>
<221> MOD_RES
<222> (17)..(17)
<223> Any L- or D-natural amino acid and any non-natural amino acid; see
specification as filed for detailed description of substitutions, R-groups

and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 539
Val Pro Leu Ser Arg Thr Val Arg Cys Thr Cys Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 540
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 540
Xaa Pro Leu Ser Arg Thr Val Arg Xaa Thr Xaa Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 541
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 541
Val Xaa Leu Ser Arg Thr Val Arg Xaa Thr Xaa Ile Ser Ile Ser Gln

1                   5                   10                  15

Xaa Xaa Xaa Xaa Xaa Leu Lys Ala Val Ser Lys Glu Met Ser Lys Arg
                20                  25                  30

Ser Pro

<210> 542
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 542
Val Pro Xaa Ser Arg Thr Val Arg Xaa Thr Xaa Ile Ser Ile Ser Gln
1                   5                   10                  15

Xaa Xaa Xaa Xaa Xaa Leu Lys Ala Val Ser Lys Glu Met Ser Lys Arg
                20                  25                  30

Ser Pro

<210> 543
<211> 34
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 543
Val Pro Leu Xaa Arg Thr Val Arg Xaa Thr Xaa Ile Ser Ile Ser Gln
1               5                   10                  15


Xaa Xaa Xaa Xaa Xaa Leu Lys Ala Val Ser Lys Glu Met Ser Lys Arg
            20                  25                  30


Ser Pro




<210> 544
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 544
Val Pro Leu Ser Xaa Thr Val Arg Xaa Thr Xaa Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 545
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 545
Val Pro Leu Ser Arg Xaa Val Arg Xaa Thr Xaa Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 546
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 546
Val Pro Leu Ser Arg Thr Xaa Arg Xaa Thr Xaa Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 547

<400> 547
000

<210> 548
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 548
Val Pro Leu Ser Arg Thr Val Xaa Xaa Thr Xaa Ile Ser Ile Ser Gln
1               5                   10                  15

Xaa Xaa Xaa Xaa Xaa Leu Lys Ala Val Ser Lys Glu Met Ser Lys Arg
            20                  25                  30

Ser Pro

<210> 549
<211> 31
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 549
Val Pro Leu Ser Arg Thr Val Arg Cys Thr Cys Ile Ser Ile Xaa Xaa
1               5                   10                  15

Xaa Xaa Leu Asn Pro Glu Ser Lys Ala Ile Lys Asn Leu Leu Lys
            20                  25                  30

<210> 550
<211> 31
<212> PRT
<213> Artificial Sequence

<220>
<223> Description of Artificial Sequence: Synthetic peptide

<220>

<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 550
Xaa Pro Leu Ser Arg Thr Val Arg Xaa Thr Xaa Ile Ser Ile Xaa Xaa
1               5                   10                  15

Xaa Xaa Leu Asn Pro Glu Ser Lys Ala Ile Lys Asn Leu Leu Lys
            20                  25                  30

<210> 551
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 551
Val Xaa Leu Ser Arg Thr Val Arg Xaa Thr Xaa Ile Ser Ile Xaa Xaa
1               5                   10                  15

Xaa Xaa Leu Asn Pro Glu Ser Lys Ala Ile Lys Asn Leu Leu Lys
            20                  25                  30

<210> 552
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 552
Val Pro Xaa Ser Arg Thr Val Arg Xaa Thr Xaa Ile Ser Ile Xaa Xaa
1               5                   10                  15

Xaa Xaa Leu Asn Pro Glu Ser Lys Ala Ile Lys Asn Leu Leu Lys
            20                  25                  30

<210> 553
<211> 31
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 553
Val Pro Leu Xaa Arg Thr Val Arg Xaa Thr Xaa Ile Ser Ile Xaa Xaa
1               5                   10                  15


Xaa Xaa Leu Asn Pro Glu Ser Lys Ala Ile Lys Asn Leu Leu Lys
            20                  25                  30


<210> 554
<211> 31
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES

<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 554
Val Pro Leu Ser Xaa Thr Val Arg Xaa Thr Xaa Ile Ser Ile Xaa Xaa
1               5                   10                  15

Xaa Xaa Leu Asn Pro Glu Ser Lys Ala Ile Lys Asn Leu Leu Lys
            20                  25                  30

<210> 555
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 555
Val Pro Leu Ser Arg Xaa Val Arg Xaa Thr Xaa Ile Ser Ile Xaa Xaa
1               5                   10                  15

Xaa Xaa Leu Asn Pro Glu Ser Lys Ala Ile Lys Asn Leu Leu Lys
            20                  25                  30

<210> 556
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 556
Val Pro Leu Ser Arg Thr Xaa Arg Xaa Thr Xaa Ile Ser Ile Xaa Xaa
1               5                   10                  15

Xaa Xaa Leu Asn Pro Glu Ser Lys Ala Ile Lys Asn Leu Leu Lys
            20                  25                  30

<210> 557
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 557
Val Pro Leu Ser Arg Thr Val Xaa Xaa Thr Xaa Ile Ser Ile Xaa Xaa
1               5                   10                  15


Xaa Xaa Leu Asn Pro Glu Ser Lys Ala Ile Lys Asn Leu Leu Lys
            20                  25                  30


<210> 558
<211> 31
<212> PRT
<213> Artificial Sequence

<220>
<223> Description of Artificial Sequence: Synthetic peptide


<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 558
Val Pro Leu Ser Arg Thr Val Arg Cys Thr Cys Xaa Ser Ile Xaa Xaa
1               5                   10                  15


Xaa Xaa Leu Asn Pro Glu Ser Lys Ala Ile Lys Asn Leu Leu Lys
            20                  25                  30

<210> 559
<211> 31
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 559
Xaa Pro Leu Ser Arg Thr Val Arg Xaa Thr Xaa Xaa Ser Ile Xaa Xaa
1               5                   10                  15


Xaa Xaa Leu Asn Pro Glu Ser Lys Ala Ile Lys Asn Leu Leu Lys
            20                  25                  30


<210> 560
<211> 31
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 560
Val Xaa Leu Ser Arg Thr Val Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 561
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 561
Val Pro Xaa Ser Arg Thr Val Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 562
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 562
Val Pro Leu Xaa Arg Thr Val Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 563
<211> 31
<212> PRT
<213> Artificial Sequence

<220>

<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 563
Val Pro Leu Ser Xaa Thr Val Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 564
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 564
Val Pro Leu Ser Arg Xaa Val Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 565
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 565
Val Pro Leu Ser Arg Thr Xaa Arg Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 566
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 566
Val Pro Leu Ser Arg Thr Val Xaa Xaa Thr Xaa Xaa Ser Ile Xaa Xaa
1               5                   10                  15

Xaa Xaa Leu Asn Pro Glu Ser Lys Ala Ile Lys Asn Leu Leu Lys
            20                  25                  30

<210> 567
<211> 31
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 567
Val Pro Leu Ser Arg Thr Val Arg Cys Thr Cys Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 568
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 568
Xaa Pro Leu Ser Arg Thr Val Arg Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 569
<211> 31
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 569
Val Xaa Leu Ser Arg Thr Val Arg Xaa Thr Xaa Ile Xaa Ile Xaa Xaa
1               5                   10                  15


Xaa Xaa Leu Asn Pro Glu Ser Lys Ala Ile Lys Asn Leu Leu Lys
            20                  25                  30


<210> 570
<211> 31
<212> PRT
<213> Artificial Sequence

<220>
<223> Description of Artificial Sequence: Synthetic peptide


<220>

<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 570
Val Pro Xaa Ser Arg Thr Val Arg Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 571
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 571
Val Pro Leu Xaa Arg Thr Val Arg Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 572
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 572
Val Pro Leu Ser Xaa Thr Val Arg Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 573
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 573
Val Pro Leu Ser Arg Xaa Val Arg Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 574
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 574
Val Pro Leu Ser Arg Thr Xaa Arg Xaa Thr Xaa Ile Xaa Ile Xaa Xaa
1               5                   10                  15

Xaa Xaa Leu Asn Pro Glu Ser Lys Ala Ile Lys Asn Leu Leu Lys
            20                  25                  30

<210> 575
<211> 31

<212> PRT
<213> Artificial Sequence

<220>
<223> Description of Artificial Sequence: Synthetic peptide


<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 575
Val Pro Leu Ser Arg Thr Val Xaa Xaa Thr Xaa Ile Xaa Ile Xaa Xaa
1               5                   10                  15


Xaa Xaa Leu Asn Pro Glu Ser Lys Ala Ile Lys Asn Leu Leu Lys
            20                  25                  30


<210> 576
<211> 31
<212> PRT
<213> Artificial Sequence

<220>
<223> Description of Artificial Sequence: Synthetic peptide


<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 576
Val Pro Leu Ser Arg Thr Val Arg Cys Thr Cys Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 577
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 577
Xaa Pro Leu Ser Arg Thr Val Arg Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 578
<211> 31
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 578
Val Xaa Leu Ser Arg Thr Val Arg Xaa Thr Xaa Ile Ser Xaa Xaa Xaa
1               5                   10                  15


Xaa Xaa Leu Asn Pro Glu Ser Lys Ala Ile Lys Asn Leu Leu Lys
            20                  25                  30


<210> 579
<211> 31
<212> PRT
<213> Artificial Sequence

<220>
<223> Description of Artificial Sequence: Synthetic peptide


<220>

<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 579
Val Pro Xaa Ser Arg Thr Val Arg Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 580
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 580
Val Pro Leu Xaa Arg Thr Val Arg Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 581
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 581
Val Pro Leu Ser Xaa Thr Val Arg Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 582
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 582
Val Pro Leu Ser Arg Xaa Val Arg Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 583
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 583
Val Pro Leu Ser Arg Thr Xaa Arg Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 584
<211> 31

<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 584
Val Pro Leu Ser Arg Thr Val Xaa Xaa Thr Xaa Ile Ser Xaa Xaa Xaa
1               5                   10                  15

Xaa Xaa Leu Asn Pro Glu Ser Lys Ala Ile Lys Asn Leu Leu Lys
            20                  25                  30

<210> 585
<211> 34
<212> PRT
<213> Artificial Sequence

<220>
<223> Description of Artificial Sequence: Synthetic peptide

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 585
Val Pro Leu Ser Arg Thr Val Arg Cys Thr Cys Ile Ser Ile Ser Asn

1                   5                   10                  15

Gln Xaa Xaa Xaa Xaa Leu Asn Pro Glu Ser Lys Ala Ile Lys Asn Leu
                20                  25                  30

Leu Lys

<210> 586
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 586
Xaa Pro Leu Ser Arg Thr Val Arg Xaa Thr Xaa Ile Ser Ile Ser Asn
1                   5                   10                  15

Gln Xaa Xaa Xaa Xaa Leu Asn Pro Glu Ser Lys Ala Ile Lys Asn Leu
                20                  25                  30

Leu Lys

<210> 587
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 587
Val Xaa Leu Ser Arg Thr Val Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 588
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)

<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 588
Val Pro Xaa Ser Arg Thr Val Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 589
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 589
Val Pro Leu Xaa Arg Thr Val Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 590
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 590
Val Pro Leu Ser Xaa Thr Val Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 591
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 591
Val Pro Leu Ser Arg Xaa Val Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 592
<211> 34
<212> PRT
<213> Artificial Sequence

<220>
<223> Description of Artificial Sequence: Synthetic peptide

<220>
<221> MOD_RES

<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 592
Val Pro Leu Ser Arg Thr Xaa Arg Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 593
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 593
Val Pro Leu Ser Arg Thr Val Xaa Xaa Thr Xaa Ile Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 594
<211> 34
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 594
Val Pro Leu Ser Arg Thr Val Arg Cys Thr Cys Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 595
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 595
Xaa Pro Leu Ser Arg Thr Val Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 596
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>

<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 596
Val Xaa Leu Ser Arg Thr Val Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 597
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 597
Val Pro Xaa Ser Arg Thr Val Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 598
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 598
Val Pro Leu Xaa Arg Thr Val Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 599

<211> 34
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 599
Val Pro Leu Ser Xaa Thr Val Arg Xaa Thr Xaa Xaa Ser Ile Ser Asn
1               5                   10                  15


Gln Xaa Xaa Xaa Xaa Leu Asn Pro Glu Ser Lys Ala Ile Lys Asn Leu
            20                  25                  30


Leu Lys




<210> 600
<211> 34
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 600
Val Pro Leu Ser Arg Xaa Val Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 601
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>

<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 601
Val Pro Leu Ser Arg Thr Xaa Arg Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 602
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 602
Val Pro Leu Ser Arg Thr Val Xaa Xaa Thr Xaa Xaa Ser Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 603
<211> 34
<212> PRT
<213> Artificial Sequence

<220>
<223> Description of Artificial Sequence: Synthetic peptide


<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 603
Val Pro Leu Ser Arg Thr Val Arg Cys Thr Cys Ile Xaa Ile Ser Asn
1               5                   10                  15


Gln Xaa Xaa Xaa Xaa Leu Asn Pro Glu Ser Lys Ala Ile Lys Asn Leu
            20                  25                  30


Leu Lys



<210> 604
<211> 34
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 604
Xaa Pro Leu Ser Arg Thr Val Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 605
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 605
Val Xaa Leu Ser Arg Thr Val Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 606
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 606
Val Pro Xaa Ser Arg Thr Val Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 607
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 607
Val Pro Leu Xaa Arg Thr Val Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu

20                  25                  30

Leu Lys

<210> 608
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 608
Val Pro Leu Ser Xaa Thr Val Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 609

<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 609
Val Pro Leu Ser Arg Xaa Val Arg Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 610
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 610
Val Pro Leu Ser Arg Thr Xaa Arg Xaa Thr Xaa Ile Xaa Ile Ser Asn
1               5                   10                  15


Gln Xaa Xaa Xaa Xaa Leu Asn Pro Glu Ser Lys Ala Ile Lys Asn Leu
            20                  25                  30


Leu Lys




<210> 611
<211> 34
<212> PRT
<213> Artificial Sequence

<220>
<223> Description of Artificial Sequence: Synthetic peptide


<220>
<221> MOD_RES
<222> (8)..(9)
<223> Any L- or D-natural amino acid and any non-natural amino acid; see
specification as filed for detailed description of substitutions, R-groups
and preferred embodiments

<220>

<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 611
Val Pro Leu Ser Arg Thr Val Xaa Xaa Thr Xaa Ile Xaa Ile Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 612
<211> 34
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 612
Val Pro Leu Ser Arg Thr Val Arg Cys Thr Cys Ile Ser Xaa Ser Asn
1               5                   10                  15

Gln Xaa Xaa Xaa Xaa Leu Asn Pro Glu Ser Lys Ala Ile Lys Asn Leu

20                  25                  30

Leu Lys

<210> 613
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 613
Xaa Pro Leu Ser Arg Thr Val Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 614

<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 614
Val Xaa Leu Ser Arg Thr Val Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 615
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 615
Val Pro Xaa Ser Arg Thr Val Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 616
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 616
Val Pro Leu Xaa Arg Thr Val Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 617
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 617
Val Pro Leu Ser Xaa Thr Val Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 618
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)

<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 618
Val Pro Leu Ser Arg Xaa Val Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 619
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may

encompass 1 or 4 residues; see specification as filed for detailed
description of substitutions, R-groups and preferred embodiments

<220>
<223> C-term may be amidated

<400> 619
Val Pro Leu Ser Arg Thr Xaa Arg Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 620
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 620
Val Pro Leu Ser Arg Thr Val Xaa Xaa Thr Xaa Ile Ser Xaa Ser Asn
1               5                   10                  15

Gln Xaa Xaa Xaa Xaa Leu Asn Pro Glu Ser Lys Ala Ile Lys Asn Leu
            20                  25                  30

Leu Lys

<210> 621
<211> 34
<212> PRT
<213> Artificial Sequence

<220>
<223> Description of Artificial Sequence: Synthetic peptide

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 621
Val Pro Leu Ser Arg Thr Val Arg Cys Thr Cys Ile Ser Ile Ser Xaa
1               5                   10                  15

Gln Xaa Xaa Xaa Xaa Leu Asn Pro Glu Ser Lys Ala Ile Lys Asn Leu
            20                  25                  30

Leu Lys

<210> 622
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 622
Xaa Pro Leu Ser Arg Thr Val Arg Xaa Thr Xaa Ile Ser Ile Ser Xaa
1               5                   10                  15

Gln Xaa Xaa Xaa Xaa Leu Asn Pro Glu Ser Lys Ala Ile Lys Asn Leu
            20                  25                  30

Leu Lys

<210> 623
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)

<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 623
Val Xaa Leu Ser Arg Thr Val Arg Xaa Thr Xaa Ile Ser Ile Ser Xaa
1               5                   10                  15

Gln Xaa Xaa Xaa Xaa Leu Asn Pro Glu Ser Lys Ala Ile Lys Asn Leu
            20                  25                  30

Leu Lys

<210> 624
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 624
Val Pro Xaa Ser Arg Thr Val Arg Xaa Thr Xaa Ile Ser Ile Ser Xaa
1               5                   10                  15

Gln Xaa Xaa Xaa Xaa Leu Asn Pro Glu Ser Lys Ala Ile Lys Asn Leu
            20                  25                  30

Leu Lys

<210> 625
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 625
Val Pro Leu Xaa Arg Thr Val Arg Xaa Thr Xaa Ile Ser Ile Ser Xaa

1                   5                   10                  15

Gln Xaa Xaa Xaa Xaa Leu Asn Pro Glu Ser Lys Ala Ile Lys Asn Leu
                20                  25                  30

Leu Lys

<210> 626
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 626
Val Pro Leu Ser Xaa Thr Val Arg Xaa Thr Xaa Ile Ser Ile Ser Xaa
1               5                   10                  15

Gln Xaa Xaa Xaa Xaa Leu Asn Pro Glu Ser Lys Ala Ile Lys Asn Leu
                20                  25                  30

Leu Lys

<210> 627
<211> 34
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 627
Val Pro Leu Ser Arg Xaa Val Arg Xaa Thr Xaa Ile Ser Ile Ser Xaa
1               5                   10                  15


Gln Xaa Xaa Xaa Xaa Leu Asn Pro Glu Ser Lys Ala Ile Lys Asn Leu
            20                  25                  30


Leu Lys



<210> 628
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 628
Val Pro Leu Ser Arg Thr Xaa Arg Xaa Thr Xaa Ile Ser Ile Ser Xaa
1               5                   10                  15

Gln Xaa Xaa Xaa Xaa Leu Asn Pro Glu Ser Lys Ala Ile Lys Asn Leu
            20                  25                  30

Leu Lys

<210> 629
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see

specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 629
Val Pro Leu Ser Arg Thr Val Xaa Xaa Thr Xaa Ile Ser Ile Ser Xaa
1               5                   10                  15

Gln Xaa Xaa Xaa Xaa Leu Asn Pro Glu Ser Lys Ala Ile Lys Asn Leu
            20                  25                  30

Leu Lys

<210> 630
<211> 34
<212> PRT
<213> Artificial Sequence

<220>
<223> Description of Artificial Sequence: Synthetic peptide

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 630
Val Pro Leu Ser Arg Thr Val Arg Cys Thr Cys Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 631
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 631
Xaa Pro Leu Ser Arg Thr Val Arg Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 632
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 632
Val Xaa Leu Ser Arg Thr Val Arg Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 633
<211> 34
<212> PRT

<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 633
Val Pro Xaa Ser Arg Thr Val Arg Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 634
<211> 34
<212> PRT
<213> Artificial Sequence

<220>
<223> Description of Artificial Sequence: Synthetic peptide

<220>
<221> MOD_RES

<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 634
Val Pro Leu Xaa Arg Thr Val Arg Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 635
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)

<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 635
Val Pro Leu Ser Xaa Thr Val Arg Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 636
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see

specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 636
Val Pro Leu Ser Arg Xaa Val Arg Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 637
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups

and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 637
Val Pro Leu Ser Arg Thr Xaa Arg Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 638
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 638
Val Pro Leu Ser Arg Thr Val Xaa Xaa Thr Xaa Ile Ser Ile Ser Gln
1               5                   10                  15

Xaa Xaa Xaa Xaa Xaa Leu Asn Pro Glu Ser Lys Ala Ile Lys Asn Leu
            20                  25                  30

Leu Lys

<210> 639
<211> 31
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 639
Val Pro Leu Ser Arg Thr Val Arg Cys Thr Cys Ile Ser Ile Xaa Xaa
1               5                   10                  15

Xaa Xaa Lys Ala Ile Lys Asn Leu Leu Lys Ala Val Ser Lys Glu
            20                  25                  30

<210> 640
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 640
Xaa Pro Leu Ser Arg Thr Val Arg Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 641
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 641
Val Xaa Leu Ser Arg Thr Val Arg Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 642
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 642
Val Pro Xaa Ser Arg Thr Val Arg Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 643
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)

<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 643
Val Pro Leu Xaa Arg Thr Val Arg Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 644
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 644
Val Pro Leu Ser Xaa Thr Val Arg Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 645
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 645
Val Pro Leu Ser Arg Xaa Val Arg Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 646
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 646
Val Pro Leu Ser Arg Thr Xaa Arg Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 647
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 647
Val Pro Leu Ser Arg Thr Val Xaa Xaa Thr Xaa Ile Ser Ile Xaa Xaa
1               5                   10                  15

Xaa Xaa Lys Ala Ile Lys Asn Leu Leu Lys Ala Val Ser Lys Glu
            20                  25                  30

<210> 648
<211> 31
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 648
Val Pro Leu Ser Arg Thr Val Arg Cys Thr Cys Xaa Ser Ile Xaa Xaa
1               5                   10                  15

Xaa Xaa Lys Ala Ile Lys Asn Leu Leu Lys Ala Val Ser Lys Glu
            20                  25                  30

<210> 649
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES

<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 649
Xaa Pro Leu Ser Arg Thr Val Arg Xaa Thr Xaa Xaa Ser Ile Xaa Xaa
1               5                   10                  15


Xaa Xaa Lys Ala Ile Lys Asn Leu Leu Lys Ala Val Ser Lys Glu
            20                  25                  30


<210> 650
<211> 31
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 650
Val Xaa Leu Ser Arg Thr Val Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 651
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 651
Val Pro Xaa Ser Arg Thr Val Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 652
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 652
Val Pro Leu Xaa Arg Thr Val Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 653
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)

<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 653
Val Pro Leu Ser Xaa Thr Val Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 654
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 654
Val Pro Leu Ser Arg Xaa Val Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 655
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 655
Val Pro Leu Ser Arg Thr Xaa Arg Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 656
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 656
Val Pro Leu Ser Arg Thr Val Xaa Xaa Thr Xaa Xaa Ser Ile Xaa Xaa
1               5                   10                  15

Xaa Xaa Lys Ala Ile Lys Asn Leu Leu Lys Ala Val Ser Lys Glu
            20                  25                  30

<210> 657
<211> 31
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 657
Val Pro Leu Ser Arg Thr Val Arg Cys Thr Cys Ile Xaa Ile Xaa Xaa
1               5                   10                  15

Xaa Xaa Lys Ala Ile Lys Asn Leu Leu Lys Ala Val Ser Lys Glu
            20                  25                  30

<210> 658
<211> 31
<212> PRT
<213> Artificial Sequence

<220>

<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 658
Xaa Pro Leu Ser Arg Thr Val Arg Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 659
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES

<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 659
Val Xaa Leu Ser Arg Thr Val Arg Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 660
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 660
Val Pro Xaa Ser Arg Thr Val Arg Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 661
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 661
Val Pro Leu Xaa Arg Thr Val Arg Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 662
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 662
Val Pro Leu Ser Xaa Thr Val Arg Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 663
<211> 31
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 663
Val Pro Leu Ser Arg Xaa Val Arg Xaa Thr Xaa Ile Xaa Ile Xaa Xaa
1               5                   10                  15


Xaa Xaa Lys Ala Ile Lys Asn Leu Leu Lys Ala Val Ser Lys Glu
            20                  25                  30


<210> 664
<211> 31
<212> PRT
<213> Artificial Sequence

<220>
<223> Description of Artificial Sequence: Synthetic peptide


<220>

<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 664
Val Pro Leu Ser Arg Thr Xaa Arg Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 665
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 665
Val Pro Leu Ser Arg Thr Val Xaa Xaa Thr Xaa Ile Xaa Ile Xaa Xaa
1               5                   10                  15

Xaa Xaa Lys Ala Ile Lys Asn Leu Leu Lys Ala Val Ser Lys Glu
            20                  25                  30

<210> 666
<211> 31
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 666
Val Pro Leu Ser Arg Thr Val Arg Cys Thr Cys Ile Ser Xaa Xaa Xaa
1               5                   10                  15

Xaa Xaa Lys Ala Ile Lys Asn Leu Leu Lys Ala Val Ser Lys Glu
            20                  25                  30

<210> 667
<211> 31
<212> PRT
<213> Artificial Sequence

<220>

<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 667
Xaa Pro Leu Ser Arg Thr Val Arg Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 668
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES

<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 668
Val Xaa Leu Ser Arg Thr Val Arg Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 669
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 669
Val Pro Xaa Ser Arg Thr Val Arg Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 670
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 670
Val Pro Leu Xaa Arg Thr Val Arg Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 671
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 671
Val Pro Leu Ser Xaa Thr Val Arg Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 672
<211> 31
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 672
Val Pro Leu Ser Arg Xaa Val Arg Xaa Thr Xaa Ile Ser Xaa Xaa Xaa
1               5                   10                  15


Xaa Xaa Lys Ala Ile Lys Asn Leu Leu Lys Ala Val Ser Lys Glu
            20                  25                  30


<210> 673
<211> 31
<212> PRT
<213> Artificial Sequence

<220>
<223> Description of Artificial Sequence: Synthetic peptide


<220>

<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 673
Val Pro Leu Ser Arg Thr Xaa Arg Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 674
<211> 31
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 674
Val Pro Leu Ser Arg Thr Val Xaa Xaa Thr Xaa Ile Ser Xaa Xaa Xaa
1               5                   10                  15

Xaa Xaa Lys Ala Ile Lys Asn Leu Leu Lys Ala Val Ser Lys Glu
            20                  25                  30

<210> 675
<211> 34
<212> PRT
<213> Artificial Sequence

<220>
<223> Description of Artificial Sequence: Synthetic peptide

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 675
Val Pro Leu Ser Arg Thr Val Arg Cys Thr Cys Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 676
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 676
Xaa Pro Leu Ser Arg Thr Val Arg Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 677
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>

<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 677
Val Xaa Leu Ser Arg Thr Val Arg Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 678
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 678
Val Pro Xaa Ser Arg Thr Val Arg Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 679
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 679
Val Pro Leu Xaa Arg Thr Val Arg Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 680

<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 680
Val Pro Leu Ser Xaa Thr Val Arg Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 681
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 681
Val Pro Leu Ser Arg Xaa Val Arg Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 682
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>

<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 682
Val Pro Leu Ser Arg Thr Xaa Arg Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 683
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 683
Val Pro Leu Ser Arg Thr Val Xaa Xaa Thr Xaa Ile Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 684
<211> 34
<212> PRT
<213> Artificial Sequence

<220>
<223> Description of Artificial Sequence: Synthetic peptide


<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 684
Val Pro Leu Ser Arg Thr Val Arg Cys Thr Cys Xaa Ser Ile Ser Asn
1               5                   10                  15


Gln Xaa Xaa Xaa Xaa Lys Ala Ile Lys Asn Leu Leu Lys Ala Val Ser
            20                  25                  30


Lys Glu




<210> 685
<211> 34
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 685
Xaa Pro Leu Ser Arg Thr Val Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 686
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 686
Val Xaa Leu Ser Arg Thr Val Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 687
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 687
Val Pro Xaa Ser Arg Thr Val Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 688
<211> 34
<212> PRT
<213> Artificial Sequence

<220>

<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 688
Val Pro Leu Xaa Arg Thr Val Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 689
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 689
Val Pro Leu Ser Xaa Thr Val Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 690
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 690
Val Pro Leu Ser Arg Xaa Val Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 691
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 691
Val Pro Leu Ser Arg Thr Xaa Arg Xaa Thr Xaa Xaa Ser Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 692
<211> 34
<212> PRT
<213> Artificial Sequence

<220>
<223> Description of Artificial Sequence: Synthetic peptide


<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 692
Val Pro Leu Ser Arg Thr Val Xaa Xaa Thr Xaa Xaa Ser Ile Ser Asn
1               5                   10                  15


Gln Xaa Xaa Xaa Xaa Lys Ala Ile Lys Asn Leu Leu Lys Ala Val Ser
            20                  25                  30


Lys Glu




<210> 693
<211> 34
<212> PRT
<213> Artificial Sequence

<220>
<223> Description of Artificial Sequence: Synthetic peptide


<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may

encompass 1 or 4 residues; see specification as filed for detailed
description of substitutions, R-groups and preferred embodiments

<220>
<223> C-term may be amidated

<400> 693
Val Pro Leu Ser Arg Thr Val Arg Cys Thr Cys Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 694
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 694
Xaa Pro Leu Ser Arg Thr Val Arg Xaa Thr Xaa Ile Xaa Ile Ser Asn

1                   5                   10                  15

Gln Xaa Xaa Xaa Xaa Lys Ala Ile Lys Asn Leu Leu Lys Ala Val Ser
            20                  25                  30

Lys Glu

<210> 695
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 695
Val Xaa Leu Ser Arg Thr Val Arg Xaa Thr Xaa Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 696
<211> 34
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 696
Val Pro Xaa Ser Arg Thr Val Arg Xaa Thr Xaa Ile Xaa Ile Ser Asn
1               5                   10                  15


Gln Xaa Xaa Xaa Xaa Lys Ala Ile Lys Asn Leu Leu Lys Ala Val Ser
            20                  25                  30


Lys Glu



<210> 697
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 697
Val Pro Leu Xaa Arg Thr Val Arg Xaa Thr Xaa Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 698
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 698
Val Pro Leu Ser Xaa Thr Val Arg Xaa Thr Xaa Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 699
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 699
Val Pro Leu Ser Arg Xaa Val Arg Xaa Thr Xaa Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 700
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 700
Val Pro Leu Ser Arg Thr Xaa Arg Xaa Thr Xaa Ile Xaa Ile Ser Asn
1               5                   10                  15


Gln Xaa Xaa Xaa Xaa Lys Ala Ile Lys Asn Leu Leu Lys Ala Val Ser
            20                  25                  30


Lys Glu



<210> 701
<211> 34
<212> PRT
<213> Artificial Sequence

<220>
<223> Description of Artificial Sequence: Synthetic peptide


<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 701
Val Pro Leu Ser Arg Thr Val Xaa Xaa Thr Xaa Ile Xaa Ile Ser Asn
1               5                   10                  15

Gln Xaa Xaa Xaa Xaa Lys Ala Ile Lys Asn Leu Leu Lys Ala Val Ser
            20                  25                  30

Lys Glu

<210> 702
<211> 34
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 702
Val Pro Leu Ser Arg Thr Val Arg Cys Thr Cys Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 703
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see

specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 703
Xaa Pro Leu Ser Arg Thr Val Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 704
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups

and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 704
Val Xaa Leu Ser Arg Thr Val Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 705
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 705
Val Pro Xaa Ser Arg Thr Val Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 706
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 706
Val Pro Leu Xaa Arg Thr Val Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 707
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> C-term may be amidated

<400> 707
Val Pro Leu Ser Xaa Thr Val Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 708
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 708
Val Pro Leu Ser Arg Xaa Val Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser

20                  25                  30

Lys Glu

<210> 709
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 709
Val Pro Leu Ser Arg Thr Xaa Arg Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 710

<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 710
Val Pro Leu Ser Arg Thr Val Xaa Xaa Thr Xaa Ile Ser Xaa Ser Asn
1               5                   10                  15

Gln Xaa Xaa Xaa Xaa Lys Ala Ile Lys Asn Leu Leu Lys Ala Val Ser
            20                  25                  30

Lys Glu

<210> 711
<211> 34
<212> PRT
<213> Artificial Sequence

<220>
<223> Description of Artificial Sequence: Synthetic peptide

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 711
Val Pro Leu Ser Arg Thr Val Arg Cys Thr Cys Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 712
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 712
Xaa Pro Leu Ser Arg Thr Val Arg Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 713
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 713
Val Xaa Leu Ser Arg Thr Val Arg Xaa Thr Xaa Ile Ser Ile Ser Xaa
1               5                   10                  15

Gln Xaa Xaa Xaa Xaa Lys Ala Ile Lys Asn Leu Leu Lys Ala Val Ser

20                  25                  30

Lys Glu

<210> 714
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 714
Val Pro Xaa Ser Arg Thr Val Arg Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 715

<211> 34
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 715
Val Pro Leu Xaa Arg Thr Val Arg Xaa Thr Xaa Ile Ser Ile Ser Xaa
1               5                   10                  15


Gln Xaa Xaa Xaa Xaa Lys Ala Ile Lys Asn Leu Leu Lys Ala Val Ser
            20                  25                  30


Lys Glu




<210> 716
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 716
Val Pro Leu Ser Xaa Thr Val Arg Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 717
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 717
Val Pro Leu Ser Arg Xaa Val Arg Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 718
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 718
Val Pro Leu Ser Arg Thr Xaa Arg Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 719
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 719
Val Pro Leu Ser Arg Thr Val Xaa Xaa Thr Xaa Ile Ser Ile Ser Xaa
1               5                   10                  15

Gln Xaa Xaa Xaa Xaa Lys Ala Ile Lys Asn Leu Leu Lys Ala Val Ser
            20                  25                  30

Lys Glu

<210> 720
<211> 34
<212> PRT
<213> Artificial Sequence

<220>
<223> Description of Artificial Sequence: Synthetic peptide

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 720
Val Pro Leu Ser Arg Thr Val Arg Cys Thr Cys Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 721
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 721
Xaa Pro Leu Ser Arg Thr Val Arg Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 722
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>

<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 722
Val Xaa Leu Ser Arg Thr Val Arg Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 723
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES

<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 723
Val Pro Xaa Ser Arg Thr Val Arg Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 724
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)

<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 724
Val Pro Leu Xaa Arg Thr Val Arg Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 725
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may

encompass 1 or 4 residues; see specification as filed for detailed
description of substitutions, R-groups and preferred embodiments

<220>
<223> C-term may be amidated

<400> 725
Val Pro Leu Ser Xaa Thr Val Arg Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 726
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 726
Val Pro Leu Ser Arg Xaa Val Arg Xaa Thr Xaa Ile Ser Ile Ser Gln

1                   5                   10                  15

Xaa Xaa Xaa Xaa Xaa Lys Ala Ile Lys Asn Leu Leu Lys Ala Val Ser
            20                  25                  30

Lys Glu

<210> 727
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 727
Val Pro Leu Ser Arg Thr Xaa Arg Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 728
<211> 34
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 728
Val Pro Leu Ser Arg Thr Val Xaa Xaa Thr Xaa Ile Ser Ile Ser Gln
1               5                   10                  15

Xaa Xaa Xaa Xaa Xaa Lys Ala Ile Lys Asn Leu Leu Lys Ala Val Ser
            20                  25                  30

Lys Glu

<210> 729
<211> 9
<212> PRT
<213> Artificial Sequence

<220>
<223> Description of Artificial Sequence: Synthetic peptide

<220>
<223> C-term may be amidated

<400> 729

Ser Leu Ala Ala Asp Thr Pro Thr Ala
1               5

<210> 730
<211> 21
<212> PRT
<213> Artificial Sequence

<220>
<223> Description of Artificial Sequence: Synthetic peptide

<220>
<223> c-term may be amidated

<400> 730
Ala Phe Ser Tyr Thr Ser Arg Gln Ile Pro Gln Asn Ala Asp Tyr Phe
1               5                   10                  15

Glu Thr Ser Ser Gln
            20

<210> 731
<211> 15
<212> PRT
<213> Artificial Sequence

<220>
<223> Description of Artificial Sequence: Synthetic peptide

<220>
<223> c-term may be amidated

<400> 731

<210> 732
<211> 30
<212> PRT
<213> Artificial Sequence

<220>
<223> Description of Artificial Sequence: Synthetic peptide

<220>
<223> c-term may be amidated

<400> 732
Ser Leu Ala Ala Asp Thr Pro Thr Cys Cys Phe Ser Tyr Thr Ser Arg
1               5                   10                  15

Gln Ile Pro Gln Asn Ala Asp Tyr Phe Glu Thr Ser Ser Gln
            20                  25                  30

<210> 733
<211> 30
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<223> c-term may be amidated

<400> 733
Xaa Leu Ala Ala Asp Thr Pro Thr Xaa Xaa Phe Ser Tyr Thr Ser Arg
1               5                   10                  15

Gln Ile Pro Gln Asn Ala Asp Tyr Phe Glu Thr Ser Ser Gln
            20                  25                  30

<210> 734
<211> 30
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<223> c-term may be amidated

<400> 734
Ser Xaa Ala Ala Asp Thr Pro Thr Xaa Xaa Phe Ser Tyr Thr Ser Arg
1               5                   10                  15

Gln Ile Pro Gln Asn Ala Asp Tyr Phe Glu Thr Ser Ser Gln
            20                  25                  30

<210> 735
<211> 30
<212> PRT
<213> Artificial Sequence

<220>

<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<223> c-term may be amidated

<400> 735
Ser Leu Xaa Ala Asp Thr Pro Thr Xaa Xaa Phe Ser Tyr Thr Ser Arg
1               5                   10                  15

Gln Ile Pro Gln Asn Ala Asp Tyr Phe Glu Thr Ser Ser Gln
            20                  25                  30

<210> 736
<211> 30
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<223> c-term may be amidated

<400> 736
Ser Leu Ala Xaa Asp Thr Pro Thr Xaa Xaa Phe Ser Tyr Thr Ser Arg
1               5                   10                  15

Gln Ile Pro Gln Asn Ala Asp Tyr Phe Glu Thr Ser Ser Gln
            20                  25                  30

<210> 737
<211> 30
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<223> c-term may be amidated

<400> 737
Ser Leu Ala Ala Xaa Thr Pro Thr Xaa Xaa Phe Ser Tyr Thr Ser Arg
1               5                   10                  15

Gln Ile Pro Gln Asn Ala Asp Tyr Phe Glu Thr Ser Ser Gln
            20                  25                  30

<210> 738
<211> 30
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<223> c-term may be amidated

<400> 738
Ser Leu Ala Ala Asp Xaa Pro Thr Xaa Xaa Phe Ser Tyr Thr Ser Arg
1               5                   10                  15

Gln Ile Pro Gln Asn Ala Asp Tyr Phe Glu Thr Ser Ser Gln
            20                  25                  30

<210> 739
<211> 30

<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<223> C-term may be amidated

<400> 739
Ser Leu Ala Ala Asp Thr Xaa Thr Xaa Xaa Phe Ser Tyr Thr Ser Arg
1               5                   10                  15


Gln Ile Pro Gln Asn Ala Asp Tyr Phe Glu Thr Ser Ser Gln
            20                  25                  30


<210> 740
<211> 30
<212> PRT
<213> Artificial Sequence

<220>
<223> Description of Artificial Sequence: Synthetic peptide


<220>
<221> MOD_RES
<222> (8)..(10)
<223> Any L- or D-natural amino acid and any non-natural amino acid; see
specification as filed for detailed description of substitutions, R-groups
and preferred embodiments

<220>
<223> C-term may be amidated

<400> 740
Ser Leu Ala Ala Asp Thr Pro Xaa Xaa Xaa Phe Ser Tyr Thr Ser Arg
1               5                   10                  15


Gln Ile Pro Gln Asn Ala Asp Tyr Phe Glu Thr Ser Ser Gln
            20                  25                  30


<210> 741
<211> 18
<212> PRT
<213> Artificial Sequence

<220>
<223> Description of Artificial Sequence: Synthetic peptide

<220>
<223> c-term may be amidated

<400> 741
Ala Asp Pro Ser Glu Glu Trp Val Gln Tyr Val Asp Asp Leu Glu Leu
1               5                   10                  15

Ser Ala

<210> 742
<211> 32
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 742
Ser Leu Ala Ala Asp Thr Pro Thr Cys Cys Phe Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 743
<211> 32
<212> PRT
<213> Artificial Sequence

<220>
<223> Description of Artificial Sequence: Synthetic peptide

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 743

Ser Leu Ala Ala Asp Thr Pro Thr Xaa Xaa Phe Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 744
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 744
Xaa Leu Ala Ala Asp Thr Pro Thr Xaa Xaa Phe Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 745
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES

<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 745
Ser Xaa Ala Ala Asp Thr Pro Thr Xaa Xaa Phe Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 746
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 746
Ser Leu Xaa Ala Asp Thr Pro Thr Xaa Xaa Phe Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 747
<211> 32

<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 747
Ser Leu Ala Xaa Asp Thr Pro Thr Xaa Xaa Phe Ser Tyr Thr Xaa Xaa
1               5                   10                  15


Xaa Xaa Glu Glu Trp Val Gln Tyr Val Asp Asp Leu Glu Leu Ser Ala
            20                  25                  30


<210> 748
<211> 32
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 748
Ser Leu Ala Ala Xaa Thr Pro Thr Xaa Xaa Phe Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 749
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 749
Ser Leu Ala Ala Asp Xaa Pro Thr Xaa Xaa Phe Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 750
<211> 32
<212> PRT
<213> Artificial Sequence

<220>
<223> Description of Artificial Sequence: Synthetic peptide

<220>
<221> MOD_RES
<222> (7)..(7)

<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 750
Ser Leu Ala Ala Asp Thr Xaa Thr Xaa Xaa Phe Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 751
<211> 32
<212> PRT
<213> Artificial Sequence

<220>
<223> Description of Artificial Sequence: Synthetic peptide

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 751
Ser Leu Ala Ala Asp Thr Pro Xaa Xaa Xaa Phe Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 752
<211> 32
<212> PRT

<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 752
Ser Leu Ala Ala Asp Thr Pro Thr Xaa Xaa Xaa Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 753
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 753
Xaa Leu Ala Ala Asp Thr Pro Thr Xaa Xaa Xaa Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 754
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 754
Ser Xaa Ala Ala Asp Thr Pro Thr Xaa Xaa Xaa Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 755
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 755
Ser Leu Xaa Ala Asp Thr Pro Thr Xaa Xaa Xaa Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 756
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 756
Ser Leu Ala Xaa Asp Thr Pro Thr Xaa Xaa Xaa Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 757
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 757
Ser Leu Ala Ala Xaa Thr Pro Thr Xaa Xaa Xaa Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 758
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 758
Ser Leu Ala Ala Asp Xaa Pro Thr Xaa Xaa Xaa Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 759
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 759
Ser Leu Ala Ala Asp Thr Xaa Thr Xaa Xaa Xaa Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 760
<211> 32
<212> PRT
<213> Artificial Sequence

<220>
<223> Description of Artificial Sequence: Synthetic peptide

<220>
<221> MOD_RES
<222> (8)..(11)
<223> Any L- or D-natural amino acid and any non-natural amino acid; see
specification as filed for detailed description of substitutions, R-groups

and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 760
Ser Leu Ala Ala Asp Thr Pro Xaa Xaa Xaa Xaa Ser Tyr Thr Xaa Xaa
1               5                   10                  15

Xaa Xaa Glu Glu Trp Val Gln Tyr Val Asp Asp Leu Glu Leu Ser Ala
            20                  25                  30

<210> 761
<211> 32
<212> PRT
<213> Artificial Sequence

<220>
<223> Description of Artificial Sequence: Synthetic peptide

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 761
Ser Leu Ala Ala Asp Thr Pro Thr Xaa Xaa Phe Xaa Tyr Thr Xaa Xaa
1               5                   10                  15

Xaa Xaa Glu Glu Trp Val Gln Tyr Val Asp Asp Leu Glu Leu Ser Ala
            20                  25                  30

<210> 762
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 762
Xaa Leu Ala Ala Asp Thr Pro Thr Xaa Xaa Phe Xaa Tyr Thr Xaa Xaa
1               5                   10                  15

Xaa Xaa Glu Glu Trp Val Gln Tyr Val Asp Asp Leu Glu Leu Ser Ala
            20                  25                  30

<210> 763
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>

<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 763
Ser Xaa Ala Ala Asp Thr Pro Thr Xaa Xaa Phe Xaa Tyr Thr Xaa Xaa
1               5                   10                  15

Xaa Xaa Glu Glu Trp Val Gln Tyr Val Asp Asp Leu Glu Leu Ser Ala
            20                  25                  30

<210> 764
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 764
Ser Leu Xaa Ala Asp Thr Pro Thr Xaa Xaa Phe Xaa Tyr Thr Xaa Xaa
1               5                   10                  15

Xaa Xaa Glu Glu Trp Val Gln Tyr Val Asp Asp Leu Glu Leu Ser Ala
            20                  25                  30

<210> 765
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 765
Ser Leu Ala Xaa Asp Thr Pro Thr Xaa Xaa Phe Xaa Tyr Thr Xaa Xaa
1               5                   10                  15

Xaa Xaa Glu Glu Trp Val Gln Tyr Val Asp Asp Leu Glu Leu Ser Ala
            20                  25                  30

<210> 766
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see

specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 766
Ser Leu Ala Ala Xaa Thr Pro Thr Xaa Xaa Phe Xaa Tyr Thr Xaa Xaa
1               5                   10                  15

Xaa Xaa Glu Glu Trp Val Gln Tyr Val Asp Asp Leu Glu Leu Ser Ala
            20                  25                  30

<210> 767
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES

<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 767
Ser Leu Ala Ala Asp Xaa Pro Thr Xaa Xaa Phe Xaa Tyr Thr Xaa Xaa
1               5                   10                  15

Xaa Xaa Glu Glu Trp Val Gln Tyr Val Asp Asp Leu Glu Leu Ser Ala
            20                  25                  30

<210> 768
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 768
Ser Leu Ala Ala Asp Thr Xaa Thr Xaa Xaa Phe Xaa Tyr Thr Xaa Xaa
1               5                   10                  15

Xaa Xaa Glu Glu Trp Val Gln Tyr Val Asp Asp Leu Glu Leu Ser Ala
            20                  25                  30

<210> 769
<211> 32

<212> PRT
<213> Artificial Sequence

<220>
<223> Description of Artificial Sequence: Synthetic peptide


<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 769
Ser Leu Ala Ala Asp Thr Pro Xaa Xaa Xaa Phe Xaa Tyr Thr Xaa Xaa
1               5                   10                  15


Xaa Xaa Glu Glu Trp Val Gln Tyr Val Asp Asp Leu Glu Leu Ser Ala
            20                  25                  30


<210> 770
<211> 32
<212> PRT
<213> Artificial Sequence

<220>
<223> Description of Artificial Sequence: Synthetic peptide


<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 770
Ser Leu Ala Ala Asp Thr Pro Thr Xaa Xaa Phe Ser Xaa Thr Xaa Xaa
1               5                   10                  15

Xaa Xaa Glu Glu Trp Val Gln Tyr Val Asp Asp Leu Glu Leu Ser Ala
            20                  25                  30

<210> 771
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 771
Xaa Leu Ala Ala Asp Thr Pro Thr Xaa Xaa Phe Ser Xaa Thr Xaa Xaa
1               5                   10                  15

Xaa Xaa Glu Glu Trp Val Gln Tyr Val Asp Asp Leu Glu Leu Ser Ala
            20                  25                  30

<210> 772
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 772
Ser Xaa Ala Ala Asp Thr Pro Thr Xaa Xaa Phe Ser Xaa Thr Xaa Xaa
1               5                   10                  15

Xaa Xaa Glu Glu Trp Val Gln Tyr Val Asp Asp Leu Glu Leu Ser Ala
            20                  25                  30

<210> 773
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>

<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 773
Ser Leu Xaa Ala Asp Thr Pro Thr Xaa Xaa Phe Ser Xaa Thr Xaa Xaa
1               5                   10                  15

Xaa Xaa Glu Glu Trp Val Gln Tyr Val Asp Asp Leu Glu Leu Ser Ala
            20                  25                  30

<210> 774
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 774
Ser Leu Ala Xaa Asp Thr Pro Thr Xaa Xaa Phe Ser Xaa Thr Xaa Xaa
1               5                   10                  15

Xaa Xaa Glu Glu Trp Val Gln Tyr Val Asp Asp Leu Glu Leu Ser Ala
            20                  25                  30

<210> 775
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 775
Ser Leu Ala Ala Xaa Thr Pro Thr Xaa Xaa Phe Ser Xaa Thr Xaa Xaa
1               5                   10                  15

Xaa Xaa Glu Glu Trp Val Gln Tyr Val Asp Asp Leu Glu Leu Ser Ala
            20                  25                  30

<210> 776
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see

specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 776
Ser Leu Ala Ala Asp Xaa Pro Thr Xaa Xaa Phe Ser Xaa Thr Xaa Xaa
1               5                   10                  15

Xaa Xaa Glu Glu Trp Val Gln Tyr Val Asp Asp Leu Glu Leu Ser Ala
            20                  25                  30

<210> 777
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES

<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 777
Ser Leu Ala Ala Asp Thr Xaa Thr Xaa Xaa Phe Ser Xaa Thr Xaa Xaa
1               5                   10                  15

Xaa Xaa Glu Glu Trp Val Gln Tyr Val Asp Asp Leu Glu Leu Ser Ala
            20                  25                  30

<210> 778
<211> 32
<212> PRT
<213> Artificial Sequence

<220>
<223> Description of Artificial Sequence: Synthetic peptide

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 778
Ser Leu Ala Ala Asp Thr Pro Xaa Xaa Xaa Phe Ser Xaa Thr Xaa Xaa
1               5                   10                  15

Xaa Xaa Glu Glu Trp Val Gln Tyr Val Asp Asp Leu Glu Leu Ser Ala
            20                  25                  30

<210> 779
<211> 32
<212> PRT
<213> Artificial Sequence

<220>
<223> Description of Artificial Sequence: Synthetic peptide

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 779
Ser Leu Ala Ala Asp Thr Pro Thr Xaa Xaa Phe Ser Tyr Xaa Xaa Xaa
1               5                   10                  15

Xaa Xaa Glu Glu Trp Val Gln Tyr Val Asp Asp Leu Glu Leu Ser Ala
            20                  25                  30

<210> 780
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 780
Xaa Leu Ala Ala Asp Thr Pro Thr Xaa Xaa Phe Ser Tyr Xaa Xaa Xaa
1               5                   10                  15

Xaa Xaa Glu Glu Trp Val Gln Tyr Val Asp Asp Leu Glu Leu Ser Ala
            20                  25                  30

<210> 781
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 781
Ser Xaa Ala Ala Asp Thr Pro Thr Xaa Xaa Phe Ser Tyr Xaa Xaa Xaa
1               5                   10                  15

Xaa Xaa Glu Glu Trp Val Gln Tyr Val Asp Asp Leu Glu Leu Ser Ala
            20                  25                  30

<210> 782
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 782
Ser Leu Xaa Ala Asp Thr Pro Thr Xaa Xaa Phe Ser Tyr Xaa Xaa Xaa
1               5                   10                  15

Xaa Xaa Glu Glu Trp Val Gln Tyr Val Asp Asp Leu Glu Leu Ser Ala
            20                  25                  30

<210> 783
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>

<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 783
Ser Leu Ala Xaa Asp Thr Pro Thr Xaa Xaa Phe Ser Tyr Xaa Xaa Xaa
1               5                   10                  15

Xaa Xaa Glu Glu Trp Val Gln Tyr Val Asp Asp Leu Glu Leu Ser Ala
            20                  25                  30

<210> 784
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 784
Ser Leu Ala Ala Xaa Thr Pro Thr Xaa Xaa Phe Ser Tyr Xaa Xaa Xaa
1               5                   10                  15

Xaa Xaa Glu Glu Trp Val Gln Tyr Val Asp Asp Leu Glu Leu Ser Ala
            20                  25                  30

<210> 785
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 785
Ser Leu Ala Ala Asp Xaa Pro Thr Xaa Xaa Phe Ser Tyr Xaa Xaa Xaa
1               5                   10                  15

Xaa Xaa Glu Glu Trp Val Gln Tyr Val Asp Asp Leu Glu Leu Ser Ala
            20                  25                  30

<210> 786
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see

specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 786
Ser Leu Ala Ala Asp Thr Xaa Thr Xaa Xaa Phe Ser Tyr Xaa Xaa Xaa
1               5                   10                  15

Xaa Xaa Glu Glu Trp Val Gln Tyr Val Asp Asp Leu Glu Leu Ser Ala
            20                  25                  30

<210> 787
<211> 32
<212> PRT
<213> Artificial Sequence

<220>
<223> Description of Artificial Sequence: Synthetic peptide

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 787
Ser Leu Ala Ala Asp Thr Pro Xaa Xaa Xaa Phe Ser Tyr Xaa Xaa Xaa
1               5                   10                  15

Xaa Xaa Glu Glu Trp Val Gln Tyr Val Asp Asp Leu Glu Leu Ser Ala
            20                  25                  30

<210> 788
<211> 32
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 788
Ser Leu Ala Ala Asp Thr Pro Thr Cys Cys Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 789
<211> 32
<212> PRT
<213> Artificial Sequence

<220>
<223> Description of Artificial Sequence: Synthetic peptide

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 789
Ser Leu Ala Ala Asp Thr Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 790
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 790
Xaa Leu Ala Ala Asp Thr Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 791
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups

and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 791
Ser Xaa Ala Ala Asp Thr Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 792
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 792
Ser Leu Xaa Ala Asp Thr Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 793
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 793
Ser Leu Ala Xaa Asp Thr Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 794
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> C-term may be amidated

<400> 794
Ser Leu Ala Ala Xaa Thr Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 795
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 795
Ser Leu Ala Ala Asp Xaa Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 796
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 796
Ser Leu Ala Ala Asp Thr Xaa Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 797
<211> 32
<212> PRT
<213> Artificial Sequence

<220>
<223> Description of Artificial Sequence: Synthetic peptide

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 797
Ser Leu Ala Ala Asp Thr Pro Xaa Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 798
<211> 32
<212> PRT
<213> Artificial Sequence

<220>

<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 798
Ser Leu Ala Ala Asp Thr Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 799
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 799
Xaa Leu Ala Ala Asp Thr Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala

20                  25                  30

<210> 800
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 800
Ser Xaa Ala Ala Asp Thr Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 801
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 801
Ser Leu Xaa Ala Asp Thr Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 802
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 802
Ser Leu Ala Xaa Asp Thr Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 803
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 803
Ser Leu Ala Ala Xaa Thr Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 804
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 804
Ser Leu Ala Ala Asp Xaa Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 805
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 805
Ser Leu Ala Ala Asp Thr Xaa Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 806
<211> 32
<212> PRT
<213> Artificial Sequence

<220>
<223> Description of Artificial Sequence: Synthetic peptide

<220>
<221> MOD_RES
<222> (8)..(11)
<223> Any L- or D-natural amino acid and any non-natural amino acid; see
specification as filed for detailed description of substitutions, R-groups
and preferred embodiments

<220>

<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 806
Ser Leu Ala Ala Asp Thr Pro Xaa Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 807
<211> 32
<212> PRT
<213> Artificial Sequence

<220>
<223> Description of Artificial Sequence: Synthetic peptide

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 807
Ser Leu Ala Ala Asp Thr Pro Thr Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala
            20                  25                  30

<210> 808
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 808
Xaa Leu Ala Ala Asp Thr Pro Thr Xaa Xaa Phe Xaa Tyr Thr Xaa Xaa
1               5                   10                  15


Xaa Xaa Glu Glu Trp Val Lys Tyr Val Asp Asp Leu Glu Leu Ser Ala
            20                  25                  30


<210> 809
<211> 32
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 809
Ser Xaa Ala Ala Asp Thr Pro Thr Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala
            20                  25                  30

<210> 810
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 810
Ser Leu Xaa Ala Asp Thr Pro Thr Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala

20                  25                  30

<210> 811
<211> 32
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 811
Ser Leu Ala Xaa Asp Thr Pro Thr Xaa Xaa Phe Xaa Tyr Thr Xaa Xaa
1               5                   10                  15


Xaa Xaa Glu Glu Trp Val Lys Tyr Val Asp Asp Leu Glu Leu Ser Ala
            20                  25                  30


<210> 812
<211> 32
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 812
Ser Leu Ala Ala Xaa Thr Pro Thr Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala
            20                  25                  30

<210> 813
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 813
Ser Leu Ala Ala Asp Xaa Pro Thr Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala
            20                  25                  30

<210> 814
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 814
Ser Leu Ala Ala Asp Thr Xaa Thr Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala
            20                  25                  30

<210> 815
<211> 32
<212> PRT
<213> Artificial Sequence

<220>
<223> Description of Artificial Sequence: Synthetic peptide

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 815
Ser Leu Ala Ala Asp Thr Pro Xaa Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala
            20                  25                  30

<210> 816
<211> 32
<212> PRT
<213> Artificial Sequence

<220>
<223> Description of Artificial Sequence: Synthetic peptide

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 816
Ser Leu Ala Ala Asp Thr Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 817
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 817
Xaa Leu Ala Ala Asp Thr Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 818
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 818
Ser Xaa Ala Ala Asp Thr Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 819
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 819
Ser Leu Xaa Ala Asp Thr Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 820
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 820
Ser Leu Ala Xaa Asp Thr Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala

            20                  25                  30

<210> 821
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 821
Ser Leu Ala Ala Xaa Thr Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 822
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 822
Ser Leu Ala Ala Asp Xaa Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 823
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> C-term may be amidated

<400> 823
Ser Leu Ala Ala Asp Thr Xaa Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 824
<211> 32
<212> PRT
<213> Artificial Sequence

<220>
<223> Description of Artificial Sequence: Synthetic peptide

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 824
Ser Leu Ala Ala Asp Thr Pro Xaa Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 825
<211> 32
<212> PRT
<213> Artificial Sequence

<220>
<223> Description of Artificial Sequence: Synthetic peptide

<220>
<221> MOD_RES
<222> (9)..(10)

<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 825
Ser Leu Ala Ala Asp Thr Pro Thr Xaa Xaa Phe Ser Tyr Xaa Xaa Xaa
1               5                   10                  15


Xaa Xaa Glu Glu Trp Val Lys Tyr Val Asp Asp Leu Glu Leu Ser Ala
            20                  25                  30


<210> 826
<211> 32
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 826
Xaa Leu Ala Ala Asp Thr Pro Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 827
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 827
Ser Xaa Ala Ala Asp Thr Pro Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 828
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 828
Ser Leu Xaa Ala Asp Thr Pro Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 829
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 829
Ser Leu Ala Xaa Asp Thr Pro Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30


<210> 830
<211> 32
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 830
Ser Leu Ala Ala Xaa Thr Pro Thr Xaa Xaa Phe Ser Tyr Xaa Xaa Xaa
1               5                   10                  15


Xaa Xaa Glu Glu Trp Val Lys Tyr Val Asp Asp Leu Glu Leu Ser Ala

20                  25                  30

<210> 831
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 831
Ser Leu Ala Ala Asp Xaa Pro Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 832
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 832
Ser Leu Ala Ala Asp Thr Xaa Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 833
<211> 32
<212> PRT
<213> Artificial Sequence

<220>
<223> Description of Artificial Sequence: Synthetic peptide

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 833
Ser Leu Ala Ala Asp Thr Pro Xaa Xaa Xaa Phe Ser Tyr Xaa Xaa Xaa

1                   5                   10                  15

Xaa Xaa Glu Glu Trp Val Lys Tyr Val Asp Asp Leu Glu Leu Ser Ala
            20                  25                  30

<210> 834
<211> 23
<212> PRT
<213> Artificial Sequence

<220>
<223> Description of Artificial Sequence: Synthetic peptide

<220>
<223> C-term may be amidated

<400> 834
Ala Cys Phe Ser Tyr Thr Ser Arg Gln Ile Pro Gln Asn Ala Asp Tyr
1               5                   10                  15

Phe Glu Thr Ser Ser Gln Cys
            20

<210> 835
<211> 23
<212> PRT
<213> Artificial Sequence

<220>
<223> Description of Artificial Sequence: Synthetic peptide

<220>
<223> C-term may be amidated

<400> 835
Ala Lys Phe Ser Tyr Thr Ser Arg Gln Ile Pro Gln Asn Ala Asp Tyr
1               5                   10                  15

Phe Glu Thr Ser Ser Gln Glu
            20

<210> 836
<211> 32
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 836
Ser Leu Ala Ala Asp Thr Pro Thr Cys Cys Phe Ser Tyr Thr Xaa Xaa

1                   5                   10                  15

Xaa Xaa Glu Glu Trp Val Lys Tyr Val Asp Asp Leu Glu Leu Ser Ala
            20                  25                  30

<210> 837
<211> 32
<212> PRT
<213> Artificial Sequence

<220>
<223> Description of Artificial Sequence: Synthetic peptide

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 837
Ser Leu Ala Ala Asp Thr Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 838
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 838
Xaa Leu Ala Ala Asp Thr Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 839
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 839
Ser Xaa Ala Ala Asp Thr Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 840
<211> 32
<212> PRT
<213> Artificial Sequence

<220>
<223> Description of Artificial Sequence: Synthetic peptide

<220>

<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 840
Ser Leu Xaa Ala Asp Thr Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 841
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 841
Ser Leu Ala Xaa Asp Thr Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 842
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 842
Ser Leu Ala Ala Xaa Thr Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 843
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 843
Ser Leu Ala Ala Asp Xaa Pro Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 844
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 844
Ser Leu Ala Ala Asp Thr Xaa Thr Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 845
<211> 32
<212> PRT
<213> Artificial Sequence

<220>
<223> Description of Artificial Sequence: Synthetic peptide

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 845
Ser Leu Ala Ala Asp Thr Pro Xaa Xaa Xaa Phe Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 846
<211> 32
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 846
Ser Leu Ala Ala Asp Thr Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 847
<211> 32

<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 847
Xaa Leu Ala Ala Asp Thr Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 848
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 848
Ser Xaa Ala Ala Asp Thr Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 849
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 849
Ser Leu Xaa Ala Asp Thr Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 850
<211> 32
<212> PRT
<213> Artificial Sequence

<220>
<223> Description of Artificial Sequence: Synthetic peptide

<220>
<221> MOD_RES
<222> (4)..(4)

<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 850
Ser Leu Ala Xaa Asp Thr Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 851
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 851
Ser Leu Ala Ala Xaa Thr Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 852
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 852
Ser Leu Ala Ala Asp Xaa Pro Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 853
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 853
Ser Leu Ala Ala Asp Thr Xaa Thr Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 854
<211> 32
<212> PRT
<213> Artificial Sequence

<220>
<223> Description of Artificial Sequence: Synthetic peptide

<220>
<221> MOD_RES
<222> (8)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 854
Ser Leu Ala Ala Asp Thr Pro Xaa Xaa Xaa Xaa Ser Tyr Thr Xaa Xaa
1               5                   10                  15

Xaa Xaa Glu Glu Trp Val Lys Tyr Val Asp Asp Leu Glu Leu Ser Ala
            20                  25                  30

<210> 855
<211> 32
<212> PRT
<213> Artificial Sequence

<220>
<223> Description of Artificial Sequence: Synthetic peptide

<220>
<221> MOD_RES
<222> (9)..(10)
<223> Any L- or D-natural amino acid and any non-natural amino acid; see

specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 855
Ser Leu Ala Ala Asp Thr Pro Thr Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala
            20                  25                  30

<210> 856
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 856
Xaa Leu Ala Ala Asp Thr Pro Thr Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala
            20                  25                  30

<210> 857
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 857
Ser Xaa Ala Ala Asp Thr Pro Thr Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala
            20                  25                  30

<210> 858
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 858
Ser Leu Xaa Ala Asp Thr Pro Thr Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala
            20                  25                  30

<210> 859
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups

and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 859
Ser Leu Ala Xaa Asp Thr Pro Thr Xaa Xaa Phe Xaa Tyr Thr Xaa Xaa
1               5                   10                  15


Xaa Xaa Glu Glu Trp Val Lys Tyr Val Asp Asp Leu Glu Leu Ser Ala
            20                  25                  30


<210> 860
<211> 32
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 860
Ser Leu Ala Ala Xaa Thr Pro Thr Xaa Xaa Phe Xaa Tyr Thr Xaa Xaa
1               5                   10                  15


Xaa Xaa Glu Glu Trp Val Lys Tyr Val Asp Asp Leu Glu Leu Ser Ala
            20                  25                  30

<210> 861
<211> 32
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 861
Ser Leu Ala Ala Asp Xaa Pro Thr Xaa Xaa Phe Xaa Tyr Thr Xaa Xaa
1               5                   10                  15


Xaa Xaa Glu Glu Trp Val Lys Tyr Val Asp Asp Leu Glu Leu Ser Ala
            20                  25                  30


<210> 862
<211> 32
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>

<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 862
Ser Leu Ala Ala Asp Thr Xaa Thr Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala
            20                  25                  30

<210> 863
<211> 32
<212> PRT
<213> Artificial Sequence

<220>
<223> Description of Artificial Sequence: Synthetic peptide

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 863
Ser Leu Ala Ala Asp Thr Pro Xaa Xaa Xaa Phe Xaa Tyr Thr Xaa Xaa
1               5                   10                  15

Xaa Xaa Glu Glu Trp Val Lys Tyr Val Asp Asp Leu Glu Leu Ser Ala
            20                  25                  30

<210> 864
<211> 32
<212> PRT
<213> Artificial Sequence

<220>
<223> Description of Artificial Sequence: Synthetic peptide

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 864
Ser Leu Ala Ala Asp Thr Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 865
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see

specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 865
Xaa Leu Ala Ala Asp Thr Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 866
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 866
Ser Xaa Ala Ala Asp Thr Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 867
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 867
Ser Leu Xaa Ala Asp Thr Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 868
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 868
Ser Leu Ala Xaa Asp Thr Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 869
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups

and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 869
Ser Leu Ala Ala Xaa Thr Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 870
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 870
Ser Leu Ala Ala Asp Xaa Pro Thr Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 871
<211> 32
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 871
Ser Leu Ala Ala Asp Thr Xaa Thr Xaa Xaa Phe Ser Xaa Thr Xaa Xaa
1               5                   10                  15


Xaa Xaa Glu Glu Trp Val Lys Tyr Val Asp Asp Leu Glu Leu Ser Ala
            20                  25                  30


<210> 872
<211> 32
<212> PRT
<213> Artificial Sequence

<220>
<223> Description of Artificial Sequence: Synthetic peptide


<220>
<221> MOD_RES
<222> (8)..(10)
<223> Any L- or D-natural amino acid and any non-natural amino acid; see
specification as filed for detailed description of substitutions, R-groups
and preferred embodiments

<220>

<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 872
Ser Leu Ala Ala Asp Thr Pro Xaa Xaa Xaa Phe Ser Xaa Thr Xaa Xaa
1               5                   10                  15

Xaa Xaa Glu Glu Trp Val Lys Tyr Val Asp Asp Leu Glu Leu Ser Ala
            20                  25                  30

<210> 873
<211> 32
<212> PRT
<213> Artificial Sequence

<220>
<223> Description of Artificial Sequence: Synthetic peptide

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 873
Ser Leu Ala Ala Asp Thr Pro Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 874

<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 874
Xaa Leu Ala Ala Asp Thr Pro Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 875
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see

specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 875
Ser Xaa Ala Ala Asp Thr Pro Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 876
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 876
Ser Leu Xaa Ala Asp Thr Pro Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 877
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 877
Ser Leu Ala Xaa Asp Thr Pro Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 878
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 878
Ser Leu Ala Ala Xaa Thr Pro Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 879
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups

and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 879
Ser Leu Ala Ala Asp Xaa Pro Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 880
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (9)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 880
Ser Leu Ala Ala Asp Thr Xaa Thr Xaa Xaa Phe Ser Tyr Xaa Xaa Xaa
1               5                   10                  15

Xaa Xaa Glu Glu Trp Val Lys Tyr Val Asp Asp Leu Glu Leu Ser Ala
            20                  25                  30

<210> 881
<211> 32
<212> PRT
<213> Artificial Sequence

<220>
<223> Description of Artificial Sequence: Synthetic peptide


<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 881
Ser Leu Ala Ala Asp Thr Pro Xaa Xaa Xaa Phe Ser Tyr Xaa Xaa Xaa
1               5                   10                  15


Xaa Xaa Glu Glu Trp Val Lys Tyr Val Asp Asp Leu Glu Leu Ser Ala
            20                  25                  30


<210> 882
<211> 9
<212> PRT
<213> Artificial Sequence

<220>
<223> Description of Artificial Sequence: Synthetic peptide

<220>
<223> c-term may be amidated

<400> 882
Ser Pro Tyr Ser Ser Asp Thr Thr Pro
1               5


<210> 883
<211> 23
<212> PRT
<213> Artificial Sequence

<220>
<223> Description of Artificial Sequence: Synthetic peptide

<220>
<223> c-term may be amidated

<400> 883
Ala Phe Ala Tyr Ile Ala Arg Pro Leu Pro Arg Ala His Ile Lys Glu
1               5                   10                  15

Tyr Phe Tyr Thr Ser Gly Lys
            20

<210> 884
<211> 15
<212> PRT
<213> Artificial Sequence

<220>
<223> Description of Artificial Sequence: Synthetic peptide

<220>
<223> c-term may be amidated

<400> 884
Ser Asn Pro Ala Val Val Phe Val Thr Arg Lys Asn Arg Gln Val
1               5                   10                  15

<210> 885
<211> 33
<212> PRT
<213> Artificial Sequence

<220>
<223> Description of Artificial Sequence: Synthetic peptide

<220>
<223> c-term may be amidated

<400> 885
Ser Pro Tyr Ser Ser Asp Thr Thr Pro Cys Cys Phe Ala Tyr Ile Ala
1               5                   10                  15

Arg Pro Leu Pro Arg Ala His Ile Lys Glu Tyr Phe Tyr Thr Ser Gly
            20                  25                  30

Lys

<210> 886
<211> 18
<212> PRT
<213> Artificial Sequence

<220>
<223> Description of Artificial Sequence: Synthetic peptide

<220>
<223> c-term may be amidated

<400> 886
Ala Asn Pro Glu Lys Lys Trp Val Arg Glu Tyr Ile Asn Ser Leu Glu
1               5                   10                  15

Met Ser

<210> 887
<211> 33
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 887
Ser Pro Tyr Ser Ser Asp Thr Thr Pro Cys Cys Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 888
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 888
Xaa Pro Tyr Ser Ser Asp Thr Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 889
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 889
Ser Xaa Tyr Ser Ser Asp Thr Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 890
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 890
Ser Pro Xaa Ser Ser Asp Thr Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 891
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 891
Ser Pro Tyr Xaa Ser Asp Thr Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 892
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 892
Ser Pro Tyr Ser Xaa Asp Thr Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 893
<211> 33
<212> PRT
<213> Artificial Sequence

<220>

<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 893
Ser Pro Tyr Ser Ser Xaa Thr Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 894
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> C-term may be amidated

<400> 894
Ser Pro Tyr Ser Ser Asp Xaa Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 895
<211> 33
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 895
Ser Pro Tyr Ser Ser Asp Thr Xaa Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 896
<211> 33
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 896
Ser Pro Tyr Ser Ser Asp Thr Thr Xaa Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 897
<211> 33
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 897
Ser Pro Tyr Ser Ser Asp Thr Thr Pro Cys Cys Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 898
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 898
Xaa Pro Tyr Ser Ser Asp Thr Thr Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 899
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 899
Ser Xaa Tyr Ser Ser Asp Thr Thr Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 900
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 900
Ser Pro Xaa Ser Ser Asp Thr Thr Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 901
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 901
Ser Pro Tyr Xaa Ser Asp Thr Thr Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 902
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups

and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 902
Ser Pro Tyr Ser Xaa Asp Thr Thr Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 903
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 903
Ser Pro Tyr Ser Ser Xaa Thr Thr Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 904
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 904
Ser Pro Tyr Ser Ser Asp Xaa Thr Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 905
<211> 33
<212> PRT
<213> Artificial Sequence

<220>
<223> Description of Artificial Sequence: Synthetic peptide

<220>
<221> MOD_RES
<222> (8)..(8)

<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 905
Ser Pro Tyr Ser Ser Asp Thr Xaa Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 906
<211> 33
<212> PRT
<213> Artificial Sequence

<220>
<223> Description of Artificial Sequence: Synthetic peptide

<220>
<221> MOD_RES
<222> (9)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 906
Ser Pro Tyr Ser Ser Asp Thr Thr Xaa Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 907
<211> 33
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 907
Ser Pro Tyr Ser Ser Asp Thr Thr Pro Cys Cys Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 908
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>

<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 908
Xaa Pro Tyr Ser Ser Asp Thr Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 909
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 909
Ser Xaa Tyr Ser Ser Asp Thr Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 910
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 910
Ser Pro Xaa Ser Ser Asp Thr Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 911

<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 911
Ser Pro Tyr Xaa Ser Asp Thr Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 912
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 912
Ser Pro Tyr Ser Xaa Asp Thr Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 913
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>

<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 913
Ser Pro Tyr Ser Ser Xaa Thr Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 914
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 914
Ser Pro Tyr Ser Ser Asp Xaa Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met

20                       25                       30

Ser

<210> 915
<211> 33
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 915
Ser Pro Tyr Ser Ser Asp Thr Xaa Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 916
<211> 33
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 916
Ser Pro Tyr Ser Ser Asp Thr Thr Xaa Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 917
<211> 33
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 917
Ser Pro Tyr Ser Ser Asp Thr Thr Pro Cys Cys Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 918
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 918
Xaa Pro Tyr Ser Ser Asp Thr Thr Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 919
<211> 33
<212> PRT
<213> Artificial Sequence

<220>

<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 919
Ser Xaa Tyr Ser Ser Asp Thr Thr Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 920
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups

and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 920
Ser Pro Xaa Ser Ser Asp Thr Thr Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 921
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 921
Ser Pro Tyr Xaa Ser Asp Thr Thr Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 922
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 922
Ser Pro Tyr Ser Xaa Asp Thr Thr Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 923
<211> 33
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 923
Ser Pro Tyr Ser Ser Xaa Thr Thr Pro Xaa Xaa Phe Ala Xaa Xaa Xaa
1               5                   10                  15


Xaa Xaa Glu Lys Lys Trp Val Arg Glu Tyr Ile Asn Ser Leu Glu Met
            20                  25                  30


Ser




<210> 924
<211> 33
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see

specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 924
Ser Pro Tyr Ser Ser Asp Xaa Thr Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 925
<211> 33
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups

and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 925
Ser Pro Tyr Ser Ser Asp Thr Xaa Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 926
<211> 33
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 926
Ser Pro Tyr Ser Ser Asp Thr Thr Xaa Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 927
<211> 25
<212> PRT
<213> Artificial Sequence

<220>
<223> Description of Artificial Sequence: Synthetic peptide

<220>
<223> C-term may be amidated

<400> 927
Ala Cys Phe Ala Tyr Ile Ala Arg Pro Leu Pro Arg Ala His Ile Lys
1               5                   10                  15

Glu Tyr Phe Tyr Thr Ser Gly Lys Cys
            20                  25

<210> 928
<211> 24
<212> PRT
<213> Artificial Sequence

<220>
<223> Description of Artificial Sequence: Synthetic peptide

<220>
<223> C-term may be amidated

<400> 928
Glu Cys Phe Ala Tyr Ile Ala Arg Pro Leu Pro Arg Ala His Ile Lys
1               5                   10                  15

Glu Tyr Phe Tyr Thr Ser Gly Lys
            20

<210> 929
<211> 24
<212> PRT
<213> Artificial Sequence

<220>
<223> Description of Artificial Sequence: Synthetic peptide

<220>
<223> C-term may be amidated

<400> 929
Ala Cys Phe Ala Tyr Ile Ala Arg Pro Leu Pro Arg Ala His Ile Lys
1               5                   10                  15

Glu Tyr Phe Tyr Thr Ser Gly Lys
            20

<210> 930
<211> 33
<212> PRT
<213> Artificial Sequence

<220>
<223> Description of Artificial Sequence: Synthetic peptide

<220>
<223> C-term may be amidated

<400> 930
Ser Pro Tyr Ser Ser Asp Thr Thr Pro Cys Cys Phe Ala Tyr Ile Ala
1               5                   10                  15

Arg Pro Leu Pro Arg Ala His Ile Lys Glu Tyr Phe Tyr Thr Ser Gly
            20                  25                  30

Lys

<210> 931
<211> 33
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<223> C-term may be amidated

<400> 931
Ser Pro Tyr Ser Ser Asp Thr Thr Pro Xaa Xaa Phe Ala Tyr Ile Ala
1               5                   10                  15

Arg Pro Leu Pro Arg Ala His Ile Lys Glu Tyr Phe Tyr Thr Ser Gly
            20                  25                  30

Lys

<210> 932
<211> 33
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 932
Ser Pro Tyr Ser Ser Asp Thr Thr Pro Cys Cys Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 933
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 933
Xaa Pro Tyr Ser Ser Asp Thr Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 934
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 934
Ser Xaa Tyr Ser Ser Asp Thr Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 935
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 935
Ser Pro Xaa Ser Ser Asp Thr Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 936
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 936
Ser Pro Tyr Xaa Ser Asp Thr Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 937
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 937
Ser Pro Tyr Ser Xaa Asp Thr Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 938
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 938
Ser Pro Tyr Ser Ser Xaa Thr Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 939
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 939
Ser Pro Tyr Ser Ser Asp Xaa Thr Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 940
<211> 33
<212> PRT

<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 940
Ser Pro Tyr Ser Ser Asp Thr Xaa Pro Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 941
<211> 33
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 941
Ser Pro Tyr Ser Ser Asp Thr Thr Xaa Xaa Xaa Phe Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 942
<211> 33
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 942
Ser Pro Tyr Ser Ser Asp Thr Thr Pro Cys Cys Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 943
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 943
Xaa Pro Tyr Ser Ser Asp Thr Thr Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 944
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 944
Ser Xaa Tyr Ser Ser Asp Thr Thr Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 945
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 945
Ser Pro Xaa Ser Ser Asp Thr Thr Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 946
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see

specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 946
Ser Pro Tyr Xaa Ser Asp Thr Thr Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 947
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 947
Ser Pro Tyr Ser Xaa Asp Thr Thr Pro Xaa Xaa Xaa Ala Tyr Xaa Xaa

1                 5                   10                  15

Xaa Xaa Glu Lys Lys Trp Val Arg Glu Tyr Ile Asn Ser Leu Glu Met
            20                  25                  30

Ser

<210> 948
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 948
Ser Pro Tyr Ser Ser Xaa Thr Thr Pro Xaa Xaa Xaa Ala Tyr Xaa Xaa
1                 5                   10                  15

Xaa Xaa Glu Lys Lys Trp Val Arg Glu Tyr Ile Asn Ser Leu Glu Met
            20                  25                  30

Ser

<210> 949
<211> 33
<212> PRT
<213> Artificial Sequence

<220>
<223> Description of Artificial Sequence: Synthetic peptide

<220>
<221> MOD_RES

<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 949
Ser Pro Tyr Ser Ser Asp Xaa Thr Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 950
<211> 33
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 950
Ser Pro Tyr Ser Ser Asp Thr Xaa Pro Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 951
<211> 33
<212> PRT
<213> Artificial Sequence

<220>
<223> Description of Artificial Sequence: Synthetic peptide

<220>
<221> MOD_RES
<222> (9)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 951
Ser Pro Tyr Ser Ser Asp Thr Thr Xaa Xaa Xaa Xaa Ala Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 952
<211> 33
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 952
Ser Pro Tyr Ser Ser Asp Thr Thr Pro Cys Cys Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 953
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 953
Xaa Pro Tyr Ser Ser Asp Thr Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 954
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 954
Ser Xaa Tyr Ser Ser Asp Thr Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 955
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 955
Ser Pro Xaa Ser Ser Asp Thr Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 956
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 956
Ser Pro Tyr Xaa Ser Asp Thr Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 957
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 957
Ser Pro Tyr Ser Xaa Asp Thr Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 958
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 958
Ser Pro Tyr Ser Ser Xaa Thr Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 959
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 959
Ser Pro Tyr Ser Ser Asp Xaa Thr Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 960
<211> 33
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups

and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 960
Ser Pro Tyr Ser Ser Asp Thr Xaa Pro Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 961
<211> 33
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 961
Ser Pro Tyr Ser Ser Asp Thr Thr Xaa Xaa Xaa Phe Xaa Tyr Xaa Xaa
1               5                   10                  15

Xaa Xaa Glu Lys Lys Trp Val Arg Glu Tyr Ile Asn Ser Leu Glu Met
            20                  25                  30

Ser

<210> 962
<211> 33
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 962
Ser Pro Tyr Ser Ser Asp Thr Thr Pro Cys Cys Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 963
<211> 33
<212> PRT
<213> Artificial Sequence

<220>
<223> Description of Artificial Sequence: Synthetic peptide

<220>
<221> MOD_RES
<222> (1)..(1)

<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 963
Xaa Pro Tyr Ser Ser Asp Thr Thr Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 964
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 964
Ser Xaa Tyr Ser Ser Asp Thr Thr Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 965
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 965
Ser Pro Xaa Ser Ser Asp Thr Thr Pro Xaa Xaa Phe Ala Xaa Xaa Xaa

1                 5                   10                  15

Xaa Xaa Glu Lys Lys Trp Val Arg Glu Tyr Ile Asn Ser Leu Glu Met
            20                  25                  30

Ser

<210> 966
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 966
Ser Pro Tyr Xaa Ser Asp Thr Thr Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 967
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 967
Ser Pro Tyr Ser Xaa Asp Thr Thr Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 968
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)

<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 968
Ser Pro Tyr Ser Ser Xaa Thr Thr Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 969
<211> 33
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 969
Ser Pro Tyr Ser Ser Asp Xaa Thr Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 970
<211> 33
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 970
Ser Pro Tyr Ser Ser Asp Thr Xaa Pro Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 971
<211> 33
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 971
Ser Pro Tyr Ser Ser Asp Thr Thr Xaa Xaa Xaa Phe Ala Xaa Xaa Xaa
1               5                   10                  15

Xaa Xaa Glu Lys Lys Trp Val Arg Glu Tyr Ile Asn Ser Leu Glu Met
            20                  25                  30

Ser

<210> 972
<211> 10
<212> PRT
<213> Artificial Sequence

<220>
<223> Description of Artificial Sequence: Synthetic peptide

<220>
<223> C-term may be amidated

<400> 972
Lys Ser Met Gln Val Pro Phe Ser Arg Ala
1               5                   10

<210> 973
<211> 15

<212> PRT
<213> Artificial Sequence

<220>
<223> Description of Artificial Sequence: Synthetic peptide

<220>
<223> c-term may be amidated

<400> 973
Ala Phe Ser Phe Ala Glu Gln Glu Ile Pro Leu Arg Ala Ile Leu
1               5                   10                  15


<210> 974
<211> 23
<212> PRT
<213> Artificial Sequence

<220>
<223> Description of Artificial Sequence: Synthetic peptide

<220>
<223> c-term may be amidated

<400> 974
Ala Phe Ser Phe Ala Glu Gln Glu Ile Pro Leu Arg Ala Ile Leu Cys
1               5                   10                  15


Tyr Arg Asn Thr Ser Ser Ile
            20


<210> 975
<211> 24
<212> PRT
<213> Artificial Sequence

<220>
<223> Description of Artificial Sequence: Synthetic peptide

<220>
<223> c-term may be amidated

<400> 975
Ala Tyr Arg Asn Thr Ser Ser Ile Cys Ser Asn Glu Gly Leu Ile Phe
1               5                   10                  15


Lys Leu Lys Arg Gly Lys Glu Ala
            20


<210> 976
<211> 25
<212> PRT
<213> Artificial Sequence

<220>
<223> Description of Artificial Sequence: Synthetic peptide

<220>
<223> c-term may be amidated

<400> 976
Lys Ser Met Gln Val Pro Phe Ser Arg Cys Cys Phe Ser Phe Ala Glu
1               5                   10                  15

Gln Glu Ile Pro Leu Arg Ala Ile Leu
            20                  25

<210> 977
<211> 25
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<223> C-term may be amidated

<400> 977
Lys Ser Met Gln Val Pro Phe Ser Arg Xaa Xaa Phe Ser Phe Ala Glu
1               5                   10                  15

Gln Glu Ile Pro Leu Arg Ala Ile Leu
            20                  25

<210> 978
<211> 25
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<223> C-term may be amidated

<400> 978
Xaa Ser Met Gln Val Pro Phe Ser Arg Xaa Xaa Phe Ser Phe Ala Glu
1               5                   10                  15

Gln Glu Ile Pro Leu Arg Ala Ile Leu
            20                  25

<210> 979
<211> 25
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<223> c-term may be amidated

<400> 979
Lys Xaa Met Gln Val Pro Phe Ser Arg Xaa Xaa Phe Ser Phe Ala Glu
1               5                   10                  15

Gln Glu Ile Pro Leu Arg Ala Ile Leu
            20                  25

<210> 980
<211> 25
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<223> c-term may be amidated

<400> 980
Lys Ser Xaa Gln Val Pro Phe Ser Arg Xaa Xaa Phe Ser Phe Ala Glu
1               5                   10                  15

Gln Glu Ile Pro Leu Arg Ala Ile Leu

20                  25

<210> 981
<211> 25
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<223> C-term may be amidated

<400> 981
Lys Ser Met Xaa Val Pro Phe Ser Arg Xaa Xaa Phe Ser Phe Ala Glu
1               5                   10                  15

Gln Glu Ile Pro Leu Arg Ala Ile Leu
            20                  25

<210> 982
<211> 25
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<223> C-term may be amidated

<400> 982
Lys Ser Met Gln Xaa Pro Phe Ser Arg Xaa Xaa Phe Ser Phe Ala Glu
1               5                   10                  15

Gln Glu Ile Pro Leu Arg Ala Ile Leu
            20                  25

<210> 983
<211> 25
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<223> C-term may be amidated

<400> 983
Lys Ser Met Gln Val Xaa Phe Ser Arg Xaa Xaa Phe Ser Phe Ala Glu
1               5                   10                  15

Gln Glu Ile Pro Leu Arg Ala Ile Leu
            20                  25

<210> 984
<211> 25
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<223> C-term may be amidated

<400> 984
Lys Ser Met Gln Val Pro Xaa Ser Arg Xaa Xaa Phe Ser Phe Ala Glu

1                5                   10                  15

Gln Glu Ile Pro Leu Arg Ala Ile Leu
            20                  25

<210> 985
<211> 25
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<223> C-term may be amidated

<400> 985
Lys Ser Met Gln Val Pro Phe Xaa Arg Xaa Xaa Phe Ser Phe Ala Glu
1               5                   10                  15

Gln Glu Ile Pro Leu Arg Ala Ile Leu
            20                  25

<210> 986
<211> 25
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<223> C-term may be amidated

<400> 986
Lys Ser Met Gln Val Pro Phe Ser Xaa Xaa Xaa Phe Ser Phe Ala Glu
1               5                   10                  15

Gln Glu Ile Pro Leu Arg Ala Ile Leu
            20                  25

<210> 987
<211> 34
<212> PRT
<213> Artificial Sequence

<220>
<223> Description of Artificial Sequence: Synthetic peptide

<220>
<223> c-term may be amidated

<400> 987
Ala Ser Asn Glu Gly Leu Ile Phe Lys Leu Lys Arg Gly Lys Glu Ala
1               5                   10                  15

Cys Ala Leu Asp Thr Val Gly Trp Val Gln Arg His Arg Lys Met Leu
            20                  25                  30

Arg His

<210> 988
<211> 24
<212> PRT
<213> Artificial Sequence

<220>
<223> Description of Artificial Sequence: Synthetic peptide

<220>
<223> c-term may be amidated

<400> 988
Ala Ala Leu Asp Thr Val Gly Trp Val Gln Arg His Arg Lys Met Leu
1               5                   10                  15

Arg His Cys Pro Ser Lys Arg Lys
            20

<210> 989
<211> 31
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 989
Lys Ser Met Gln Val Pro Phe Ser Arg Cys Cys Phe Ser Phe Xaa Xaa
1               5                   10                  15

Xaa Xaa Thr Val Gly Trp Val Gln Arg His Arg Lys Met Leu Arg
            20                  25                  30

<210> 990
<211> 31
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 990
Lys Ser Met Gln Val Pro Phe Ser Arg Xaa Xaa Phe Ser Phe Xaa Xaa
1               5                   10                  15

Xaa Xaa Thr Val Gly Trp Val Gln Arg His Arg Lys Met Leu Arg
            20                  25                  30

<210> 991
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 991
Xaa Ser Met Gln Val Pro Phe Ser Arg Xaa Xaa Phe Ser Phe Xaa Xaa
1               5                   10                  15

Xaa Xaa Thr Val Gly Trp Val Gln Arg His Arg Lys Met Leu Arg
            20                  25                  30

<210> 992
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 992
Lys Xaa Met Gln Val Pro Phe Ser Arg Xaa Xaa Phe Ser Phe Xaa Xaa
1               5                   10                  15

Xaa Xaa Thr Val Gly Trp Val Gln Arg His Arg Lys Met Leu Arg
            20                  25                  30

<210> 993
<211> 31
<212> PRT
<213> Artificial Sequence

<220>
<223> Description of Artificial Sequence: Synthetic peptide

<220>
<221> MOD_RES

<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 993
Lys Ser Xaa Gln Val Pro Phe Ser Arg Xaa Xaa Phe Ser Phe Xaa Xaa
1               5                   10                  15

Xaa Xaa Thr Val Gly Trp Val Gln Arg His Arg Lys Met Leu Arg
            20                  25                  30

<210> 994
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 994
Lys Ser Met Xaa Val Pro Phe Ser Arg Xaa Xaa Phe Ser Phe Xaa Xaa
1               5                   10                  15

Xaa Xaa Thr Val Gly Trp Val Gln Arg His Arg Lys Met Leu Arg
            20                  25                  30

<210> 995
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 995
Lys Ser Met Gln Xaa Pro Phe Ser Arg Xaa Xaa Phe Ser Phe Xaa Xaa
1               5                   10                  15

Xaa Xaa Thr Val Gly Trp Val Gln Arg His Arg Lys Met Leu Arg
            20                  25                  30

<210> 996
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups

and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 996
Lys Ser Met Gln Val Xaa Phe Ser Arg Xaa Xaa Phe Ser Phe Xaa Xaa
1               5                   10                  15

Xaa Xaa Thr Val Gly Trp Val Gln Arg His Arg Lys Met Leu Arg
            20                  25                  30

<210> 997
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 997
Lys Ser Met Gln Val Pro Xaa Ser Arg Xaa Xaa Phe Ser Phe Xaa Xaa
1               5                   10                  15

Xaa Xaa Thr Val Gly Trp Val Gln Arg His Arg Lys Met Leu Arg
            20                  25                  30

<210> 998
<211> 31
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 998
Lys Ser Met Gln Val Pro Phe Xaa Arg Xaa Xaa Phe Ser Phe Xaa Xaa
1               5                   10                  15

Xaa Xaa Thr Val Gly Trp Val Gln Arg His Arg Lys Met Leu Arg
            20                  25                  30

<210> 999
<211> 31
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 999
Lys Ser Met Gln Val Pro Phe Ser Xaa Xaa Xaa Phe Ser Phe Xaa Xaa
1               5                   10                  15

Xaa Xaa Thr Val Gly Trp Val Gln Arg His Arg Lys Met Leu Arg
            20                  25                  30

<210> 1000
<211> 31
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1000
Lys Ser Met Gln Val Pro Phe Ser Arg Cys Cys Xaa Ser Phe Xaa Xaa
1               5                   10                  15

Xaa Xaa Thr Val Gly Trp Val Gln Arg His Arg Lys Met Leu Arg
            20                  25                  30

<210> 1001
<211> 31
<212> PRT
<213> Artificial Sequence

<220>
<223> Description of Artificial Sequence: Synthetic peptide

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1001
Lys Ser Met Gln Val Pro Phe Ser Arg Xaa Xaa Xaa Ser Phe Xaa Xaa
1               5                   10                  15

Xaa Xaa Thr Val Gly Trp Val Gln Arg His Arg Lys Met Leu Arg
            20                  25                  30

<210> 1002
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1002
Xaa Ser Met Gln Val Pro Phe Ser Arg Xaa Xaa Xaa Ser Phe Xaa Xaa
1               5                   10                  15

Xaa Xaa Thr Val Gly Trp Val Gln Arg His Arg Lys Met Leu Arg
            20                  25                  30

<210> 1003
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1003
Lys Xaa Met Gln Val Pro Phe Ser Arg Xaa Xaa Xaa Ser Phe Xaa Xaa
1               5                   10                  15

Xaa Xaa Thr Val Gly Trp Val Gln Arg His Arg Lys Met Leu Arg
            20                  25                  30

<210> 1004
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1004
Lys Ser Xaa Gln Val Pro Phe Ser Arg Xaa Xaa Xaa Ser Phe Xaa Xaa
1               5                   10                  15

Xaa Xaa Thr Val Gly Trp Val Gln Arg His Arg Lys Met Leu Arg
            20                  25                  30

<210> 1005
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1005
Lys Ser Met Xaa Val Pro Phe Ser Arg Xaa Xaa Xaa Ser Phe Xaa Xaa
1               5                   10                  15

Xaa Xaa Thr Val Gly Trp Val Gln Arg His Arg Lys Met Leu Arg
            20                  25                  30

<210> 1006
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1006
Lys Ser Met Gln Xaa Pro Phe Ser Arg Xaa Xaa Xaa Ser Phe Xaa Xaa
1               5                   10                  15

Xaa Xaa Thr Val Gly Trp Val Gln Arg His Arg Lys Met Leu Arg
            20                  25                  30

<210> 1007
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1007
Lys Ser Met Gln Val Xaa Phe Ser Arg Xaa Xaa Xaa Ser Phe Xaa Xaa
1               5                   10                  15

Xaa Xaa Thr Val Gly Trp Val Gln Arg His Arg Lys Met Leu Arg
            20                  25                  30

<210> 1008
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups

and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1008
Lys Ser Met Gln Val Pro Xaa Ser Arg Xaa Xaa Xaa Ser Phe Xaa Xaa
1               5                   10                  15

Xaa Xaa Thr Val Gly Trp Val Gln Arg His Arg Lys Met Leu Arg
            20                  25                  30

<210> 1009
<211> 31
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1009
Lys Ser Met Gln Val Pro Phe Xaa Arg Xaa Xaa Xaa Ser Phe Xaa Xaa
1               5                   10                  15

Xaa Xaa Thr Val Gly Trp Val Gln Arg His Arg Lys Met Leu Arg
            20                  25                  30

<210> 1010
<211> 31
<212> PRT
<213> Artificial Sequence

<220>
<223> Description of Artificial Sequence: Synthetic peptide


<220>
<221> MOD_RES
<222> (9)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1010
Lys Ser Met Gln Val Pro Phe Ser Xaa Xaa Xaa Xaa Ser Phe Xaa Xaa
1               5                   10                  15


Xaa Xaa Thr Val Gly Trp Val Gln Arg His Arg Lys Met Leu Arg
            20                  25                  30


<210> 1011
<211> 31
<212> PRT
<213> Artificial Sequence

<220>
<223> Description of Artificial Sequence: Synthetic peptide


<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1011
Lys Ser Met Gln Val Pro Phe Ser Arg Cys Cys Phe Xaa Phe Xaa Xaa
1               5                   10                  15

Xaa Xaa Thr Val Gly Trp Val Gln Arg His Arg Lys Met Leu Arg
            20                  25                  30

<210> 1012
<211> 31
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1012
Lys Ser Met Gln Val Pro Phe Ser Arg Xaa Xaa Phe Xaa Phe Xaa Xaa
1               5                   10                  15

Xaa Xaa Thr Val Gly Trp Val Gln Arg His Arg Lys Met Leu Arg
            20                  25                  30

<210> 1013
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1013
Xaa Ser Met Gln Val Pro Phe Ser Arg Xaa Xaa Phe Xaa Phe Xaa Xaa
1               5                   10                  15


Xaa Xaa Thr Val Gly Trp Val Gln Arg His Arg Lys Met Leu Arg
            20                  25                  30


<210> 1014
<211> 31
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1014

Lys Xaa Met Gln Val Pro Phe Ser Arg Xaa Xaa Phe Xaa Phe Xaa Xaa
1               5                   10                  15

Xaa Xaa Thr Val Gly Trp Val Gln Arg His Arg Lys Met Leu Arg
            20                  25                  30

<210> 1015
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1015
Lys Ser Xaa Gln Val Pro Phe Ser Arg Xaa Xaa Phe Xaa Phe Xaa Xaa
1               5                   10                  15

Xaa Xaa Thr Val Gly Trp Val Gln Arg His Arg Lys Met Leu Arg
            20                  25                  30

<210> 1016
<211> 31
<212> PRT
<213> Artificial Sequence

<220>
<223> Description of Artificial Sequence: Synthetic peptide

<220>
<221> MOD_RES

<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1016
Lys Ser Met Xaa Val Pro Phe Ser Arg Xaa Xaa Phe Xaa Phe Xaa Xaa
1               5                   10                  15

Xaa Xaa Thr Val Gly Trp Val Gln Arg His Arg Lys Met Leu Arg
            20                  25                  30

<210> 1017
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1017
Lys Ser Met Gln Xaa Pro Phe Ser Arg Xaa Xaa Phe Xaa Phe Xaa Xaa
1               5                   10                  15

Xaa Xaa Thr Val Gly Trp Val Gln Arg His Arg Lys Met Leu Arg
            20                  25                  30

<210> 1018
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1018
Lys Ser Met Gln Val Xaa Phe Ser Arg Xaa Xaa Phe Xaa Phe Xaa Xaa
1               5                   10                  15

Xaa Xaa Thr Val Gly Trp Val Gln Arg His Arg Lys Met Leu Arg
            20                  25                  30

<210> 1019
<211> 31
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1019
Lys Ser Met Gln Val Pro Xaa Ser Arg Xaa Xaa Phe Xaa Phe Xaa Xaa
1               5                   10                  15


Xaa Xaa Thr Val Gly Trp Val Gln Arg His Arg Lys Met Leu Arg
            20                  25                  30


<210> 1020
<211> 31
<212> PRT
<213> Artificial Sequence

<220>
<223> Description of Artificial Sequence: Synthetic peptide


<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)

<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1020
Lys Ser Met Gln Val Pro Phe Xaa Arg Xaa Xaa Phe Xaa Phe Xaa Xaa
1               5                   10                  15


Xaa Xaa Thr Val Gly Trp Val Gln Arg His Arg Lys Met Leu Arg
            20                  25                  30


<210> 1021
<211> 31
<212> PRT
<213> Artificial Sequence

<220>
<223> Description of Artificial Sequence: Synthetic peptide


<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1021
Lys Ser Met Gln Val Pro Phe Ser Xaa Xaa Xaa Phe Xaa Phe Xaa Xaa
1               5                   10                  15

Xaa Xaa Thr Val Gly Trp Val Gln Arg His Arg Lys Met Leu Arg
            20                  25                  30

<210> 1022
<211> 31
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1022
Lys Ser Met Gln Val Pro Phe Ser Arg Cys Cys Phe Ser Xaa Xaa Xaa
1               5                   10                  15

Xaa Xaa Thr Val Gly Trp Val Gln Arg His Arg Lys Met Leu Arg
            20                  25                  30

<210> 1023
<211> 31
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1023
Lys Ser Met Gln Val Pro Phe Ser Arg Xaa Xaa Phe Ser Xaa Xaa Xaa
1               5                   10                  15

Xaa Xaa Thr Val Gly Trp Val Gln Arg His Arg Lys Met Leu Arg
            20                  25                  30

<210> 1024
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1024
Xaa Ser Met Gln Val Pro Phe Ser Arg Xaa Xaa Phe Ser Xaa Xaa Xaa
1               5                   10                  15

Xaa Xaa Thr Val Gly Trp Val Gln Arg His Arg Lys Met Leu Arg
            20                  25                  30

<210> 1025
<211> 31
<212> PRT
<213> Artificial Sequence

<220>

<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1025
Lys Xaa Met Gln Val Pro Phe Ser Arg Xaa Xaa Phe Ser Xaa Xaa Xaa
1               5                   10                  15

Xaa Xaa Thr Val Gly Trp Val Gln Arg His Arg Lys Met Leu Arg
            20                  25                  30

<210> 1026
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES

<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1026
Lys Ser Xaa Gln Val Pro Phe Ser Arg Xaa Xaa Phe Ser Xaa Xaa Xaa
1               5                   10                  15

Xaa Xaa Thr Val Gly Trp Val Gln Arg His Arg Lys Met Leu Arg
            20                  25                  30

<210> 1027
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1027
Lys Ser Met Xaa Val Pro Phe Ser Arg Xaa Xaa Phe Ser Xaa Xaa Xaa
1               5                   10                  15

Xaa Xaa Thr Val Gly Trp Val Gln Arg His Arg Lys Met Leu Arg
            20                  25                  30

<210> 1028
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1028
Lys Ser Met Gln Xaa Pro Phe Ser Arg Xaa Xaa Phe Ser Xaa Xaa Xaa
1               5                   10                  15

Xaa Xaa Thr Val Gly Trp Val Gln Arg His Arg Lys Met Leu Arg
            20                  25                  30

<210> 1029
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups

and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1029
Lys Ser Met Gln Val Xaa Phe Ser Arg Xaa Xaa Phe Ser Xaa Xaa Xaa
1               5                   10                  15


Xaa Xaa Thr Val Gly Trp Val Gln Arg His Arg Lys Met Leu Arg
            20                  25                  30


<210> 1030
<211> 31
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1030
Lys Ser Met Gln Val Pro Xaa Ser Arg Xaa Xaa Phe Ser Xaa Xaa Xaa
1               5                   10                  15

Xaa Xaa Thr Val Gly Trp Val Gln Arg His Arg Lys Met Leu Arg
            20                  25                  30

<210> 1031
<211> 31
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1031
Lys Ser Met Gln Val Pro Phe Xaa Arg Xaa Xaa Phe Ser Xaa Xaa Xaa
1               5                   10                  15

Xaa Xaa Thr Val Gly Trp Val Gln Arg His Arg Lys Met Leu Arg
            20                  25                  30

<210> 1032
<211> 31
<212> PRT

<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1032
Lys Ser Met Gln Val Pro Phe Ser Xaa Xaa Xaa Phe Ser Xaa Xaa Xaa
1               5                   10                  15

Xaa Xaa Thr Val Gly Trp Val Gln Arg His Arg Lys Met Leu Arg
            20                  25                  30

<210> 1033
<211> 34
<212> PRT
<213> Artificial Sequence

<220>
<223> Description of Artificial Sequence: Synthetic peptide

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1033
Lys Ser Met Gln Val Pro Phe Ser Arg Cys Cys Phe Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1034
<211> 34
<212> PRT
<213> Artificial Sequence

<220>
<223> Description of Artificial Sequence: Synthetic peptide


<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1034
Lys Ser Met Gln Val Pro Phe Ser Arg Xaa Xaa Phe Ser Phe Ala Glu
1               5                   10                  15


Gln Xaa Xaa Xaa Xaa Thr Val Gly Trp Val Gln Arg His Arg Lys Met
            20                  25                  30


Leu Arg



<210> 1035
<211> 34
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1035
Xaa Ser Met Gln Val Pro Phe Ser Arg Xaa Xaa Phe Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1036
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1036
Lys Xaa Met Gln Val Pro Phe Ser Arg Xaa Xaa Phe Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1037
<211> 34

<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1037
Lys Ser Xaa Gln Val Pro Phe Ser Arg Xaa Xaa Phe Ser Phe Ala Glu
1               5                   10                  15


Gln Xaa Xaa Xaa Xaa Thr Val Gly Trp Val Gln Arg His Arg Lys Met
            20                  25                  30


Leu Arg



<210> 1038
<211> 34
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>

<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1038
Lys Ser Met Xaa Val Pro Phe Ser Arg Xaa Xaa Phe Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1039
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1039
Lys Ser Met Gln Xaa Pro Phe Ser Arg Xaa Xaa Phe Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1040
<211> 34
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1040
Lys Ser Met Gln Val Xaa Phe Ser Arg Xaa Xaa Phe Ser Phe Ala Glu
1               5                   10                  15


Gln Xaa Xaa Xaa Xaa Thr Val Gly Trp Val Gln Arg His Arg Lys Met
            20                  25                  30


Leu Arg




<210> 1041
<211> 34
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1041
Lys Ser Met Gln Val Pro Xaa Ser Arg Xaa Xaa Phe Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1042
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1042
Lys Ser Met Gln Val Pro Phe Xaa Arg Xaa Xaa Phe Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1043
<211> 34
<212> PRT
<213> Artificial Sequence

<220>
<223> Description of Artificial Sequence: Synthetic peptide


<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1043
Lys Ser Met Gln Val Pro Phe Ser Xaa Xaa Xaa Phe Ser Phe Ala Glu
1               5                   10                  15


Gln Xaa Xaa Xaa Xaa Thr Val Gly Trp Val Gln Arg His Arg Lys Met
            20                  25                  30


Leu Arg



<210> 1044
<211> 34
<212> PRT
<213> Artificial Sequence

<220>
<223> Description of Artificial Sequence: Synthetic peptide


<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1044

Lys Ser Met Gln Val Pro Phe Ser Arg Cys Cys Xaa Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1045
<211> 34
<212> PRT
<213> Artificial Sequence

<220>
<223> Description of Artificial Sequence: Synthetic peptide

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1045
Lys Ser Met Gln Val Pro Phe Ser Arg Xaa Xaa Xaa Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1046
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>

<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1046
Xaa Ser Met Gln Val Pro Phe Ser Arg Xaa Xaa Xaa Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1047
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1047
Lys Xaa Met Gln Val Pro Phe Ser Arg Xaa Xaa Xaa Ser Phe Ala Glu
1               5                   10                  15

Gln Xaa Xaa Xaa Xaa Thr Val Gly Trp Val Gln Arg His Arg Lys Met

20                  25                  30

Leu Arg

<210> 1048
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1048
Lys Ser Xaa Gln Val Pro Phe Ser Arg Xaa Xaa Xaa Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1049
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1049
Lys Ser Met Xaa Val Pro Phe Ser Arg Xaa Xaa Xaa Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1050
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1050
Lys Ser Met Gln Xaa Pro Phe Ser Arg Xaa Xaa Xaa Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1051
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1051
Lys Ser Met Gln Val Xaa Phe Ser Arg Xaa Xaa Xaa Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1052
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see

specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1052
Lys Ser Met Gln Val Pro Xaa Ser Arg Xaa Xaa Xaa Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1053
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1053
Lys Ser Met Gln Val Pro Phe Xaa Arg Xaa Xaa Xaa Ser Phe Ala Glu

1                   5                   10                  15

Gln Xaa Xaa Xaa Xaa Thr Val Gly Trp Val Gln Arg His Arg Lys Met
            20                  25                  30

Leu Arg

<210> 1054
<211> 34
<212> PRT
<213> Artificial Sequence

<220>
<223> Description of Artificial Sequence: Synthetic peptide

<220>
<221> MOD_RES
<222> (9)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1054
Lys Ser Met Gln Val Pro Phe Ser Xaa Xaa Xaa Xaa Ser Phe Ala Glu
1               5                   10                  15

Gln Xaa Xaa Xaa Xaa Thr Val Gly Trp Val Gln Arg His Arg Lys Met
            20                  25                  30

Leu Arg

<210> 1055
<211> 34
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES

<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1055
Lys Ser Met Gln Val Pro Phe Ser Arg Cys Cys Phe Xaa Phe Ala Glu
1               5                   10                  15

Gln Xaa Xaa Xaa Xaa Thr Val Gly Trp Val Gln Arg His Arg Lys Met
            20                  25                  30

Leu Arg

<210> 1056
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1056
Lys Ser Met Gln Val Pro Phe Ser Arg Xaa Xaa Phe Xaa Phe Ala Glu
1               5                   10                  15

Gln Xaa Xaa Xaa Xaa Thr Val Gly Trp Val Gln Arg His Arg Lys Met
            20                  25                  30

Leu Arg

<210> 1057

<211> 34
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1057
Xaa Ser Met Gln Val Pro Phe Ser Arg Xaa Xaa Phe Xaa Phe Ala Glu
1               5                   10                  15


Gln Xaa Xaa Xaa Xaa Thr Val Gly Trp Val Gln Arg His Arg Lys Met
            20                  25                  30


Leu Arg




<210> 1058
<211> 34
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1058
Lys Xaa Met Gln Val Pro Phe Ser Arg Xaa Xaa Phe Xaa Phe Ala Glu
1               5                   10                  15

Gln Xaa Xaa Xaa Xaa Thr Val Gly Trp Val Gln Arg His Arg Lys Met
            20                  25                  30

Leu Arg

<210> 1059
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>

<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1059
Lys Ser Xaa Gln Val Pro Phe Ser Arg Xaa Xaa Phe Xaa Phe Ala Glu
1               5                   10                  15

Gln Xaa Xaa Xaa Xaa Thr Val Gly Trp Val Gln Arg His Arg Lys Met
            20                  25                  30

Leu Arg

<210> 1060
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1060
Lys Ser Met Xaa Val Pro Phe Ser Arg Xaa Xaa Phe Xaa Phe Ala Glu
1               5                   10                  15

Gln Xaa Xaa Xaa Xaa Thr Val Gly Trp Val Gln Arg His Arg Lys Met

20                    25                    30

Leu Arg

<210> 1061
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1061
Lys Ser Met Gln Xaa Pro Phe Ser Arg Xaa Xaa Phe Xaa Phe Ala Glu
1               5                   10                  15

Gln Xaa Xaa Xaa Xaa Thr Val Gly Trp Val Gln Arg His Arg Lys Met
            20                  25                  30

Leu Arg

<210> 1062
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1062
Lys Ser Met Gln Val Xaa Phe Ser Arg Xaa Xaa Phe Xaa Phe Ala Glu
1               5                   10                  15

Gln Xaa Xaa Xaa Xaa Thr Val Gly Trp Val Gln Arg His Arg Lys Met
            20                  25                  30

Leu Arg

<210> 1063
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1063
Lys Ser Met Gln Val Pro Xaa Ser Arg Xaa Xaa Phe Xaa Phe Ala Glu
1               5                   10                  15

Gln Xaa Xaa Xaa Xaa Thr Val Gly Trp Val Gln Arg His Arg Lys Met
            20                  25                  30

Leu Arg

<210> 1064
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 1064
Lys Ser Met Gln Val Pro Phe Xaa Arg Xaa Xaa Phe Xaa Phe Ala Glu
1               5                   10                  15

Gln Xaa Xaa Xaa Xaa Thr Val Gly Trp Val Gln Arg His Arg Lys Met
            20                  25                  30

Leu Arg

<210> 1065
<211> 34
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1065
Lys Ser Met Gln Val Pro Phe Ser Xaa Xaa Xaa Phe Xaa Phe Ala Glu
1               5                   10                  15

Gln Xaa Xaa Xaa Xaa Thr Val Gly Trp Val Gln Arg His Arg Lys Met
            20                  25                  30

Leu Arg

<210> 1066
<211> 34
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1066
Lys Ser Met Gln Val Pro Phe Ser Arg Cys Cys Phe Ser Xaa Ala Glu
1               5                   10                  15

Gln Xaa Xaa Xaa Xaa Thr Val Gly Trp Val Gln Arg His Arg Lys Met
            20                  25                  30

Leu Arg

<210> 1067
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1067
Lys Ser Met Gln Val Pro Phe Ser Arg Xaa Xaa Phe Ser Xaa Ala Glu
1               5                   10                  15

Gln Xaa Xaa Xaa Xaa Thr Val Gly Trp Val Gln Arg His Arg Lys Met
            20                  25                  30

Leu Arg

<210> 1068
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1068
Xaa Ser Met Gln Val Pro Phe Ser Arg Xaa Xaa Phe Ser Xaa Ala Glu
1               5                   10                  15

Gln Xaa Xaa Xaa Xaa Thr Val Gly Trp Val Gln Arg His Arg Lys Met
            20                  25                  30

Leu Arg

<210> 1069
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1069
Lys Xaa Met Gln Val Pro Phe Ser Arg Xaa Xaa Phe Ser Xaa Ala Glu
1               5                   10                  15

Gln Xaa Xaa Xaa Xaa Thr Val Gly Trp Val Gln Arg His Arg Lys Met
            20                  25                  30

Leu Arg

<210> 1070
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see

specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1070
Lys Ser Xaa Gln Val Pro Phe Ser Arg Xaa Xaa Phe Ser Xaa Ala Glu
1               5                   10                  15

Gln Xaa Xaa Xaa Xaa Thr Val Gly Trp Val Gln Arg His Arg Lys Met
            20                  25                  30

Leu Arg

<210> 1071
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 1071
Lys Ser Met Xaa Val Pro Phe Ser Arg Xaa Xaa Phe Ser Xaa Ala Glu
1               5                   10                  15

Gln Xaa Xaa Xaa Xaa Thr Val Gly Trp Val Gln Arg His Arg Lys Met
            20                  25                  30

Leu Arg

<210> 1072
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1072
Lys Ser Met Gln Xaa Pro Phe Ser Arg Xaa Xaa Phe Ser Xaa Ala Glu
1               5                   10                  15

Gln Xaa Xaa Xaa Xaa Thr Val Gly Trp Val Gln Arg His Arg Lys Met
            20                  25                  30

Leu Arg

<210> 1073
<211> 34
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1073
Lys Ser Met Gln Val Xaa Phe Ser Arg Xaa Xaa Phe Ser Xaa Ala Glu
1               5                   10                  15


Gln Xaa Xaa Xaa Xaa Thr Val Gly Trp Val Gln Arg His Arg Lys Met
            20                  25                  30


Leu Arg




<210> 1074
<211> 34
<212> PRT
<213> Artificial Sequence

<220>
<223> Description of Artificial Sequence: Synthetic peptide


<220>
<221> MOD_RES
<222> (7)..(7)

<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1074
Lys Ser Met Gln Val Pro Xaa Ser Arg Xaa Xaa Phe Ser Xaa Ala Glu
1               5                   10                  15

Gln Xaa Xaa Xaa Xaa Thr Val Gly Trp Val Gln Arg His Arg Lys Met
            20                  25                  30

Leu Arg

<210> 1075
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see

specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1075
Lys Ser Met Gln Val Pro Phe Xaa Arg Xaa Xaa Phe Ser Xaa Ala Glu
1               5                   10                  15

Gln Xaa Xaa Xaa Xaa Thr Val Gly Trp Val Gln Arg His Arg Lys Met
            20                  25                  30

Leu Arg

<210> 1076
<211> 34
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1076
Lys Ser Met Gln Val Pro Phe Ser Xaa Xaa Xaa Phe Ser Xaa Ala Glu
1               5                   10                  15

Gln Xaa Xaa Xaa Xaa Thr Val Gly Trp Val Gln Arg His Arg Lys Met
            20                  25                  30

Leu Arg

<210> 1077
<211> 34
<212> PRT
<213> Artificial Sequence

<220>
<223> Description of Artificial Sequence: Synthetic peptide

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1077
Lys Ser Met Gln Val Pro Phe Ser Arg Cys Cys Phe Ser Phe Xaa Glu
1               5                   10                  15

Gln Xaa Xaa Xaa Xaa Thr Val Gly Trp Val Gln Arg His Arg Lys Met
            20                  25                  30

Leu Arg

<210> 1078
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES

<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1078
Lys Ser Met Gln Val Pro Phe Ser Arg Xaa Xaa Phe Ser Phe Xaa Glu
1               5                   10                  15

Gln Xaa Xaa Xaa Xaa Thr Val Gly Trp Val Gln Arg His Arg Lys Met
            20                  25                  30

Leu Arg

<210> 1079
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1079
Xaa Ser Met Gln Val Pro Phe Ser Arg Xaa Xaa Phe Ser Phe Xaa Glu
1               5                   10                  15

Gln Xaa Xaa Xaa Xaa Thr Val Gly Trp Val Gln Arg His Arg Lys Met
            20                  25                  30

Leu Arg

<210> 1080
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1080
Lys Xaa Met Gln Val Pro Phe Ser Arg Xaa Xaa Phe Ser Phe Xaa Glu
1               5                   10                  15

Gln Xaa Xaa Xaa Xaa Thr Val Gly Trp Val Gln Arg His Arg Lys Met
            20                  25                  30

Leu Arg

<210> 1081
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1081
Lys Ser Xaa Gln Val Pro Phe Ser Arg Xaa Xaa Phe Ser Phe Xaa Glu
1               5                   10                  15

Gln Xaa Xaa Xaa Xaa Thr Val Gly Trp Val Gln Arg His Arg Lys Met
            20                  25                  30

Leu Arg

<210> 1082
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>

<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1082
Lys Ser Met Xaa Val Pro Phe Ser Arg Xaa Xaa Phe Ser Phe Xaa Glu
1               5                   10                  15

Gln Xaa Xaa Xaa Xaa Thr Val Gly Trp Val Gln Arg His Arg Lys Met
            20                  25                  30

Leu Arg

<210> 1083
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1083
Lys Ser Met Gln Xaa Pro Phe Ser Arg Xaa Xaa Phe Ser Phe Xaa Glu
1               5                   10                  15

Gln Xaa Xaa Xaa Xaa Thr Val Gly Trp Val Gln Arg His Arg Lys Met
            20                  25                  30

Leu Arg

<210> 1084
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1084
Lys Ser Met Gln Val Xaa Phe Ser Arg Xaa Xaa Phe Ser Phe Xaa Glu
1               5                   10                  15

Gln Xaa Xaa Xaa Xaa Thr Val Gly Trp Val Gln Arg His Arg Lys Met
            20                  25                  30

Leu Arg

<210> 1085

<211> 34
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1085
Lys Ser Met Gln Val Pro Xaa Ser Arg Xaa Xaa Phe Ser Phe Xaa Glu
1               5                   10                  15


Gln Xaa Xaa Xaa Xaa Thr Val Gly Trp Val Gln Arg His Arg Lys Met
            20                  25                  30


Leu Arg




<210> 1086
<211> 34
<212> PRT
<213> Artificial Sequence

<220>
<223> Description of Artificial Sequence: Synthetic peptide


<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1086
Lys Ser Met Gln Val Pro Phe Xaa Arg Xaa Xaa Phe Ser Phe Xaa Glu
1               5                   10                  15

Gln Xaa Xaa Xaa Xaa Thr Val Gly Trp Val Gln Arg His Arg Lys Met
            20                  25                  30

Leu Arg

<210> 1087
<211> 34
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 1087
Lys Ser Met Gln Val Pro Phe Ser Xaa Xaa Xaa Phe Ser Phe Xaa Glu
1               5                   10                  15

Gln Xaa Xaa Xaa Xaa Thr Val Gly Trp Val Gln Arg His Arg Lys Met
            20                  25                  30

Leu Arg

<210> 1088
<211> 34
<212> PRT
<213> Artificial Sequence

<220>
<223> Description of Artificial Sequence: Synthetic peptide

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1088
Lys Ser Met Gln Val Pro Phe Ser Arg Cys Cys Phe Ser Phe Ala Xaa
1               5                   10                  15

Gln Xaa Xaa Xaa Xaa Thr Val Gly Trp Val Gln Arg His Arg Lys Met
            20                  25                  30

Leu Arg

<210> 1089
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups

and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1089
Lys Ser Met Gln Val Pro Phe Ser Arg Xaa Xaa Phe Ser Phe Ala Xaa
1               5                   10                  15

Gln Xaa Xaa Xaa Xaa Thr Val Gly Trp Val Gln Arg His Arg Lys Met
            20                  25                  30

Leu Arg

<210> 1090
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1090
Xaa Ser Met Gln Val Pro Phe Ser Arg Xaa Xaa Phe Ser Phe Ala Xaa
1               5                   10                  15

Gln Xaa Xaa Xaa Xaa Thr Val Gly Trp Val Gln Arg His Arg Lys Met
            20                  25                  30

Leu Arg

<210> 1091
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1091
Lys Xaa Met Gln Val Pro Phe Ser Arg Xaa Xaa Phe Ser Phe Ala Xaa
1               5                   10                  15

Gln Xaa Xaa Xaa Xaa Thr Val Gly Trp Val Gln Arg His Arg Lys Met
            20                  25                  30

Leu Arg

<210> 1092
<211> 34
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1092
Lys Ser Xaa Gln Val Pro Phe Ser Arg Xaa Xaa Phe Ser Phe Ala Xaa
1               5                   10                  15


Gln Xaa Xaa Xaa Xaa Thr Val Gly Trp Val Gln Arg His Arg Lys Met
            20                  25                  30


Leu Arg



<210> 1093
<211> 34
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see

specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1093
Lys Ser Met Xaa Val Pro Phe Ser Arg Xaa Xaa Phe Ser Phe Ala Xaa
1               5                   10                  15

Gln Xaa Xaa Xaa Xaa Thr Val Gly Trp Val Gln Arg His Arg Lys Met
            20                  25                  30

Leu Arg

<210> 1094
<211> 34
<212> PRT
<213> Artificial Sequence

<220>
<223> Description of Artificial Sequence: Synthetic peptide

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1094
Lys Ser Met Gln Val Pro Phe Ser Arg Cys Cys Phe Ser Phe Ala Glu
1               5                   10                  15

Xaa Xaa Xaa Xaa Xaa Thr Val Gly Trp Val Gln Arg His Arg Lys Met
            20                  25                  30

Leu Arg

<210> 1095
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1095
Lys Ser Met Gln Val Pro Phe Ser Arg Xaa Xaa Phe Ser Phe Ala Glu
1               5                   10                  15

Xaa Xaa Xaa Xaa Xaa Thr Val Gly Trp Val Gln Arg His Arg Lys Met
            20                  25                  30

Leu Arg

<210> 1096
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1096
Xaa Ser Met Gln Val Pro Phe Ser Arg Xaa Xaa Phe Ser Phe Ala Glu
1               5                   10                  15

Xaa Xaa Xaa Xaa Xaa Thr Val Gly Trp Val Gln Arg His Arg Lys Met
            20                  25                  30

Leu Arg

<210> 1097
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>

<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1097
Lys Xaa Met Gln Val Pro Phe Ser Arg Xaa Xaa Phe Ser Phe Ala Glu
1               5                   10                  15

Xaa Xaa Xaa Xaa Xaa Thr Val Gly Trp Val Gln Arg His Arg Lys Met
            20                  25                  30

Leu Arg

<210> 1098
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1098
Lys Ser Xaa Gln Val Pro Phe Ser Arg Xaa Xaa Phe Ser Phe Ala Glu
1               5                   10                  15

Xaa Xaa Xaa Xaa Xaa Thr Val Gly Trp Val Gln Arg His Arg Lys Met
            20                  25                  30

Leu Arg

<210> 1099
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1099
Lys Ser Met Xaa Val Pro Phe Ser Arg Xaa Xaa Phe Ser Phe Ala Glu
1               5                   10                  15

Xaa Xaa Xaa Xaa Xaa Thr Val Gly Trp Val Gln Arg His Arg Lys Met
            20                  25                  30

Leu Arg

<210> 1100

<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1100
Lys Ser Met Gln Xaa Pro Phe Ser Arg Xaa Xaa Phe Ser Phe Ala Glu
1               5                   10                  15

Xaa Xaa Xaa Xaa Xaa Thr Val Gly Trp Val Gln Arg His Arg Lys Met
            20                  25                  30

Leu Arg

<210> 1101
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1101
Lys Ser Met Gln Val Xaa Phe Ser Arg Xaa Xaa Phe Ser Phe Ala Glu
1               5                   10                  15

Xaa Xaa Xaa Xaa Xaa Thr Val Gly Trp Val Gln Arg His Arg Lys Met
            20                  25                  30

Leu Arg

<210> 1102
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>

<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1102
Lys Ser Met Gln Val Pro Xaa Ser Arg Xaa Xaa Phe Ser Phe Ala Glu
1               5                   10                  15

Xaa Xaa Xaa Xaa Xaa Thr Val Gly Trp Val Gln Arg His Arg Lys Met
            20                  25                  30

Leu Arg

<210> 1103
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1103
Lys Ser Met Gln Val Pro Phe Xaa Arg Xaa Xaa Phe Ser Phe Ala Glu
1               5                   10                  15

Xaa Xaa Xaa Xaa Xaa Thr Val Gly Trp Val Gln Arg His Arg Lys Met

20                  25                  30

Leu Arg

<210> 1104
<211> 34
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1104
Lys Ser Met Gln Val Pro Phe Ser Xaa Xaa Xaa Phe Ser Phe Ala Glu
1               5                   10                  15

Xaa Xaa Xaa Xaa Xaa Thr Val Gly Trp Val Gln Arg His Arg Lys Met
            20                  25                  30

Leu Arg

<210> 1105
<211> 31
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1105
Lys Ser Met Gln Val Pro Phe Ser Arg Cys Cys Phe Ser Phe Xaa Xaa
1               5                   10                  15

Xaa Xaa Thr Val Gly Trp Val Glu Arg His Arg Lys Met Leu Arg
            20                  25                  30

<210> 1106
<211> 31
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1106
Lys Ser Met Gln Val Pro Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1107
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1107
Xaa Ser Met Gln Val Pro Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1108
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1108
Lys Xaa Met Gln Val Pro Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1109
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1109
Lys Ser Xaa Gln Val Pro Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1110
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1110
Lys Ser Met Xaa Val Pro Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1111
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1111
Lys Ser Met Gln Xaa Pro Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1112
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups

and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1112
Lys Ser Met Gln Val Xaa Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1113
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1113
Lys Ser Met Gln Val Pro Xaa Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1114
<211> 31
<212> PRT
<213> Artificial Sequence

<220>
<223> Description of Artificial Sequence: Synthetic peptide


<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1114
Lys Ser Met Gln Val Pro Phe Xaa Arg Xaa Xaa Phe Ser Phe Xaa Xaa
1               5                   10                  15


Xaa Xaa Thr Val Gly Trp Val Glu Arg His Arg Lys Met Leu Arg
            20                  25                  30


<210> 1115
<211> 31
<212> PRT
<213> Artificial Sequence

<220>
<223> Description of Artificial Sequence: Synthetic peptide


<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 1115
Lys Ser Met Gln Val Pro Phe Ser Xaa Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1116
<211> 31
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1116
Lys Ser Met Gln Val Pro Phe Ser Arg Cys Cys Xaa Ser Phe Xaa Xaa
1               5                   10                  15

Xaa Xaa Thr Val Gly Trp Val Glu Arg His Arg Lys Met Leu Arg
            20                  25                  30

<210> 1117
<211> 31
<212> PRT
<213> Artificial Sequence

<220>
<223> Description of Artificial Sequence: Synthetic peptide

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1117
Lys Ser Met Gln Val Pro Phe Ser Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1118
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1118
Xaa Ser Met Gln Val Pro Phe Ser Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1119
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see

specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1119
Lys Xaa Met Gln Val Pro Phe Ser Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1120
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1120
Lys Ser Xaa Gln Val Pro Phe Ser Arg Xaa Xaa Xaa Ser Phe Xaa Xaa
1               5                   10                  15

Xaa Xaa Thr Val Gly Trp Val Glu Arg His Arg Lys Met Leu Arg

20                   25                   30

<210> 1121
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1121
Lys Ser Met Xaa Val Pro Phe Ser Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1122
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1122
Lys Ser Met Gln Xaa Pro Phe Ser Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1123
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1123
Lys Ser Met Gln Val Xaa Phe Ser Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1124
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1124
Lys Ser Met Gln Val Pro Xaa Ser Arg Xaa Xaa Xaa Ser Phe Xaa Xaa
1               5                   10                  15


Xaa Xaa Thr Val Gly Trp Val Glu Arg His Arg Lys Met Leu Arg
            20                  25                  30


<210> 1125
<211> 31
<212> PRT
<213> Artificial Sequence

<220>
<223> Description of Artificial Sequence: Synthetic peptide


<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1125
Lys Ser Met Gln Val Pro Phe Xaa Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1126
<211> 31
<212> PRT
<213> Artificial Sequence

<220>
<223> Description of Artificial Sequence: Synthetic peptide

<220>
<221> MOD_RES
<222> (9)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1126
Lys Ser Met Gln Val Pro Phe Ser Xaa Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1127
<211> 31
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 1127
Lys Ser Met Gln Val Pro Phe Ser Arg Cys Cys Phe Xaa Phe Xaa Xaa
1               5                   10                  15

Xaa Xaa Thr Val Gly Trp Val Glu Arg His Arg Lys Met Leu Arg
            20                  25                  30

<210> 1128
<211> 31
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1128
Lys Ser Met Gln Val Pro Phe Ser Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1129
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1129
Xaa Ser Met Gln Val Pro Phe Ser Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1130
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 1130
Lys Xaa Met Gln Val Pro Phe Ser Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1131
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1131
Lys Ser Xaa Gln Val Pro Phe Ser Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1132
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1132
Lys Ser Met Xaa Val Pro Phe Ser Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1133
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1133
Lys Ser Met Gln Xaa Pro Phe Ser Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1134
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1134
Lys Ser Met Gln Val Xaa Phe Ser Arg Xaa Xaa Phe Xaa Phe Xaa Xaa

1                   5                        10                       15

Xaa Xaa Thr Val Gly Trp Val Glu Arg His Arg Lys Met Leu Arg
                20                       25                       30

<210> 1135
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1135
Lys Ser Met Gln Val Pro Xaa Ser Arg Xaa Xaa Phe Xaa Phe Xaa Xaa
1                   5                        10                       15

Xaa Xaa Thr Val Gly Trp Val Glu Arg His Arg Lys Met Leu Arg
                20                       25                       30

<210> 1136
<211> 31
<212> PRT
<213> Artificial Sequence

<220>
<223> Description of Artificial Sequence: Synthetic peptide

<220>
<221> MOD_RES
<222> (8)..(8)

<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1136
Lys Ser Met Gln Val Pro Phe Xaa Arg Xaa Xaa Phe Xaa Phe Xaa Xaa
1               5                   10                  15


Xaa Xaa Thr Val Gly Trp Val Glu Arg His Arg Lys Met Leu Arg
            20                  25                  30


<210> 1137
<211> 31
<212> PRT
<213> Artificial Sequence

<220>
<223> Description of Artificial Sequence: Synthetic peptide


<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 1137
Lys Ser Met Gln Val Pro Phe Ser Xaa Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1138
<211> 31
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1138
Lys Ser Met Gln Val Pro Phe Ser Arg Cys Cys Phe Ser Xaa Xaa Xaa
1               5                   10                  15

Xaa Xaa Thr Val Gly Trp Val Glu Arg His Arg Lys Met Leu Arg
            20                  25                  30

<210> 1139
<211> 31
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1139
Lys Ser Met Gln Val Pro Phe Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1140
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1140
Xaa Ser Met Gln Val Pro Phe Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1141
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1141
Lys Xaa Met Gln Val Pro Phe Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1142
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES

<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1142
Lys Ser Xaa Gln Val Pro Phe Ser Arg Xaa Xaa Phe Ser Xaa Xaa Xaa
1               5                   10                  15


Xaa Xaa Thr Val Gly Trp Val Glu Arg His Arg Lys Met Leu Arg
            20                  25                  30


<210> 1143
<211> 31
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments
<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 1143
Lys Ser Met Xaa Val Pro Phe Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1144
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1144
Lys Ser Met Gln Xaa Pro Phe Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1145
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1145
Lys Ser Met Gln Val Xaa Phe Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1146
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1146
Lys Ser Met Gln Val Pro Xaa Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1147
<211> 31
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1147
Lys Ser Met Gln Val Pro Phe Xaa Arg Xaa Xaa Phe Ser Xaa Xaa Xaa
1               5                   10                  15

Xaa Xaa Thr Val Gly Trp Val Glu Arg His Arg Lys Met Leu Arg

20                  25                  30

<210> 1148
<211> 31
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1148
Lys Ser Met Gln Val Pro Phe Ser Xaa Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1149
<211> 34
<212> PRT
<213> Artificial Sequence

<220>
<223> Description of Artificial Sequence: Synthetic peptide

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1149
Lys Ser Met Gln Val Pro Phe Ser Arg Cys Cys Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1150
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1150
Lys Ser Met Gln Val Pro Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1151
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see

specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1151
Xaa Ser Met Gln Val Pro Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1152
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1152
Lys Xaa Met Gln Val Pro Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1153
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1153
Lys Ser Xaa Gln Val Pro Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1154
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES

<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1154
Lys Ser Met Xaa Val Pro Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1155
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1155
Lys Ser Met Gln Xaa Pro Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1156
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1156
Lys Ser Met Gln Val Xaa Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1157
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1157
Lys Ser Met Gln Val Pro Xaa Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1158
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1158
Lys Ser Met Gln Val Pro Phe Xaa Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1159
<211> 34
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1159
Lys Ser Met Gln Val Pro Phe Ser Xaa Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1160
<211> 34
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 1160
Lys Ser Met Gln Val Pro Phe Ser Arg Cys Cys Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1161
<211> 34
<212> PRT
<213> Artificial Sequence

<220>
<223> Description of Artificial Sequence: Synthetic peptide

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1161
Lys Ser Met Gln Val Pro Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1162
<211> 34
<212> PRT
<213> Artificial Sequence

<220>
<223> Description of Artificial Sequence: Synthetic peptide

<220>
<221> MOD_RES

<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1162
Xaa Ser Met Gln Val Pro Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1163
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1163
Lys Xaa Met Gln Val Pro Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1164
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see specification as filed for detailed description of substitutions, R-groups and preferred embodiments

<220>
<221> MOD_RES
<222> (10)..(12)
<223> Any L- or D-natural amino acid and any non-natural amino acid; see specification as filed for detailed description of substitutions, R-groups and preferred embodiments

<220>
<221> MOD_RES
<222> (18)..(21)
<223> Any L- or D-natural or non-natural amino acid, and this range may encompass 1 or 4 residues; see specification as filed for detailed description of substitutions, R-groups and preferred embodiments

<220>
<223> C-term may be amidated

<400> 1164
Lys Ser Xaa Gln Val Pro Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1165
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1165
Lys Ser Met Xaa Val Pro Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1166
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 1166
Lys Ser Met Gln Xaa Pro Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1167
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1167
Lys Ser Met Gln Val Xaa Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1168
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1168
Lys Ser Met Gln Val Pro Xaa Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1169
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1169
Lys Ser Met Gln Val Pro Phe Xaa Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1170
<211> 34
<212> PRT
<213> Artificial Sequence

<220>
<223> Description of Artificial Sequence: Synthetic peptide

<220>
<221> MOD_RES
<222> (9)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1170
Lys Ser Met Gln Val Pro Phe Ser Xaa Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1171
<211> 34
<212> PRT
<213> Artificial Sequence

<220>
<223> Description of Artificial Sequence: Synthetic peptide

<220>
<221> MOD_RES
<222> (13)..(13)

<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1171
Lys Ser Met Gln Val Pro Phe Ser Arg Cys Cys Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1172
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1172
Lys Ser Met Gln Val Pro Phe Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1173
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1173
Xaa Ser Met Gln Val Pro Phe Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1174
<211> 34
<212> PRT
<213> Artificial Sequence

<220>
<223> Description of Artificial Sequence: Synthetic peptide

<220>

<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1174
Lys Xaa Met Gln Val Pro Phe Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1175
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES

<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1175
Lys Ser Xaa Gln Val Pro Phe Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1176
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1176
Lys Ser Met Xaa Val Pro Phe Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1177
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1177
Lys Ser Met Gln Xaa Pro Phe Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1178
<211> 34

<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1178
Lys Ser Met Gln Val Xaa Phe Ser Arg Xaa Xaa Phe Xaa Phe Ala Glu
1               5                   10                  15


Gln Xaa Xaa Xaa Xaa Thr Val Gly Trp Val Glu Arg His Arg Lys Met
            20                  25                  30


Leu Arg




<210> 1179
<211> 34
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>

<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1179
Lys Ser Met Gln Val Pro Xaa Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1180
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES

<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1180
Lys Ser Met Gln Val Pro Phe Xaa Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1181
<211> 34
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1181
Lys Ser Met Gln Val Pro Phe Ser Xaa Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1182

<211> 34
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1182
Lys Ser Met Gln Val Pro Phe Ser Arg Cys Cys Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1183
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1183
Lys Ser Met Gln Val Pro Phe Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1184
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1184
Xaa Ser Met Gln Val Pro Phe Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1185
<211> 34
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1185
Lys Xaa Met Gln Val Pro Phe Ser Arg Xaa Xaa Phe Ser Xaa Ala Glu
1               5                   10                  15


Gln Xaa Xaa Xaa Xaa Thr Val Gly Trp Val Glu Arg His Arg Lys Met
            20                  25                  30


Leu Arg




<210> 1186
<211> 34
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups

and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1186
Lys Ser Xaa Gln Val Pro Phe Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1187
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1187
Lys Ser Met Xaa Val Pro Phe Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1188
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1188
Lys Ser Met Gln Xaa Pro Phe Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1189
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1189
Lys Ser Met Gln Val Xaa Phe Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1190
<211> 34
<212> PRT
<213> Artificial Sequence

<220>

<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1190
Lys Ser Met Gln Val Pro Xaa Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1191
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups

and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1191
Lys Ser Met Gln Val Pro Phe Xaa Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1192
<211> 34
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1192
Lys Ser Met Gln Val Pro Phe Ser Xaa Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1193
<211> 34
<212> PRT
<213> Artificial Sequence

<220>
<223> Description of Artificial Sequence: Synthetic peptide

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1193
Lys Ser Met Gln Val Pro Phe Ser Arg Cys Cys Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1194
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)

<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1194
Lys Ser Met Gln Val Pro Phe Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1195
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1195

Xaa Ser Met Gln Val Pro Phe Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1196
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1196
Lys Xaa Met Gln Val Pro Phe Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1197
<211> 34
<212> PRT

<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1197
Lys Ser Xaa Gln Val Pro Phe Ser Arg Xaa Xaa Phe Ser Phe Xaa Glu
1               5                   10                  15


Gln Xaa Xaa Xaa Xaa Thr Val Gly Trp Val Glu Arg His Arg Lys Met
            20                  25                  30


Leu Arg




<210> 1198
<211> 34
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES

<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1198
Lys Ser Met Xaa Val Pro Phe Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1199
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1199
Lys Ser Met Gln Xaa Pro Phe Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1200
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1200
Lys Ser Met Gln Val Xaa Phe Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1201
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1201
Lys Ser Met Gln Val Pro Xaa Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1202
<211> 34
<212> PRT
<213> Artificial Sequence

<220>
<223> Description of Artificial Sequence: Synthetic peptide

<220>

<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1202
Lys Ser Met Gln Val Pro Phe Xaa Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1203
<211> 34
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES

<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1203
Lys Ser Met Gln Val Pro Phe Ser Xaa Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1204
<211> 34
<212> PRT
<213> Artificial Sequence

<220>
<223> Description of Artificial Sequence: Synthetic peptide

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1204
Lys Ser Met Gln Val Pro Phe Ser Arg Cys Cys Phe Ser Phe Ala Xaa
1               5                   10                  15

Gln Xaa Xaa Xaa Xaa Thr Val Gly Trp Val Glu Arg His Arg Lys Met
            20                  25                  30

Leu Arg

<210> 1205
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1205
Lys Ser Met Gln Val Pro Phe Ser Arg Xaa Xaa Phe Ser Phe Ala Xaa
1               5                   10                  15

Gln Xaa Xaa Xaa Xaa Thr Val Gly Trp Val Glu Arg His Arg Lys Met
            20                  25                  30

Leu Arg

<210> 1206
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1206
Xaa Ser Met Gln Val Pro Phe Ser Arg Xaa Xaa Phe Ser Phe Ala Xaa
1               5                   10                  15

Gln Xaa Xaa Xaa Xaa Thr Val Gly Trp Val Glu Arg His Arg Lys Met
            20                  25                  30

Leu Arg

<210> 1207
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1207
Lys Xaa Met Gln Val Pro Phe Ser Arg Xaa Xaa Phe Ser Phe Ala Xaa
1               5                   10                  15

Gln Xaa Xaa Xaa Xaa Thr Val Gly Trp Val Glu Arg His Arg Lys Met
            20                  25                  30

Leu Arg

<210> 1208
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1208
Lys Ser Xaa Gln Val Pro Phe Ser Arg Xaa Xaa Phe Ser Phe Ala Xaa
1               5                   10                  15

Gln Xaa Xaa Xaa Xaa Thr Val Gly Trp Val Glu Arg His Arg Lys Met
            20                  25                  30

Leu Arg

<210> 1209
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1209
Lys Ser Met Xaa Val Pro Phe Ser Arg Xaa Xaa Phe Ser Phe Ala Xaa
1               5                   10                  15

Gln Xaa Xaa Xaa Xaa Thr Val Gly Trp Val Glu Arg His Arg Lys Met
            20                  25                  30

Leu Arg

<210> 1210
<211> 34
<212> PRT
<213> Artificial Sequence

<220>
<223> Description of Artificial Sequence: Synthetic peptide

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1210
Lys Ser Met Gln Val Pro Phe Ser Arg Cys Cys Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1211
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1211
Lys Ser Met Gln Val Pro Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1212
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1212
Xaa Ser Met Gln Val Pro Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1213
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see

specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1213
Lys Xaa Met Gln Val Pro Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1214
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 1214
Lys Ser Xaa Gln Val Pro Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1215
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1215
Lys Ser Met Xaa Val Pro Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1216
<211> 34
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1216
Lys Ser Met Gln Xaa Pro Phe Ser Arg Xaa Xaa Phe Ser Phe Ala Glu
1               5                   10                  15


Xaa Xaa Xaa Xaa Xaa Thr Val Gly Trp Val Glu Arg His Arg Lys Met
            20                  25                  30


Leu Arg



<210> 1217
<211> 34
<212> PRT
<213> Artificial Sequence

<220>
<223> Description of Artificial Sequence: Synthetic peptide


<220>
<221> MOD_RES
<222> (6)..(6)

<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1217
Lys Ser Met Gln Val Xaa Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1218
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see

specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1218
Lys Ser Met Gln Val Pro Xaa Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1219
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1219
Lys Ser Met Gln Val Pro Phe Xaa Arg Xaa Xaa Phe Ser Phe Ala Glu

1                   5                   10                  15

Xaa Xaa Xaa Xaa Xaa Thr Val Gly Trp Val Glu Arg His Arg Lys Met
            20                  25                  30

Leu Arg

<210> 1220
<211> 34
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1220
Lys Ser Met Gln Val Pro Phe Ser Xaa Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1221
<211> 24
<212> PRT
<213> Artificial Sequence

<220>
<223> Description of Artificial Sequence: Synthetic peptide

<220>
<223> C-term may be amidated

<400> 1221
Ala Tyr Arg Asn Thr Ser Ser Ile Cys Ser Asn Glu Gly Leu Ile Phe
1               5                   10                  15

Lys Leu Lys Arg Gly Lys Glu Ala
            20

<210> 1222
<211> 24
<212> PRT
<213> Artificial Sequence

<220>
<223> Description of Artificial Sequence: Synthetic peptide

<220>
<223> C-term may be amidated

<400> 1222
Ala Tyr Arg Asn Thr Ser Ser Ile Cys Ser Asn Glu Gly Leu Ile Phe
1               5                   10                  15

Lys Leu Lys Arg Gly Lys Glu Ala
            20

<210> 1223
<211> 24
<212> PRT
<213> Artificial Sequence

<220>
<223> Description of Artificial Sequence: Synthetic peptide

<220>
<223> C-term may be amidated

<400> 1223
Ala Tyr Arg Asn Thr Ser Ser Ile Cys Ser Asn Glu Gly Leu Ile Phe
1               5                   10                  15

Lys Leu Lys Arg Gly Lys Glu Ala
            20

<210> 1224
<211> 25
<212> PRT
<213> Artificial Sequence

<220>
<223> Description of Artificial Sequence: Synthetic peptide

<220>
<223> C-term may be amidated

<400> 1224
Lys Ser Met Gln Val Pro Phe Ser Arg Cys Cys Phe Ser Phe Ala Glu
1               5                   10                  15

Gln Glu Ile Pro Leu Arg Ala Ile Leu
            20                  25

<210> 1225
<211> 25
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<223> C-term may be amidated

<400> 1225
Lys Ser Met Gln Val Pro Phe Ser Arg Xaa Xaa Phe Ser Phe Ala Glu
1               5                   10                  15

Gln Glu Ile Pro Leu Arg Ala Ile Leu
            20                  25

<210> 1226
<211> 25
<212> PRT
<213> Artificial Sequence

<220>
<223> Description of Artificial Sequence: Synthetic peptide

<220>
<223> C-term may be amidated

<400> 1226
Lys Ser Met Gln Val Pro Phe Ser Arg Cys Cys Phe Ser Phe Ala Glu
1               5                   10                  15

Gln Glu Ile Pro Leu Arg Ala Ile Leu
            20                  25

<210> 1227
<211> 25
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<223> C-term may be amidated

<400> 1227
Lys Ser Met Gln Val Pro Phe Ser Arg Xaa Xaa Phe Ser Phe Ala Glu
1               5                   10                  15

Gln Glu Ile Pro Leu Arg Ala Ile Leu
            20                  25

<210> 1228
<211> 34
<212> PRT
<213> Artificial Sequence

<220>
<223> Description of Artificial Sequence: Synthetic peptide

<220>
<223> c-term may be amidated

<400> 1228
Ala Ser Asn Glu Gly Leu Ile Phe Lys Leu Lys Arg Gly Lys Glu Ala
1               5                   10                  15

Cys Ala Leu Asp Thr Val Gly Trp Val Gln Arg His Arg Lys Met Leu
            20                  25                  30

Arg His

<210> 1229
<211> 33
<212> PRT
<213> Artificial Sequence

<220>
<223> Description of Artificial Sequence: Synthetic peptide

<220>
<223> c-term may be amidated

<400> 1229
Ala Ser Asn Glu Gly Leu Ile Phe Lys Leu Lys Arg Gly Lys Glu Ala
1               5                   10                  15

Cys Ala Leu Asp Thr Val Gly Trp Val Gln Arg His Arg Lys Met Leu
            20                  25                  30

Arg

<210> 1230
<211> 33
<212> PRT
<213> Artificial Sequence

<220>
<223> Description of Artificial Sequence: Synthetic peptide

<220>
<223> c-term may be amidated

<400> 1230
Ala Ser Asn Glu Gly Leu Ile Phe Lys Leu Lys Arg Gly Lys Glu Ala
1               5                   10                  15

Cys Ala Leu Asp Thr Val Gly Trp Val Gln Arg His Arg Lys Met Leu
            20                  25                  30

Arg

<210> 1231
<211> 33
<212> PRT
<213> Artificial Sequence

<220>
<223> Description of Artificial Sequence: Synthetic peptide

<220>
<223> C-term may be amidated

<400> 1231
Ala Ser Asn Glu Gly Leu Ile Phe Lys Leu Lys Arg Gly Lys Glu Ala
1               5                   10                  15

Cys Ala Leu Asp Thr Val Gly Trp Val Gln Arg His Arg Lys Met Leu
            20                  25                  30

Arg

<210> 1232
<211> 33
<212> PRT
<213> Artificial Sequence

<220>
<223> Description of Artificial Sequence: Synthetic peptide

<220>
<223> C-term may be amidated

<400> 1232
Ala Ser Asn Glu Gly Leu Ile Phe Lys Leu Lys Arg Gly Lys Glu Ala
1               5                   10                  15

Cys Ala Leu Asp Thr Val Gly Trp Val Gln Arg His Arg Lys Met Leu
            20                  25                  30

Arg

<210> 1233
<211> 33
<212> PRT
<213> Artificial Sequence

<220>
<223> Description of Artificial Sequence: Synthetic peptide

<220>
<223> c-term may be amidated

<400> 1233
Ala Ser Asn Glu Gly Leu Ile Phe Lys Leu Lys Arg Gly Lys Glu Ala
1               5                   10                  15

Cys Ala Leu Asp Thr Val Gly Trp Val Gln Arg His Arg Lys Met Leu
            20                  25                  30

Arg

<210> 1234
<211> 33
<212> PRT
<213> Artificial Sequence

<220>
<223> Description of Artificial Sequence: Synthetic peptide

<220>
<223> c-term may be amidated

<400> 1234
Ala Ser Asn Glu Gly Leu Ile Phe Lys Leu Lys Arg Gly Lys Glu Ala
1               5                   10                  15

Cys Ala Leu Asp Thr Val Gly Trp Val Gln Arg His Arg Lys Met Leu
            20                  25                  30

Arg

<210> 1235
<211> 31
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1235
Lys Ser Met Gln Val Pro Phe Ser Arg Cys Cys Phe Ser Phe Xaa Xaa
1               5                   10                  15

Xaa Xaa Thr Val Gly Trp Val Glu Arg His Arg Lys Met Leu Arg
            20                  25                  30

<210> 1236
<211> 31
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1236
Lys Ser Met Gln Val Pro Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1237
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1237
Xaa Ser Met Gln Val Pro Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1238
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1238
Lys Xaa Met Gln Val Pro Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1239
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1239
Lys Ser Xaa Gln Val Pro Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1240
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1240
Lys Ser Met Xaa Val Pro Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1241
<211> 31
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1241
Lys Ser Met Gln Xaa Pro Phe Ser Arg Xaa Xaa Phe Ser Phe Xaa Xaa
1               5                   10                  15


Xaa Xaa Thr Val Gly Trp Val Glu Arg His Arg Lys Met Leu Arg
            20                  25                  30


<210> 1242
<211> 31
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1242
Lys Ser Met Gln Val Xaa Phe Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1243
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1243
Lys Ser Met Gln Val Pro Xaa Ser Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1244
<211> 31
<212> PRT
<213> Artificial Sequence

<220>
<223> Description of Artificial Sequence: Synthetic peptide

<220>

<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1244
Lys Ser Met Gln Val Pro Phe Xaa Arg Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1245
<211> 31
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1245
Lys Ser Met Gln Val Pro Phe Ser Xaa Xaa Xaa Phe Ser Phe Xaa Xaa
1               5                   10                  15

Xaa Xaa Thr Val Gly Trp Val Glu Arg His Arg Lys Met Leu Arg
            20                  25                  30

<210> 1246

<211> 31
<212> PRT
<213> Artificial Sequence

<220>
<223> Description of Artificial Sequence: Synthetic peptide


<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1246
Lys Ser Met Gln Val Pro Phe Ser Arg Cys Cys Xaa Ser Phe Xaa Xaa
1               5                   10                  15


Xaa Xaa Thr Val Gly Trp Val Glu Arg His Arg Lys Met Leu Arg
            20                  25                  30


<210> 1247
<211> 31
<212> PRT
<213> Artificial Sequence

<220>
<223> Description of Artificial Sequence: Synthetic peptide


<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1247
Lys Ser Met Gln Val Pro Phe Ser Arg Xaa Xaa Xaa Ser Phe Xaa Xaa
1               5                   10                  15


Xaa Xaa Thr Val Gly Trp Val Glu Arg His Arg Lys Met Leu Arg
            20                  25                  30

<210> 1248
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1248
Xaa Ser Met Gln Val Pro Phe Ser Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1249
<211> 31
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES

<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1249
Lys Xaa Met Gln Val Pro Phe Ser Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1250
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1250
Lys Ser Xaa Gln Val Pro Phe Ser Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1251
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1251
Lys Ser Met Xaa Val Pro Phe Ser Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1252
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1252
Lys Ser Met Gln Xaa Pro Phe Ser Arg Xaa Xaa Xaa Ser Phe Xaa Xaa

1                   5                   10                  15

Xaa Xaa Thr Val Gly Trp Val Glu Arg His Arg Lys Met Leu Arg
            20                  25                  30

<210> 1253
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1253
Lys Ser Met Gln Val Xaa Phe Ser Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1254
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(12)

<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1254
Lys Ser Met Gln Val Pro Xaa Ser Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1255
<211> 31
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1255
Lys Ser Met Gln Val Pro Phe Xaa Arg Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1256
<211> 31
<212> PRT

<213> Artificial Sequence

<220>
<223> Description of Artificial Sequence: Synthetic peptide

<220>
<221> MOD_RES
<222> (9)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1256
Lys Ser Met Gln Val Pro Phe Ser Xaa Xaa Xaa Xaa Ser Phe Xaa Xaa
1               5                   10                  15

Xaa Xaa Thr Val Gly Trp Val Glu Arg His Arg Lys Met Leu Arg
            20                  25                  30

<210> 1257
<211> 31
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1257
Lys Ser Met Gln Val Pro Phe Ser Arg Cys Cys Phe Xaa Phe Xaa Xaa
1               5                   10                  15

Xaa Xaa Thr Val Gly Trp Val Glu Arg His Arg Lys Met Leu Arg
            20                  25                  30

<210> 1258

<211> 31
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1258
Lys Ser Met Gln Val Pro Phe Ser Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1259
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1259
Xaa Ser Met Gln Val Pro Phe Ser Arg Xaa Xaa Phe Xaa Phe Xaa Xaa
1               5                   10                  15


Xaa Xaa Thr Val Gly Trp Val Glu Arg His Arg Lys Met Leu Arg
            20                  25                  30


<210> 1260
<211> 31
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1260
Lys Xaa Met Gln Val Pro Phe Ser Arg Xaa Xaa Phe Xaa Phe Xaa Xaa
1               5                   10                  15


Xaa Xaa Thr Val Gly Trp Val Glu Arg His Arg Lys Met Leu Arg

20                    25                    30

<210> 1261
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1261
Lys Ser Xaa Gln Val Pro Phe Ser Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1262
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1262
Lys Ser Met Xaa Val Pro Phe Ser Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1263
<211> 31
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 1263
Lys Ser Met Gln Xaa Pro Phe Ser Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1264
<211> 31
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1264
Lys Ser Met Gln Val Xaa Phe Ser Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1265
<211> 31
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1265
Lys Ser Met Gln Val Pro Xaa Ser Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1266
<211> 31
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>

<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1266
Lys Ser Met Gln Val Pro Phe Xaa Arg Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1267
<211> 31
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1267
Lys Ser Met Gln Val Pro Phe Ser Xaa Xaa Xaa Phe Xaa Phe Xaa Xaa
1               5                   10                  15

Xaa Xaa Thr Val Gly Trp Val Glu Arg His Arg Lys Met Leu Arg
            20                  25                  30

<210> 1268

<211> 31
<212> PRT
<213> Artificial Sequence

<220>
<223> Description of Artificial Sequence: Synthetic peptide

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1268
Lys Ser Met Gln Val Pro Phe Ser Arg Cys Cys Phe Ser Xaa Xaa Xaa
1               5                   10                  15

Xaa Xaa Thr Val Gly Trp Val Glu Arg His Arg Lys Met Leu Arg
            20                  25                  30

<210> 1269
<211> 31
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1269

Lys Ser Met Gln Val Pro Phe Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1270
<211> 31
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1270
Xaa Ser Met Gln Val Pro Phe Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1271
<211> 31
<212> PRT
<213> Artificial Sequence

<220>
<223> Description of Artificial Sequence: Synthetic peptide

<220>
<221> MOD_RES

<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1271
Lys Xaa Met Gln Val Pro Phe Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1272
<211> 31
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1272
Lys Ser Xaa Gln Val Pro Phe Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1273
<211> 31
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1273
Lys Ser Met Xaa Val Pro Phe Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1274
<211> 31
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1274
Lys Ser Met Gln Xaa Pro Phe Ser Arg Xaa Xaa Phe Ser Xaa Xaa Xaa
1               5                   10                  15


Xaa Xaa Thr Val Gly Trp Val Glu Arg His Arg Lys Met Leu Arg
            20                  25                  30


<210> 1275
<211> 31
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)

<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1275
Lys Ser Met Gln Val Xaa Phe Ser Arg Xaa Xaa Phe Ser Xaa Xaa Xaa
1               5                   10                  15


Xaa Xaa Thr Val Gly Trp Val Glu Arg His Arg Lys Met Leu Arg
            20                  25                  30


<210> 1276
<211> 31
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 1276
Lys Ser Met Gln Val Pro Xaa Ser Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1277
<211> 31
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1277
Lys Ser Met Gln Val Pro Phe Xaa Arg Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1278
<211> 31
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1278
Lys Ser Met Gln Val Pro Phe Ser Xaa Xaa Xaa Phe Ser Xaa Xaa Xaa
1               5                   10                  15

Xaa Xaa Thr Val Gly Trp Val Glu Arg His Arg Lys Met Leu Arg
            20                  25                  30

<210> 1279
<211> 34
<212> PRT
<213> Artificial Sequence

<220>
<223> Description of Artificial Sequence: Synthetic peptide

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1279
Lys Ser Met Gln Val Pro Phe Ser Arg Cys Cys Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1280
<211> 34

<212> PRT
<213> Artificial Sequence

<220>
<223> Description of Artificial Sequence: Synthetic peptide


<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1280
Lys Ser Met Gln Val Pro Phe Ser Arg Xaa Xaa Phe Ser Phe Ala Glu
1               5                   10                  15


Gln Xaa Xaa Xaa Xaa Thr Val Gly Trp Val Glu Arg His Arg Lys Met
            20                  25                  30


Leu Arg



<210> 1281
<211> 34
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> C-term may be amidated

<400> 1281
Xaa Ser Met Gln Val Pro Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1282
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1282
Lys Xaa Met Gln Val Pro Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1283
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1283
Lys Ser Xaa Gln Val Pro Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1284
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1284
Lys Ser Met Xaa Val Pro Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1285
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1285
Lys Ser Met Gln Xaa Pro Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1286
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1286
Lys Ser Met Gln Val Xaa Phe Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1287
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may

encompass 1 or 4 residues; see specification as filed for detailed
description of substitutions, R-groups and preferred embodiments

<220>
<223> C-term may be amidated

<400> 1287
Lys Ser Met Gln Val Pro Xaa Ser Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1288
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1288
Lys Ser Met Gln Val Pro Phe Xaa Arg Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1289
<211> 34
<212> PRT

<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1289
Lys Ser Met Gln Val Pro Phe Ser Xaa Xaa Xaa Phe Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1290
<211> 34
<212> PRT
<213> Artificial Sequence

<220>
<223> Description of Artificial Sequence: Synthetic peptide

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1290
Lys Ser Met Gln Val Pro Phe Ser Arg Cys Cys Xaa Ser Phe Ala Glu
1               5                   10                  15

Gln Xaa Xaa Xaa Xaa Thr Val Gly Trp Val Glu Arg His Arg Lys Met

20                  25                  30

Leu Arg

<210> 1291
<211> 34
<212> PRT
<213> Artificial Sequence

<220>
<223> Description of Artificial Sequence: Synthetic peptide

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1291
Lys Ser Met Gln Val Pro Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1292
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1292
Xaa Ser Met Gln Val Pro Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1293
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1293
Lys Xaa Met Gln Val Pro Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1294
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1294
Lys Ser Xaa Gln Val Pro Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1295
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see

specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1295
Lys Ser Met Xaa Val Pro Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1296
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1296
Lys Ser Met Gln Xaa Pro Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1297
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1297
Lys Ser Met Gln Val Xaa Phe Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1298
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES

<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1298
Lys Ser Met Gln Val Pro Xaa Ser Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1299
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1299
Lys Ser Met Gln Val Pro Phe Xaa Arg Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1300
<211> 34
<212> PRT
<213> Artificial Sequence

<220>
<223> Description of Artificial Sequence: Synthetic peptide

<220>
<221> MOD_RES
<222> (9)..(12)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1300
Lys Ser Met Gln Val Pro Phe Ser Xaa Xaa Xaa Xaa Ser Phe Ala Glu
1               5                   10                  15

Gln Xaa Xaa Xaa Xaa Thr Val Gly Trp Val Glu Arg His Arg Lys Met
            20                  25                  30

Leu Arg

<210> 1301
<211> 34
<212> PRT
<213> Artificial Sequence

<220>
<223> Description of Artificial Sequence: Synthetic peptide

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1301
Lys Ser Met Gln Val Pro Phe Ser Arg Cys Cys Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1302
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1302
Lys Ser Met Gln Val Pro Phe Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1303
<211> 34
<212> PRT
<213> Artificial Sequence

<220>

<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1303
Xaa Ser Met Gln Val Pro Phe Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1304
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups

and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1304
Lys Xaa Met Gln Val Pro Phe Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1305
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1305
Lys Ser Xaa Gln Val Pro Phe Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1306
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1306
Lys Ser Met Xaa Val Pro Phe Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1307
<211> 34
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1307
Lys Ser Met Gln Xaa Pro Phe Ser Arg Xaa Xaa Phe Xaa Phe Ala Glu
1               5                   10                  15


Gln Xaa Xaa Xaa Xaa Thr Val Gly Trp Val Glu Arg His Arg Lys Met
            20                  25                  30


Leu Arg



<210> 1308
<211> 34
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see

specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1308
Lys Ser Met Gln Val Xaa Phe Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1309
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups

and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1309
Lys Ser Met Gln Val Pro Xaa Ser Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1310
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1310
Lys Ser Met Gln Val Pro Phe Xaa Arg Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1311
<211> 34
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1311
Lys Ser Met Gln Val Pro Phe Ser Xaa Xaa Xaa Phe Xaa Phe Ala Glu
1               5                   10                  15

Gln Xaa Xaa Xaa Xaa Thr Val Gly Trp Val Glu Arg His Arg Lys Met
            20                  25                  30

Leu Arg

<210> 1312
<211> 34
<212> PRT
<213> Artificial Sequence

<220>
<223> Description of Artificial Sequence: Synthetic peptide

<220>
<221> MOD_RES
<222> (14)..(14)

<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1312
Lys Ser Met Gln Val Pro Phe Ser Arg Cys Cys Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1313
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1313
Lys Ser Met Gln Val Pro Phe Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1314
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1314
Xaa Ser Met Gln Val Pro Phe Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1315
<211> 34
<212> PRT
<213> Artificial Sequence

<220>
<223> Description of Artificial Sequence: Synthetic peptide

<220>

<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1315
Lys Xaa Met Gln Val Pro Phe Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1316
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES

<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1316
Lys Ser Xaa Gln Val Pro Phe Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1317
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1317
Lys Ser Met Xaa Val Pro Phe Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1318
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1318
Lys Ser Met Gln Xaa Pro Phe Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1319
<211> 34

<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1319
Lys Ser Met Gln Val Xaa Phe Ser Arg Xaa Xaa Phe Ser Xaa Ala Glu
1               5                   10                  15


Gln Xaa Xaa Xaa Xaa Thr Val Gly Trp Val Glu Arg His Arg Lys Met
            20                  25                  30


Leu Arg




<210> 1320
<211> 34
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>

<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1320
Lys Ser Met Gln Val Pro Xaa Ser Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1321
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES

<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1321
Lys Ser Met Gln Val Pro Phe Xaa Arg Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1322
<211> 34
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1322
Lys Ser Met Gln Val Pro Phe Ser Xaa Xaa Xaa Phe Ser Xaa Ala Glu
1               5                   10                  15

Gln Xaa Xaa Xaa Xaa Thr Val Gly Trp Val Glu Arg His Arg Lys Met
            20                  25                  30

Leu Arg

<210> 1323

<211> 34
<212> PRT
<213> Artificial Sequence

<220>
<223> Description of Artificial Sequence: Synthetic peptide

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1323
Lys Ser Met Gln Val Pro Phe Ser Arg Cys Cys Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1324
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1324
Lys Ser Met Gln Val Pro Phe Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1325
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1325
Xaa Ser Met Gln Val Pro Phe Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1326
<211> 34
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1326
Lys Xaa Met Gln Val Pro Phe Ser Arg Xaa Xaa Phe Ser Phe Xaa Glu
1               5                   10                  15


Gln Xaa Xaa Xaa Xaa Thr Val Gly Trp Val Glu Arg His Arg Lys Met
            20                  25                  30


Leu Arg




<210> 1327
<211> 34
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups

and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1327
Lys Ser Xaa Gln Val Pro Phe Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1328
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1328
Lys Ser Met Xaa Val Pro Phe Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1329
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1329
Lys Ser Met Gln Xaa Pro Phe Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1330
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1330
Lys Ser Met Gln Val Xaa Phe Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1331
<211> 34
<212> PRT
<213> Artificial Sequence

<220>

<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1331
Lys Ser Met Gln Val Pro Xaa Ser Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1332
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups

and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1332
Lys Ser Met Gln Val Pro Phe Xaa Arg Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1333
<211> 34
<212> PRT
<213> Artificial Sequence

<220>
<223> Description of Artificial Sequence: Synthetic peptide

<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (15)..(15)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1333
Lys Ser Met Gln Val Pro Phe Ser Xaa Xaa Xaa Phe Ser Phe Xaa Glu
1               5                   10                  15

Gln Xaa Xaa Xaa Xaa Thr Val Gly Trp Val Glu Arg His Arg Lys Met
            20                  25                  30

Leu Arg

<210> 1334
<211> 34
<212> PRT
<213> Artificial Sequence

<220>
<223> Description of Artificial Sequence: Synthetic peptide

<220>
<221> MOD_RES
<222> (16)..(16)
<223> Any L- or D-natural amino acid and any non-natural amino acid; see specification as filed for detailed description of substitutions, R-groups and preferred embodiments

<220>
<221> MOD_RES
<222> (18)..(21)
<223> Any L- or D-natural or non-natural amino acid, and this range may encompass 1 or 4 residues; see specification as filed for detailed description of substitutions, R-groups and preferred embodiments

<220>
<223> C-term may be amidated

<400> 1334
Lys Ser Met Gln Val Pro Phe Ser Arg Cys Cys Phe Ser Phe Ala Xaa
1               5                   10                  15

Gln Xaa Xaa Xaa Xaa Thr Val Gly Trp Val Glu Arg His Arg Lys Met
            20                  25                  30

Leu Arg

<210> 1335
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see specification as filed for detailed description of substitutions, R-groups and preferred embodiments

<220>
<221> MOD_RES
<222> (16)..(16)

<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1335
Lys Ser Met Gln Val Pro Phe Ser Arg Xaa Xaa Phe Ser Phe Ala Xaa
1               5                   10                  15

Gln Xaa Xaa Xaa Xaa Thr Val Gly Trp Val Glu Arg His Arg Lys Met
            20                  25                  30

Leu Arg

<210> 1336
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1336

Xaa Ser Met Gln Val Pro Phe Ser Arg Xaa Xaa Phe Ser Phe Ala Xaa
1               5                   10                  15

Gln Xaa Xaa Xaa Xaa Thr Val Gly Trp Val Glu Arg His Arg Lys Met
            20                  25                  30

Leu Arg

<210> 1337
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1337
Lys Xaa Met Gln Val Pro Phe Ser Arg Xaa Xaa Phe Ser Phe Ala Xaa
1               5                   10                  15

Gln Xaa Xaa Xaa Xaa Thr Val Gly Trp Val Glu Arg His Arg Lys Met
            20                  25                  30

Leu Arg

<210> 1338
<211> 34
<212> PRT

<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1338
Lys Ser Xaa Gln Val Pro Phe Ser Arg Xaa Xaa Phe Ser Phe Ala Xaa
1               5                   10                  15

Gln Xaa Xaa Xaa Xaa Thr Val Gly Trp Val Glu Arg His Arg Lys Met
            20                  25                  30

Leu Arg

<210> 1339
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES

<222> (10)..(11)
<223> Any L- or D-natural amino acid and any non-natural amino acid; see
specification as filed for detailed description of substitutions, R-groups
and preferred embodiments

<220>
<221> MOD_RES
<222> (16)..(16)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1339
Lys Ser Met Xaa Val Pro Phe Ser Arg Xaa Xaa Phe Ser Phe Ala Xaa
1               5                   10                  15

Gln Xaa Xaa Xaa Xaa Thr Val Gly Trp Val Glu Arg His Arg Lys Met
            20                  25                  30

Leu Arg

<210> 1340
<211> 34
<212> PRT
<213> Artificial Sequence

<220>
<223> Description of Artificial Sequence: Synthetic peptide

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1340
Lys Ser Met Gln Val Pro Phe Ser Arg Cys Cys Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1341
<211> 34
<212> PRT
<213> Artificial Sequence

<220>
<223> Description of Artificial Sequence: Synthetic peptide

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1341
Lys Ser Met Gln Val Pro Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1342
<211> 34
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1342
Xaa Ser Met Gln Val Pro Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1343
<211> 34
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>

<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1343
Lys Xaa Met Gln Val Pro Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1344
<211> 34
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1344
Lys Ser Xaa Gln Val Pro Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met

20                  25                  30

Leu Arg

<210> 1345
<211> 34
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1345
Lys Ser Met Xaa Val Pro Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1346
<211> 34
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1346
Lys Ser Met Gln Xaa Pro Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1347
<211> 34
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1347
Lys Ser Met Gln Val Xaa Phe Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1348
<211> 34
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 1348
Lys Ser Met Gln Val Pro Xaa Ser Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1349
<211> 34
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (10)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1349
Lys Ser Met Gln Val Pro Phe Xaa Arg Xaa Xaa Phe Ser Phe Ala Glu
1               5                   10                  15

Xaa Xaa Xaa Xaa Xaa Thr Val Gly Trp Val Glu Arg His Arg Lys Met
            20                  25                  30

Leu Arg

<210> 1350
<211> 34
<212> PRT
<213> Artificial Sequence

<220>
<223> Description of Artificial Sequence: Synthetic peptide


<220>
<221> MOD_RES
<222> (9)..(11)
<223> Any L- or D-natural amino acid and any non-natural amino acid; see
specification as filed for detailed description of substitutions, R-groups
and preferred embodiments

<220>
<221> MOD_RES
<222> (17)..(17)
<223> Any L- or D-natural amino acid and any non-natural amino acid; see
specification as filed for detailed description of substitutions, R-groups
and preferred embodiments

<220>
<221> MOD_RES
<222> (18)..(21)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1350
Lys Ser Met Gln Val Pro Phe Ser Xaa Xaa Xaa Phe Ser Phe Ala Glu
1               5                   10                  15


Xaa Xaa Xaa Xaa Xaa Thr Val Gly Trp Val Glu Arg His Arg Lys Met
            20                  25                  30


Leu Arg




<210> 1351
<211> 35
<212> PRT
<213> Artificial Sequence

<220>
<223> Description of Artificial Sequence: Synthetic peptide

<220>
<223> C-term may be amidated

<400> 1351
Gln Pro Asp Ala Ile Asn Ala Pro Val Thr Cys Cys Tyr Asn Phe Thr
1               5                   10                  15


Asn Arg Lys Ile Ser Val Gln Arg Leu Ala Ser Tyr Arg Arg Ile Thr
            20                  25                  30


Ser Ser Lys
        35

<210> 1352
<211> 35
<212> PRT
<213> Artificial Sequence

<220>
<223> Description of Artificial Sequence: Synthetic peptide

<220>
<221> MOD_RES
<222> (11)..(12)
<223> Any L- or D-natural amino acid and any non-natural amino acid; see specification as filed for detailed description of substitutions, R-groups and preferred embodiments

<220>
<223> c-term may be amidated

<400> 1352
Gln Pro Asp Ala Ile Asn Ala Pro Val Thr Xaa Xaa Tyr Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1353
<211> 35
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see specification as filed for detailed description of substitutions, R-groups and preferred embodiments

<220>
<221> MOD_RES
<222> (11)..(12)
<223> Any L- or D-natural amino acid and any non-natural amino acid; see specification as filed for detailed description of substitutions, R-groups and preferred embodiments

<220>
<223> c-term may be amidated

<400> 1353
Xaa Pro Asp Ala Ile Asn Ala Pro Val Thr Xaa Xaa Tyr Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35


<210> 1354
<211> 35
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> C-term may be amidated

<400> 1354
Gln Xaa Asp Ala Ile Asn Ala Pro Val Thr Xaa Xaa Tyr Asn Phe Thr
1               5                   10                  15


Asn Arg Lys Ile Ser Val Gln Arg Leu Ala Ser Tyr Arg Arg Ile Thr
            20                  25                  30


Ser Ser Lys
        35


<210> 1355
<211> 35
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>

<223> c-term may be amidated

<400> 1355
Gln Pro Xaa Ala Ile Asn Ala Pro Val Thr Xaa Xaa Tyr Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1356
<211> 35
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> c-term may be amidated

<400> 1356
Gln Pro Asp Xaa Ile Asn Ala Pro Val Thr Xaa Xaa Tyr Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1357
<211> 35
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups

and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> c-term may be amidated

<400> 1357
Gln Pro Asp Ala Xaa Asn Ala Pro Val Thr Xaa Xaa Tyr Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1358
<211> 35
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> c-term may be amidated

<400> 1358
Gln Pro Asp Ala Ile Xaa Ala Pro Val Thr Xaa Xaa Tyr Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1359
<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> c-term may be amidated

<400> 1359
Gln Pro Asp Ala Ile Asn Xaa Pro Val Thr Xaa Xaa Tyr Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1360
<211> 35
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> c-term may be amidated

<400> 1360
Gln Pro Asp Ala Ile Asn Ala Xaa Val Thr Xaa Xaa Tyr Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1361
<211> 35
<212> PRT
<213> Artificial Sequence

<220>
<223> Description of Artificial Sequence: Synthetic peptide

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> C-term may be amidated

<400> 1361
Gln Pro Asp Ala Ile Asn Ala Pro Xaa Thr Xaa Xaa Tyr Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1362
<211> 35
<212> PRT
<213> Artificial Sequence

<220>
<223> Description of Artificial Sequence: Synthetic peptide

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<223> C-term may be amidated

<400> 1362
Gln Pro Asp Ala Ile Asn Ala Pro Val Xaa Xaa Xaa Tyr Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1363
<211> 35
<212> PRT
<213> Artificial Sequence

<220>
<223> Description of Artificial Sequence: Synthetic peptide

<220>
<223> c-term may be amidated

<400> 1363
Gln Pro Asp Ala Ile Asn Ala Pro Val Thr Cys Cys Glu Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1364
<211> 35
<212> PRT
<213> Artificial Sequence

<220>
<223> Description of Artificial Sequence: Synthetic peptide

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> c-term may be amidated

<400> 1364
Gln Pro Asp Ala Ile Asn Ala Pro Val Thr Xaa Xaa Glu Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1365
<211> 35
<212> PRT

<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> C-term may be amidated

<400> 1365
Xaa Pro Asp Ala Ile Asn Ala Pro Val Thr Xaa Xaa Glu Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1366
<211> 35
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> C-term may be amidated

<400> 1366
Gln Xaa Asp Ala Ile Asn Ala Pro Val Thr Xaa Xaa Glu Asn Phe Thr
1               5                   10                  15

Asn Arg Lys Ile Ser Val Gln Arg Leu Ala Ser Tyr Arg Arg Ile Thr

20                  25                  30

Ser Ser Lys
        35

<210> 1367
<211> 35
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> C-term may be amidated

<400> 1367
Gln Pro Xaa Ala Ile Asn Ala Pro Val Thr Xaa Xaa Glu Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1368
<211> 35
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> c-term may be amidated

<400> 1368
Gln Pro Asp Xaa Ile Asn Ala Pro Val Thr Xaa Xaa Glu Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1369
<211> 35
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> c-term may be amidated

<400> 1369
Gln Pro Asp Ala Xaa Asn Ala Pro Val Thr Xaa Xaa Glu Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1370
<211> 35
<212> PRT
<213> Artificial Sequence

<220>
<223> Description of Artificial Sequence: Synthetic peptide

<220>
<221> MOD_RES
<222> (6)..(6)

<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> c-term may be amidated

<400> 1370
Gln Pro Asp Ala Ile Xaa Ala Pro Val Thr Xaa Xaa Glu Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1371
<211> 35
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> c-term may be amidated

<400> 1371
Gln Pro Asp Ala Ile Asn Xaa Pro Val Thr Xaa Xaa Glu Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1372
<211> 35

<212> PRT
<213> Artificial Sequence

<220>
<223> Description of Artificial Sequence: Synthetic peptide


<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> C-term may be amidated

<400> 1372
Gln Pro Asp Ala Ile Asn Ala Xaa Val Thr Xaa Xaa Glu Asn Phe Thr
1               5                   10                  15


Asn Arg Lys Ile Ser Val Gln Arg Leu Ala Ser Tyr Arg Arg Ile Thr
            20                  25                  30


Ser Ser Lys
        35


<210> 1373
<211> 35
<212> PRT
<213> Artificial Sequence

<220>
<223> Description of Artificial Sequence: Synthetic peptide


<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> C-term may be amidated

<400> 1373
Gln Pro Asp Ala Ile Asn Ala Pro Xaa Thr Xaa Xaa Glu Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1374
<211> 35
<212> PRT
<213> Artificial Sequence

<220>
<223> Description of Artificial Sequence: Synthetic peptide

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<223> C-term may be amidated

<400> 1374
Gln Pro Asp Ala Ile Asn Ala Pro Val Xaa Xaa Xaa Glu Asn Phe Thr
1               5                   10                  15

Asn Arg Lys Ile Ser Val Gln Arg Leu Ala Ser Tyr Arg Arg Ile Thr
            20                  25                  30

Ser Ser Lys
        35

<210> 1375
<211> 26
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<223> C-term may be amidated

<400> 1375
Ala Lys Xaa Asn Phe Thr Asn Arg Lys Ile Ser Val Gln Arg Leu Ala
1               5                   10                  15

Ser Tyr Arg Arg Ile Thr Ser Ser Lys Glu
            20                  25

<210> 1376
<211> 15
<212> PRT
<213> Artificial Sequence

<220>
<223> Description of Artificial Sequence: Synthetic peptide

<220>
<223> c-term may be amidated

<400> 1376
Pro Lys Glu Ala Val Ile Phe Lys Thr Ile Val Ala Lys Glu Ile
1               5                   10                  15


<210> 1377
<211> 19
<212> PRT
<213> Artificial Sequence

<220>
<223> Description of Artificial Sequence: Synthetic peptide

<220>
<223> c-term may be amidated

<400> 1377
Ala Asp Pro Lys Gln Lys Trp Val Gln Asp Ser Met Asp His Leu Asp
1               5                   10                  15


Lys Gln Thr



<210> 1378
<211> 41
<212> PRT
<213> Artificial Sequence

<220>
<223> Description of Artificial Sequence: Synthetic peptide


<220>
<221> MOD_RES
<222> (24)..(27)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1378
Tyr Asn Phe Thr Asn Arg Lys Ile Ser Val Gln Arg Leu Ala Ser Tyr
1               5                   10                  15


Arg Arg Ile Thr Ser Ser Lys Xaa Xaa Xaa Xaa Lys Trp Val Gln Asp
            20                  25                  30


Ser Met Asp His Leu Asp Lys Gln Thr
        35                  40

<210> 1379
<211> 32
<212> PRT
<213> Artificial Sequence

<220>
<223> Description of Artificial Sequence: Synthetic peptide


<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1379
Gln Pro Asp Ala Ile Asn Ala Pro Val Thr Cys Cys Tyr Asn Xaa Xaa
1               5                   10                  15


Xaa Xaa Lys Trp Val Gln Asp Ser Met Asp His Leu Asp Lys Gln Thr
            20                  25                  30


<210> 1380
<211> 32
<212> PRT
<213> Artificial Sequence

<220>
<223> Description of Artificial Sequence: Synthetic peptide


<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1380
Gln Pro Asp Ala Ile Asn Ala Pro Val Thr Xaa Xaa Tyr Asn Xaa Xaa
1               5                   10                  15


Xaa Xaa Lys Trp Val Gln Asp Ser Met Asp His Leu Asp Lys Gln Thr
            20                  25                  30


<210> 1381
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1381
Xaa Pro Asp Ala Ile Asn Ala Pro Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1382
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 1382
Gln Xaa Asp Ala Ile Asn Ala Pro Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1383
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1383
Gln Pro Xaa Ala Ile Asn Ala Pro Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1384
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1384
Gln Pro Asp Xaa Ile Asn Ala Pro Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1385
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1385
Gln Pro Asp Ala Xaa Asn Ala Pro Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1386
<211> 32
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1386
Gln Pro Asp Ala Ile Xaa Ala Pro Val Thr Xaa Xaa Tyr Asn Xaa Xaa
1               5                   10                  15


Xaa Xaa Lys Trp Val Gln Asp Ser Met Asp His Leu Asp Lys Gln Thr
            20                  25                  30


<210> 1387
<211> 32
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES

<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1387
Gln Pro Asp Ala Ile Asn Xaa Pro Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1388
<211> 32
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1388
Gln Pro Asp Ala Ile Asn Ala Xaa Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1389
<211> 32
<212> PRT
<213> Artificial Sequence

<220>
<223> Description of Artificial Sequence: Synthetic peptide

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1389
Gln Pro Asp Ala Ile Asn Ala Pro Xaa Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1390
<211> 32
<212> PRT
<213> Artificial Sequence

<220>
<223> Description of Artificial Sequence: Synthetic peptide

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1390
Gln Pro Asp Ala Ile Asn Ala Pro Val Xaa Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1391
<211> 32
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1391
Xaa Pro Asp Ala Ile Asn Ala Pro Val Thr Xaa Xaa Xaa Asn Xaa Xaa
1               5                   10                  15


Xaa Xaa Lys Trp Val Gln Asp Ser Met Asp His Leu Asp Lys Gln Thr
            20                  25                  30


<210> 1392
<211> 32
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1392
Gln Xaa Asp Ala Ile Asn Ala Pro Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1393
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1393
Gln Pro Xaa Ala Ile Asn Ala Pro Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1394
<211> 32
<212> PRT
<213> Artificial Sequence

<220>
<223> Description of Artificial Sequence: Synthetic peptide

<220>

<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1394
Gln Pro Asp Xaa Ile Asn Ala Pro Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1395
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1395
Gln Pro Asp Ala Xaa Asn Ala Pro Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1396
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1396
Gln Pro Asp Ala Ile Xaa Ala Pro Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1397
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1397
Gln Pro Asp Ala Ile Asn Xaa Pro Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1398
<211> 32
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1398
Gln Pro Asp Ala Ile Asn Ala Xaa Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1399
<211> 32
<212> PRT
<213> Artificial Sequence

<220>
<223> Description of Artificial Sequence: Synthetic peptide

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1399
Gln Pro Asp Ala Ile Asn Ala Pro Xaa Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1400
<211> 32
<212> PRT
<213> Artificial Sequence

<220>
<223> Description of Artificial Sequence: Synthetic peptide

<220>
<221> MOD_RES
<222> (10)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1400
Gln Pro Asp Ala Ile Asn Ala Pro Val Xaa Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1401
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1401
Xaa Pro Asp Ala Ile Asn Ala Pro Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1402
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups

and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1402
Gln Xaa Asp Ala Ile Asn Ala Pro Val Thr Xaa Xaa Tyr Xaa Xaa Xaa
1               5                   10                  15


Xaa Xaa Lys Trp Val Gln Asp Ser Met Asp His Leu Asp Lys Gln Thr
            20                  25                  30


<210> 1403
<211> 32
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1403
Gln Pro Xaa Ala Ile Asn Ala Pro Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1404
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1404
Gln Pro Asp Xaa Ile Asn Ala Pro Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1405
<211> 32
<212> PRT

<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1405
Gln Pro Asp Ala Xaa Asn Ala Pro Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1406
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1406
Gln Pro Asp Ala Ile Xaa Ala Pro Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1407
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1407

Gln Pro Asp Ala Ile Asn Xaa Pro Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1408
<211> 32
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1408
Gln Pro Asp Ala Ile Asn Ala Xaa Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1409
<211> 32
<212> PRT
<213> Artificial Sequence

<220>
<223> Description of Artificial Sequence: Synthetic peptide

<220>
<221> MOD_RES

<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1409
Gln Pro Asp Ala Ile Asn Ala Pro Xaa Thr Xaa Xaa Tyr Xaa Xaa Xaa
1               5                   10                  15


Xaa Xaa Lys Trp Val Gln Asp Ser Met Asp His Leu Asp Lys Gln Thr
            20                  25                  30


<210> 1410
<211> 32
<212> PRT
<213> Artificial Sequence

<220>
<223> Description of Artificial Sequence: Synthetic peptide


<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1410
Gln Pro Asp Ala Ile Asn Ala Pro Val Xaa Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1411
<211> 26
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<223> c-term may be amidated

<400> 1411
Ala Lys Xaa Asn Phe Thr Asn Arg Lys Ile Ser Val Gln Arg Leu Ala
1               5                   10                  15

Ser Tyr Arg Arg Ile Thr Ser Ser Lys Glu
            20                  25

<210> 1412
<211> 26
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<223> c-term may be amidated

<400> 1412
Ala Lys Xaa Asn Phe Thr Asn Arg Lys Ile Ser Val Gln Arg Leu Ala
1               5                   10                  15

Ser Tyr Arg Arg Ile Thr Ser Ser Lys Glu
            20                  25

<210> 1413
<211> 35
<212> PRT
<213> Artificial Sequence

<220>
<223> Description of Artificial Sequence: Synthetic peptide


<220>
<221> MOD_RES
<222> (11)..(12)
<223> Any L- or D-natural amino acid and any non-natural amino acid; see
specification as filed for detailed description of substitutions, R-groups
and preferred embodiments

<220>
<223> c-term may be amidated

<400> 1413
Gln Pro Asp Ala Ile Asn Ala Pro Val Thr Xaa Xaa Glu Asn Phe Thr
1               5                   10                  15


Asn Arg Lys Ile Ser Val Gln Arg Leu Ala Ser Tyr Arg Arg Ile Thr
            20                  25                  30


Ser Ser Lys
        35


<210> 1414
<211> 32
<212> PRT
<213> Artificial Sequence

<220>
<223> Description of Artificial Sequence: Synthetic peptide


<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1414
Gln Pro Asp Ala Ile Asn Ala Pro Val Thr Cys Cys Tyr Asn Xaa Xaa
1               5                   10                  15


Xaa Xaa Lys Trp Val Gln Asp Ser Met Asp His Leu Asp Lys Gln Thr
            20                  25                  30


<210> 1415
<211> 32
<212> PRT
<213> Artificial Sequence

<220>
<223> Description of Artificial Sequence: Synthetic peptide

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1415
Gln Pro Asp Ala Ile Asn Ala Pro Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1416
<211> 41
<212> PRT
<213> Artificial Sequence

<220>
<223> Description of Artificial Sequence: Synthetic peptide

<220>
<221> MOD_RES
<222> (24)..(27)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1416
Tyr Asn Phe Thr Asn Arg Lys Ile Ser Val Gln Arg Leu Ala Ser Tyr
1               5                   10                  15

Arg Arg Ile Thr Ser Ser Lys Xaa Xaa Xaa Xaa Lys Trp Val Gln Asp
            20                  25                  30

Ser Met Asp His Leu Asp Lys Gln Thr
        35                  40

<210> 1417
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1417
Xaa Pro Asp Ala Ile Asn Ala Pro Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1418
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1418
Gln Xaa Asp Ala Ile Asn Ala Pro Val Thr Xaa Xaa Tyr Asn Xaa Xaa

1                   5                   10                  15

Xaa Xaa Lys Trp Val Gln Asp Ser Met Asp His Leu Asp Lys Gln Thr
            20                  25                  30

<210> 1419
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1419
Gln Pro Xaa Ala Ile Asn Ala Pro Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1420
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (11)..(12)

<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1420
Gln Pro Asp Xaa Ile Asn Ala Pro Val Thr Xaa Xaa Tyr Asn Xaa Xaa
1               5                   10                  15


Xaa Xaa Lys Trp Val Gln Asp Ser Met Asp His Leu Asp Lys Gln Thr
            20                  25                  30


<210> 1421
<211> 32
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1421
Gln Pro Asp Ala Xaa Asn Ala Pro Val Thr Xaa Xaa Tyr Asn Xaa Xaa
1               5                   10                  15


Xaa Xaa Lys Trp Val Gln Asp Ser Met Asp His Leu Asp Lys Gln Thr
            20                  25                  30


<210> 1422
<211> 32
<212> PRT

<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1422
Gln Pro Asp Ala Ile Xaa Ala Pro Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1423
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1423
Gln Pro Asp Ala Ile Asn Xaa Pro Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1424
<211> 32
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1424
Gln Pro Asp Ala Ile Asn Ala Xaa Val Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1425
<211> 32
<212> PRT
<213> Artificial Sequence

<220>
<223> Description of Artificial Sequence: Synthetic peptide

<220>
<221> MOD_RES
<222> (9)..(9)
<223> Any L- or D-natural amino acid and any non-natural amino acid; see

specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1425
Gln Pro Asp Ala Ile Asn Ala Pro Xaa Thr Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1426
<211> 32
<212> PRT
<213> Artificial Sequence

<220>
<223> Description of Artificial Sequence: Synthetic peptide

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1426
Gln Pro Asp Ala Ile Asn Ala Pro Val Xaa Xaa Xaa Tyr Asn Xaa Xaa
1               5                   10                  15

Xaa Xaa Lys Trp Val Gln Asp Ser Met Asp His Leu Asp Lys Gln Thr
            20                  25                  30

<210> 1427
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1427
Xaa Pro Asp Ala Ile Asn Ala Pro Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1428
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1428
Gln Xaa Asp Ala Ile Asn Ala Pro Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1429
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1429
Gln Pro Xaa Ala Ile Asn Ala Pro Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1430
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups

and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1430
Gln Pro Asp Xaa Ile Asn Ala Pro Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1431
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1431
Gln Pro Asp Ala Xaa Asn Ala Pro Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1432
<211> 32
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1432
Gln Pro Asp Ala Ile Xaa Ala Pro Val Thr Xaa Xaa Xaa Asn Xaa Xaa
1               5                   10                  15


Xaa Xaa Lys Trp Val Gln Asp Ser Met Asp His Leu Asp Lys Gln Thr
            20                  25                  30


<210> 1433
<211> 32
<212> PRT
<213> Artificial Sequence

<220>
<223> Description of Artificial Sequence: Synthetic peptide


<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>

<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1433
Gln Pro Asp Ala Ile Asn Xaa Pro Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1434
<211> 32
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1434
Gln Pro Asp Ala Ile Asn Ala Xaa Val Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1435
<211> 32
<212> PRT
<213> Artificial Sequence

<220>
<223> Description of Artificial Sequence: Synthetic peptide

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1435
Gln Pro Asp Ala Ile Asn Ala Pro Xaa Thr Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1436
<211> 32
<212> PRT
<213> Artificial Sequence

<220>
<223> Description of Artificial Sequence: Synthetic peptide

<220>
<221> MOD_RES
<222> (10)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1436
Gln Pro Asp Ala Ile Asn Ala Pro Val Xaa Xaa Xaa Xaa Asn Xaa Xaa
1               5                   10                  15

Xaa Xaa Lys Trp Val Gln Asp Ser Met Asp His Leu Asp Lys Gln Thr
            20                  25                  30

<210> 1437
<211> 32
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1437
Xaa Pro Asp Ala Ile Asn Ala Pro Val Thr Xaa Xaa Tyr Xaa Xaa Xaa
1               5                   10                  15


Xaa Xaa Lys Trp Val Gln Asp Ser Met Asp His Leu Asp Lys Gln Thr
            20                  25                  30


<210> 1438
<211> 32
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES

<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1438
Gln Xaa Asp Ala Ile Asn Ala Pro Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1439
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1439
Gln Pro Xaa Ala Ile Asn Ala Pro Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1440
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1440
Gln Pro Asp Xaa Ile Asn Ala Pro Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1441
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1441
Gln Pro Asp Ala Xaa Asn Ala Pro Val Thr Xaa Xaa Tyr Xaa Xaa Xaa
1               5                   10                  15


Xaa Xaa Lys Trp Val Gln Asp Ser Met Asp His Leu Asp Lys Gln Thr
            20                  25                  30


<210> 1442
<211> 32
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)

<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1442
Gln Pro Asp Ala Ile Xaa Ala Pro Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1443
<211> 32
<212> PRT
<213> Artificial Sequence

<220>
<223> Description of Artificial Sequence: Synthetic peptide

<220>
<221> MOD_RES
<222> (7)..(7)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1443
Gln Pro Asp Ala Ile Asn Xaa Pro Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1444
<211> 32
<212> PRT
<213> Artificial Sequence

<220>
<223> Description of Artificial Sequence: Synthetic peptide

<220>
<221> MOD_RES
<222> (8)..(8)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1444
Gln Pro Asp Ala Ile Asn Ala Xaa Val Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1445
<211> 32
<212> PRT
<213> Artificial Sequence

<220>
<223> Description of Artificial Sequence: Synthetic peptide

<220>
<221> MOD_RES
<222> (9)..(9)
<223> Any L- or D-natural amino acid and any non-natural amino acid; see
specification as filed for detailed description of substitutions, R-groups
and preferred embodiments

<220>
<221> MOD_RES
<222> (11)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1445
Gln Pro Asp Ala Ile Asn Ala Pro Xaa Thr Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1446
<211> 32
<212> PRT
<213> Artificial Sequence

<220>
<223> Description of Artificial Sequence: Synthetic peptide

<220>
<221> MOD_RES
<222> (10)..(12)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1446

Gln Pro Asp Ala Ile Asn Ala Pro Val Xaa Xaa Xaa Tyr Xaa Xaa Xaa
1               5                   10                  15

Xaa Xaa Lys Trp Val Gln Asp Ser Met Asp His Leu Asp Lys Gln Thr
            20                  25                  30

<210> 1447
<211> 7
<212> PRT
<213> Artificial Sequence

<220>
<223> Description of Artificial Sequence: Synthetic peptide

<220>
<223> c-term may be amidated

<400> 1447
Ile Leu Pro Ile Ala Ser Ser
1               5

<210> 1448
<211> 26
<212> PRT
<213> Artificial Sequence

<220>
<223> Description of Artificial Sequence: Synthetic peptide

<220>
<223> c-term may be amidated

<400> 1448
Ile Leu Pro Ile Ala Ser Ser Cys Cys Thr Glu Val Ser His His Ile
1               5                   10                  15

Ser Arg Arg Leu Leu Glu Arg Val Asn Met
            20                  25

<210> 1449
<211> 26
<212> PRT
<213> Artificial Sequence

<220>
<223> Description of Artificial Sequence: Synthetic peptide

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<223> c-term may be amidated

<400> 1449
Ile Leu Pro Ile Ala Ser Ser Xaa Xaa Thr Glu Val Ser His His Ile
1               5                   10                  15

Ser Arg Arg Leu Leu Glu Arg Val Asn Met
            20                  25

<210> 1450
<211> 26
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<223> C-term may be amidated

<400> 1450
Xaa Leu Pro Ile Ala Ser Ser Xaa Xaa Thr Glu Val Ser His His Ile
1               5                   10                  15

Ser Arg Arg Leu Leu Glu Arg Val Asn Met
            20                  25

<210> 1451
<211> 26
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<223> C-term may be amidated

<400> 1451
Ile Xaa Pro Ile Ala Ser Ser Xaa Xaa Thr Glu Val Ser His His Ile
1               5                   10                  15

Ser Arg Arg Leu Leu Glu Arg Val Asn Met
            20                  25


<210> 1452
<211> 26
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<223> C-term may be amidated

<400> 1452
Ile Leu Xaa Ile Ala Ser Ser Xaa Xaa Thr Glu Val Ser His His Ile
1               5                   10                  15


Ser Arg Arg Leu Leu Glu Arg Val Asn Met
            20                  25


<210> 1453
<211> 26
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<223> C-term may be amidated

<400> 1453

Ile Leu Pro Xaa Ala Ser Ser Xaa Xaa Thr Glu Val Ser His His Ile
1               5                   10                  15

Ser Arg Arg Leu Leu Glu Arg Val Asn Met
            20                  25

<210> 1454
<211> 26
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<223> C-term may be amidated

<400> 1454
Ile Leu Pro Ile Xaa Ser Ser Xaa Xaa Thr Glu Val Ser His His Ile
1               5                   10                  15

Ser Arg Arg Leu Leu Glu Arg Val Asn Met
            20                  25

<210> 1455
<211> 26
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<223> C-term may be amidated

<400> 1455
Ile Leu Pro Ile Ala Xaa Ser Xaa Xaa Thr Glu Val Ser His His Ile
1               5                   10                  15

Ser Arg Arg Leu Leu Glu Arg Val Asn Met
            20                  25

<210> 1456
<211> 26
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<223> C-term may be amidated

<400> 1456
Ile Leu Pro Ile Ala Ser Xaa Xaa Xaa Thr Glu Val Ser His His Ile
1               5                   10                  15

Ser Arg Arg Leu Leu Glu Arg Val Asn Met
            20                  25

<210> 1457
<211> 17
<212> PRT
<213> Artificial Sequence

<220>
<223> Description of Artificial Sequence: Synthetic peptide

<220>
<223> C-term may be amidated

<400> 1457
Thr Glu Val Ser His His Ile Ser Arg Arg Leu Leu Glu Arg Val Asn
1               5                   10                  15

Met

<210> 1458
<211> 23
<212> PRT
<213> Artificial Sequence

<220>
<223> Description of Artificial Sequence: Synthetic peptide

<220>
<223> C-term may be amidated

<400> 1458
Arg Ile Gln Arg Ala Asp Gly Asp Cys Asp Leu Ala Ala Val Ile Leu
1               5                   10                  15

His Val Lys Arg Arg Arg Ile
            20

<210> 1459
<211> 25
<212> PRT
<213> Artificial Sequence

<220>
<223> Description of Artificial Sequence: Synthetic peptide

<220>
<223> c-term may be amidated

<400> 1459
Val Ser Pro His Asn His Thr Val Lys Gln Trp Met Lys Val Gln Ala
1               5                   10                  15

Ala Lys Lys Asn Gly Lys Gly Asn Val
            20                  25

<210> 1460
<211> 28
<212> PRT
<213> Artificial Sequence

<220>
<223> Description of Artificial Sequence: Synthetic peptide

<220>
<223> c-term may be amidated

<400> 1460
His Arg Lys Lys His His Gly Lys Arg Asn Ser Asn Arg Ala His Gln
1               5                   10                  15

Gly Lys His Glu Thr Tyr Gly His Lys Thr Pro Tyr
            20                  25

<210> 1461
<211> 32
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1461
Ile Leu Pro Ile Ala Ser Ser Cys Cys Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1462
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1462
Xaa Leu Pro Ile Ala Ser Ser Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1463
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1463
Ile Xaa Pro Ile Ala Ser Ser Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1464
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1464
Ile Leu Xaa Ile Ala Ser Ser Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1465
<211> 32
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1465
Ile Leu Pro Xaa Ala Ser Ser Xaa Xaa Thr Glu Val Ser His Xaa Xaa
1               5                   10                  15


Xaa Xaa His Arg Lys Lys His His Gly Lys Arg Asn Ser Asp Arg Ala
            20                  25                  30


<210> 1466
<211> 32
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1466
Ile Leu Pro Ile Xaa Ser Ser Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1467
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1467
Ile Leu Pro Ile Ala Xaa Ser Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1468
<211> 32
<212> PRT
<213> Artificial Sequence

<220>
<223> Description of Artificial Sequence: Synthetic peptide

<220>

<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1468
Ile Leu Pro Ile Ala Ser Xaa Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1469
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1469
Xaa Leu Pro Ile Ala Ser Ser Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1470

<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1470
Ile Xaa Pro Ile Ala Ser Ser Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1471
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 1471
Ile Leu Xaa Ile Ala Ser Ser Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1472
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1472
Ile Leu Pro Xaa Ala Ser Ser Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1473
<211> 32
<212> PRT
<213> Artificial Sequence

<220>
<223> Description of Artificial Sequence: Synthetic peptide

<220>
<221> MOD_RES

<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1473
Ile Leu Pro Ile Xaa Ser Ser Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1474
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1474
Ile Leu Pro Ile Ala Xaa Ser Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1475
<211> 32
<212> PRT
<213> Artificial Sequence

<220>
<223> Description of Artificial Sequence: Synthetic peptide

<220>
<221> MOD_RES
<222> (7)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1475
Ile Leu Pro Ile Ala Ser Xaa Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1476
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups

and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1476
Xaa Leu Pro Ile Ala Ser Ser Xaa Xaa Thr Xaa Val Ser His Xaa Xaa
1               5                   10                  15

Xaa Xaa His Arg Lys Lys His His Gly Lys Arg Asn Ser Asp Arg Ala
            20                  25                  30

<210> 1477
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1477
Ile Xaa Pro Ile Ala Ser Ser Xaa Xaa Thr Xaa Val Ser His Xaa Xaa
1               5                   10                  15

Xaa Xaa His Arg Lys Lys His His Gly Lys Arg Asn Ser Asp Arg Ala
            20                  25                  30

<210> 1478
<211> 32
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1478
Ile Leu Xaa Ile Ala Ser Ser Xaa Xaa Thr Xaa Val Ser His Xaa Xaa
1               5                   10                  15


Xaa Xaa His Arg Lys Lys His His Gly Lys Arg Asn Ser Asp Arg Ala
            20                  25                  30


<210> 1479
<211> 32
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>

<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1479
Ile Leu Pro Xaa Ala Ser Ser Xaa Xaa Thr Xaa Val Ser His Xaa Xaa
1               5                   10                  15

Xaa Xaa His Arg Lys Lys His His Gly Lys Arg Asn Ser Asp Arg Ala
            20                  25                  30

<210> 1480
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1480
Ile Leu Pro Ile Xaa Ser Ser Xaa Xaa Thr Xaa Val Ser His Xaa Xaa
1               5                   10                  15

Xaa Xaa His Arg Lys Lys His His Gly Lys Arg Asn Ser Asp Arg Ala
            20                  25                  30

<210> 1481
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1481
Ile Leu Pro Ile Ala Xaa Ser Xaa Xaa Thr Xaa Val Ser His Xaa Xaa
1               5                   10                  15

Xaa Xaa His Arg Lys Lys His His Gly Lys Arg Asn Ser Asp Arg Ala
            20                  25                  30

<210> 1482
<211> 32
<212> PRT
<213> Artificial Sequence

<220>

<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1482
Ile Leu Pro Ile Ala Ser Xaa Xaa Xaa Thr Xaa Val Ser His Xaa Xaa
1               5                   10                  15

Xaa Xaa His Arg Lys Lys His His Gly Lys Arg Asn Ser Asp Arg Ala
            20                  25                  30

<210> 1483
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES

<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1483
Xaa Leu Pro Ile Ala Ser Ser Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1484
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1484
Ile Xaa Pro Ile Ala Ser Ser Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1485
<211> 32

<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1485
Ile Leu Xaa Ile Ala Ser Ser Xaa Xaa Thr Glu Xaa Ser His Xaa Xaa
1               5                   10                  15


Xaa Xaa His Arg Lys Lys His His Gly Lys Arg Asn Ser Asp Arg Ala
            20                  25                  30


<210> 1486
<211> 32
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups

and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1486
Ile Leu Pro Xaa Ala Ser Ser Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1487
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1487
Ile Leu Pro Ile Xaa Ser Ser Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1488
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1488
Ile Leu Pro Ile Ala Xaa Ser Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1489
<211> 32
<212> PRT
<213> Artificial Sequence

<220>
<223> Description of Artificial Sequence: Synthetic peptide

<220>

<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1489
Ile Leu Pro Ile Ala Ser Xaa Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1490
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1490
Xaa Leu Pro Ile Ala Ser Ser Xaa Xaa Thr Glu Val Xaa His Xaa Xaa
1               5                   10                  15

Xaa Xaa His Arg Lys Lys His His Gly Lys Arg Asn Ser Asp Arg Ala
            20                  25                  30

<210> 1491
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1491
Ile Xaa Pro Ile Ala Ser Ser Xaa Xaa Thr Glu Val Xaa His Xaa Xaa
1               5                   10                  15

Xaa Xaa His Arg Lys Lys His His Gly Lys Arg Asn Ser Asp Arg Ala
            20                  25                  30

<210> 1492
<211> 32
<212> PRT
<213> Artificial Sequence

<220>

<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1492
Ile Leu Xaa Ile Ala Ser Ser Xaa Xaa Thr Glu Val Xaa His Xaa Xaa
1               5                   10                  15

Xaa Xaa His Arg Lys Lys His His Gly Lys Arg Asn Ser Asp Arg Ala
            20                  25                  30

<210> 1493
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES

<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1493
Ile Leu Pro Xaa Ala Ser Ser Xaa Xaa Thr Glu Val Xaa His Xaa Xaa
1               5                   10                  15

Xaa Xaa His Arg Lys Lys His His Gly Lys Arg Asn Ser Asp Arg Ala
            20                  25                  30

<210> 1494
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1494
Ile Leu Pro Ile Xaa Ser Ser Xaa Xaa Thr Glu Val Xaa His Xaa Xaa
1               5                   10                  15

Xaa Xaa His Arg Lys Lys His His Gly Lys Arg Asn Ser Asp Arg Ala
            20                  25                  30

<210> 1495
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1495
Ile Leu Pro Ile Ala Xaa Ser Xaa Xaa Thr Glu Val Xaa His Xaa Xaa
1               5                   10                  15

Xaa Xaa His Arg Lys Lys His His Gly Lys Arg Asn Ser Asp Arg Ala
            20                  25                  30

<210> 1496
<211> 32
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups

and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1496
Ile Leu Pro Ile Ala Ser Xaa Xaa Xaa Thr Glu Val Xaa His Xaa Xaa
1               5                   10                  15


Xaa Xaa His Arg Lys Lys His His Gly Lys Arg Asn Ser Asp Arg Ala
            20                  25                  30


<210> 1497
<211> 32
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1497
Xaa Leu Pro Ile Ala Ser Ser Xaa Xaa Thr Glu Val Ser Xaa Xaa Xaa
1               5                   10                  15

Xaa Xaa His Arg Lys Lys His His Gly Lys Arg Asn Ser Asp Arg Ala
            20                  25                  30

<210> 1498
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1498
Ile Xaa Pro Ile Ala Ser Ser Xaa Xaa Thr Glu Val Ser Xaa Xaa Xaa
1               5                   10                  15

Xaa Xaa His Arg Lys Lys His His Gly Lys Arg Asn Ser Asp Arg Ala
            20                  25                  30

<210> 1499
<211> 32
<212> PRT
<213> Artificial Sequence

<220>
<223> Description of Artificial Sequence: Synthetic peptide

<220>

<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1499
Ile Leu Xaa Ile Ala Ser Ser Xaa Xaa Thr Glu Val Ser Xaa Xaa Xaa
1               5                   10                  15

Xaa Xaa His Arg Lys Lys His His Gly Lys Arg Asn Ser Asp Arg Ala
            20                  25                  30

<210> 1500
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1500
Ile Leu Pro Xaa Ala Ser Ser Xaa Xaa Thr Glu Val Ser Xaa Xaa Xaa
1               5                   10                  15

Xaa Xaa His Arg Lys Lys His His Gly Lys Arg Asn Ser Asp Arg Ala
            20                  25                  30

<210> 1501
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1501
Ile Leu Pro Ile Xaa Ser Ser Xaa Xaa Thr Glu Val Ser Xaa Xaa Xaa
1               5                   10                  15

Xaa Xaa His Arg Lys Lys His His Gly Lys Arg Asn Ser Asp Arg Ala
            20                  25                  30

<210> 1502
<211> 32
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1502
Ile Leu Pro Ile Ala Xaa Ser Xaa Xaa Thr Glu Val Ser Xaa Xaa Xaa
1               5                   10                  15


Xaa Xaa His Arg Lys Lys His His Gly Lys Arg Asn Ser Asp Arg Ala
            20                  25                  30


<210> 1503
<211> 32
<212> PRT
<213> Artificial Sequence

<220>
<223> Description of Artificial Sequence: Synthetic peptide


<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES

<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1503
Ile Leu Pro Ile Ala Ser Xaa Xaa Xaa Thr Glu Val Ser Xaa Xaa Xaa
1               5                   10                  15

Xaa Xaa His Arg Lys Lys His His Gly Lys Arg Asn Ser Asp Arg Ala
            20                  25                  30

<210> 1504
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1504
Xaa Leu Pro Ile Ala Ser Ser Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1505
<211> 33
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1505
Ile Xaa Pro Ile Ala Ser Ser Xaa Xaa Thr Glu Val Ser His Xaa Xaa
1               5                   10                  15


Xaa Xaa Ala His Gln Gly Lys His Glu Thr Tyr Gly His Lys Thr Pro
            20                  25                  30


Tyr




<210> 1506
<211> 33
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1506
Ile Leu Xaa Ile Ala Ser Ser Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1507
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1507
Ile Leu Pro Xaa Ala Ser Ser Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1508
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1508
Ile Leu Pro Ile Xaa Ser Ser Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1509
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES

<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1509
Ile Leu Pro Ile Ala Xaa Ser Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1510
<211> 33
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1510
Ile Leu Pro Ile Ala Ser Xaa Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1511

<211> 33
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1511
Xaa Leu Pro Ile Ala Ser Ser Xaa Xaa Xaa Glu Val Ser His Xaa Xaa
1               5                   10                  15


Xaa Xaa Ala His Gln Gly Lys His Glu Thr Tyr Gly His Lys Thr Pro
            20                  25                  30


Tyr



<210> 1512
<211> 33
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1512
Ile Xaa Pro Ile Ala Ser Ser Xaa Xaa Xaa Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1513
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1513
Ile Leu Xaa Ile Ala Ser Ser Xaa Xaa Xaa Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1514
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1514
Ile Leu Pro Xaa Ala Ser Ser Xaa Xaa Xaa Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1515
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups

and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1515
Ile Leu Pro Ile Xaa Ser Ser Xaa Xaa Xaa Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1516
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1516
Ile Leu Pro Ile Ala Xaa Ser Xaa Xaa Xaa Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1517
<211> 33
<212> PRT
<213> Artificial Sequence

<220>
<223> Description of Artificial Sequence: Synthetic peptide


<220>
<221> MOD_RES
<222> (7)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1517
Ile Leu Pro Ile Ala Ser Xaa Xaa Xaa Xaa Glu Val Ser His Xaa Xaa
1               5                   10                  15


Xaa Xaa Ala His Gln Gly Lys His Glu Thr Tyr Gly His Lys Thr Pro
            20                  25                  30


Tyr




<210> 1518
<211> 33
<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)

<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1518
Xaa Leu Pro Ile Ala Ser Ser Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1519
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1519

Ile Xaa Pro Ile Ala Ser Ser Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1520
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1520
Ile Leu Xaa Ile Ala Ser Ser Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1521
<211> 33
<212> PRT

<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1521
Ile Leu Pro Xaa Ala Ser Ser Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1522
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES

<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1522
Ile Leu Pro Ile Xaa Ser Ser Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1523
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)

<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1523
Ile Leu Pro Ile Ala Xaa Ser Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1524
<211> 33
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1524
Ile Leu Pro Ile Ala Ser Xaa Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1525
<211> 33

<212> PRT
<213> Artificial Sequence

<220>
<223> Description of Artificial Sequence: Synthetic peptide


<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1525
Xaa Leu Pro Ile Ala Ser Ser Xaa Xaa Thr Glu Xaa Ser His Xaa Xaa
1               5                   10                  15


Xaa Xaa Ala His Gln Gly Lys His Glu Thr Tyr Gly His Lys Thr Pro
            20                  25                  30


Tyr




<210> 1526
<211> 33
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>

<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1526
Ile Xaa Pro Ile Ala Ser Ser Xaa Xaa Thr Glu Xaa Ser His Xaa Xaa
1               5                   10                  15


Xaa Xaa Ala His Gln Gly Lys His Glu Thr Tyr Gly His Lys Thr Pro
            20                  25                  30


Tyr




<210> 1527
<211> 33
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES

<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1527
Ile Leu Xaa Ile Ala Ser Ser Xaa Xaa Thr Glu Xaa Ser His Xaa Xaa
1               5                   10                  15

Xaa Xaa Ala His Gln Gly Lys His Glu Thr Tyr Gly His Lys Thr Pro
            20                  25                  30

Tyr

<210> 1528
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1528
Ile Leu Pro Xaa Ala Ser Ser Xaa Xaa Thr Glu Xaa Ser His Xaa Xaa
1               5                   10                  15

Xaa Xaa Ala His Gln Gly Lys His Glu Thr Tyr Gly His Lys Thr Pro
            20                  25                  30

Tyr

<210> 1529
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1529
Ile Leu Pro Ile Xaa Ser Ser Xaa Xaa Thr Glu Xaa Ser His Xaa Xaa
1               5                   10                  15

Xaa Xaa Ala His Gln Gly Lys His Glu Thr Tyr Gly His Lys Thr Pro
            20                  25                  30

Tyr

<210> 1530
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1530
Ile Leu Pro Ile Ala Xaa Ser Xaa Xaa Thr Glu Xaa Ser His Xaa Xaa
1               5                   10                  15

Xaa Xaa Ala His Gln Gly Lys His Glu Thr Tyr Gly His Lys Thr Pro
            20                  25                  30

Tyr

<210> 1531
<211> 33
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>

<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1531
Ile Leu Pro Ile Ala Ser Xaa Xaa Xaa Thr Glu Xaa Ser His Xaa Xaa
1               5                   10                  15

Xaa Xaa Ala His Gln Gly Lys His Glu Thr Tyr Gly His Lys Thr Pro
            20                  25                  30

Tyr

<210> 1532
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1532
Xaa Leu Pro Ile Ala Ser Ser Xaa Xaa Thr Glu Val Xaa His Xaa Xaa
1               5                   10                  15

Xaa Xaa Ala His Gln Gly Lys His Glu Thr Tyr Gly His Lys Thr Pro

20                  25                  30

Tyr

<210> 1533
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1533
Ile Xaa Pro Ile Ala Ser Ser Xaa Xaa Thr Glu Val Xaa His Xaa Xaa
1               5                   10                  15

Xaa Xaa Ala His Gln Gly Lys His Glu Thr Tyr Gly His Lys Thr Pro
            20                  25                  30

Tyr

<210> 1534
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1534
Ile Leu Xaa Ile Ala Ser Ser Xaa Xaa Thr Glu Val Xaa His Xaa Xaa
1               5                   10                  15

Xaa Xaa Ala His Gln Gly Lys His Glu Thr Tyr Gly His Lys Thr Pro
            20                  25                  30

Tyr

<210> 1535
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1535
Ile Leu Pro Xaa Ala Ser Ser Xaa Xaa Thr Glu Val Xaa His Xaa Xaa
1               5                   10                  15

Xaa Xaa Ala His Gln Gly Lys His Glu Thr Tyr Gly His Lys Thr Pro
            20                  25                  30

Tyr

<210> 1536
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 1536
Ile Leu Pro Ile Xaa Ser Ser Xaa Xaa Thr Glu Val Xaa His Xaa Xaa
1               5                   10                  15

Xaa Xaa Ala His Gln Gly Lys His Glu Thr Tyr Gly His Lys Thr Pro
            20                  25                  30

Tyr

<210> 1537
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1537
Ile Leu Pro Ile Ala Xaa Ser Xaa Xaa Thr Glu Val Xaa His Xaa Xaa
1               5                   10                  15

Xaa Xaa Ala His Gln Gly Lys His Glu Thr Tyr Gly His Lys Thr Pro
            20                  25                  30

Tyr

<210> 1538
<211> 33
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1538
Ile Leu Pro Ile Ala Ser Xaa Xaa Xaa Thr Glu Val Xaa His Xaa Xaa
1               5                   10                  15

Xaa Xaa Ala His Gln Gly Lys His Glu Thr Tyr Gly His Lys Thr Pro
            20                  25                  30

Tyr

<210> 1539
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1539
Xaa Leu Pro Ile Ala Ser Ser Xaa Xaa Thr Glu Val Ser Xaa Xaa Xaa
1               5                   10                  15


Xaa Xaa Ala His Gln Gly Lys His Glu Thr Tyr Gly His Lys Thr Pro
            20                  25                  30


Tyr




<210> 1540
<211> 33
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1540
Ile Xaa Pro Ile Ala Ser Ser Xaa Xaa Thr Glu Val Ser Xaa Xaa Xaa
1               5                   10                  15

Xaa Xaa Ala His Gln Gly Lys His Glu Thr Tyr Gly His Lys Thr Pro
            20                  25                  30

Tyr

<210> 1541
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1541
Ile Leu Xaa Ile Ala Ser Ser Xaa Xaa Thr Glu Val Ser Xaa Xaa Xaa
1               5                   10                  15

Xaa Xaa Ala His Gln Gly Lys His Glu Thr Tyr Gly His Lys Thr Pro
            20                  25                  30

Tyr

<210> 1542
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1542
Ile Leu Pro Xaa Ala Ser Ser Xaa Xaa Thr Glu Val Ser Xaa Xaa Xaa
1               5                   10                  15

Xaa Xaa Ala His Gln Gly Lys His Glu Thr Tyr Gly His Lys Thr Pro
            20                  25                  30

Tyr

<210> 1543
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups

and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1543
Ile Leu Pro Ile Xaa Ser Ser Xaa Xaa Thr Glu Val Ser Xaa Xaa Xaa
1               5                   10                  15

Xaa Xaa Ala His Gln Gly Lys His Glu Thr Tyr Gly His Lys Thr Pro
            20                  25                  30

Tyr

<210> 1544
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1544
Ile Leu Pro Ile Ala Xaa Ser Xaa Xaa Thr Glu Val Ser Xaa Xaa Xaa
1               5                   10                  15

Xaa Xaa Ala His Gln Gly Lys His Glu Thr Tyr Gly His Lys Thr Pro
            20                  25                  30

Tyr

<210> 1545
<211> 33
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1545
Ile Leu Pro Ile Ala Ser Xaa Xaa Xaa Thr Glu Val Ser Xaa Xaa Xaa
1               5                   10                  15

Xaa Xaa Ala His Gln Gly Lys His Glu Thr Tyr Gly His Lys Thr Pro
            20                  25                  30

Tyr

<210> 1546
<211> 32
<212> PRT
<213> Artificial Sequence

<220>
<223> Description of Artificial Sequence: Synthetic peptide

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1546
Ile Leu Pro Ile Ala Ser Ser Cys Cys Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1547
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1547
Xaa Leu Pro Ile Ala Ser Ser Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1548
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1548
Ile Xaa Pro Ile Ala Ser Ser Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1549
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1549
Ile Leu Xaa Ile Ala Ser Ser Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1550
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1550
Ile Leu Pro Xaa Ala Ser Ser Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1551
<211> 32
<212> PRT
<213> Artificial Sequence

<220>

<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1551
Ile Leu Pro Ile Xaa Ser Ser Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1552
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1552
Ile Leu Pro Ile Ala Xaa Ser Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1553
<211> 32
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1553
Ile Leu Pro Ile Ala Ser Xaa Xaa Xaa Thr Glu Val Ser His Xaa Xaa
1               5                   10                  15

Xaa Xaa His Arg Lys Lys His His Gly Lys Arg Asn Ser Asp Arg Ala
            20                  25                  30

<210> 1554
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1554
Xaa Leu Pro Ile Ala Ser Ser Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1555
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1555
Ile Xaa Pro Ile Ala Ser Ser Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1556
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1556
Ile Leu Xaa Ile Ala Ser Ser Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1557
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1557
Ile Leu Pro Xaa Ala Ser Ser Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1558
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1558
Ile Leu Pro Ile Xaa Ser Ser Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1559
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>

<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1559
Ile Leu Pro Ile Ala Xaa Ser Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1560
<211> 32
<212> PRT
<213> Artificial Sequence

<220>
<223> Description of Artificial Sequence: Synthetic peptide

<220>
<221> MOD_RES
<222> (7)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1560
Ile Leu Pro Ile Ala Ser Xaa Xaa Xaa Xaa Glu Val Ser His Xaa Xaa
1               5                   10                  15

Xaa Xaa His Arg Lys Lys His His Gly Lys Arg Asn Ser Asp Arg Ala
            20                  25                  30

<210> 1561
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1561
Xaa Leu Pro Ile Ala Ser Ser Xaa Xaa Thr Xaa Val Ser His Xaa Xaa
1               5                   10                  15


Xaa Xaa His Arg Lys Lys His His Gly Lys Arg Asn Ser Asp Arg Ala
            20                  25                  30


<210> 1562
<211> 32
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1562
Ile Xaa Pro Ile Ala Ser Ser Xaa Xaa Thr Xaa Val Ser His Xaa Xaa
1               5                   10                  15

Xaa Xaa His Arg Lys Lys His His Gly Lys Arg Asn Ser Asp Arg Ala
            20                  25                  30

<210> 1563
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1563
Ile Leu Xaa Ile Ala Ser Ser Xaa Xaa Thr Xaa Val Ser His Xaa Xaa
1               5                   10                  15

Xaa Xaa His Arg Lys Lys His His Gly Lys Arg Asn Ser Asp Arg Ala

20                  25                  30

<210> 1564
<211> 32
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1564
Ile Leu Pro Xaa Ala Ser Ser Xaa Xaa Thr Xaa Val Ser His Xaa Xaa
1               5                   10                  15


Xaa Xaa His Arg Lys Lys His His Gly Lys Arg Asn Ser Asp Arg Ala
            20                  25                  30


<210> 1565
<211> 32
<212> PRT
<213> Artificial Sequence

<220>
<223> Description of Artificial Sequence: Synthetic peptide


<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1565
Ile Leu Pro Ile Xaa Ser Ser Xaa Xaa Thr Xaa Val Ser His Xaa Xaa
1               5                   10                  15

Xaa Xaa His Arg Lys Lys His His Gly Lys Arg Asn Ser Asp Arg Ala
            20                  25                  30

<210> 1566
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 1566
Ile Leu Pro Ile Ala Xaa Ser Xaa Xaa Thr Xaa Val Ser His Xaa Xaa
1               5                   10                  15

Xaa Xaa His Arg Lys Lys His His Gly Lys Arg Asn Ser Asp Arg Ala
            20                  25                  30

<210> 1567
<211> 32
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1567
Ile Leu Pro Ile Ala Ser Xaa Xaa Xaa Thr Xaa Val Ser His Xaa Xaa
1               5                   10                  15

Xaa Xaa His Arg Lys Lys His His Gly Lys Arg Asn Ser Asp Arg Ala
            20                  25                  30

<210> 1568
<211> 32
<212> PRT
<213> Artificial Sequence

<220>
<223> Description of Artificial Sequence: Synthetic peptide

<220>
<221> MOD_RES
<222> (1)..(1)

<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1568
Xaa Leu Pro Ile Ala Ser Ser Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1569
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>

<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1569
Ile Xaa Pro Ile Ala Ser Ser Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1570
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1570
Ile Leu Xaa Ile Ala Ser Ser Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1571

<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1571
Ile Leu Pro Xaa Ala Ser Ser Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1572
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see

specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1572
Ile Leu Pro Ile Xaa Ser Ser Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1573
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1573
Ile Leu Pro Ile Ala Xaa Ser Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1574
<211> 32
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1574
Ile Leu Pro Ile Ala Ser Xaa Xaa Xaa Thr Glu Xaa Ser His Xaa Xaa
1               5                   10                  15

Xaa Xaa His Arg Lys Lys His His Gly Lys Arg Asn Ser Asp Arg Ala
            20                  25                  30

<210> 1575
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1575
Xaa Leu Pro Ile Ala Ser Ser Xaa Xaa Thr Glu Val Xaa His Xaa Xaa
1               5                   10                  15


Xaa Xaa His Arg Lys Lys His His Gly Lys Arg Asn Ser Asp Arg Ala
            20                  25                  30


<210> 1576
<211> 32
<212> PRT
<213> Artificial Sequence

<220>
<223> Description of Artificial Sequence: Synthetic peptide


<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed

description of substitutions, R-groups and preferred embodiments

<220>
<223> c-term may be amidated

<400> 1576
Ile Xaa Pro Ile Ala Ser Ser Xaa Xaa Thr Glu Val Xaa His Xaa Xaa
1               5                   10                  15

Xaa Xaa His Arg Lys Lys His His Gly Lys Arg Asn Ser Asp Arg Ala
            20                  25                  30

<210> 1577
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1577
Ile Leu Xaa Ile Ala Ser Ser Xaa Xaa Thr Glu Val Xaa His Xaa Xaa
1               5                   10                  15

Xaa Xaa His Arg Lys Lys His His Gly Lys Arg Asn Ser Asp Arg Ala
            20                  25                  30

<210> 1578
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1578
Ile Leu Pro Xaa Ala Ser Ser Xaa Xaa Thr Glu Val Xaa His Xaa Xaa
1               5                   10                  15

Xaa Xaa His Arg Lys Lys His His Gly Lys Arg Asn Ser Asp Arg Ala
            20                  25                  30

<210> 1579
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>

<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1579
Ile Leu Pro Ile Xaa Ser Ser Xaa Xaa Thr Glu Val Xaa His Xaa Xaa
1               5                   10                  15


Xaa Xaa His Arg Lys Lys His His Gly Lys Arg Asn Ser Asp Arg Ala
            20                  25                  30


<210> 1580
<211> 32
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1580
Ile Leu Pro Ile Ala Xaa Ser Xaa Xaa Thr Glu Val Xaa His Xaa Xaa
1               5                   10                  15

Xaa Xaa His Arg Lys Lys His His Gly Lys Arg Asn Ser Asp Arg Ala
            20                  25                  30

<210> 1581
<211> 32
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1581
Ile Leu Pro Ile Ala Ser Xaa Xaa Xaa Thr Glu Val Xaa His Xaa Xaa
1               5                   10                  15

Xaa Xaa His Arg Lys Lys His His Gly Lys Arg Asn Ser Asp Arg Ala
            20                  25                  30

<210> 1582
<211> 32
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see

specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1582
Xaa Leu Pro Ile Ala Ser Ser Xaa Xaa Thr Glu Val Ser Xaa Xaa Xaa
1               5                   10                  15

Xaa Xaa His Arg Lys Lys His His Gly Lys Arg Asn Ser Asp Arg Ala
            20                  25                  30

<210> 1583
<211> 32
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1583
Ile Xaa Pro Ile Ala Ser Ser Xaa Xaa Thr Glu Val Ser Xaa Xaa Xaa
1               5                   10                  15

Xaa Xaa His Arg Lys Lys His His Gly Lys Arg Asn Ser Asp Arg Ala
            20                  25                  30

<210> 1584
<211> 32
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1584
Ile Leu Xaa Ile Ala Ser Ser Xaa Xaa Thr Glu Val Ser Xaa Xaa Xaa
1               5                   10                  15

Xaa Xaa His Arg Lys Lys His His Gly Lys Arg Asn Ser Asp Arg Ala
            20                  25                  30

<210> 1585
<211> 32
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1585
Ile Leu Pro Xaa Ala Ser Ser Xaa Xaa Thr Glu Val Ser Xaa Xaa Xaa
1               5                   10                  15

Xaa Xaa His Arg Lys Lys His His Gly Lys Arg Asn Ser Asp Arg Ala
            20                  25                  30

<210> 1586
<211> 32
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups

and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1586
Ile Leu Pro Ile Xaa Ser Ser Xaa Xaa Thr Glu Val Ser Xaa Xaa Xaa
1               5                   10                  15

Xaa Xaa His Arg Lys Lys His His Gly Lys Arg Asn Ser Asp Arg Ala
            20                  25                  30

<210> 1587
<211> 32
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1587
Ile Leu Pro Ile Ala Xaa Ser Xaa Xaa Thr Glu Val Ser Xaa Xaa Xaa
1               5                   10                  15

Xaa Xaa His Arg Lys Lys His His Gly Lys Arg Asn Ser Asp Arg Ala
            20                  25                  30

<210> 1588
<211> 32
<212> PRT
<213> Artificial Sequence

<220>
<223> Description of Artificial Sequence: Synthetic peptide


<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1588
Ile Leu Pro Ile Ala Ser Xaa Xaa Xaa Thr Glu Val Ser Xaa Xaa Xaa
1               5                   10                  15


Xaa Xaa His Arg Lys Lys His His Gly Lys Arg Asn Ser Asp Arg Ala
            20                  25                  30


<210> 1589
<211> 33
<212> PRT
<213> Artificial Sequence

<220>
<223> Description of Artificial Sequence: Synthetic peptide


<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1589
Ile Leu Pro Ile Ala Ser Ser Cys Cys Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1590
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1590
Xaa Leu Pro Ile Ala Ser Ser Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1591
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups

and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1591
Ile Xaa Pro Ile Ala Ser Ser Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1592
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1592
Ile Leu Xaa Ile Ala Ser Ser Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1593
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1593
Ile Leu Pro Xaa Ala Ser Ser Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1594
<211> 33
<212> PRT
<213> Artificial Sequence

<220>
<223> Description of Artificial Sequence: Synthetic peptide

<220>
<221> MOD_RES
<222> (5)..(5)

<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1594
Ile Leu Pro Ile Xaa Ser Ser Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1595
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1595

Ile Leu Pro Ile Ala Xaa Ser Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1596
<211> 33
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1596
Ile Leu Pro Ile Ala Ser Xaa Xaa Xaa Thr Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1597
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>

<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1597
Xaa Leu Pro Ile Ala Ser Ser Xaa Xaa Xaa Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1598
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1598
Ile Xaa Pro Ile Ala Ser Ser Xaa Xaa Xaa Glu Val Ser His Xaa Xaa
1               5                   10                  15

Xaa Xaa Ala His Gln Gly Lys His Glu Thr Tyr Gly His Lys Thr Pro

20                  25                  30

Tyr

<210> 1599
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1599
Ile Leu Xaa Ile Ala Ser Ser Xaa Xaa Xaa Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1600
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1600
Ile Leu Pro Xaa Ala Ser Ser Xaa Xaa Xaa Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1601
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1601
Ile Leu Pro Ile Xaa Ser Ser Xaa Xaa Xaa Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1602
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(10)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1602
Ile Leu Pro Ile Ala Xaa Ser Xaa Xaa Xaa Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1603
<211> 33
<212> PRT
<213> Artificial Sequence

<220>
<223> Description of Artificial Sequence: Synthetic peptide

<220>
<221> MOD_RES
<222> (7)..(10)
<223> Any L- or D-natural amino acid and any non-natural amino acid; see

specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1603
Ile Leu Pro Ile Ala Ser Xaa Xaa Xaa Xaa Glu Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1604
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1604
Xaa Leu Pro Ile Ala Ser Ser Xaa Xaa Thr Xaa Val Ser His Xaa Xaa

1               5                   10                  15

Xaa Xaa Ala His Gln Gly Lys His Glu Thr Tyr Gly His Lys Thr Pro
            20                  25                  30

Tyr

<210> 1605
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1605
Ile Xaa Pro Ile Ala Ser Ser Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1606
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1606
Ile Leu Xaa Ile Ala Ser Ser Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1607
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)

<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1607
Ile Leu Pro Xaa Ala Ser Ser Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1608
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may

encompass 1 or 4 residues; see specification as filed for detailed
description of substitutions, R-groups and preferred embodiments

<220>
<223> c-term may be amidated

<400> 1608
Ile Leu Pro Ile Xaa Ser Ser Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1609
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1609
Ile Leu Pro Ile Ala Xaa Ser Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1610
<211> 33
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (11)..(11)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1610
Ile Leu Pro Ile Ala Ser Xaa Xaa Xaa Thr Xaa Val Ser His Xaa Xaa
1               5                   10                  15

Xaa Xaa Ala His Gln Gly Lys His Glu Thr Tyr Gly His Lys Thr Pro
            20                  25                  30

Tyr

<210> 1611
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES

<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1611
Xaa Leu Pro Ile Ala Ser Ser Xaa Xaa Thr Glu Xaa Ser His Xaa Xaa
1               5                   10                  15

Xaa Xaa Ala His Gln Gly Lys His Glu Thr Tyr Gly His Lys Thr Pro
            20                  25                  30

Tyr

<210> 1612
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)

<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1612
Ile Xaa Pro Ile Ala Ser Ser Xaa Xaa Thr Glu Xaa Ser His Xaa Xaa
1               5                   10                  15

Xaa Xaa Ala His Gln Gly Lys His Glu Thr Tyr Gly His Lys Thr Pro
            20                  25                  30

Tyr

<210> 1613
<211> 33
<212> PRT
<213> Artificial Sequence

<220>
<223> Description of Artificial Sequence: Synthetic peptide

<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1613
Ile Leu Xaa Ile Ala Ser Ser Xaa Xaa Thr Glu Xaa Ser His Xaa Xaa
1               5                   10                  15

Xaa Xaa Ala His Gln Gly Lys His Glu Thr Tyr Gly His Lys Thr Pro
            20                  25                  30

Tyr

<210> 1614
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1614
Ile Leu Pro Xaa Ala Ser Ser Xaa Xaa Thr Glu Xaa Ser His Xaa Xaa
1               5                   10                  15

Xaa Xaa Ala His Gln Gly Lys His Glu Thr Tyr Gly His Lys Thr Pro
            20                  25                  30

Tyr

<210> 1615
<211> 33
<212> PRT
<213> Artificial Sequence

<220>
<223> Description of Artificial Sequence: Synthetic peptide

<220>

<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1615
Ile Leu Pro Ile Xaa Ser Ser Xaa Xaa Thr Glu Xaa Ser His Xaa Xaa
1               5                   10                  15

Xaa Xaa Ala His Gln Gly Lys His Glu Thr Tyr Gly His Lys Thr Pro
            20                  25                  30

Tyr

<210> 1616
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES

<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1616
Ile Leu Pro Ile Ala Xaa Ser Xaa Xaa Thr Glu Xaa Ser His Xaa Xaa
1               5                   10                  15

Xaa Xaa Ala His Gln Gly Lys His Glu Thr Tyr Gly His Lys Thr Pro
            20                  25                  30

Tyr

<210> 1617
<211> 33
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (12)..(12)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1617
Ile Leu Pro Ile Ala Ser Xaa Xaa Xaa Thr Glu Xaa Ser His Xaa Xaa
1               5                   10                  15

Xaa Xaa Ala His Gln Gly Lys His Glu Thr Tyr Gly His Lys Thr Pro
            20                  25                  30

Tyr

<210> 1618
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1618
Xaa Leu Pro Ile Ala Ser Ser Xaa Xaa Thr Glu Val Xaa His Xaa Xaa
1               5                   10                  15

Xaa Xaa Ala His Gln Gly Lys His Glu Thr Tyr Gly His Lys Thr Pro
            20                  25                  30

Tyr

<210> 1619
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1619
Ile Xaa Pro Ile Ala Ser Ser Xaa Xaa Thr Glu Val Xaa His Xaa Xaa
1               5                   10                  15


Xaa Xaa Ala His Gln Gly Lys His Glu Thr Tyr Gly His Lys Thr Pro
            20                  25                  30


Tyr




<210> 1620
<211> 33
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>

<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1620
Ile Leu Xaa Ile Ala Ser Ser Xaa Xaa Thr Glu Val Xaa His Xaa Xaa
1               5                   10                  15

Xaa Xaa Ala His Gln Gly Lys His Glu Thr Tyr Gly His Lys Thr Pro
            20                  25                  30

Tyr

<210> 1621
<211> 33
<212> PRT
<213> Artificial Sequence

<220>
<223> Description of Artificial Sequence: Synthetic peptide

<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1621
Ile Leu Pro Xaa Ala Ser Ser Xaa Xaa Thr Glu Val Xaa His Xaa Xaa
1               5                   10                  15

Xaa Xaa Ala His Gln Gly Lys His Glu Thr Tyr Gly His Lys Thr Pro
            20                  25                  30

Tyr

<210> 1622
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1622
Ile Leu Pro Ile Xaa Ser Ser Xaa Xaa Thr Glu Val Xaa His Xaa Xaa
1               5                   10                  15

Xaa Xaa Ala His Gln Gly Lys His Glu Thr Tyr Gly His Lys Thr Pro
            20                  25                  30

Tyr

<210> 1623

<211> 33
<212> PRT
<213> Artificial Sequence

<220>
<223> Description of Artificial Sequence: Synthetic peptide


<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1623
Ile Leu Pro Ile Ala Xaa Ser Xaa Xaa Thr Glu Val Xaa His Xaa Xaa
1               5                   10                  15


Xaa Xaa Ala His Gln Gly Lys His Glu Thr Tyr Gly His Lys Thr Pro
            20                  25                  30


Tyr




<210> 1624
<211> 33
<212> PRT
<213> Artificial Sequence

<220>
<223> Description of Artificial Sequence: Synthetic peptide


<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (13)..(13)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1624
Ile Leu Pro Ile Ala Ser Xaa Xaa Xaa Thr Glu Val Xaa His Xaa Xaa
1               5                   10                  15

Xaa Xaa Ala His Gln Gly Lys His Glu Thr Tyr Gly His Lys Thr Pro
            20                  25                  30

Tyr

<210> 1625
<211> 33
<212> PRT
<213> Artificial Sequence

<220>
<223> Description of Artificial Sequence: Synthetic peptide

<220>
<221> MOD_RES
<222> (1)..(1)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>

<223> c-term may be amidated

<400> 1625
Xaa Leu Pro Ile Ala Ser Ser Xaa Xaa Thr Glu Val Ser Xaa Xaa Xaa
1               5                   10                  15

Xaa Xaa Ala His Gln Gly Lys His Glu Thr Tyr Gly His Lys Thr Pro
            20                  25                  30

Tyr

<210> 1626
<211> 33
<212> PRT
<213> Artificial Sequence

<220>
<223> Description of Artificial Sequence: Synthetic peptide

<220>
<221> MOD_RES
<222> (2)..(2)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1626
Ile Xaa Pro Ile Ala Ser Ser Xaa Xaa Thr Glu Val Ser Xaa Xaa Xaa
1               5                   10                  15

Xaa Xaa Ala His Gln Gly Lys His Glu Thr Tyr Gly His Lys Thr Pro
            20                  25                  30

Tyr

<210> 1627
<211> 33
<212> PRT
<213> Artificial Sequence

<220>
<223> Description of Artificial Sequence: Synthetic peptide


<220>
<221> MOD_RES
<222> (3)..(3)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> C-term may be amidated

<400> 1627
Ile Leu Xaa Ile Ala Ser Ser Xaa Xaa Thr Glu Val Ser Xaa Xaa Xaa
1               5                   10                  15


Xaa Xaa Ala His Gln Gly Lys His Glu Thr Tyr Gly His Lys Thr Pro
            20                  25                  30


Tyr




<210> 1628
<211> 33
<212> PRT
<213> Artificial Sequence

<220>
<223> Description of Artificial Sequence: Synthetic peptide


<220>
<221> MOD_RES
<222> (4)..(4)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1628
Ile Leu Pro Xaa Ala Ser Ser Xaa Xaa Thr Glu Val Ser Xaa Xaa Xaa
1               5                   10                  15

Xaa Xaa Ala His Gln Gly Lys His Glu Thr Tyr Gly His Lys Thr Pro
            20                  25                  30

Tyr

<210> 1629
<211> 33
<212> PRT
<213> Artificial Sequence

<220>
<223> Description of Artificial Sequence: Synthetic peptide

<220>
<221> MOD_RES
<222> (5)..(5)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1629
Ile Leu Pro Ile Xaa Ser Ser Xaa Xaa Thr Glu Val Ser Xaa Xaa Xaa
1               5                   10                  15

Xaa Xaa Ala His Gln Gly Lys His Glu Thr Tyr Gly His Lys Thr Pro
            20                  25                  30

Tyr

<210> 1630
<211> 33
<212> PRT
<213> Artificial Sequence

<220>
<223> Description of Artificial Sequence: Synthetic peptide

<220>
<221> MOD_RES
<222> (6)..(6)
<223> Any L- or D-natural amino acid and any non-natural amino acid; see
specification as filed for detailed description of substitutions, R-groups
and preferred embodiments

<220>
<221> MOD_RES
<222> (8)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1630
Ile Leu Pro Ile Ala Xaa Ser Xaa Xaa Thr Glu Val Ser Xaa Xaa Xaa
1               5                   10                  15

Xaa Xaa Ala His Gln Gly Lys His Glu Thr Tyr Gly His Lys Thr Pro
            20                  25                  30

Tyr

<210> 1631
<211> 33
<212> PRT
<213> Artificial Sequence

<220>
<223> Description of Artificial Sequence: Synthetic peptide

<220>
<221> MOD_RES
<222> (7)..(9)
<223> Any L- or D-natural amino acid and any non-natural amino acid; see
specification as filed for detailed description of substitutions, R-groups
and preferred embodiments

<220>
<221> MOD_RES
<222> (14)..(14)
<223> Any L- or D-natural amino acid and any non-natural amino acid; see
specification as filed for detailed description of substitutions, R-groups
and preferred embodiments

<220>
<221> MOD_RES
<222> (15)..(18)
<223> Any L- or D-natural or non-natural amino acid, and this range may
encompass 1 or 4 residues; see specification as filed for detailed
description of substitutions, R-groups and preferred embodiments

<220>
<223> c-term may be amidated

<400> 1631
Ile Leu Pro Ile Ala Ser Xaa Xaa Xaa Thr Glu Val Ser Xaa Xaa Xaa
1               5                   10                  15

Xaa Xaa Ala His Gln Gly Lys His Glu Thr Tyr Gly His Lys Thr Pro
            20                  25                  30

Tyr

<210> 1632
<211> 35
<212> PRT
<213> Artificial Sequence

<220>
<223> Description of Artificial Sequence: Synthetic peptide

<220>
<223> c-term may be amidated

<400> 1632
Ser Ala Lys Glu Leu Arg Cys Gln Cys Ile Lys Thr Tyr Ser Lys Gly
1               5                   10                  15

Gly Gly Gly Asn Trp Val Gln Arg Val Val Glu Lys Phe Leu Lys Arg
            20                  25                  30

Ala Glu Asn
        35

<210> 1633
<211> 33
<212> PRT
<213> Artificial Sequence

<220>
<223> Description of Artificial Sequence: Synthetic peptide

<220>
<223> c-term may be amidated

<400> 1633
Ser Ala Lys Glu Leu Arg Cys Gln Cys Ile Lys Thr Tyr Gly Gly Gly
1               5                   10                  15

Gly Asn Trp Val Gln Arg Val Val Glu Lys Phe Leu Lys Arg Ala Glu
            20                  25                  30

Asn

<210> 1634
<211> 31
<212> PRT
<213> Artificial Sequence

<220>
<223> Description of Artificial Sequence: Synthetic peptide

<220>
<223> c-term may be amidated

<400> 1634
Ser Ala Lys Glu Leu Arg Cys Gln Cys Ile Lys Gly Gly Gly Gly Asn
1               5                   10                  15

Trp Val Gln Arg Val Val Glu Lys Phe Leu Lys Arg Ala Glu Asn
            20                  25                  30

<210> 1635
<211> 10
<212> PRT
<213> Artificial Sequence

<220>
<223> Description of Artificial Sequence: Synthetic peptide

<220>
<223> c-term may be amidated

<400> 1635
Lys Ser Met Gln Val Pro Phe Ser Arg Ala
1               5                   10

<210> 1636
<211> 15
<212> PRT
<213> Artificial Sequence

<220>
<223> Description of Artificial Sequence: Synthetic peptide

<220>
<223> c-term may be amidated

<400> 1636
Ala Phe Ser Phe Ala Glu Gln Glu Ile Pro Leu Arg Ala Ile Leu
1               5                   10                  15


<210> 1637
<211> 23
<212> PRT
<213> Artificial Sequence

<220>
<223> Description of Artificial Sequence: Synthetic peptide

<220>
<223> c-term may be amidated

<400> 1637
Ala Phe Ser Phe Ala Glu Gln Glu Ile Pro Leu Arg Ala Ile Leu Cys
1               5                   10                  15


Tyr Arg Asn Thr Ser Ser Ile
            20


<210> 1638
<211> 24
<212> PRT
<213> Artificial Sequence

<220>
<223> Description of Artificial Sequence: Synthetic peptide

<220>
<223> c-term may be amidated

<400> 1638
Ala Tyr Arg Asn Thr Ser Ser Ile Cys Ser Asn Glu Gly Leu Ile Phe
1               5                   10                  15


Lys Leu Lys Arg Gly Lys Glu Ala
            20


<210> 1639
<211> 24
<212> PRT
<213> Artificial Sequence

<220>
<223> Description of Artificial Sequence: Synthetic peptide

<220>
<223> c-term may be amidated

<400> 1639
Ala Ala Leu Asp Thr Val Gly Trp Val Gln Arg His Arg Lys Met Leu
1               5                   10                  15

Arg His Cys Pro Ser Lys Arg Lys
            20

<210> 1640
<211> 4
<212> PRT
<213> Artificial Sequence

<220>
<223> Description of Artificial Sequence: Synthetic peptide

<400> 1640
Gly Gly Gly Gly
1

<210> 1641
<211> 30
<212> PRT
<213> Artificial Sequence

<220>
<223> Description of Artificial Sequence: Synthetic peptide

<220>
<221> MOD_RES
<222> (17)..(17)
<223> 11-amino undecanoic acid

<400> 1641
Val Pro Leu Ser Arg Thr Val Arg Cys Thr Cys Ile Ser Ile Ser Asn
1               5                   10                  15

Xaa Leu Lys Ala Val Ser Lys Glu Met Ser Lys Arg Ser Pro
            20                  25                  30

<210> 1642
<211> 30
<212> PRT
<213> Artificial Sequence

<220>
<223> Description of Artificial Sequence: Synthetic peptide

<220>
<221> MOD_RES
<222> (17)..(17)
<223> 11-amino undecanoic acid

<400> 1642
Val Pro Leu Ser Arg Thr Val Arg Ala Thr Phe Ile Ser Ile Ser Asn
1               5                   10                  15

Xaa Leu Lys Ala Val Ser Lys Glu Met Ser Lys Arg Ser Pro
            20                  25                  30

<210> 1643
<211> 30
<212> PRT
<213> Artificial Sequence

<220>
<223> Description of Artificial Sequence: Synthetic peptide


<220>
<221> MOD_RES
<222> (16)..(16)
<223> 11-amino undecanoic acid

<400> 1643
Val Pro Leu Ser Arg Thr Pro Arg Cys Thr Cys Ile Ser Ile Ser Xaa
1               5                   10                  15


Glu Ser Lys Ala Ile Lys Asn Leu Leu Lys Ala Val Ser Lys
            20                  25                  30


<210> 1644
<211> 30
<212> PRT
<213> Artificial Sequence

<220>
<223> Description of Artificial Sequence: Synthetic peptide


<220>
<221> MOD_RES
<222> (16)..(16)
<223> 11-amino undecanoic acid

<400> 1644
Val Pro Leu Ser Arg Thr Val Arg Ser Thr Ser Ile Ser Ile Ser Xaa
1               5                   10                  15


Glu Ser Lys Ala Ile Glu Asn Leu Leu Lys Ala Val Ser Lys
            20                  25                  30


<210> 1645
<211> 30
<212> PRT
<213> Artificial Sequence

<220>
<223> Description of Artificial Sequence: Synthetic peptide


<220>
<221> MOD_RES
<222> (16)..(16)
<223> 11-amino undecanoic acid

<400> 1645
Val Pro Leu Ser Arg Thr Val Arg Ser Thr Ser Ile Ser Ile Ser Xaa
1               5                   10                  15


Glu Ser Lys Ala Ile Lys Asn Leu Leu Glu Ala Val Ser Lys
            20                  25                  30

<210> 1646
<211> 15
<212> PRT
<213> Artificial Sequence

<220>
<223> Description of Artificial Sequence: Synthetic peptide

<220>
<223> c-term may be amidated

<400> 1646
Val Pro Leu Ser Arg Thr Val Arg Cys Thr Cys Ile Ser Ile Ser
1               5                   10                  15


<210> 1647
<211> 6
<212> PRT
<213> Artificial Sequence

<220>
<223> Description of Artificial Sequence: Synthetic peptide

<220>
<223> c-term may be amidated

<400> 1647
Leu Lys Ala Val Ser Lys
1               5

## Description

### FIELD OF THE INVENTION

This invention is directed to peptide analogs of interferon-inducible protein-10 (IP-10 or CXCL10) chemokine that bind to the CXCR3 receptor or any other receptor in which IP-10 analogs can bind to as a ligand, such that the analogs can be designed to serve as agonists or antagonists of IP-10 chemokine. The analogs can be used to prevent, treat, or ameliorate the symptoms of, a disease.

### BACKGROUND OF THE INVENTION

Chemokines (chemoattractant cytokines) are a family of homologous serum proteins of between 7 and 16 kDa, which were originally characterized by their ability to induce migration of leukocytes. Most chemokines have four characteristic cysteines (Cys), and depending on the motif displayed by the first two cysteines, they have been classified into CXC or alpha, CC or beta, C or gamma, and CX3C or delta chemokine classes. Two disulfide bonds are formed between the first and third cysteine and between the second and fourth cysteine. In general, it was thought that the disulfide bridges were required, and Clark-Lewis and co-workers reported that, at least for IP-10, the disulfide bridges are critical for chemokine activity (Clark-Lewis et al., J. Biol. Chem. 269:16075-16081, 1994). The only exception to having four cysteines is lymphotactin, which has only two cysteine residues. Thus, lymphotactin manages to retain a functional structure with only one disulfide bond.

In addition, the CXC, or alpha, subfamily has been divided into two groups depending on the presence of the ELR motif (Glu-Leu-Arg) preceding the first cysteine: the ELR-CXC chemokines and the non-ELR-CXC chemokines (*see, e.g.,* Clark-Lewis, *supra,* and Belperio et al., "CXC Chemokines in Angiogenesis," J. Leukoc. Biol. 68:1-8, 2000).

Chemokines have been shown to be useful in therapeutic applications. For example, the chemokine SDF-1 has been shown to enhance platelet production (Lane et al., Blood 96:4152-59, 2000) and B-cell production (Nagasawa, T., Int. J. Hematol. 72:408-11, 2000), inter alia. Other chemokine functions are reviewed in Schwarz and Wells (Schwarz and Wells, Nat. Rev. Drug Discov. 1:347-58, 2002). Glimm and colleagues reported for example, that SDF-1 arrests hematopoietic stem cell cycling, thus allowing a better transfection of these cells with gene constructs for the purpose of gene therapy (Glimm H. et al., "Ex vivo treatment of proliferating human cord blood stem cells with stroma-derived factor-1 enhances their ability to engraft NOD/SCID mice," Blood 99(9):3454-57, 2002). All of the above references are incorporated by reference herein their entirety, including any drawings, tables, and figures.

Interferon-inducible protein-10 (IP-10 or CXCL10) is induced by interferon-gamma and TNF-alpha, and is produced by keratinocytes, endothelial cells, fibroblasts and monocytes. IP-10 is thought to play a role in recruiting activated T cells to sites of tissue inflammation (Dufour, et al., "IFN-gamma-inducible protein 10 (IP-10; CXCL10)-deficient mice reveal a role for IP-10 in effector T cell generation and trafficking," J Immunol., 168:3195-204, 2002). In addition, IP-10 may play a role in hypersensitivity. It may also play a role in the genesis of inflammatory demyelinating neuropathies (Kieseier, et al., "Chemokines and chemokine receptors in inflammatory demyelinating neuropathies: a central role for IP-10," Brain 125:823-34, 2002).

Research has shown that IP-10s may be useful in improving the engraftment of stem cells following transplantation (Nagasawa, 2000), as well as in mobilizing stem cells (Gazitt, Y., J. Hematother Stem Cell Res 10:229-36, 2001; Hattori et al., Blood 97:3354-59, 2001) and enhancing anti-tumor immunity (Nomura et al., Int. J. Cancer 91:597-606, 2001; Mach and Dranoff, Curr. Opin. Immunol. 12:571-75, 2000). The biological activity of chemokines has been discussed, for example, in reports known to those of skill in the art (Bruce, L. et al., "Radiolabeled Chemokine binding assays," Methods in Molecular Biology (2000) vol. 138, pp129-134, Raphaele, B. et al. "Calcium Mobilization," Methods in Molecular Biology (2000) vol. 138, pp143-148, Paul D. Ponath et al., "Transwell Chemotaxis," Methods in Molecular Biology (2000) vol. 138, pp113-120 Humana Press. Totowa, New Jersey).

Accordingly, one of skill will appreciate novel IP-10 analogs that bind to the CXCR3 receptor or any other receptor in which IP-10 analogs can be a ligand, such that the analogs can be designed to serve as agonists or antagonists of IP-10 chemokine.

### SUMMARY OF THE INVENTION

The present invention is directed to an IP-10 analog that can serve as an agonist or antagonist in the treatment of disease. More particularly, the invention is directed to a composition comprising an IP-10 chemokine analog having a length ranging from about 21 to about 34 amino acids and comprising an N-terminal region having a first conserved sequence consisting of the IP-10 chemokine residues 1-15 (SEQ ID NO:1646), and conservatively modified variants thereof; a C-terminal region having a second conserved sequence consisting of the IP-10 chemokine residues 66-71 (SEQ ID NO:1647), and conservatively modified variants thereof; and, a linker consisting of up to 4 amino acids. If the N-terminal region is composed of more than 16 residues, then at least Lys⁶³ or Lys⁶⁷ of the C-terminal region is substituted with Glu.

The N-terminus of the N-terminal region can consist of a hydrogen or can be modified using an N-terminal modifier comprising a component selected from a group consisting of a poly(ethylene glycol) or derivative thereof, a glycosaminoglycan, a diagnostic label, a radioactive group, an acyl group, an acetyl group, a peptide, and a modifier capable of reducing the ability of the IP-10 analog to act as a substrate for aminopeptidases. The linker can be selected from a group consisting of (a) up to four natural amino acids, and (b) any non-natural amino acid having the following structure: 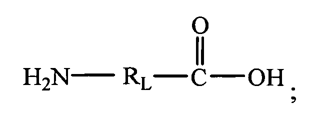 wherein, R_{L} is selected from a group consisting of saturated and unsaturated aliphatics and heteroaliphatics consisting of 20 or fewer carbon atoms that are optionally substituted with a hydroxyl, carboxyl, amino, amido, or imino group; or an aromatic group having from 5 to 7 members in the ring; and -(CH₂)ₙ-, wherein n is an integer ranging from 1 to 20.

In some embodiments, the IP-10 analog can have a length of about 26 to 32 amino acid residues and comprise an N-terminal region consisting essentially of SEQ ID NO:1646 and conservatively modified variants thereof in residues 1-15, and a C-terminal region comprising SEQ ID NO:1647 and conservatively modified variants thereof. In some embodiments, the linker is 11-aminoundecanoic acid.

In many embodiments, the IP-10 analogs can be comprised of an amino acid sequence selected from a group consisting of SEQ ID NO:1641, SEQ ID NO:1642, SEQ ID NO:1643, SEQ ID NO:1644, and SEQ ID NO:1645. In some embodiments, the IP-10 analogs can be comprised of an amino acid sequence selected from a group consisting of SEQ ID NOs:296 through 349, 404 through 457, 494 through 548, 675 through 728, variants b134 through b187, b242 through b295, b332 through b385, and b512 through b566, inclusive. These amino acid sequences can contain variables Xaa₁, Xaa₂, Xaa₃, and Xaa₄, wherein, Xaa₁, Xaa₂, Xaa₃, and Xaa₄ can each be independently selected from a group consisting of any natural amino acid or any non-natural amino acid having the following structure: 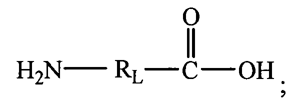 wherein, R_{L} is selected from a group consisting of saturated and unsaturated aliphatics and heteroaliphatics consisting of 20 or fewer carbon atoms that are optionally substituted with a hydroxyl, carboxyl, amino, amido, or imino group; or an aromatic group having from 5 to 7 members in the ring; and -(CH₂)ₙ-, wherein n is an integer ranging from 1 to 20.

In many embodiments, the invention includes a method of increasing the IP-10-mediated activity of a cell having a receptor capable of binding to an IP-10 analog, comprising binding the receptor to the IP-10 analog described above. In some embodiments, the invention includes an antibody produced using the IP-10 analog described above as an antigen. In these embodiments, the antibody can be polyclonal, monoclonal, and/or humanized.

### BRIEF DESCRIPTION OF THE DRAWINGS

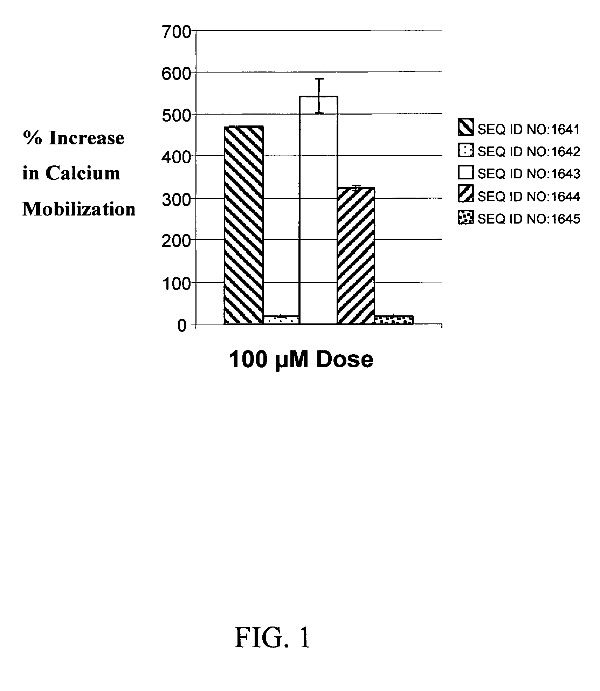

FIG. 1 illustates the induction of [Ca²⁺]i mobilization by select IP-10 analogs at a concentration of 100µM according to some embodiments of the present invention.

### DETAILED DESCRIPTION OF THE INVENTION

The present invention generally relates to the design, preparation, derivation, and use of amino acid sequences in the prevention, treatment, and ameliorization of diseases and disorders. Generally speaking, this invention is directed to the design, synthesis, and use of IP-10 analogs which bind to an IP-10 chemokine receptor, such as a CXCR3 receptor or any other receptor to which IP-10 binds, such that the IP-10 analogs can be designed to affect the activity of the receptor.

An IP-10 chemokine receptor, for example, can by any receptor recognized in the art as binding to an IP-10 chemokine, as well as any other binding moiety capable of binding to an IP-10 chemokine or IP-10 analog, wherein the activity of the receptor can be increased or decreased. In some embodiments, the terms "activity," "activate," "activated," "activating," "activation," and the like, can refer to a cellular or extracellular function of an IP-10 chemokine receptor.

In some embodiments, biological activity can include, for example, receptor binding, chemotaxis, and calcium mobilization, as well as other activities known to those of skill in the art that are affected by the presence of a binding peptide. The IP-10 chemokine receptor function can include catalytic activity from the interaction with a natural binding partner. In some embodiments, an IP-10 mimetic activates the catalytic activity of a chemokine receptor. In some embodiments, an IP-10 mimetic inhibits the catalytic activity of a chemokine receptor. In some embodiments, the activation or inhibition of a chemokine receptor can be dependent on the concentration of an IP-10 mimetic.

A recognized sequence of the human IP-10 chemokine is as follows:

Val-Pro-Leu-Ser-Arg-Thr-Val-Arg-Cys-Thr-Cys-Ile-Ser-Ile-Ser-Asn-
Gln-Pro-Val-Asn-Pro-Pro-Arg-Ser-Leu-Glu-Lys-Leu-Glu-Ile-Ile-Pro-
Ala-Ser-Gln-Phe-Cys-Pro-Arg-Val-Glu-Ile-Ile-Ala-Thr-Met-Lys-Lys-

Lys-Gly-Glu-Lys-Arg-Cys-Leu-Asn-Pro-Glu-Ser-Lys-Ala-Ile-Lys-Asn-
Leu-Leu-Lys-Ala-Val-Ser-Lys-Glu-Met-Ser-Lys-Arg-Ser-Pro
(SEQ ID NO:2)

The products of the present invention can be referred to by various terms including, but not limited to, "analogs," "mimetics,"peptidomimetics," "peptides," "polypeptides," "designer peptides," "IP-10 chemokine mimetics," "IP-10 mimetics," "IP-10 chemokine analogs," "IP-10 analogs," and "IP-10 chemokine derivatives." These terms can be used interchangeably in many embodiments and, in some embodiments, denote equivalent compounds. The IP-10 analogs can include, but are not limited to, the sequences described herein, variants and conservately modified variants thereof, and can also include additional elements such as R-group substituents and linkers, examples of which are taught in the present application.

The term "variant" refers to modifications to a peptide that allows the peptide to retain its binding properties, and such modifications include, but are not limited to, conservative substitutions in which one or more amino acids are substituted for other amino acids; deletion or addition of amino acids that have minimal influence on the binding properties or secondary structure; conjugation of a linker; post-translational modifications such as, for example, the addition of functional groups. Examples of such post-translational modifications can include, but are not limited to, the addition of modifying groups described below through processes such as, for example, glycosylation, acetylation, phosphorylation, modifications with fatty acids, formation of disulfide bonds within and between peptides, biotinylation, PEGylation, and combinations thereof.

The term "conservatively modified variant" refers to a conservative amino acid substitution, which is an amino acid substituted by an amino acid of similar charge density, hydrophilicity/hydrophobicity, size, and/or configuration such as, for example, substituting valine for isoleucine. In comparison, a "non-conservatively modified variant" can refer to a non-conservative amino acid substitution, which can include, in some embodiments, an amino acid substituted by an amino acid of differing charge density, hydrophilicity/hydrophobicity, size, and/or configuration such as, for example, substituting valine for phenyalanine. One of skill will recognize that there is more than one convention for identifying conservatively modified variants.

In some embodiments, the amino acid residues can be conservatively modified. In some embodiments, the amino acid residues can be non-conservatively modified. In some embodiments, for example, a lysine residue can be substituted with glutamic acid, or a glutamic acid residue can be substituted with lysine and, in some embodiments, these substitutions can be considered conservative. In some embodiments, if the N-terminal region is composed of more than 16 residues, then at least Lys⁶³ or Lys⁶⁷ of the C-terminal region is substituted with Glu;

The IP-10 chemokine and its analogs can affect biological funcions and can be designed to include a wide variety of modifications to provide, for example, a diagnostic, therapeutic and/or prophylactic activity in a subject. The term "subject" and "patient" can be used interchangeably in the present invention and, in most embodiments, refer to an animal such as a mammal including, but not limited to, non-primates such as, for example, a cow, pig, horse, cat, dog, rat and mouse; and primates such as, for example, a monkey or a human.

The IP-10 analogs can act as agonists or antagonists for an IP-10 chemokine in many embodiments. In some embodiments, the N-terminal region, the C-terminal region, or both, of an IP-10 chemokine can bind to an IP-10 receptor such as, for example, a CXCR3 receptor. In some embodiments, the beta sheet structure that connects the two termini may play a role in the stabilization of the IP-10 receptor and can help assure that the termini are in the proper conformation.

Examples of these analogs include compounds that contain structures corresponding to various select regions and combinations of regions within the IP-10 chemokine. In some embodiments, the IP-10 analog comprises an N-terminal region joined with a C-terminal region, wherein the means for joining the two regions is a linker. In some embodiments, the amino acid residues of the IP-10 analog can be cyclized, e.g., by etherification of lysine and serine residues or by other means described *infra* or known in the art.

In some embodiments, the IP-10 analog comprises a sequence derived from a wild-type IP-10 chemokine sequence but with one or more of the cysteines replaced with another amino acid, which can be a natural or non-natural amino acid. Some embodiments include IP-10 analogs comprising an N-terminal region, an internal region containing up to three anti-parallel β- sheets, a C-terminal region containing an α-helical structure, a combination of the N- and C-terminal regions linked together directly, a combination of an N-terminal and an internal region, a combination of a C-terminal region and an internal region, a combination of an N-terminal, internal and C-terminal region, and any combination thereof.

In some embodiments, the N-terminal, internal and C-terminal regions of the IP-10 analogs may be 2, 3, 4, 5, 6, 7, 8, 9, 10, 11, 12, 13, 14, 15, 16, 17, 20, 25, 30, 35, 40, 41, or 45 amino acids in length. In some embodiments, the IP-10 analogs can be 10-15, 12-17, 15-20, 20-25, 25-30, 30-35, 35-40, 30-50, 30-70, or any range therein, amino acids in length. It should be appreciated that, in some embodiments, the IP-10 analogs do not consist of more that 16 amino acids in the N-terminal region. In some embodiments, the N-terminal regions consist of 15 or 16 amino acids and, in these embodiments, the 15 or 16 amino acids can be the first 15 or 16 amino acids of the native IP-10 chemokine.

### Methods of Use

The IP-10 analogs can be used in treating, preventing, or ameliorating the symptoms of, several diseases. The terms "disease," "disorder," and "abnormal condition" can be used interchangeably in many embodiments and generally refer to a deviation from a normal function in an organism. An abnormal condition can comprise deviations, for example, in cell proliferation, cell differentiation, cell survival, cell migration or movement, or the activities of enzymes within a cell.

In some embodiments, the diseases and disorders may include, but are not limited to, hyperproliferative disorders, such as inflammatory conditions, autoimmune disorders, cancers, and vascular conditions; and neuropathies, such as inflammatory demyelinating neuropathies. In some embodiments, the diseases or disorders can include, but are not limited to, cardiovascular disease, neurological disorders, infectious disease, and inflammatory disorders that include rheumatoid arthritis, chronic inflammatory bowel disease, chronic inflammatory pelvic disease, multiple sclerosis, asthma, osteoarthritis, atherosclerosis, psoriasis, rhinitis, autoimmunity, organ transplant rejection, and genetic diseases. In some embodiments, the compositions can be used to increase the hemocrit, assist in mobilizing and recovering stem cells or progenitor cells, stimulate the production of blood cells, assist in vaccine production, assist in gene therapy, or increase apoptosis in tumors, or otherwise stimulate the immune system to effectuate tumor destruction.

The therapeutic and prophylactic effects of IP-10 mimetics can include, but are not limited to, one or more of the following: (a) an increase or decrease in the number of cells present at a specified location; (b) an increase or decrease in the ability of cells to migrate; (c) an increase or decrease in the response of cells to a stimulus; (d) an increase or decrease in the proliferation, growth, and/or differentiation of cells; (e) an inhibition or acceleration of apoptosis; (f) an ameliorization of one or more symptoms of disease; (g) an enhancement or inhibition of a cell function; and (h) an activation or inhibition of enzyme activity in cells. An increase in the number of cells is "cell expansion," and the IP-10 mimetics can cause this increase to occur *in vivo* or *ex vivo.* Accordingly, the IP-10 mimetics can be used to create mammal cells for diagnostic, prophylactic, therapeutic applications as well as commercial applications that include, but are not limited to, medicinal research and development of products such as, for example, vaccines.

Generally speaking, a function or activity of a chemokine receptor can be increased or decreased by contacting the receptor with an IP-10 mimetic to increase or decrease the probability of forming a complex between the receptor and a natural binding partner. The term "natural binding partner" includes, but is not limited to, G proteins, polypeptides, lipids, small molecules, or nucleic acids that bind to chemokine receptors in cells or in the extracellular environment. The term natural binding partner also includes substrates that can be acted upon by a chemokine receptor. The terms "modulate," "modulating," and the like, can be used in some embodiments to refer to altering the function or activity of a chemokine receptor by contacting it with a chemokine or chemokine analog and thus increasing or decreasing the probability that a complex forms between the receptor and a natural binding partner. The term "complex" refers to an assembly of at least two molecules bound to one another. The concentration of the IP-10 analog can be adjusted to control the degree of the effect.

The term "natural binding partner" refers to G proteins, polypeptides, lipids, small molecules, or nucleic acids that bind to IP-10 chemokine receptors in cells or in the extracellular environment. The term natural binding partner includes a substrate to be acted upon by the IP-10 chemokine receptor. A change in the interaction between an IP-10 chemokine receptor and a natural binding partner can manifest itself as an increased or decreased probability that the interaction forms, or an increased or decreased concentration of IP-10 chemokine receptor/natural binding partner complex. This can result in a decreased or increased activity of the IP-10 chemokine receptor.

In some embodiments, the terms "contact," "contacting," and the like, can refer to binding an IP-10 analog to a cell, and this can occur in vivo or in vitro, for example. In these embodiments, for example, an IP-10 analog can be contacted with a receptor by adding together a solution or a composition comprising the IP-10 chemokine or IP-10 analog with a liquid medium bath containing a polypeptide or a cell comprising an IP-10 chemokine receptor. The solution comprising the IP-10 chemokine or IP-10 analog may also comprise another component, such as dimethyl sulfoxide (DMSO), which facilitates the uptake of the IP-10 chemokine or IP-10 analog into the cells of the methods. The solution comprising the IP-10 chemokine or IP-10 analog may be added to the medium bathing the cells by utilizing a delivery apparatus, such as a pipette-based device or syringe-based device.

In some embodiments, an IP-10 mimetic can increase the probability of forming a complex between a chemokine receptor and a natural binding partner. In some embodiments, an IP-10 mimetic can decrease the probability of forming a complex between a chemokine receptor and a natural binding partner. In some embodiments, the concentration of an IP-10 mimetic can control the probability that a complex between a chemokine receptor and a natural binding partner will form. The term "complex" refers to a binding between at least two molecules.

In some embodiments, the IP-10 analogs can affect the formation of signal transduction complexes. A signal transduction complex often contains at least two protein molecules bound to one another. For instance, a protein tyrosine receptor protein kinase, GRB2, Sos (son of sevenless protein), Raf, and Ras assemble to form a signal transduction complex in response to a mitogenic ligand. In some embodiments, for example, a G protein can be bound to an IP-10 chemokine receptor, and the IP-10 analog can affect the formation of the complex.

The IP-10 mimetics of the present invention can be administered to treat, prevent, or ameliorate the symptoms of neurological disorders. Due to a traditional view that cells cannot be replaced, current treatments have focused on preventing cell death, but evidence has shown that some cells have the ability to regenerate. Recent research in the use of human stem cells and progenitor cells has catapulted cell replacement therapy to the position of a real alternative to current treatments such as, for example, treatments for neurodegenerative and other diseases. The term "stem cell" can refer to a cell that can be self-renewing over a prolonged time period and can generate multiple phenotypes in response to an exogenous signal. The term "progenitor cell" can refer to a cell that is more restricted in its differentiation capability and undergoes only limited self-renewal. For example, an eosinophil progenitor must become an eosinophil - it can't become a neutrophil, although both are granulocytes.

Due to the belief that brain and spinal cord cells cannot be replaced, treatments of neurological disorders have, as described above, focused on preventing neuronal death. Evidence has proven that some cells of the central nervous system such as, for example, neuronal stem cells, can regenerate. The term "neuronal stem cell" can refer to a cell that can self-renew, give rise to other cells through asymmetric cell division/differentiation, generate neural tissue, or are derived from the nervous system. Neuronal stem cells exist in a post-implantation developing mammalian nervous system as fetal stem cells and in the adult nervous system as adult stem cells. Neurones and neuronal progenitors can also be derived from the more primitive embryonic stem cell of the inner cell mass (ICM) of the pre- or peri-implantation embryo. For example, progenitor cells are found in an adult mammalian spinal cord and can be manipulated to mature into all of the major cell types that are found in the brain and spinal cord such as, for example, neurons and glial cells. These progenitor cells can be manipulated within the brain and spinal cord, for example, by administering growth factors.

The term "embryonic stem cell" refers to a primitive form of cell that can be isolated from an embryo days after conception, is unformed and unprogrammed, and has an innate ability to develop into *any* other type of cell in the body such as, for example, new brain cells, insulin-producing pancreas cells, heart muscle, and other tissues that could take the place of damaged or diseased cells. Accordingly, the implanting of embryonic stem cells can create not only neurons but also cells, tissues and organs of all other systems. When implanted into degenerating areas of the brain, for example, these cells have the capacity to acquire relevant cellular phenotype of the degenerated region and take on functions of cells that were lost through disease.

Examples of neurological disorders include, but are not limited to, Parkinson's disease, Alzheimer's disease, multiple sclerosis, and any other conditions associated with neuronal stem cells. Parkinson's disease, for example, can be treated with limited success in some cases by drugs including, but not limited to, L-beta-3,4-dihydroxyphenylalanine hydrochloride (L-Dopa). Alzheimer's disease, for example, is irreversible despite treatments with drugs including, but not limited to, ARICEPT^{®}. Such treatments have had limited success in ameliorating symptoms and slowing progression of the disease, but most patients with Alzheimer's disease require some sort of palliative care. Multiple sclerosis, for example, is sometimes responsive to the anti-inflammatory drugs including, but not limited to, interferon-β, but patients with multiple sclerosis remain incurable over the long term. As a result, subjects with multiple sclerosis develop permanent motor, sensory and cognitive deficits. In general, the treatment and reversal of neurodegenerative diseases of the brain and spinal cord remains a formidable challenge.

In some embodiments, a treatment of a neurological disorder can include administration of a composition comprising an IP-10 mimetic. In some embodiments, a treatment of a neurological disorder can include administering a composition comprising an IP-10 mimetic before, during or after administering one more other treatments or agents. In one example, an IP-10 mimetic can be administered as an agonist to mobilize stem cells in the treatment of a disease. In these embodiments, the IP-10 mimetic can contain the N-terminal portion of the IP-10 and the C-terminal portion of the IP-10 connected by a linker.

The IP-10 mimetics of the present invention can be administered to treat, prevent, or ameliorate the symptoms of autoimmune disorders. Autoimmune disorders can be organ-specific or systemic and are provoked by different pathogenic mechanisms. Examples of organ-specific autoimmune disorders include, but are not limited to, diabetes, hyperthyroidism, autoimmune adrenal insufficiency, pure red cell anemia, multiple sclerosis, and rheumatic carditis. Examples of systemic autoimmune diseases include systemic lupus erythematosus, rheumatoid arthritis, chronic inflammation, Sjogren's syndrome polymyositis, dermatomyositis and scleroderma. In some embodiments, a treatment of an autoimmune disorder can include administering a composition comprising an IP-10 mimetic. In some embodiments, a treatment of an autoimmune disorder can include administering a composition comprising an IP-10 mimetic before, during or after administering one or more other agents that are diagnostic, therapeutic, or a combination thereof.

The IP-10 mimetics of the present invention can be administered to treat, prevent, or ameliorate the symptoms of inflammatory disorders. Inflammatory disorders are manifested by an inflammatory response, which is initiated by injury that can be a result of trauma, ischemia or the introduction of foreign particles; and infection, which can be bacterial or viral. Inflammation includes a complex series of events including chemical mediators such as cytokines and prostaglandins; and inflammatory cells such as, for example, leukocytes.

The inflammatory response is a delicate interplay between the humoral and cellular immune elements that enables elimination of harmful agents and initiation of tissue repair. Unfortunately, the inflammatory response can be a disorder in its own right by causing considerable, and potentially more, damage to tissue than the disorder that initiated the inflammatory response. Examples of inflammatory disorders include, but are not limited to, acute and chronic inflammatory diseases such as, for example, arthritis, atheromas, colitis, chronic inflammatory bowel disease, chronic inflammatory pelvic disease, asthma, psoriasis, and rhinitis. Current treatments for inflammatory disorders include the use of non-steroidal anti-inflammatory drugs, which can cause, *inter alia,* gastrointestinal side effects; corticosteroids, which can cause, *inter alia,* an increased risk of infection; and immunosuppressive agents, which can leave a subject defenseless to infections.

In some embodiments, a treatment of an inflammatory disorder can include administering a composition comprising an IP-10 mimetic. In some embodiments, a treatment of an inflammatory disorder can include administering a composition comprising an IP-10 mimetic, either an agonist or antagonist, before, during or after administering one or more other agents. In some embodiments, the IP-10 mimetic can contain the N-terminal portion of the IP-10 and the C-terminal portion of the IP-10 connected by a linker.

The IP-10 mimetics of the present invention can be administered to transplant recipients. Transplant rejections occur in recipients receiving tissue from donors that differ genetically, and such rejections are mediated by T-cell dependent mechanisms. Immunosuppressive agents such as calcineurin phosphatase inhibitors and glucocorticosteroids are administered to transplant recipients to prevent allograft rejection. Immunosuppressive agents have a short lasting effect, so transplant recipients normally require life-long treatment with such agents. Life-long treatment with immunosuppressive agents creates serious adverse effects in a recipient such as, for example, the development of infections and tumors.

In some embodiments, the IP-10 analogs can be used in the treatment of graft rejection. The IP-10 mimetics of the present invention can be administered to transplant recipients to modulate cellular response and achieve effects that are diagnostic, therapeutic, prophylactic, ameliorative or a combination thereof. In some embodiments, a treatment of a transplant rejection can include administering a composition comprising an IP-10 mimetic for stem cell mobilization for transplantations. In some embodiments, a treatment of a transplant rejection can include administering a composition comprising an IP-10 mimetic before, during or after administering one or more other agents. In these embodiments, the IP-10 mimetic can contain the N-terminal portion of the IP-10 and the C-terminal portion of the IP-10 connected by a linker.

The IP-10 mimetics of the present invention can be administered to treat, prevent, or ameliorate the symptoms of cardiovascular disease. Cardiovascular disease is a broad, all-encompassing term that refers to a collection of diseases and conditions and is any disorder of the heart and blood vessels. Examples of cardiovascular disorders and treatments include, but are not limited to, any disease involving heart or vascular tissue such as, for example, atherosclerosis, and ischemic heart or vascular tissue requiring reconstruction and/or management. Traditional therapies used to treat cardiovascular disease have used either angioplasty procedures to compress blockages in arteries or coronary artery bypass grafts to provide an alternate path for blood flow around clogged vascular passageways. A latest generation of treatments includes "therapeutic angiogenesis," which is an inducement of angiogenesis to produce new blood vessels that supplement or replace a diseased vascular passageway.

In some embodiments, the IP-10 analogs can be used to treat or manage a wide variety of vascular conditions that include, for example, atherosclerosis, restenosis, vascular disorders associated with autoimmune diseases such as systemic lupus erythematosis, and ischemia-reperfusion. In some embodiments, the IP-10 analogs can be useful in either the promotion or inhibition of angiogenesis.

In some embodiments, a treatment of a cardiovascular disorder can include administering a composition comprising an IP-10 mimetic to modulate angiogenesis and assist in the reconstruction of heart or vascular tissue, which can include the proliferation and/or mobilization of endothelial cells such as, for example, vascular endothelial cells. In some embodiments, a treatment of a cardiovascular disorder can include administering compositions comprising an IP-10 mimetic before, during or after administering one more other agents. In these embodiments, the IP-10 mimetic can contain the N-terminal portion of the IP-10 and the C-terminal portion of the IP-10 connected by a linker.

The IP-10 mimetics can be used to treat conditions associated with cancer. The term "cancer" can refer to all types of cancer or neoplasm or malignant tumors found in mammals, especially humans, and include, but are not limited to, sarcomas, leukemias, carcinomas and melanoma. The methods of the present invention include treatments that slow the growth of tumors, prevent tumor growth, induce partial regression of tumors, and induce complete regression of tumors, to the point of complete disappearance. The methods also include preventing the outgrowth of metastases derived from solid tumors.

The term "leukemia," for example, refers broadly to progressive, malignant diseases of the blood-forming organs and is generally characterized by a distorted proliferation and development of leukocytes and their precursors in the blood and bone marrow. The major types of leukemias include acute myeloid leukemia, acute lymphoid leukemia, chronic myeloid leukemia, and chronic lymphoid leukemia. Acute leukemias are those that, if left untreated, can cause fatal complications in a very short period of time. Chronic leukemias, on the other hand, may not cause any problems at all for years. Hairy cell leukemia, a disease due to overproduction of mature lymphoid cells, affects blood, bone marrow, lymph glands, spleen and liver and is more commonly seen after age of 50.

The term "sarcoma" generally refers to a tumor which is made up of a substance like the embryonic connective tissue and is generally composed of closely packed cells embedded in a fibrillar or homogeneous substance. The term "melanoma" is taken to mean a tumor arising from the melanocytic system of the skin and other organs. The term "carcinoma" refers to a malignant new growth made up of epithelial cells tending to infiltrate the surrounding tissues and give rise to metastases.

The IP-10 analogs can be used to treat or manage a wide variety of cancers that include, but are not limited to, any of a variety of malignancies and their potential metastases, particularly solid and liquid human malignancies; and, relapses of the same. In some embodiments, the cell proliferation disorders include, but are not limited to, lung large cell carcinoma, colon adenocarcinoma, skin cancer (basal cell carcinoma and malignant melanoma), renal adenocarcinoma, promyelocytic leukemia, T cell lymphoma, cutaneous T cell lymphoma, breast adenocarcinoma, steroid sensitive tumors, hairy cell leukemia, Kaposi's Sarcoma, chronic myelogenous leukemia, multiple myeloma, superficial bladder cancer, ovarian cancer, and glioma.

In some embodiments, the cell proliferation disorders include cells derived from reproductive tissue (such as Sertoli cells, germ cells, developing or more mature spermatogonia, spermatids or spermatocytes and nurse cells, germ cells and other cells of the ovary), the lymphoid or immune systems (such as Hodgkin's disease and non-Hodgkin's lymphomas), the hematopoietic system, and epithelium (such as skin, including malignant melanoma, and gastrointestinal tract), solid organs, the nervous system, e.g. glioma (see Y.X. Zhou et al., 2002), and musculo-skeletal tissue.

In some embodiments, the cell proliferation disorders include solid tumor types, including, but not limited to, brain, including medulloblastoma, head and neck, breast, colon, small cell lung, large cell lung, thyroid, testicle, bladder, prostate, liver, kidney, pancreatic, esophogeal, stomach, ovarian, pr cervical tumors. Treatment of breast, colon, lung, and prostate tumors is particularly contemplated.

The IP-10 mimetics can be used to prevent, treat, or ameliorate the symptoms of, a variety of haematological disorders. Examples of hematological disorders include, but are not limited to, bone marrow depression, aplastic anemia, agranulocytosis, leukopenia, pancytopenia, thrombocytopenia, macrocytic or megaloblastic anemia. In some embodiments, a treatment of a hematological disorder can include managing white blood cells, platelets, red blood cells, stem cells and various progenitor subsets by administering a composition comprising an IP-10 mimetic. In some embodiments, an IP-10 mimetic can be administered as an agonist to mobilize stem cells in the treatment of a disease. In one example, an IP-10 can be administered to increase the proliferation of blood cells and/or mobilize the blood cells into the bloodstream. In some embodiments, a treatment of a hematological disorder can include administering a composition comprising an IP-10 mimetic before, during or after administration of one or more other treatments or agents. In these embodiments, the IP-10 mimetic can contain the N-terminal portion of the IP-10 and the C-terminal portion of the IP-10 connected by a linker.

The IP-10 analogs can be used to treat or manage a wide variety of conditions associated with cancers that include, but are not limited to, a diverse group of hematopoietic stem cell disorders known as myelodysplastic syndrome (MDS). Such disorders are characterized by a cellular marrow with an impaired morphology and maturation (dysmyelopoiesis), peripheral blood cytopenias, and a variable risk of progression to acute leukemia that results from an ineffective blood cell production. *See* The Merck Manual 953, (17^{th} ed. 1999).

Disorders associated with cancers can result from an initial hematopoietic stem cell injury that can be from a variety of causes including, but not limited to, cytotoxic chemotherapy, radiation therapy, virus, chemical exposure and genetic predisposition. A clonal mutation predominates over bone marrow and suppresses healthy stem cells. In the early stages of MDS, for example, a main cause of cytopenia is an increase in programmed cell death, or apoptosis. As the disease progresses and converts into leukemia, gene mutation rarely occurs and a proliferation of leukemic cells overwhelms the healthy marrow. The course of the disease can vary, and some cases can behave as an indolent disease, whereas others can behave aggressively with a short clinical progression into an acute form of leukemia. Subjects that survive a malignancy treatment with chemotherapy drugs such as, for example, alkylating agents, with or without radiotherapy, can have a high incidence of developing MDS or secondary acute leukemia. Examples of treatments for MDS include, but are not limited to, bone marrow transplantation, transfusions, and administration of hematopoietic growth factors and cytokines. Since the IP-10 mimetics are chemokines, they are a subspecies of cytokines.

In some embodiments, a treatment of a condition associated with cancer can include the administrating a composition comprising an IP-10 mimetic before, during or after chemotherapy or radiotherapy. In some embodiments, a treatment of a condition associated with cancer can include administering a composition comprising an IP-10 mimetic before, during or after administering one more other treatments or agents. In one example, an IP-10 mimetic can be administered as an antagonist to inhibit or prevent the proliferation of cancer cells. In another example, an IP-10 mimetic can be administered during recovery from chemotherapy and/or radiotherapy to expedite recovery of the blood count. In these embodiments, the IP-10 mimetic can contain the N-terminal portion of the IP-10 and the C-terminal portion of the IP-10 connected by a linker.

In some embodiments, the IP-10 analogs can be administered to prevent, treat, or ameliorate the symptoms of a bacterial infection, viral infection, and sepsis. The IP-10 analogs can also be instrumental in controlling tumirogenesis. Moreover, IP-10 chemokines have also been identified as playing a role in osteoporosis and, as such, the IP-10 analogs can be instrumental in the treatment of osteoporosis.

In some embodiments, the IP-10 analogs can be used in gene therapy to treat genetic disease. For example, the IP-10 analogs may prove useful in arresting the cell cycle for conducting gene therapy.

In some embodiments, the IP-10 analogs can be used to prepare vaccines, to enhance humoral antibody production, to increase antigen-presenting T-cells, to increase dendritic cells and immunological features known as vaccine induction, and combinations thereof. The term "antibody" can refer to any antibody-like molecule that has an antigen binding region, and includes antibody fragments such as Fab', Fab, F(ab')₂, single domain antibodies (DABs), Fv, scFv (single chain Fv), and the like. Techniques for preparing and using various antibody-based constructs and fragments are well known in the art as are techniques for preparing and characterizing antibodies. In some embodiments, the IP-10 analogs taught herein can be used as antigens to produce antibodies using methods well-known to those skilled in the art. In these embodiments, the antibody can be polyclonal or monoclonal. In some embodiments, the antibody is humanized.

### Synthesis and Design of the IP-10 Analogs

The amino acids used in the present invention may comprise an amino group and a carboxyl group, and the amino group may be primary or secondary. Examples of amino acids include, but are not limited to, glycine, alanine, valine, leucine, isoleucine, methionine, phenylalanine, tyrosine, aspartic acid, glutamic acid, lysine, arginine, serine, threonine, cysteine, asparagine, proline, tryptophan, histidine and combinations thereof.

In some embodiments, the amino acids may be limited to bifunctional amino acids, trifunctional amino acids, diamines, triamines, monocarboxylics, or dicarboxylics. In some embodiments, an amino acids may be limited to containing aliphatics or aromatics. In some embodiments, the amino acids may not include lysine. It is to be appreciated that one skilled in the art will recognize that some of the groups, subgroups, and individual amino acids may not be used in some embodiments of the present invention.

The natural amino acids are identified in the present application by the conventional one-letter and three-letter abbreviations as indicated below, and are preceded by "L-" to indicate their L-form and by "D-" to refer to their D form. These abbreviations are generally accepted in the peptide art as recommended by the IUPAC-IUB commission in biochemical nomenclature:

| | | | | | |
|---|---|---|---|---|---|
| Alanine | A | Ala | Leucine | L | Leu |
| Arginine | R | Arg | Lysine | K | Lys |
| Asparagine | N | Asn | Methionine | M | Met |
| Aspartic acid | D | Asp | Phenylalanine | F | Phe |
| Cysteine | C | Cys | Proline | P | Pro |
| Glutamic acid | E | Glu | Serine | S | Ser |
| Glutamine | Q | Gln | Threonine | T | Thr |
| Glycine | G | Gly | Tryptophan | W | Trp |
| Histidine | H | His | Tyrosine | Y | Tyr |
| Isoleucine | I | Ile | Valine | V | Val |
| Ornithine | O | Orn | | | |

All of the peptide sequences set out herein are written according to the generally accepted convention, whereby the N-terminal amino acid is on the left of a described sequence and the C-terminal amino acid is on the right of the described sequence.

### Synthesis of the IP-10 analogs

The IP-10 analog compounds may be prepared by standard techniques known in the art. A peptide or polypeptide component of an IP-10 analog may comprise, at least in part, a peptide synthesized using standard techniques (such as those described by Clark-Lewis, I., Dewald, B., Loetscher, M., Moser, B., and Baggiolini, M., (1994) J. Biol. Chem., 269, 16075-16081, Merrifield R. B. (1963) J Am Chem Soc., 85, 2149-2154). Automated peptide synthesizers are commercially available (e.g., Advanced ChemTech Model 396; Milligen/Biosearch 9600, Appliedbiosystems/Pioneer). The peptides and polypeptides may be assayed for IP-10 chemokine receptor agonist or antagonist activity in accordance with standard methods.

Peptides and polypeptides may be purified by HPLC and analyzed by mass spectrometry. Peptides and polypeptides may be dimerized. In one embodiment, peptides and polypeptides are dimerized via a disulfide bridge formed by gentle oxidation of the cysteines using 10% DMSO in water. Following HPLC purification, dimer formation may be verified, by mass spectrometry. One or more modifying groups may be attached to an IP-10 derived peptidic component by standard methods, for example, using methods for reaction through an amino group (e.g., the alpha-amino group at the amino-terminus of a peptide), a carboxyl group (*e.g*., at the carboxy terminus of a peptide), a hydroxyl group (*e.g*., on a tyrosine, serine or threonine residue) or other suitable reactive group on an amino acid side chain.

In some embodiments, the IP-10 analogs comprising the C-terminal and N-terminal regions joined by a linker could be cyclized. In these embodiments, the cyclization can occur in the C-terminal region using side-chain to side-chain; side-chain to scaffold or, scaffold to scaffold cyclization. In some embodiments, lactamization, etherification, or RCM (Ring Closing Methatesis) can be used to carry out this reaction.

For instance, IP-10 analogs may be cyclized using a lactam formation procedure by joining the γ-carboxy side chain or the α-carboxy moiety of glutamate (Glu) residue to the ε-amino side chain of lysine (Lys) residue, as indicated in the following sequences by underlining of linked residues. Lactams may for example be formed between glutamic acid and lysine (Lys) in the C-terminal portion of the polypeptide.

As described above, the IP-10 analogs can include conservative amino acid substitutions. In some embodiments, a conservatively modified sequence has one or more of the amino acid residues replaced by an amino acid residue having a side chain with similar properties. Families of amino acid residues having side chains with similar properties are well known in the art and there are a few different conventions for comparing amino acids. Generally speaking, these conventions typically include a characterization of the amino acids using some form of grouping by chemical structure. (Note that the numbering schemes used for the amino acids can also vary and do not always correspond to the native sequence; most often the numbering corresponds to the native sequence or to that of the analog). Any of the analogs taught herein can have 75, 80, 85, 90, 95, 97, 99%, or any range therein, homology to the corresponding regions of the native chemokine sequence, so long as the function of the respective analog is preserved. Percent homology can be determined using any method known to one of skill, such as the NCBI BLAST tool and techniques, for example, which is available at www.ncbi.nlm.nih.gov.

In some embodiments, the amino acids can be grouped as follows: amino acids with acidic side chains (*e.g*., aspartic acid, glutamic acid), basic side chains (*e.g*., lysine, arginine, histidine), uncharged polar side chains (*e.g*., glycine, asparagine, glutamine, serine, threonine, tyrosine, cysteine), nonpolar side chains (*e.g*., alanine, valine, leucine, isoleucine, proline, phenylalanine,methionine, tryptophan), beta-branched side chains (*e.g*., threonine, valine, isoleucine) and aromatic side chains (e.g., tyrosine, phenylalanine, tryptophan, histidine). Thus, in some embodiments, an amino acid residue in a chemokine can be replaced with another amino acid residue from the same side chain family. In some embodiments, the conservative substitutions include, but are not limited to, substituting a Lys with Orn or any other (L or D) natural or (L or D) non-natural amino acid having an amino group on its side chain, or a Glu with Asp. In some embodiments, the conservative substitutions include, but are not limited to, the following: Gly⇔Ala, Val⇔Ile⇔Leu, Asp⇔Glu, Lys⇔Arg, Asn⇔Gln, and Phe⇔Trp⇔Tyr.

### Recombinant Synthesis

IP-10 chemokine, IP-10 chemokine fragments, or IP-10 analogs may also be synthesized, in whole or in part, by recombinant methods using expression vectors encoding all or part of a IP-10 chemokine.

Vectors, or preferably expression vectors, may contain a gene encoding a polypeptide of the invention, a functional derivative thereof, or another useful polypeptide. These vectors may be employed to express the encoded polypeptide in either prokaryotic or eukaryotic cells.

The term "vector" in this application refers to a DNA molecule into which another DNA of interest can be inserted by incorporation into the DNA of the vector. One skilled in the art is familiar with the term. Examples of classes of vectors can be plasmids, cosmids, viruses, and bacteriophage. Typically, vectors are designed to accept a wide variety of inserted DNA molecules and then used to transfer or transmit the DNA of interest into a host cell (e.g., bacterium, yeast, higher eukaryotic cell). A vector may be chosen based on the size of the DNA molecule to be inserted, as well as based on the intended use. For transcription into RNA or transcription followed by translation to produce an encoded polypeptide, an expression vector would be chosen. For the preservation or identification of a specific DNA sequence (e.g., one DNA sequence in a cDNA library) or for producing a large number of copies of the specific DNA sequence, a cloning vector would be chosen. If the vector is a virus or bacteriophage, the term vector may include the viral/bacteriophage coat.

Following entry into a cell, all or part of the vector DNA, including the insert DNA, may be incorporated into the host cell chromosome, or the vector may be maintained extrachromosomally. Those vectors that are maintained extrachromosomally are frequently capable of autonomous replication in a host cell into which they are introduced (e.g., many plasmids having a bacterial origin of replication). Other vectors are integrated into the genome of a host cell upon introduction into the host cell, and thereby are replicated along with the host genome.

The term "expression vector" refers to a DNA construct which allows one to place a gene encoding a gene product of interest, usually a protein, into a specific location in a vector from which the selected gene product can be expressed by the machinery of the host cell, or alternately, by in vitro expression system. This type of vector is frequently a plasmid, but other forms of expression vectors, such as bacteriophage vectors and viral vectors (e.g., adenoviruses, replication defective retroviruses, and adeno-associated viruses), may be employed. The selection of expression vectors, control sequences, transformation methods, and the like, are dependent on the type of host cell used to express the gene.

### Prokaryotic hosts

Prokaryotic hosts are, in generally, very efficient and convenient for the production of recombinant polypeptides and are, therefore, one type of preferred expression system. Prokaryotes most frequently are represented by various strains of E. *coli,* but other microbial strains may be used, including other bacterial strains. Recognized prokaryotic hosts include bacteria such as *E. coli, Bacillus, Streptomyces, Pseudomonas, Salmonella, Serratia,* and the like. However, under such conditions, recombinantly-produced polypeptides will not be glycosylated.

In prokaryotic systems, vectors that contain replication sites and control sequences derived from a species compatible with the host may be used. Preferred prokaryotic vectors include plasmids such as those capable of replication in E. *coli* (such as, for example, pBR322, ColEl, pSC101, pACYC 184, pVX, pUC118, pUC119 and the like). Suitable phage or bacteriophage vectors may include λgt10, λgt11, vectors derived from filamentous bacteriophage such as m13, and the like. Suitable *Streptomyces* plasmids include p1J101, and streptomyces bacteriophages such as fC31. *Bacillus* plasmids include pC194, pC221, pT127, and the like. Suitable *Pseudomonas* plasmids have been reviewed by Izaki (Jpn. J. Bacteriol. 33:729-742, 1978) and John et al. (Rev. Infect. Dis. 8:693-704, 1986).

To express a protease of the invention (or a functional derivative thereof) in a prokaryotic cell, it is necessary to operably link the sequence encoding the protease of the invention to a functional prokaryotic promoter. Such promoters are either constitutive or inducible promoters, but commonly inducible promoters are used. Examples of constitutive promoters include the int promoter of bacteriophage λ, the bla promoter of the β-lactamase gene sequence of pBR322, and the cat promoter of the chloramphenicol acetyl transferase gene sequence of pPR325, and the like. Examples of inducible prokaryotic promoters include the major right and left promoters of bacteriophage λ (PL and PR), the trp, recA, lacZ, lacI, and gal promoters of E. *coli,* the α-amylase and the V-28-specific promoters of B. subtilis, the promoters of the bacteriophages of Bacillus, and Streptomyces promoters. Prokaryotic promoters are reviewed by Glick (Ind. Microbiot. 1:277-282, 1987), Cenatiempo (Biochimie 68:505-516, 1986), and Gottesman (Ann. Rev. Genet. 18:415-442, 1984). Additionally, proper expression in a prokaryotic cell also requires the presence of a ribosome-binding site upstream of the encoding sequence. Such ribosome-binding sites are disclosed, for example, by Gold et al. (Ann. Rev. Microbiol. 35:365-404, 1981).

### Fusion Protein

Proteins may be expressed as fusion proteins. Genes for proteins expressed as fusion proteins ligated into expression vectors that add a number of amino acids to a protein encoded and expressed, usually to the amino terminus of the recombinant protein. Such a strategy of producing fusion proteins is usually adopted for three purposes: (1) to assist in the purification by acting as a ligand in affinity purification, (2) to increase the solubility of the product, and (3) to increase the expression of the product. Often, expression vectors for use in fusion protein production, a proteolytic cleavage site is included at the junction of the fusion region and the protein of interest to enable purification of the recombinant protein away from the fusion region following affinity purification of the fusion protein. Such enzymes, and their cognate recognition sequences, include Factor Xa, thrombin and enterokinase, and may also include trypsin or chymotrypsin. Typical fusion expression vectors include pGEX (Pharmacia Biotech Inc; Smith, D. B. and Johnson, K. S. (1988) Gene 67:31-40), pMAL (New England Biolabs, Beverly, Mass.) and pRIT5 (Pharmacia, Piscataway, N.J.) which fuse glutathione S-transferase (GST), maltose E binding protein, or protein A, respectively, to the target recombinant protein.

### Improving Yield

Maximizing recombinant protein expression in E. *coli* can be assisted by expressing the protein or fusion protein in host bacteria with an impaired proteolytic system so as to reduce the post-synthesis degradation of the recombinant protein (Gottesman, S., Gene Expression Technology: Methods in Enzymology 185, Academic Press, San Diego, Calif. (1990) 119-128). Another strategy is to alter the mix of codons used in the coding sequence to reflect the usage of the individual codons for each amino acid in the host (*e.g., E*. *coli* (Wada et al., (1992) Nucleic Acids Res. 20:2111-2118)). Such alteration of nucleic acid sequences of the invention can be carried out by standard DNA synthesis techniques and may prove useful for a variety of prokaryotic and eukaryotic expression systems.

### Eukaryotic Hosts

Suitable hosts may include eukaryotic cells. Preferred eukaryotic hosts include, for example, yeast, fungi, insect cells, and mammalian cells both in vivo and in tissue culture. Useful mammalian cell hosts include HeLa cells, cells of fibroblast origin such as VERO or CHO-K1, and cells of lymphoid origin and their derivatives. Preferred mammalian host cells include SP2/0 and J558L, as well as neuroblastoma cell lines such as IMR 332, which may provide better capacities for correct post-translational processing. In general, eukaryotic organisms such as yeast provide substantial advantages in that they can also carry out post-translational modifications.

A large number of yeast expression systems may be potentially utilized which incorporate promoter and termination elements from the actively expressed sequences coding for glycolytic enzymes. These expression systems produce in large quantities of proteins when yeast are grown in mediums rich in glucose. Known glycolytic gene sequences can also provide very efficient transcriptional control signals. A number of recombinant DNA strategies exist utilizing strong promoter sequences and high copy number plasmids which can be utilized for production of the desired proteins in yeast. Examples of vectors suitable for expression in S. cerivisae include pYepSecl (Baldari, et al., (1987) Embo J. 6:229-234), pMFa (Kurjan and Herskowitz, (1982) Cell 30:933-943), pJRY88 (Schultz et al., (1987) Gene 54:113-123), pYES2 (InVitrogen Corporation, San Diego, Calif.), and picZ (InVitrogen Corp, San Diego, Calif.).

In another embodiment, the protein of interest may be expressed in insect cells for example the Drosophila larvae. Using insect cells as hosts, the Drosophila alcohol dehydrogenase promoter may be used (Rubin, Science 240:1453-1459, 1988). Additionally, baculovirus vectors can be engineered to express large amounts of the protein of interest in cultured insect cells (*e.g.*, Sf 9 cells)(Jasny, Science 238:1653, 1987; Miller et al., in: Genetic Engineering, Vol. 8, Plenum, Setlow et al., eds., pp. 277-297, 1986). Vectors which may be used include the pAc series (Smith et al. (1983) Mol. Cell Biol. 3:2156-2165) and the pVL series (Lucklow and Summers (1989) Virology 170:31-39).

Plant cells may also be utilized as hosts, and control sequences compatible with plant cells are available, such as the cauliflower mosaic virus 35S and 19S promoters, and nopaline synthase promoter and polyadenylation signal sequences. Furthermore, the protein of interest may be expressed in plants which have incorporated the expression vector into their germ line.

In yet another embodiment, a nucleic acid of the invention may be expressed in mammalian cells using a mammalian expression vector. Possibilities and techniques for expression in mammalian cells has recently been summarized (Colosimo, et al., "Transfer and expression of foreign genes in mammalian cells," Biotechniques 29(2):314-8, 320-2, 324 passim, 2000; which is hereby incorporated by reference in its entirety including any drawings, tables, and figures.). Examples of mammalian expression vectors include pCDM8 (Seed, B. (1987) Nature 329:840) and pMT2PC (Kaufinan et al. (1987) EMBO J. 6:187-195). For use in mammalian cells, the regulatory sequences of the expression vector are often derived from viral regulatory elements. For example, commonly used promoters are derived from Simian Virus 40 (SV40), polyoma, Adenovirus 2, and cytomegalovirus (CMV) viruses. Preferred eukaryotic promoters include, for example, the promoter of the mouse metallothionein I gene sequence (Hamer et al., J. Mol. Appl. Gen. 1:273-288, 1982); the TK promoter of Herpes virus (McKnight, Cell 31:355-365, 1982); the SV40 early promoter (Benoist et al., Nature (London) 290:304-31, 1981); and the yeast gal4 gene sequence promoter (Johnston et al., Proc. Natl. Acad. Sci. (USA) 79:6971-6975, 1982; Silver et al., Proc. Natl. Acad. Sci. (USA) 81:5951-5955, 1984). Alternatively, promoters from mammalian expression products, such as actin, collagen, myosin, and the like, may be employed. Regulatory elements may also be derived from adenovirus, bovine papilloma virus, cytomegalovirus, simian virus, or the like.

Transcriptional initiation regulatory signals may be selected which allow for repression or activation, so that expression of the gene sequences can be modulated. Of interest are regulatory signals which are temperature-sensitive so that by varying the temperature, expression can be repressed or initiated, or are subject to chemical (such as metabolite) regulation. Expression of proteins of interest in eukaryotic hosts requires the use of eukaryotic regulatory regions. Such regions will, in general, include a promoter region sufficient to direct the initiation of RNA synthesis.

The recombinant mammalian expression vector may also be designed to be capable of directing expression of the nucleic acid preferentially in a particular cell type (i.e., tissue-specific regulatory elements are used to control the expression). Such tissue-specific promoters include the liver-specific albumin promoter (Pinkert et al. (1987) Genes Dev. 1:268-277); lymphoid-specific promoters (*e.g*., Calame and Eaton (1988) Adv. Immunol. 43:235-275), and in particular promoters of immunoglobulins and T cell receptors (Winoto and Baltimore (1989) EMBO J. 8:729-733, Banerji et al. (1983) Cell 33:729-740; Queen and Baltimore (1983) Cell 33:741-748); mammary gland-specific promoters (*e.g*., milk whey promoter; U.S. Pat. No. 4,873,316 and European Application Publication No. 264,166); and pancreas-specific promoters (Edlund et al. (1985) Science 230:912-916). Developmentally-regulated promoters may also be utilized, for example, the α-fetoprotein promoter (Campes and Tilghman (1989) Genes Dev. 3:537-546), and the murine hox promoters (Kessel and Gruss (1990) Science 249:374-379).

Preferred eukaryotic plasmids include, for example, SV40, BPV, pMAM-neo, pKRC, vaccinia, 2-micron circle, and the like, or their derivatives. Such plasmids are well known in the art (Botstein et al., Miami Wntr. Symp. 19:265-274, 1982; Broach, In: "The Molecular Biology of the Yeast Saccharomyces: Life Cycle and Inheritance," Cold Spring Harbor Laboratory, Cold Spring Harbor, NY, p. 445-470, 1981; Broach, Cell 28:203-204, 1982; Bollon et al., J. Clin. Hematol. Oncol. 10:39-48, 1980; Maniatis, In: Cell Biology: A Comprehensive Treatise, Vol. 3, Gene Sequence Expression, Academic Press, NY, pp. 563-608, 1980).

Once the vector or nucleic acid molecule containing the construct(s) has been prepared for expression, the DNA construct(s) may be introduced into an appropriate host cell by any of a variety of suitable means, i.e., transformation, transfection, conjugation, protoplast fusion, electroporation, particle gun technology, DEAE-dextran-mediated transfection, lipofection, calcium phosphate-precipitation, direct microinjection, and the like. Suitable methods for transforming or transfecting host cells can be found in Sambrook, *et al.* (2001). After the introduction of the vector, recipient cells are grown in a selective medium, which selects for the growth of vector-containing cells. Expression of the cloned gene(s) results in the production of a protein of interest, or fragments thereof.

For other suitable expression systems for both prokaryotic and eukaryotic cells see Sambrook, et al., "Molecular Cloning: A Laboratory Manual," 3rd ed., Cold Spring Harbor Laboratory, Cold Spring Harbor Laboratory Press, Cold Spring Harbor, N.Y., 2001, which is hereby incorporated by reference in its entirety, including any drawings, figures, and tables.

For transformation of eukaryotic cells, it is known that, depending upon the expression vector and transfection technique used, only a small fraction of cells may integrate the foreign DNA into their genome. In order to identify and select these integrants, a gene that encodes a selectable marker (*e.g*., resistance to antibiotics) is generally introduced into the host cells along with the gene of interest. Preferred selectable markers include those which confer resistance to drugs, such as G418, hygromycin, neomycin, methotrexate, glyphosate, and bialophos. Nucleic acid encoding a selectable marker can be introduced into a host cell on the same vector as that encoding the protein of interest or can be introduced on a separate vector. Cells stably transformed with the introduced nucleic acid can be identified by drug selection (*e.g*., cells that have incorporated the selectable marker gene will survive, while the other cells die).

A host cell of the invention, such as a prokaryotic or eukaryotic host cell in culture, can be used to produce (i.e., express) the protein of interest. Accordingly, the invention further provides methods for producing the protein of interest using the host cells of the invention. In one embodiment, the method comprises culturing the host cell into which a recombinant expression vector encoding the protein of interest has been introduced in a suitable medium such that the protein of interest is produced, and may be purified by one skilled in the art.

In many embodiments, the IP-10 analogs are designed using one or more of the following: (1) replacing all or part of the beta-sheet domain of an IP-10 chemokine with a linker; (2) connecting all or a portion of the amino-terminal domain and all or a portion of the carboxy-terminal domain of an IP-10 chemokine with a linker; (3) cyclizing residues within an analog; (4) replacing the cysteines of an IP-10 chemokine with other amino acids; and (5) attaching modifying groups to an IP-10 analog such as, for example, to the amino terminus, carboxy terminus, or a combination thereof.

### Modifying Groups

Generally speaking, the IP-10 mimetics may include derivatives of IP-10 chemokines, analogs of IP-10 chemokines, and conservatively modified variants thereof. Examples of such mimetics include C-terminal hydroxymethyl derivatives; O-modified derivatives (e.g., C-terminal hydroxymethyl benzyl ether); N-terminally modified derivatives including substituted amides such as alkylamides and hydrazides and compounds in which a C-terminal phenylalanine residue is replaced with a phenethylamide analogue (*e.g*., Ser-Ile-phenethylamide as an analog of the tripeptide Ser-Ile-Phe); glycosylated IP-10 chemokine derivatives; polyethylene glycol modified derivatives; biotinylated derivatives; and combinations thereof. The IP-10 analogs may also include a pharmaceutically acceptable salt of an IP-10 analog.

In some embodiments, the IP-10 analogs may be coupled directly or indirectly to at least one modifying group. The term "modifying group" can be used in some embodiments to describe structures that are directly attached to the peptidic structure (*e.g*., by covalent bonding or covalent coupling), as well as those that are indirectly attached to the peptidic structure (*e.g*., by a stable non-covalent bond association or by covalent coupling through a linker to additional amino acid residues). The term "modifying group" may also refer to mimetics, analogues or derivatives thereof, which may flank the IP-10 core peptidic structure.

In some embodiments, the modifying groups can be coupled to the amino-terminus or carboxy-terminus of an IP-10 peptidic structure, or to a peptidic or peptidomimetic region flanking the core structure. Alternatively, the modifying group can be coupled to a side chain of at least one amino acid residue of an IP-10 peptidic structure, or to a peptidic or peptido-mimetic region flanking the core domain (*e.g*., through the epsilon amino group of a lysyl residue(s); through the carboxyl group of an aspartic acid residue(s) or a glutamic acid residue(s); through a hydroxy group of a tyrosyl residue(s), a serine residue(s) or a threonine residue(s); or any other suitable reactive group on an amino acid side chain). The modifying groups can be covalently coupled to the peptidic structure and can be attached by means and methods well known in the art for linking chemical structures, including, for example, amide, alkylamino, sulfide, carbamate or urea bonds.

In some embodiments, the modifying group may comprise a cyclic, heterocyclic or polycyclic group. The term "cyclic group," as used herein, includes cyclic saturated or unsaturated (i.e., aromatic) group having from 3 to 10; from 4 to 8; or 5, 6, or 7 carbon atoms. Exemplary non-aromatic cyclic groups include cyclopropyl, cyclobutyl, cyclopentyl, cyclohexyl, and cyclooctyl groups. The term "heterocyclic group" includes optionally substituted, saturated or unsaturated, three- to eight-membered cyclic structures in which one or more skeletal atoms is oxygen, nitrogen, sulfur, or combinations thereof.

Cyclic groups may be unsubstituted or substituted at one or more ring positions. A cyclic group may for example be substituted with halogens, alkyls, cycloalkyls, alkenyls, alkynyls, aryls, arylalkyls, heterocycles, hydroxyls, aminos, nitros, thiols amines, imines, amides, phosphonates, phosphines, carbonyls, carboxyls, silyls, ethers, thioethers, sulfonyls, sulfonates, selenoethers, ketones, aldehydes, esters, -CF₃, -CN. The cyclic group may also be linked to a substituent, such as halogens, alkyls, cycloalkyls, alkenyls, alkynyls, aryls, arylalkyls, heterocycles, hydroxyls, aminos, nitros, thiols amines, imines, amides, phosphonates, phosphines, carbonyls, carboxyls, silyls, ethers, thioethers, sulfonyls, sulfonates, selenoethers, ketones, aldehydes, esters, -CF₃, or -CN, by means of a saturated or unsaturated chain of 1, 2, 3, 4, 5, 6, 7, 8, or more carbon atoms; additionally, one or more of the carbon atoms may be replaced with an oxygen, nitrogen, or sulfur atom. One of skill will appreciate that these means of linking are taught for example only, and that other means of linking are available in the art.

The term "heterocyclic group" includes cyclic saturated, unsaturated and aromatic groups having from 3 to 10; from 4 to 8; or 5, 6, or 7 carbon atoms, wherein the ring structure includes about one or more heteroatoms. Heterocyclic groups include pyrrolidine, oxolane, thiolane, imidazole, oxazole, piperidine, piperazine, morpholine. The heterocyclic ring may be substituted at one or more positions with such substituents as, for example, halogens, alkyls, cycloalkyls, alkenyls, alkynyls, aryls, arylalkyls, other heterocycles, hydroxyl, amino, nitro, thiol, amines, imines, amides, phosphonates, phosphines, carbonyls, carboxyls, silyls, ethers, thioethers, sulfonyls, selenoethers, ketones, aldehydes, esters, -CF₃, - CN. Heterocycles may also be bridged or fused to other cyclic groups as described below. A linker may also link the heterocyclic group to such substituents as, for example, halogens, alkyls, cycloalkyls, alkenyls, alkynyls, aryls, arylalkyls, heterocycles, hydroxyls, aminos, nitros, thiols amines, imines, amides, phosphonates, phosphines, carbonyls, carboxyls, silyls, ethers, thioethers, sulfonyls, sulfonates, selenoethers, ketones, aldehydes, esters, -CF₃, -CN.

The term "polycyclic group" as used herein is intended to refer to two or more saturated, unsaturated, or aromatic cyclic rings in which two or more carbons are common to two adjoining rings, so that the rings are "fused rings." Rings that are joined through non-adjacent atoms are termed "bridged" rings. Each of the rings of the polycyclic group may be substituted with such substituents as described above, as for example, halogens, alkyls, cycloalkyls, alkenyls, alkynyls, hydroxyl, amino, nitro, thiol, amines, imines, amides, phosphonates, phosphines, carbonyls, carboxyls, silyls, ethers, thioethers, sulfonyls, selenoethers, ketones, aldehydes, esters, -CF₃, or -CN.

The term "alkyl" refers to saturated aliphatic groups, including straight chain alkyl groups, branched-chain alkyl groups, cycloalkyl (alicyclic) groups, alkyl substituted cycloalkyl groups, and cycloalkyl substituted alkyl groups. In some embodiments, a straight chain or branched chain alkyl has 20 or fewer carbon atoms in its backbone (C₁-C₂₀ for straight chain, C₃-C₂₀ for branched chain), 15 or few carbon atoms; 12 or fewer carbon atoms; or 10 or fewer carbon atoms. In some embodiments, cycloalkyls may have from 4-10 carbon atoms in their ring structure, such as rings made from 5, 6 or 7 carbon atoms. Unless the number of carbons is otherwise specified, "lower alkyl" as used herein means an alkyl group, as defined above, having from one to ten carbon atoms in its backbone structure. Likewise, "lower alkenyl" and "lower alkynyl" have chain lengths of ten or less carbons.

The term "alkyl" (or "lower alkyl") as used throughout the specification and claims is intended to include both "unsubstituted alkyls" and "substituted alkyls," the latter of which refers to alkyl moieties having substituents replacing a hydrogen on one or more carbons of the hydrocarbon backbone. Such substituents can include, for example, halogen, hydroxyl, carbonyl (such as carboxyl, ketones (including alkylcarbonyl and arylcarbonyl groups)), and esters (including alkyloxycarbonyl and aryloxycarbonyl groups), thiocarbonyl, acyloxy, alkoxyl, phosphoryl, phosphonate, phosphinate, amino, acylamino, amido, amidine, imino, cyano, nitro, azido, sulfhydryl, alkylthio, sulfate, sulfonate, sulfamoyl, sulfonamido, heterocyclyl, aralkyl, or an aromatic or heteroaromatic moiety.

The moieties substituted on the hydrocarbon chain can themselves be substituted, if appropriate. For instance, the substituents of a substituted alkyl may include substituted and unsubstituted forms of aminos, azidos, iminos, amidos, phosphoryls (including phosphonates and phosphinates), sulfonyls (including sulfates, sulfonamidos, sulfamoyls and sulfonates), or silyl groups, as well as ethers, alkylthios, carbonyls (including ketones, aldehydes, carboxylates, and esters), -CF₃, -CN and the like. Exemplary substituted alkyls are described below. Cycloalkyls can be further substituted with alkyls, alkenyls, alkoxys, alkylthios, aminoalkyls, carbonyl-substituted alkyls, -CF3, -CN, and the like.

The terms "alkenyl" and "alkynyl" refer to unsaturated aliphatic groups analogous in length and possible substitution to the alkyls described above, but that contain at least one double or triple bond respectively. The term "aralkyl," as used herein, refers to an alkyl or alkylenyl group substituted with at least one aryl group. Exemplary aralkyls include benzyl (i.e., phenylmethyl), 2-naphthylethyl, 2-(2-pyridyl)propyl, 5-dibenzosuberyl, and the like. The term "alkylcarbonyl," as used herein, refers to -C(O)-alkyl. Similarly, the term "arylcarbonyl" refers to -C(O)-aryl. The term "alkyloxycarbonyl," as used herein, refers to the group -C(O)-O-alkyl, and the term "aryloxycarbonyl" refers to -C(O)-O-aryl. The term "acyloxy" refers to -O-C(O)-R₇, in which R₇ is alkyl, alkenyl, alkynyl, aryl, aralkyl or heterocyclyl.

The term "amino," as used herein, refers to -N(R_{α})(R_{β}), in which R_{α} and R_{β} are each independently hydrogen, alkyl, alkyenyl, alkynyl, aralkyl, aryl, or in which R_{α} and R_{β} together with the nitrogen atom to which they are attached form a ring having 4-8 atoms. Thus, the term "amino," as used herein, includes unsubstituted, monosubstituted (e.g., monoalkylamino or monoarylamino), and disubstituted (e.g., dialkylamino or alkylarylamino) amino groups. The term "amido" refers to -C(O)-N(R_{α})(R_{β}), in which R_{α} and R_{β} are as defined above. The term "acylamino" refers to -N(R'_{α})C(O)-R₇, in which R₇ is as defined above and R_{α} is alkyl. As used herein, the term "nitro" means -NO₂; the term "halogen" designates -F, -Cl, -Br or -I; the term "sulfhydryl" means -SH; and the term "hydroxyl" means -OH.

The term "aryl" as used herein includes 5-, 6- and 7-membered aromatic groups that may include from zero to four heteroatoms in the ring, for example, phenyl, pyrrolyl, furyl, thiophenyl, imidazolyl, oxazole, thiazolyl, triazolyl, pyrazolyl, pyridyl, pyrazinyl, pyridazinyl and pyrimidinyl, and the like. Those aryl groups having heteroatoms in the ring structure may also be referred to as "aryl heterocycles" or "heteroaromatics." The aromatic ring can be substituted at one or more ring positions with such substituents as described above, as for example, halogen, azide, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, sulfonamido, ketone, aldehyde, ester, a heterocyclyl, an aromatic or heteroaromatic moiety, -CF₃, -CN, or the like. Aryl groups can also be part of a polycyclic group. For example, aryl groups include fused aromatic moieties such as naphthyl, anthracenyl, quinolyl, indolyl, and the like.

In some embodiments, the molecular weight of a modifying group should be at or below about 40,000 Daltons. In some embodiments, the molecular weight ranges from about 100 Daltons to about 40,000 Daltons, from about 300 Daltons to about 30,000 Daltons, from about 200 Daltons to about 20,000 Daltons, from about 200 Daltons to about 10,000 Daltons, from about 300 Daltons to about 5,000 Daltons, from about 500 Daltons to about 500 Daltons, or any range therein. It is to be appreciated that one skilled in the art should recognize that some of the groups, subgroups, and individual biobeneficial agents may not be used in some embodiments of the present invention.

### Functional Enhancement

An IP-10 chemokine analog compound of the invention may be further modified to alter the specific properties of the compound while retaining the desired functionality of the compound. For example, in one embodiment, the compound may be modified to alter a pharmacokinetic property of the compound, such as in vivo stability, solubility, bioavailability or half-life. The compound may be modified to label the compound with a detectable substance. In addition, the compound may be modified to couple the compound to an additional therapeutic moiety.

To further chemically modify the compound, such as to alter its pharmacokinetic properties, reactive groups can be derivatized. For example, when the modifying group is attached to the amino-terminal end of the IP-10 core domain, the carboxy-terminal end of the compound may be further modified. Potential C-terminal modifications include those that reduce the ability of the compound to act as a substrate for carboxypeptidases. Examples of C-terminal modifiers include an amide group, an ethylamide group and various non-natural amino acids, such as D-amino acids, β-alanine, C-terminal decarboxylation, and a C-terminal alcohol. Alternatively, when the modifying group is attached to the carboxy-terminal end of the aggregation core domain, the amino-terminal end of the compound may be further modified, for example, to reduce the ability of the compound to act as a substrate for aminopeptidases.

IP-10 chemokines and IP-10 analogs of the invention may be modified by the addition of polyethylene glycol (PEG). PEG modification may lead to improved circulation time, improved solubility, improved resistance to proteolysis, reduced antigenicity and immunogenicity, improved bioavailability, reduced toxicity, improved stability, and easier formulation (For a review see, Francis et al., International Journal of Hematology 68:1-18, 1998). PEGylation may also result in a substantial reduction in bioactivity. In some embodiments, the molecular weight of the PEG can be 40,000 Daltons or less, 30,000 Daltons or less, 20,000 Daltons or less, 10,000 Daltons or less, 5000 Daltons or less, 3000 Daltons or less 1000 Daltons or less, or any range therein. In some embodiments, the molecular weight of the PEG can range from about 100 Daltons to about 20,000 Daltons, from about 200 Daltons to about 5,000 Daltons, from about 300 Daltons to about 3000 Daltons, from about 400 Daltons to about 2000 Daltons, from about 500 Daltons to about 1000 Daltons, or any range therein.

The IP-10 analogs of the invention may also be coupled to a radioisotope such as yttrium-90, F-18 or iodine-131 for therapeutic purposes (*see*, *e.g.,* DeNardo et al., "Choosing an optimal radioimmunotherapy dose for clinical response, "Cancer 94(4 Suppl):1275-86, 2002; Kaltsas et al., "The value of radiolabelled MIBG and octreotide in the diagnosis and management of neuroendocrine tumours," Ann Oncol 12 Suppl 2:S47-50, 2001).

An IP-10 chemokine mimetic compound can be further modified to label the compound by combining the compound with a detectable substance. In some aspects of the invention, suitable detectable substances include various enzymes, prosthetic groups, fluorescent materials, luminescent materials, light scattering or plasmon resonant materials, and radioactive materials. Modifying groups may also include groups comprising biochemical labels or structures, such as biotin, a diethylene-triaminepentaacetyl group, a (O)-menthoxyacetyl group, a N-acetylneuraminyl group, a cholyl structure or an iminobiotinyl group.

Examples of suitable enzymes include horseradish peroxidase, alkaline phosphatase, beta-galactosidase, or acetylcholinesterase. Examples of suitable prosthetic groups which are members of a binding pair and are capable of forming complexes include streptavidin/biotin, avidin/biotin and an antigen/antibody complex (e.g., rabbit IgG and anti-rabbit IgG).

Examples of suitable fluorescent materials include umbelliferone, fluorescein, fluorescein isothiocyanate, rhodamine, dichlorotriazinylamine fluorescein, dansyl chloride or phycoerythrin and energy transfer fluorescent dyes. An example of a luminescent material includes luminol. Examples of light scattering or plasmon resonant materials include gold or silver particles and quantum dots. Examples of suitable radioactive material include ¹⁴C, ¹²³I, ¹²⁴I, ¹²⁵I, ¹³¹I, Tc99m, ³⁵S or ³H. A chemokine mimetic compound may be radioactively labeled with ¹⁴C, either by incorporation of ¹⁴C into the modifying group or one or more amino acid structures in the chemokine mimetic compound.

Labeled chemokine mimetic compounds may be used to assess the in vivo pharmacokinetics of the compounds, as well as to detect disease progression or propensity of a subject to develop a disease, for example for diagnostic purposes. Tissue distribution can be detected using a labeled chemokine mimetic compound either in vivo or in an in vitro sample derived from a subject. A modifying group can be chosen that provides a site at which a chelation group for the label can be introduced, such as the Aic derivative of cholic acid, which has a free amino group. For example, a tyrosine residue within IP-10 sequence may be substituted with radioactive iodotyrosyl. Any of the various isotopes of radioactive iodine may be incorporated to create a diagnostic or therapeutic agent. For example, a chemokine mimetic compound of the invention may be labeled with radioactive technetium or iodine for use as a diagnostic agent: ¹²³I (half-life=13.2 hours) may be used for whole body scintigraphy, ¹²⁴I (half life=4 days) may be used for positron emission tomography (PET), ¹²⁵I (half life=60 days) may be used for metabolic turnover studies and ¹³¹I (half life=8 days) may be used for whole body counting and delayed low resolution imaging studies.

In some embodiments, the IP-10 analog may be modified at its carboxy terminus with a cholyl group according to methods known in the art. Cholyl derivatives and analogs may also be used as modifying groups. For example, a preferred cholyl derivative is Aic (3-(O-aminoethyl-iso)-cholyl), which has a free amino group that can be used to further modify the chemokine mimetic compound. A modifying group may be a "biotinyl structure," which includes biotinyl groups and analogues and derivatives thereof (such as a 2-iminobiotinyl group).

In some embodiments, the modifying group may comprise a fluorescent-label group, e.g., a fluorescein-containing group, such as a group derived from reacting an IP-10 derived peptidic structure with 5-(and 6-)-carboxyfluorescein, succinimidyl ester or fluorescein isothiocyanate. The IP-10 analogs may also be modified by attaching other fluorescent labels including rhodamine, dichlorotriazinylamine fluorescein, dansyl chloride or phycoerythrin and energy transfer fluorescent dyes or fluorescent ion indicators.

In various embodiments, the modifying group(s) may comprise an N-acetylneuraminyl group, a trans-4-cotininecarboxyl group, a 2-imino-1-imidazolidineacetyl group, an (S)-(-)-indoline-2-carboxyl group, a (-)-menthoxyacetyl group, a 2-norbomaneacetyl group, a γ-oxo-5-acenaphthenebutyryl, a (-)-2-oxo-4-thiazolidinecarboxyl group, a tetrahydro-3-furoyl group, a 2-iminobiotinyl group, a diethylenetriaminepentaacetyl group, a 4-morpholinecarbonyl group, a 2-thiopheneacetyl group or a 2-thiophenesulfonyl group.

In some embodiments, light scattering groups, magnetic groups, nanogold, peptides, proteins, a solid matrix, radiolabels, or carbohydrates may be attached. In some embodiments, the modifying group may be an oligomer such as, for example, an oligonucleotide, or an oligopeptide (which may or may not be derived from an IP-10 chemokine). In some embodiments, the modifying group can include a polysaccharide, a glycosaminoglycan, a poly(alkylene glycol), or a combination thereof.

In some embodiments, the modifying group can be an RDG or RGD peptide, a hydrolyzable chemical moiety for a prodrug form of the IP-10 analog, or another therapeutic agent connected to the IP-10 analog through a hydrolysable chemical moiety to form a codrug form of the IP-10 analog. The prodrugs and codrugs are capable of being transformed, upon metabolism in vivo, into an IP-10 analog agonist or antagonist. Such a form of a modifying group can be referred to as a "secondary modifying group." A variety of strategies are known in the art for preparing peptide prodrugs and codrugs that limit metabolism in order to optimize delivery of the active form of the peptide-based drug.

### IP-10 Sequence Listings:

Using the teachings provided herein, one of skill can create a variety of IP-10 analogs. Preferred embodiments of linear IP-10 analogs of the present invention corresponding to a portion ofN-terminal have the following structures, wherein the underlined residues are cyclized, and in some embodiments the cyclization may include, for example, formation of a disulphide bond, an alkenyl bond, an ether bond, or a lactam; and, any additional amino acids identified in square brackets in a header indicates that the peptide described by the header is at least an analog of a native fragment, and the position of the amino acid substitutions are noted by the standard abbreviation for the amino acid substituent with a superscript number indicating the residue positions at which the modification has occurred, corresponding to the native sequence:
IP-10-(1-14) acid or amide
   b1) RNH-Val-Pro-Leu-Ser-Arg-Thr-Val-Arg-Cys-Thr-Cys-Ile-Ser-Ile-(OH)NH₂ (SEQ ID NO:163)
   b2) RNH-Val-Pro-Xaa₃-Ser-Arg-Thr-Val-Arg-Xaa₁-Thr-Xaa₂-Ile-Ser-Ile-(OH)NH₂ (SEQ ID NO:164)
   b3) RNH-Val-Pro-Leu-Xaa₃-Arg-Thr-Val-Arg-Xaa₁-Thr-Xaa₂-Ile-Ser-Ile-(OH)NH₂ (SEQ ID NO:165)
   b4) RNH-Val-Pro-Leu-Ser-Xaa₃-Thr-Val-Arg-Xaa₁-Thr-Xaa₂-Ile-Ser-Ile-(OH)NH₂ (SEQ ID NO:166)
   b5) RNH-Val-Pro-Leu-Ser-Arg-Xaa₃-Val-Arg-Xaa₁-Thr-Xaa₂-Ile-Ser-Ile-(OH)NH₂ (SEQ ID N0:167)
   b6) RNH-Val-Pro-Leu-Ser-Arg-Thr-Xaa₃-Arg-Xaa₁-Thr-Xaa₂-Ile-Ser-Ile-(OH)NH₂ (SEQ ID N0:168)
   b7) RNH-Val-Pro-Leu-Ser-Arg-Thr-Val-Arg-Xaa₃-Xaa₁-Thr-Xaa₂-Ile-Ser-Ile-(OH)NH₂ (SEQ ID NO:169)
IP-10-(1-17) acid or amide
   b8) RNH-Val-Pro-Leu-Ser-Arg-Thr-Val-Arg-Cys-Thr-Cys-Ile-Ser-Ile-Ser-Asn-Gln-(OH)NH₂ (SEQ ID NO:170)
   b9) RNH-Val-Pro-Xaa₃-Ser-Arg-Thr-Val-Arg-Xaa₁-Thr-Xaa₂-Ile-Ser-Ile-Ser-Asn-Gln-(OH)NH₂ (SEQ ID NO:171)
   b10) RNH-Val-Pro-Leu-Xaa₃-Arg-Thr-Val-Arg-Xaa₁-Thr-Xaa₂-Ile-Ser-Ile-Ser-Asn-Gln-(OH)NH₂ (SEQ ID NO: 172)
   b11) RNH-Val-Pro-Leu-Ser-Xaa₃-Thr-Val-Arg-Xaa₁-Thr-Xaa₂-Ile-Ser-Ile-Ser-Asn-Gln-(OH)NH₂ (SEQ ID NO: 173)
   b12) RNH-Val-Pro-Leu-Ser-Arg-Xaa₃-Val-Arg-Xaa₁-Thr-Xaa₂-Ile-Ser-Ile-Ser-Asn-Gln-(OH)NH₂ (SEQ ID N0:174)
   b13) RNH-Val-Pro-Leu-Ser-Arg-Thr-Xaa₃-Arg-Xaa₁-Thr-Xaa₂-Ile-Ser-Ile-Ser-Asn-Gln-(OH)NH₂ (SEQ ID N0:175)
   b14) RNH-Val-Pro-Leu-Ser-Arg-Thr-Val-Arg-Xaa₃-Xaa₁-Thr-Xaa₂-Ile-Ser-Ile-Ser-Asn-Gln-(OH)NH₂ (SEQ ID NO: 176)

Preferred embodiments of linear IP-10 analogs of the present invention corresponding to a portion of the internal region of IP-10 having the following structures:
[A¹¹]-IP-10-(11-35) acid or amide
   b15) RNH-Ala-Ile-Ser-Ile-Ser-Asn-Gln-Pro-Val-Asn-Pro-Arg-Ser-Leu-Glu-Lys-Leu-Glu-Ile-Ile-Pro-Ala-Ser-Gln-Phe-(OH)NH₂ (SEQ ID NO:177)

Preferred embodiments of linear IP-10 analogs of the present invention corresponding to a portion of the N-terminal region and the internal region of IP-10 having the following structures:
IP-10-(1-35) acid or amide
   b16) RNH-Val-Pro-Leu-Ser-Arg-Thr-Val-Arg-Cys-Thr-Cys-Ile-Ser-Ile-Ser-Asn-Gln-Pro-Val-Asn-Pro-Arg-Ser-Leu-Glu-Lys-Leu-Glu-Ile-Ile-Pro-Ala-Ser-Gln-Phe-(OH)NH₂ (SEQ ID NO:178)
   b17) RNH-Xaa₃-Pro-Leu-Ser-Arg-Thr-Val-Arg-Xaa₁-Thr-Xaa₂-Ile-Ser-Ile-Ser-Asn-Gln-Pro-Val-Asn-Pro-Arg-Ser-Leu-Glu-Lys-Leu-Glu-Ile-Ile-Pro-Ala-Ser-Gln-Phe-(OH)NH₂ (SEQ ID NO: 179)
   b18) RNH-Val-Xaa₃-Leu-Ser-Arg-Thr-Val-Arg-Xaa₁-Thr-Xaa₂-Ile-Ser-Ile-Ser-Asn-Gln-Pro-Val-Asn-Pro-Arg-Ser-Leu-Glu-Lys-Leu-Glu-Ile-Ile-Pro-Ala-Ser-Gln-Phe-(OH)NH₂ (SEQ ID NO:180)
   b19) RNH-Val-Pro-Xaa₃-Ser-Arg-Thr-Val-Arg-Xaa₁-Thr-Xaa₂-Ile-Ser-Ile-Ser-Asn-Gln-Pro-Val-Asn-Pro-Arg-Ser-Leu-Glu-Lys-Leu-Glu-Ile-Ile-Pro-Ala-Ser-Gln-Phe-(OH)NH₂ (SEQ ID NO:181)
   b20) RNH-Val-Pro-Leu-Xaa₃-Arg-Thr-Val-Arg-Xaa₁-Thr-Xaa₂-Ile-Ser-Ile-Ser-Asn-Gln-Pro-Val-Asn-Pro-Arg-Ser-Leu-Glu-Lys-Leu-Glu-Ile-Ile-Pro-Ala-Ser-Gln-Phe-(OH)NH₂ (SEQ ID NO: 182)
   b21) RNH-Val-Pro-Leu-Ser-Xaa₃-Thr-Val-Arg-Xaa₁-Thr-Xaa₂-Ile-Ser-Ile-Ser-Asn-Gln-Pro-Val-Asn-Pro-Arg-Ser-Leu-Glu-Lys-Leu-Glu-Ile-Ile-Pro-Ala-Ser-Gln-Phe-(OH)NH₂ (SEQ ID NO: 183)
   b22) RNH-Val-Pro-Leu-Ser-Arg-Xaa₃-Val-Arg-Xaa₁-Thr-Xaa₂-Ile-Ser-Ile-Ser-Asn-Gln-Pro-Val-Asn-Pro-Arg-Ser-Leu-Glu-Lys-Leu-Glu-Ile-Ile-Pro-Ala-Ser-Gln-Phe-(OH)NH₂ (SEQ ID NO:184)
   b23) RNH-Val-Pro-Leu-Ser-Arg-Thr-Xaa₃-Arg-Xaa₁-Thr-Xaa₂-Ile-Ser-Ile-Ser-Asn-Gln-Pro-Val-Asn-Pro-Arg-Ser-Leu-Glu-Lys-Leu-Glu-Ile-Ile-Pro-Ala-Ser-Gln-Phe-(OH)NH₂ (SEQ ID NO:185)
   b24) RNH-Val-Pro-Leu-Ser-Arg-Thr-Val-Xaa₃-Xaa₁-Thr-Xaa₂-Ile-Ser-Ile-Ser-Asn-Gln-Pro-Val-Asn-Pro-Arg-Ser-Leu-Glu-Lys-Leu-Glu-Ile-Ile-Pro-Ala-Ser-Gln-Phe-(OH)NH₂ (SEQ ID NO:186)

Preferred embodiments of linear IP-10 analogs of the present invention corresponding to a portion of the C-terminal region of IP-10 having the following sequence:
IP-10-(53-77) acid or amide
   b25) RNH-Leu-Asn-Pro-Glu-Ser-Lys-Ala-Ile-Lys-Asn-Leu-Leu-Lys-Ala-Val-Ser-Lys-Glu-Met-Ser-Lys-Arg-Ser-Pro-(OH)NH₂ (SEQ ID NO:187)

Preferred embodiments of cyclic IP-10 analogs of the present invention corresponding to a portion of the N-terminal region joined with a linker to a cyclic portion of the C-terminal region of IP-10 having the following structures:
IP-10-(1-14)-[linker]-IP-10-(65-77)-acid or amide
   b26) RNH-Val-Pro-Leu-Ser-Arg-Thr-Val-Arg-Cys-Thr-Cys-Ile-Ser-Ile-**[linker]**-Leu-Lys-Ala-Val-Ser-Lys-Glu-Met-Ser-Lys-Arg-Ser-Pro-(OH)NH₂ (SEQ ID NO:188)
   b27) RNH-Xaa₃-Pro-Leu-Ser-Arg-Thr-Val-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:189)
   b28) RNH-Val-Xaa₃-Leu-Ser-Arg-Thr-Val-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:190)
   b29) RNH-Val-Pro-Xaa₃-Ser-Arg-Thr-Val-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:191)
   b30) RNH-Val-Pro-Leu-Xaa₃-Arg-Thr-Val-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:192)
   b31) RNH-Val-Pro-Leu-Ser-Xaa₃-Thr-Val-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:193)
   b32) RNH-Val-Pro-Leu-Ser-Arg-Xaa₃-Val-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:194)
   b33) RNH-Val-Pro-Leu-Ser-Arg-Thr-Xaa₃-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:195)
   b34) RNH-Val-Pro-Leu-Ser-Arg-Thr-Val-Xaa₃-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:196)
   b35) RNH-Val-Pro-Leu-Ser-Arg-Thr-Val-Arg-Cys-Thr-Cys-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:197)
   b36) RNH-Xaa₃-Pro-Leu-Ser-Arg-Thr-Val-Arg-Xaa₁-Thr-Xaa₂-Xaa₄-Ser-Ile-[**linker**]-Leu-Lys-Ala-Val-Ser-Lys-Glu-Met-Ser-Lys-Arg-Ser-Pro-(OH)NH₂ (SEQ ID NO: 198)
   b37) RNH-Val-Xaa₃-Leu-Ser-Arg-Thr-Val-Arg-Xaa₁-Thr-Xaa₂-Xaa₄-Ser-Ile-[**linker**]-Leu-Lys-Ala-Val-Ser-Lys-Glu-Met-Ser-Lys-Arg-Ser-Pro-(OH)NH₂ (SEQ ID NO: 199)
   b38) RNH-Val-Pro-Xaa₃-Ser-Arg-Thr-Val-Arg-Xaa₁-Thr-Xaa₂-Xaa_{d}-Ser-Ile-[**linker**]-Leu-Lys-Ala-Val-Ser-Lys-Glu-Met-Ser-Lys-Arg-Ser-Pro-(OH)NH₂ (SEQ ID NO:200)
   b39) RNH-Val-Pro-Leu-Xaa₃-Arg-Thr-Val-Arg-Xaa₁-Thr-Xaa₂-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:201)
   b40) RNH-Val-Pro-Leu-Ser-Xaa₃-Thr-Val-Arg-Xaa₁-Thr-Xaa₂-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:202)
   b41) RNH-Val-Pro-Leu-Ser-Arg-Xaa₃-Val-Arg-Xaa₁-Thr-Xaa₂-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:203)
   b42) RNH-Val-Pro-Leu-Ser-Arg-Thr-Xaa₃-Arg-Xaa₁-Thr-Xaa₂-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:204)
   b43) RNH-Val-Pro-Leu-Ser-Arg-Thr-Val-Xaa₃-Xaa₁-Thr-Xaa₂-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:205)
   b44) RNH-Val-Pro-Leu-Ser-Arg-Thr-Val-Arg-Cys-Thr-Cys-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:206)
   b45) RNH-Xaa₃-Pro-Leu-Ser-Arg-Thr-Val-Arg-Xaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:207)
   b46) RNH-Val-Xaa₃-Leu-Ser-Arg-Thr-Val-Arg-Xaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:208)
   b47) RNH-Val-Pro-Xaa₃-Ser-Arg-Thr-Val-Arg-Xaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:209)
   b48) RNH-Val-Pro-Leu-Xaa₃-Arg-Thr-Val-Arg-Xaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:210)
   b49) RNH-Val-Pro-Leu-Ser-Xaa₃-Thr-Val-Arg-Xaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:211)
   b50) RNH-Val-Pro-Leu-Ser-Arg-Xaa₃-Val-Arg-Xaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:212)
   b51) RNH-Val-Pro-Leu-Ser-Arg-Thr-Xaa₃-Arg-Xaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:213)
   b52) RNH-Val-Pro-Leu-Ser-Arg-Thr-Val-Xaa₃-Xaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:214)
   b53) RNH-Val-Pro-Leu-Ser-Arg-Thr-Val-Arg-Cys-Thr-Cys-Ile-Ser-Xaa₄-[**linker**]-Leu-Lys-Ala-Val-Ser-Lys-Glu-Met-Ser-Lys-Arg-Ser-Pro-(OH)NH₂ (SEQ ID NO:215)
   b54) RNH-Xaa₃-Pro-Leu-Ser-Arg-Thr-Val-Arg-Xaa₁-Thr-Xaa₂-Ile-Ser-Xaa₄-[**linker**]-Leu-Lys-Ala-Val-Ser-Lys-Glu-Met-Ser-Lys-Arg-Ser-Pro-(OH)NH₂ (SEQ ID NO:216)
   b55) RNH-Val-Xaa₃-Leu-Ser-Arg-Thr-Val-Arg-Xaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:217)
   b56) RNH-Val-Pro-Xaa₃-Ser-Arg-Thr-Val-Arg-Xaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:218)
   b57) RNH-Val-Pro-Leu-Xaa₃-Arg-Thr-Val-Arg-Xaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:219)
   b58) RNH-Val-Pro-Leu-Ser-Xaa₃-Thr-Val-Arg-Xaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:220)
   b59) RNH-Val-Pro-Leu-Ser-Arg-Xaa₃-Val-Arg-Xaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:221)
   b60) RNH-Val-Pro-Leu-Ser-Arg-Thr-Xaa₃-Arg-Xaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:222)
   b61) RNH-Val-Pro-Leu-Ser-Arg-Thr-Val-Xaa₃-Xaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:223)
IP-10-(1-14)-[linker]-IP-10-(54-66)-acid or amide
   b62) RNH-Val-Pro-Leu-Ser-Arg-Thr-Val-Arg-Cys-Thr-Cys-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:224)
   b63) RNH-Xaa₃-Pro-Leu-Ser-Arg-Thr-Val-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:225)
   b64) RNH-Val-Xaa₃-Leu-Ser-Arg-Thr-Val-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:226)
   b65) RNH-Val-Pro-Xaa₃-Ser-Arg-Thr-Vat-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:227)
   b66) RNH-Val-Pro-Leu-Xaa₃-Arg-Thr-Val-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:228)
   b67) RNH-Val-Pro-Leu-Ser-Xaa₃-Thr-Val-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:229)
   b68) RNH-Val-Pro-Leu-Ser-Arg-Xaa₃-Val-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:230)
   b69) RNH-Val-Pro-Leu-Ser-Arg-Thr-Xaa₃-Arg-Xaa₁-Thr-Xaa₂-Ile-Ser-Ile-[**linker**]-Leu-Asn-Pro-Glu-Ser-Lys-Ala-Ile-Lys-Asn-Leu-Leu-Lys-(OH)NH₂ (SEQ ID NO:231)
   b70) RNH-Val-Pro-Leu-Ser-Arg-Thr-Val-Xaa₃-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:232)
   b71) RNH-Val-Pro-Leu-Ser-Arg-Thr-Val-Arg-Cys-Thr-Cys-Xaa₄-Ser-Ile-[**linker**]-Leu-Asn-Pro-Glu-Ser-Lys-Ala-Ile-Lys-Asn-Leu-Leu-Lys-(OH)NH₂ (SEQ ID NO:233)
   b72) RNH-Xaa₃-Pro-Leu-Ser-Arg-Thr-Val-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:234)
   b73) RNH-Val-Xaa₃-Leu-Ser-Arg-Thr-Val-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:235)
   b74) RNH-Val-Pro-Xaa₃-Ser-Arg-Thr-Val-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:236)
   b75) RNH-Val-Pro-Leu-Xaa₃-Arg-Thr-Val-Arg-Xaa₁-Thr-Xaa_{z}-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID N0:237)
   b76) RNH-Val-Pro-Leu-Ser-Xaa₃-Thr-Val-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:238)
   b77) RNH-Val-Pro-Leu-Ser-Arg-Xaa₃-Val-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:239)
   b78) RNH-Val-Pro-Leu-Ser-Arg-Thr-Xaa₃-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:240)
   b79) RNH-Val-Pro-Leu-Ser-Arg-Thr-Val-Xaa₃-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:241)
   b80) RNH-Val-Pro-Leu-Ser-Arg-Thr-Val-Arg-Cys-Thr-Cys-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:242)
   b81) RNH-Xaa₃-Pro-Leu-Ser-Arg-Thr-Val-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:243)
   b82) RNH-Val-Xaa₃-Leu-Ser-Arg-Thr-Val-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:244)
   b83) RNH-Val-Pro-Xaa₃-Ser-Arg-Thr-Val-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:245)
   b84) RNH-Val-Pro-Leu-Xaa₃-Arg-Thr-Val-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:246)
   b85) RNH-Val-Pro-Leu-Ser-Xaa₃-Thr-Val-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:247)
   b86) RNH-Val-Pro-Leu-Ser-Arg-Xaa₃-Val-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:248)
   b87) RNH-Val-Pro-Leu-Ser-Arg-Thr-Xaa₃-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:249)
   b88) RNH-Val-Pro-Leu-Ser-Arg-Thr-Val-Xaa₃-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:250)
   b89) RNH-Val-Pro-Leu-Ser-Arg-Thr-Val-Arg-Cys-Thr-Cys-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:251)
   b90) RNH-Xaa₃-Pro-Leu-Ser-Arg-Thr-Val-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:252)
   b91) RNH-Val-Xaa₃-Leu-Ser-Arg-Thr-Val-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:253)
   b92) RNH-Val-Pro-Xaa₃-Ser-Arg-Thr-Val-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:254)
   b93) RNH-Val-Pro-Leu-Xaa₃-Arg-Thr-Val-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:255)
   b94) RNH-Val-Pro-Leu-Ser-Xaa₃-Thr-Val-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:256)
   b95) RNH-Val-Pro-Leu-Ser-Arg-Xaa₃-Val-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:257)
   b96) RNH-Val-Pro-Leu-Ser-Arg-Thr-Xaa₃-Arg-Xaa)-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:258)
   b97) RNH-Val-Pro-Leu-Ser-Arg-Thr-Val-Xaa₃-Xaa)-Thr-Xaa₂-IIe-Ser-Xaa₄-[**linker**]-Leu-Asn-Pro-Glu-Ser-Lys-Ala-Ile-Lys-Asn-Leu-Leu-Lys-(OH)NH₂ (SEQ ID NO:259)
IP-10-(1-14)-[linker]-IP-10-(59-71)-acid or amide
   b98) RNH-Val-Pro-Leu-Ser-Arg-Thr-Val-Arg-Cys-Thr-Cys- Ile-Ser-Ile -**[linker]**- Lys-Ala-Ile-Lys-Asn-Leu-Leu-Lys-Ala-Val-Ser-Lys-Glu-(OH)NH₂ (SEQ ID NO:260)
   b99) RNH-Xaa₃-Pro-Leu-Ser-Arg-Thr-Val-Arg-Xaa₁-Thr-Xaa₂-Ile-Ser-Ile -[linker]- Lys-Ala-Ile-Lys-Asn-Leu-Leu-Lys-Ala-Val-Ser-Lys-Glu -(OH)NH₂ (SEQ ID NO:261)
   b100) RNH-Val-Xaa₃-Leu-Ser-Arg-Thr-Val-Arg-Xaa₁-Thr-Xaa₂-Ile-Ser-Ile **-[linker]-** Lys-Ala-Ile-Lys-Asn-Leu-Leu-Lys-Ala-Val-Ser-Lys-Glu -(OH)NH₂ (SEQ ID NO:262)
   b101) RNH-Val-Pro Xaa₃-Ser-Arg-Thr-Val-Arg-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:263)
   b102) RNH-Val-Pro-Leu-Xaa₃-Arg-Thr-Val-Arg-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:264)
   b103) RNH-Val-Pro-Leu-Ser-Xaa₃-Thr-Val-Arg-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:265)
   b104) RNH-Val-Pro-Leu-Ser-Arg-Xaa₃-Val-Arg-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:266)
   b105) RNH-Val-Pro-Leu-Ser-Arg-Thr-Xaa₃-Arg-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:267)
   b106) RNH-Val-Pro-Leu-Ser-Arg-Thr-Val-Xaa₃-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:268)
   b107) RNH-Val-Pro-Leu-Ser-Arg-Thr-Val-Arg-Cys-Thr-Cys-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:269)
   b108) RNH-Xaa₃-Pro-Leu-Ser-Arg-Thr-Val-Arg-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:270)
   b109) RNH-Val-Xaa₃-Leu-Ser-Arg-Thr-Val-Arg-Xaa₁-Thr-Xaa₂-Xaa_{d}-Ser-Ile-[linker]-Lys-Ala-Ile-Lys-Asn-Leu-Leu-Lys-Ala-Val-Ser-Lys-Glu-(OH)NH₂ (SEQ ID NO:271)
   b110) RNH-Val-Pro-Xaa₃-Ser-Arg-Thr-Val-Arg-Xaa₁-Thr-Xaa₂-Xaa_{d}-Ser-Ile-[**linker**]-Lys-Ala-Ile-Lys-Asn-Leu-Leu-Lys-Ala-Val-Ser-Lys-Glu-(OH)NH₂ (SEQ ID NO:272)
   b111) RNH-Val-Pro-Leu-Xaa₃-Arg-Thr-Val-Arg-Xaal-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:273)
   b112) RNH-Val-Pro-Leu-Ser-Xaa₃-Thr-Val-Arg-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:274)
   b113) RNH-Val-Pro-Leu-Ser-Arg-Xaa₃-Val-Arg-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:275)
   b114) RNH-Val-Pro-Leu-Ser-Arg-Thr-Xaa₃-Arg-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:276)
   b115) RNH-Val-Pro-Leu-Ser-Arg-Thr-Val-Xaa₃-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:277)
   b116) RNH-Val-Pro-Leu-Ser-Arg-Thr-Val-Arg-Cys-Thr-Cys-Ile-Xaa₄-Ile-[linker]-Lys-Ala-Ile-Lys-Asn-Leu-Leu-Lys-Ala-Val-Ser-Lys-Glu-(OH)NH₂ (SEQ ID NO:278)
   b117) RNH-Xaa₃-Pro-Leu-Ser-Arg-Thr-Val-Arg-Xaa₁ -Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:279)
   b118) RNH-Val-Xaa₃-Leu-Ser-Arg-Thr-Val-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:280)
   b119) RNH-Val-Pro-Xaa₃-Ser-Arg-Thr-Val-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:281)
   b120) RNH-Val-Pro-Leu-Xaa₃-Arg-Thr-Val-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:282)
   b121) RNH-Val-Pro-Leu-Ser-Xaa₃-Thr-Val-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:283)
   b122) RNH-Val-Pro-Leu-Ser-Arg-Xaa₃-Val-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:284)
   b123) RNH-Val-Pro-Leu-Ser-Arg-Thr-Xaa₃-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:285)
   b124) RNH-Val-Pro-Leu-Ser-Arg-Thr-Val-Xaa₃-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:286)
   b125) RNH-Val-Pro-Leu-Ser-Arg-Thr-Val-Arg-Cys-Thr-Cys-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:287)
   b126) RNH-Xaa₃-Pro-Leu-Ser-Arg-Thr-Val-Arg-Xaa₁-Thr-Xaa₂-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:288)
   b127) RNH-Val-Xaa₃-Leu-Ser-Arg-Thr-Val-Arg-Xaa₁-Thr-Xaa₂-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:289)
   b128) RNH-Val-Pro-Xaa₃-Ser-Arg-Thr-Val-Arg-Xaa₁-Thr-Xaa₂-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:290)
   b129) RNH-Val-Pro-Leu-Xaa₃-Arg-Thr-Val-Arg-Xaa₁-Thr-Xaa₂-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:291)
   b130) RNH-Val-Pro-Leu-Ser-Xaa₃-Thr-Val-Arg-Xaa₁-Thr-Xaa₂-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:292)
   b131) RNH-Val-Pro-Leu-Ser-Arg-Xaa₃-Val-Arg-Xaa₁-Thr-Xaa₂-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:293)
   b132) RNH-Val-Pro-Leu-Ser-Arg-Thr-Xaa₃-Arg-Xaa₁-Thr-Xaa₂-Ile-Ser-Xaa₄-[**linker**]-Lys-Ala-Ile-Lys-Asn-Leu-Leu-Lys-Ala-Val-Ser-Lys-Glu-(OH)NH₂ (SEQ ID NO:294)
   b133) RNH-Val-Pro-Leu-Ser-Arg-Thr-Val-Xaa₃-Xaa₁-Thr-Xaa₂-Ile-Ser-Xaa₄-[**linker**]-Lys-Ala-Ile-Lys-Asn-Leu-Leu-Lys-Ala-Val-Ser-Lys-Glu-(OH)NH₂ (SEQ ID NO:295)
IP-10-(1-17)-[linker]-IP-10-(65-77)-acid or amide
   b134) RNH-Val-Pro-Leu-Ser-Arg-Thr-Val-Arg-Cys-Thr-Cys-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:296)
   b135) RNH-Xaa₃-Pro-Leu-Ser-Arg-Thr-Val-Arg-Xaa₁-Thr-Xaa₂-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:297)
   b136) RNH-Val-Xaa₃-Leu-Ser-Arg-Thr-Val-Arg-Xaa₁-Thr-Xaa₂-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:298)
   b137) RNH-Val-Pro-Xaa₃-Ser-Arg-Thr-Val-Arg-Xaa₁-Thr-Xaa₂-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:299)
   b138) RNH-Val-Pro-Leu-Xaa₃-Arg-Thr-Val-Arg-Xaa₁-Thr-Xaa₂-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:300)
   b139) RNH-Val-Pro-Leu-Ser-Xaa₃-Thr-Val-Arg-Xaa₁-Thr-Xaa₂-Ile-Ser-Ile-Ser-Asn-Gln-[linker] -Leu-Lys-Ala-Val-Ser-Lys-Glu-Met-Ser-Lys-Arg-Ser-Pro-(OH)NH₂ (SEQ ID NO:301)
   b140) RNH-Val-Pro-Leu-Ser-Arg-Xaa₃-Val-Arg-Xaa₁-Thr-Xaa₂-Ile-Ser-Ile-Ser-Asn-Gln-[Iinker]-Leu-Lys-Ala-Val-Ser-Lys-Glu-Met-Ser-Lys-Arg-Ser-Pro-(OH)NH₂ (SEQ ID NO:302)
   b141) RNH-Val-Pro-Leu-Ser-Arg-Thr-Xaa₃-Arg-Xaa₁-Thr-Xaa₂-Ile-Ser-Ile-Ser-Asn-Gln-**[linker]** -Leu-Lys-Ala-Val- Ser-Lys-Glu-Met-Ser-Lys-Arg- Ser-Pro-(OH)NH₂ (SEQ ID NO:303)
   b142) RNH-Val-Pro-Leu-Ser-Arg-Thr-Val-Xaa₃-Xaa₁-Thr-Xaa₂-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:304)
   b143) RNH-Val-Pro-Leu-Ser-Arg-Thr-Val-Arg-Cys-Thr-Cys-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:305)
   b144) RNH-Xaa₃-Pro-Leu-Ser-Arg-Thr-Val-Arg-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:306)
   b145) RNH-Val-Xaa₃-Leu-Ser-Arg-Thr-Val-Arg-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:307)
   b146) RNH-Val-Pro-Xaa₃-Ser-Arg-Thr-Val-Arg-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:308)
   b147) RNH-Val-Pro-Leu-Xaa₃-Arg-Thr-Val-Arg-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:309)
   b148) RNH-Val-Pro-Leu-Ser-Xaa₃-Thr-Val-Arg-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:310)
   b149) RNH-Val-Pro-Leu-Ser-Arg-Xaa₃-Val-Arg-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:311)
   b150) RNH-Val-Pro-Leu-Ser-Arg-Thr-Xaa₃-Arg-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:312)
   b151) RNH-Val-Pro-Leu-Ser-Arg-Thr-Val-Xaa₃-Xaa₁-Thr-Xaa₂-Xaa₄-Ser-Ile-Ser-Asn-Gln-**[linker]** -Leu-Lys-Ala-Val-Ser-Lys-Glu-Met-Ser-Lys-Arg-Ser-Pro-(OH)NH₂ (SEQ ID NO:313)
   b152) RNH-Val-Pro-Leu-Ser-Arg-Thr-Val-Arg-Cys-Thr-Cys-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:314)
   b153) RNH-Xaa₃-Pro-Leu-Ser-Arg-Thr-Val-ArgXaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:315)
   b154) RNH-Val-Xaa₃-Leu-Ser-Arg-Thr-Val-ArgXaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:316)
   b155) RNH-Val-Pro-Xaa₃-Ser-Arg-Thr-Val-ArgXaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:317)
   b156) RNH-Val-Pro-Leu-Xaa₃-Arg-Thr-Val-ArgXaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:318)
   b157) RNH-Val-Pro-Leu-Ser-Xaa₃-Thr-Val-Arg-Xaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:319)
   b158) RNH-Val-Pro-Leu-Ser-Arg-Xaa₃-Val-Arg-Xaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:320)
   b159) RNH-Val-Pro-Leu-Ser-Arg-Thr-Xaa₃-Arg-Xaa₁Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:321)
   b160) RNH-Val-Pro-Leu-Ser-Arg-Thr-Val-Xaa₃-Xaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:322)
   b161) RNH-Val-Pro-Leu-Ser-Arg-Thr-Val-Arg-Cys-Thr-Cys-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:323)
   b162) RNH-Xaa₃-Pro-Leu-Ser-Arg-Thr-Val-Arg-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:324)
   b163) RNH-Val-Xaa₃-Leu-Ser-Arg-Thr-Val-Arg-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:325)
   b164) RNH-Val-Pro-Xaa₃-Ser-Arg-Thr-Val-Arg-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:326)
   b165) RNH-Val-Pro-Leu-Xaa₃-Arg-Thr-Val-Arg-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:327)
   b166) RNH-Val-Pro-Leu-Ser-Xaa₃-Thr-Val-Arg-Xaa₁-Thr-Xaa₂-Ile-Ser-Xaa₄-Ser-Asn-Gln-**[linker]-**Leu-Lys-Ala-Val-Ser-Lys-Glu-Met-Ser-Lys-Arg-Ser-Pro-(OH)NH₂ (SEQ ID NO:328)
   b167) RNH-Val-Pro-Leu-Ser-Arg-Xaa₃-Val-Arg-Xaa₁-Thr-Xaa₂-Ile-Ser-Xaa₄-Ser-Asn-Gln-**[linker]-**Leu-Lys-Ala-Val-Ser-Lys-Glu-Met-Ser-Lys-Arg-Ser-Pro-(OH)NH₂ (SEQ ID NO:329)
   b168) RNH-Val-Pro-Leu-Ser-Arg-Thr-Xaa₃-Arg-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:330)
   b169) RNH-Val-Pro-Leu-Ser-Arg-Thr-Val-Xaa₃-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:331)
   b170) RNH-Val-Pro-Leu-Ser-Arg-Thr-Val-Arg-Cys-Thr-Cys-Ile-Ser-Ile-Ser-Xaa₄-Gln-**[linker]-**Leu-Lys-Ala-Val-Ser-Lys-Glu-Met-Ser-Lys-Arg-Ser-Pro-(OH)NH₂ (SEQ ID NO:332)
   b171) RNH-Xaa₃-Pro-Leu-Ser-Arg-Thr-Val-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:333)
   b172) RNH-Val-Xaa₃-Leu-Ser-Arg-Thr-Val-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:334)
   b173) RNH-Val-Pro-Xaa₃-Ser-Arg-Thr-Val-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:335)
   b174) RNH-Val-Pro-Leu-Xaa₃-Arg-Thr-Val-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:336)
   b175) RNH-Val-Pro-Leu-Ser-Xaa₃-Thr-Val-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH2 (SEQ ID NO:337)
   b176) RNH-Val-Pro-Leu-Ser-Arg-Xaa₃-Val-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:338)
   b177) RNH-Val-Pro-Leu-Ser-Arg-Thr-Xaa₃-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:339)
   b178) RNH-Val-Pro-Leu-Ser-Arg-Thr-Val-Xaa₃-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:340)
   b179) RNH-Val-Pro-Leu-Ser-Arg-Thr-Val-Arg-Cys-Thr-Cys-Ile-Ser-Ile-Ser-Gln-Xaa₄-**[linker]**-Leu-Lys-Ala-Val-Ser-Lys-Glu-Met-Ser-Lys-Arg-Ser-Pro-(OH)NH₂ (SEQ ID NO:341)
   b180) RNH-Xaa₃-Pro-Leu-Ser-Arg-Thr-Val-ArgXaa₁-Thr-Xaa₂-Ile-Ser-Ile-Ser-Gln-Xaa₄-**[linker]**-Leu-Lys-Ala-Val-Ser-Lys-Glu-Met-Ser-Lys-Arg-Ser-Pro-(OH)NH₂ (SEQ ID NO:342)
   b181) RNH-Val-Xaa₃-Leu-Ser-Arg-Thr-Val-ArgXaa₁-Thr-Xaa₂-Ile-Ser-Ile-Ser-Gln-Xaa₄-**[linker]**-Leu-Lys-Ala-Val-Ser-Lys-Glu-Met-Ser-Lys-Arg-Ser-Pro-(OH)NH₂ (SEQ ID NO:343)
   b182) RNH-Val-Pro-Xaa₃-Ser-Arg-Thr-Val-ArgXaa₁-Thr-Xaa₂-Ile-Ser-Ile-Ser-Gln-Xaa₄-**[linker]**-Leu-Lys-Ala-Val-Ser-Lys-Glu-Met-Ser-Lys-Arg-Ser-Pro-(OH)NH₂ (SEQ ID NO:344)
   b183) RNH-Val-Pro-Leu-Xaa₃-Arg-Thr-Val-ArgXaa₁-Thr-Xaa₂-Ile-Ser-Ile-Ser-Gln-Xaa₄-**[linker]**-Leu-Lys-Ala-Val-Ser-Lys-Glu-Met-Ser-Lys-Arg-Ser-Pro-(OH)NH₂ (SEQ ID NO:345)
   b184) RNH-Val-Pro-Leu-Ser-Xaa₃-Thr-Val-Arg-Xaa₁-Thr-Xaa₂-Ile-Ser-Ile-Ser-Gln-Xaa₄-[linker]-Leu-Lys-Ala-Val-Ser-Lys-Glu-Met-Ser-Lys-Arg-Ser-Pro-(OH)NH2 (SEQ ID NO:346)
   b185) RNH-Val-Pro-Leu-Ser-Arg-Xaa₃-Val-Arg-Xaa₁-Thr-Xaa₂-Ile-Ser-Ile-Ser-Gln-Xaa₄-**[linker]**-Leu-Lys-Ala-Val-Ser-Lys-Glu-Met-Ser-Lys-Arg-Ser-Pro-(OH)NH₂ (SEQ ID NO:347)
   b186) RNH-Val-Pro-Leu-Ser-Arg-Thr-Xaa₃-Arg-Xaa₁-Thr-Xaa₂-Ile-Ser-Ile-Ser-Gln-Xaa₄-**[linker]-**Leu-Lys-Ala-Val-Ser-Lys-Glu-Met-Ser-Lys-Arg-Ser-Pro-(OH)NH₂ (SEQ ID NO:348)
   b187) RNH-Val-Pro-Leu-Ser-Arg-Thr-Val-Xaa₃-Xaa₁-Thr-Xaa₂-Ile-Ser-Ile-Ser-Gln-Xaa₄-**[linker]**-Leu-Lys-Ala-Val-Ser-Lys-Glu-Met-Ser-Lys-Arg-Ser-Pro-(OH)NH₂ (SEQ ID NO:349)
IP-10-(1-17)-[linker]-IP-10-(54-66)-acid or amide
   b188) RNH-Val-Pro-Leu-Ser-Arg-Thr-Val-Arg-Cys-Thr-Cys-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:350)
   b189) RNH-Xaa₃-Pro-Leu-Ser-Arg-Thr-Val-Arg-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:351)
   b190) RNH-Val-Xaa₃-Leu-Ser-Arg-Thr-Val-Arg-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:352)
   b191) RNH-Val-Pro-Xaa₃-Ser-Arg-Thr-Val-ArgXaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:353)
   b192) RNH-Val-Pro-Leu-Xaa₃-Arg-Thr-Val-ArgXaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:354)
   b193) RNH-Val-Pro-Leu-Ser-Xaa₃-Thr-Val-ArgXaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:355)
   b194) RNH-Val-Pro-Leu-Ser-Arg-Xaa₃-Val-Arg-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:356)
   b195) RNH-Val-Pro-Leu-Ser-Arg-Thr-Xaa₃-Arg-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:357)
   b196) RNH-Val-Pro-Leu-Ser-Arg-Thr-Val-Xaa₃-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:358)
   b197) RNH-Val-Pro-Leu-Ser-Arg-Thr-Val-Arg-Cys-Thr-Cys-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:359)
   b198) RNH-Xaa₃-Pro-Leu-Ser-Arg-Thr-Val-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:360)
   b199) RNH-Val-Xaa₃-Leu-Ser-Arg-Thr-Val-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:361)
   b200) RNH-Val-Pro-Xaa₃-Ser-Arg-Thr-Val-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:362)
   b201) RNH-Val-Pro-Leu-Xaa₃-Arg-Thr-Val-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:363)
   b202) RNH-Val-Pro-Leu-Ser-Xaa₃-Thr-Val-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:364)
   b203) RNH-Val-Pro-Leu-Ser-Arg-Xaa₃-Val-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:365)
   b204) RNH-Val-Pro-Leu-Ser-Arg-Thr-Xaa₃-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:366)
   b205) RNH-Val-Pro-Leu-Ser-Arg-Thr-Val-Xaa₃-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:367)
   b206) RNH-Val-Pro-Leu-Ser-Arg-Thr-Val-Arg-Cys-Thr-Cys-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:368)
   b207) RNH-Xaa₃-Pro-Leu-Ser-Arg-Thr-Val-ArgXaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:369)
   b208) RNH-Val-Xaa₃-Leu-Ser-Arg-Thr-Val-ArgXaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH2 (SEQ ID NO:370)
   b209) RNH-Val-Pro-Xaa₃-Ser-Arg-Thr-Val-ArgXaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:371)
   b210) RNH-Val-Pro-Leu-Xaa₃-Arg-Thr-Val-ArgXaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:372)
   b211) RNH-Val-Pro-Leu-Ser-Xaa₃-Thr-Val-Arg-Xaa₁-Thr-Xaa₂-Ile-Xaa₄-Ile-Ser-Asn-Gln-[linker]-Leu-Asn-Pro-Glu-Ser-Lys-Ala-Ile-Lys-Asn-Leu-Leu-Lys-(OH)NH2 (SEQ ID NO:373)
   b212) RNH-Val-Pro-Leu-Ser-Arg-Xaa₃-Val-Arg-Xaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:374)
   b213) RNH-Val-Pro-Leu-Ser-Arg-Thr-Xaa₃-Arg-Xaa₁-Thr-Xaa₂-Ile-Xaa₄-Ile-Ser-Asn-Gln-**[linker]** -Leu-Asn-Pro-Glu-Ser-Lys-Ala-Ile-Lys-Asn-Leu-Leu-Lys-(OH)NH₂ (SEQ ID NO:375)
   b214) RNH-Val-Pro-Leu-Ser-Arg-Thr-Val-Xaa₃-Xaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO: 376)
   b215) RNH-Val-Pro-Leu-Ser-Arg-Thr-Va!-Arg-Cys-Thr-Cys-Ile-Ser-Xaa₄-Ser-Asn-Gln-**[linker]** -Leu-Asn-Pro-Glu-Ser-Lys-Ala-Ile-Lys-Asn-Leu-Leu-Lys-(OH)NH₂ (SEQ ID NO:377)
   b216) RNH-Xaa₃-Pro-Leu-Ser-Arg-Thr-Val-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:378)
   b217) RNH-Val-Xaa₃-Leu-Ser-Arg-Thr-Val-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:379)
   b218) RNH-Val-Pro-Xaa₃-Ser-Arg-Thr-Val-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:380)
   b219) RNH-Val-Pro-Leu-Xaa₃-Arg-Thr-Val-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:381)
   b220) RNH-Val-Pro-Leu-Ser-Xaa₃-Thr-Val-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:382)
   b221) RNH-Val-Pro-Leu-Ser-Arg-Xaa₃-Val-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:383)
   b222) RNH-Val-Pro-Leu-Ser-Arg-Thr-Xaa₃-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:384)
   b223) RNH-Val-Pro-Leu-Ser-Arg-Thr-Val-Xaa₃-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:385)
   b224) RNH-Val-Pro-Leu-Ser-Arg-Thr-Val-Arg-Cys-Thr-Cys-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:386)
   b225) RNH-Xaa₃-Pro-Leu-Ser-Arg-Thr-Val-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH2 (SEQ ID NO:387)
   b226) RNH-Val-Xaa₃-Leu-Ser-Arg-Thr-Val-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:388)
   b227) RNH-Val-Pro-Xaa₃-Ser-Arg-Thr-Val-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:389)
   b228) RNH-Val-Pro-Leu-Xaa₃-Arg-Thr-Val-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:390)
   b229) RNH-Val-Pro-Leu-Ser-Xaa₃-Thr-Val-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:391)
   b230) RNH-Val-Pro-Leu-Ser-Arg-Xaa₃-Val-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:392)
   b231) RNH-Val-Pro-Leu-Ser-Arg-Thr-Xaa₃-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:393)
   b232) RNH-Val-Pro-Leu-Ser-Arg-Thr-Val-Xaa₃-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:394)
   b233) RNH-Val-Pro-Leu-Ser-Arg-Thr-Val-Arg-Cys-Thr-Cys-Ile-Ser-Ile-Ser--Gln-Xaa₄-[**linker**]-Leu-Asn-Pro-Glu-Ser-Lys-Ala-Ile-Lys-Asn-Leu-Leu-Lys-(OH)NH₂ (SEQ ID NO: 395)
   b234) RNH-Xaa₃-Pro-Leu-Ser-Arg-Thr-Val-ArgXaa₁-Thr-Xaa₂-Ile-Ser-Ile-Ser-Gln-Xaa₄-[**linker**]-Leu-Asn-Pro-Glu-Ser-Lys-Ala-Ile-Lys-Asn-Leu-Leu-Lys-(OH)NH₂ (SEQ ID NO:396)
   b235) RNH-Val-Xaa₃-Leu-Ser-Arg-Thr-Val-ArgXaa₁-Thr-Xaa₂-Ile-Ser-Ile-Ser-Gln-Xaa₄-**[linker]**-Leu-Asn-Pro-Glu-Ser-Lys-Ala-Ile-Lys-Asn-Leu-Leu-Lys-(OH)NH₂ (SEQ ID NO:397)
   b236) RNH-Val-Pro-Xaa₃-Ser-Arg-Thr-Val-ArgXaa₁-Thr-Xaa₂-Ile-Ser-Ile-Ser-Gln-Xaa₄-**[linker]**-Leu-Asn-Pro-Glu-Ser-Lys-Ala-Ile-Lys-Asn-Leu-Leu-Lys-(OH)NH₂ (SEQ ID NO:398)
   b237) RNH-Val-Pro-Leu-Xaa₃-Arg-Thr-Val-ArgXaa₁-Thr-Xaa₂-Ile-Ser-Ile-Ser-Gln-Xaa₄-**[linker]**-Leu-Asn-Pro-Glu-Ser-Lys-Ala-Ile-Lys-Asn-Leu-Leu-Lys-(OH)NH₂ (SEQ ID NO:399)
   b238) RNH-Val-Pro-Leu-Ser-Xaa₃-Thr-Val-Arg-Xaa₁-Thr-Xaa₂-Ile-Ser-Ile-Ser-Gln-Xaa₄-**[linker]**-Leu-Asn-Pro-Glu-Ser-Lys-Ala-Ile-Lys-Asn-Leu-Leu-Lys-(OH)NH₂ (SEQ ID NO:400)
   b239) RNH-Val-Pro-Leu-Ser-Arg-Xaa₃-Val-Arg-Xaa₁-Thr-Xaa₂-Ile-Ser-Ile-Ser-Gln-Xaa₄-**[linker]**-Leu-Asn-Pro-Glu-Ser-Lys-Ala-Ile-Lys-Asn-Leu-Leu-Lys-(OH)NH₂ (SEQ ID NO:401)
   b240) RNH-Val-Pro-Leu-Ser-Arg-Thr-Xaa₃-Arg-Xaa₁-Thr-Xaa₂-Ile-Ser-Ile-Ser-Gln-Xaa₄-**[linker]**-Leu-Asn-Pro-Glu-Ser-Lys-Ala-Ile-Lys-Asn-Leu-Leu-Lys-(OH)NH₂ (SEQ ID NO:402)
   b241) RNH-Val-Pro-Leu-Ser-Arg-Thr-Val-Xaa₃-Xaa₁-Thr-Xaa₂-Ile-Ser-Ile-Ser-Gln-Xaa₄-**[linker]**-Leu-Asn-Pro-Glu-Ser-Lys-Ala-Ile-Lys-Asn-Leu-Leu-Lys-(OH)NH₂ (SEQ ID NO:403)
IP-10-(1-17)-[linker]-IP-10-(59-71)-acid or amide
   b242) RNH-Val-Pro-Leu-Ser-Arg-Thr-Val-Arg-Cys-Thr-Cys-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:404)
   b243) RNH-Xaa₃-Pro-Leu-Ser-Arg-Thr-Val-Arg-Xaa₁-Thr-Xaa₂-Ile-Ser-Ile-Ser-Asn-Gln-[**linker**]-Lys-Ala-Ile-Lys-Asn-Leu-Leu-Lys-Ala-Val-Ser-Lys-Glu-(OH)NH₂ (SEQ ID NO:405)
   b244) RNH-Val-Xaa₃-Leu-Ser-Arg-Thr-Val-Arg-Xaa₁-Thr-Xaa₂-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:406)
   b245) RNH-Val-Pro-Xaa₃-Ser-Arg-Thr-Val-Arg-Xaa₁-Thr-Xaa₂-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:407)
   b246) RNH-Val-Pro-Leu-Xaa₃-Arg-Thr-Val-Arg-Xaa₁-Thr-Xaa₂-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:408)
   b247) RNH-Val-Pro-Leu-Ser-Xaa₃-Thr-Val-Arg-Xaa₁-Thr-Xaa₂-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:409)
   b248) RNH-Val-Pro-Leu-Ser-Arg-Xaa₃-Val-Arg-Xaa₁-Thr-Xaa₂-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:410)
   b249) RNH-Val-Pro-Leu-Ser-Arg-Thr-Xaa₃-Arg-Xaa₁-Thr-Xaa₂-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:411)
   b250) RNH-Val-Pro-Leu-Ser-Arg-Thr-Val-Xaa₃-Xaa₁-Thr-Xaa₂-Ile-Ser-Ile-Ser-Asn-Gln-**[linker]-**Lys-Ala-Ile-Lys-Asn-Leu-Leu-Lys-Ala-Val-Ser-Lys-Glu-(OH)NH₂ (SEQ ID NO:412)
   b251) RNH-Val-Pro-Leu-Ser-Arg-Thr-Val-Arg-Cys-Thr-Cys-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:413)
   b252) RNH-Xaa₃-Pro-Leu-Ser-Arg-Thr-Val-Arg-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:414)
   b253) RNH-Val-Xaa₃-Leu-Ser-Arg-Thr-Val-Arg-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:415)
   b254) RNH-Val-Pro-Xaa₃-Ser-Arg-Thr-Val-Arg-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID N0:416)
   b255) RNH-Val-Pro-Leu-Xaa₃-Arg-Thr-Val-Arg-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:417)
   b256) RNH-Val-Pro-Leu-Ser-Xaa₃-Thr-Val-Arg-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:418)
   b257) RNH-Val-Pro-Leu-Ser-Arg-Xaa₃-Val-Arg-Xaa₁-Thr-Xaa₂-Xaa₄-Ser-Ile-Ser-Asn-Gln-**[linker]-**Lys-Ala-Ile-Lys-Asn-Leu-Leu-Lys-Ala-Val-Ser-Lys-Glu-(OH)NH₂ (SEQ ID NO:419)
   b258) RNH-Val-Pro-Leu-Ser-Arg-Thr-Xaa₃-Arg-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:420)
   b259) RNH-Val-Pro-Leu-Ser-Arg-Thr-Val-Xaa₃-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:421)
   b260) RNH-Val-Pro-Leu-Ser-Arg-Thr-Val-Arg-Cys-Thr-Cys-Ile-Xaa₄-Ile-Ser-Asn-Gln-**[linker]-**Lys-Ala-Ile-Lys-Asn-Leu-Leu-Lys-Ala-Val-Ser-Lys-Glu-(OH)NH₂ (SEQ ID NO:422)
   b261) RNH-Xaa₃-Pro-Leu-Ser-Arg-Thr-Val-Arg-Xaa₁-Thr-Xaa₂-Ile-Xaa₄-Ile-Ser-Asn-Gln-**[linker]-**Lys-Ala-Ile-Lys-Asn-Leu-Leu-Lys-Ala-Val-Ser-Lys-Glu-(OH)NH₂ (SEQ ID NO:423)
   b262) RNH-Val-Xaa₃-Leu-Ser-Arg-Thr-Val-Arg-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:424)
   b263) RNH-Val-Pro-Xaa₃-Ser-Arg-Thr-Val-Arg-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:425)
   b264) RNH-Val-Pro-Leu-Xaa₃-Arg-Thr-Val-Arg-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:426)
   b265) RNH-Val-Pro-Leu-Ser-Xaa₃-Thr-Val-Arg-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:427)
   b266) RNH-Val-Pro-Leu-Ser-Arg-Xaa₃-Val-Arg-Xaa₁-Thr-Xaa₂-Ile-Xaa₄-Ile-Ser-Asn-Gln-**[linker]** -Lys-Ala-Ile-Lys-Asn-Leu-Leu-Lys-Ala-Val-Ser-Lys-Glu-(OH)NH₂ (SEQ ID NO:428)
   b267) RNH-Val-Pro-Leu-Ser-Arg-Thr-Xaa₃-Arg-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:429)
   b268) RNH-Val-Pro-Leu-Ser-Arg-Thr-Val-Xaa₃-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:430)
   b269) RNH-Val-Pro-Leu-Ser-Arg-Thr-Val-Arg-Cys-Thr-Cys-Ile-Ser-Xaa₄-Ser-Asn-Gln-**[linker]** -Lys-Ala-Ile-Lys-Asn-Leu-Leu-Lys-Ala-Val-Ser-Lys-Glu-(OH)NH₂ (SEQ ID NO:431)
   b270) RNH-Xaa₃-Pro-Leu-Ser-Arg-Thr-Val-Arg-Xaa₁-Thr-Xaa₂-Ile-Ser-Xaa₄-Ser-Asn-Gln-**[linker]** -Lys-Ala-Ile-Lys-Asn-Leu-Leu-Lys-Ala-Val-Ser-Lys-Glu-(OH)NH₂ (SEQ ID NO:432)
   b271) RNH-Val-Xaa₃-Leu-Ser-Arg-Thr-Val-Arg-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:433)
   b272) RNH-Val-Pro-Xaa₃-Ser-Arg-Thr-Val-Arg-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:434)
   b273) RNH-Val-Pro-Leu-Xaa₃-Arg-Thr-Val-Arg-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH2 (SEQ ID NO:435)
   b274) RNH-Val-Pro-Leu-Ser-Xaa₃-Thr-Val-Arg-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:436)
   b275) RNH-Val-Pro-Leu-Ser-Arg-Xaa₃-Val-Arg-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:437)
   b276) RNH-Val-Pro-Leu-Ser-Arg-Thr-Xaa₃-Arg-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:438)
   b277) RNH-Val-Pro-Leu-Ser-Arg-Thr-Val-Xaa₃-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:439)
   b278) RNH-Val-Pro-Leu-Ser-Arg-Thr-Val-Arg-Cys-Thr-Cys-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:440)
   b279) RNH-Xaa₃-Pro-Leu-Ser-Arg-Thr-Val-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID N0:441)
   b280) RNH-Val-Xaa₃-Leu-Ser-Arg-Thr-Val-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:442)
   b281) RNH-Val-Pro-Xaa₃-Ser-Arg-Thr-Val-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:443)
   b282) RNH-Val-Pro-Leu-Xaa₃-Arg-Thr-Val-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:444)
   b283) RNH-Val-Pro-Leu-Ser-Xaa₃-Thr-Val-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:445)
   b284) RNH-Val-Pro-Leu-Ser-Arg-Xaa₃-Val-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:446)
   b285) RNH-Val-Pro-Leu-Ser-Arg-Thr-Xaa₃-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:447)
   b286) RNH-Val-Pro-Leu-Ser-Arg-Thr-Val-Xaa₃-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:448)
   b287) RNH-Val-Pro-Leu-Ser-Arg-Thr-Val-Arg-Cys-Thr-Cys-Ile-Ser-Ile-Ser-Gln-Xaa₄-**[linker]**-Lys-Ala-Ile-Lys-Asn-Leu-Leu-Lys-Ala-Val-Ser-Lys-Glu-(OH)NH₂ (SEQ ID NO:449)
   b288) RNH-Xaa₃-Pro-Leu-Ser-Arg-Thr-Val-Arg-Xaa₁-Thr-Xaa₂-Ile-Ser-Ile-Ser-Gln-Xaa₄-**[linker]**-Lys-Ala-Ile-Lys-Asn-Leu-Leu-Lys-Ala-Val-Ser-Lys-Glu-(OH)NH₂ (SEQ ID NO:450)
   b289) RNH-Val-Xaa₃-Leu-Ser-Arg-Thr-Val-Arg-Xaa₁-Thr-Xaa₂-Ile-Ser-Ile-Ser-Gln-Xaa₄-**[linker]** -Lys-Ala-Ile-Lys-Asn-Leu-Leu-Lys-Ala-Val-Ser-Lys-Glu-(OH)NH₂ (SEQ ID NO:45 1)
   b290) RNH-Val-Pro-Xaa₃-Ser-Arg-Thr-Val-Arg-Xaa₁-Thr-Xaa₂-Ile-Ser-Ile-Ser-GIn-Xaa₄-**[linker]** -Lys-Ala-Ile-Lys-Asn-Leu-Leu-Lys-Ala-Val-Ser-Lys-Glu-(OH)NH₂ (SEQ ID NO:452)
   b291) RNH-Val-Pro-Leu-Xaa₃-Arg-Thr-Val-Arg-Xaa₁-Thr-Xaa₂-Ile-Ser-Ile-Ser-Gln-Xaa₄-**[linker]**-Lys-Ala-Ile-Lys-Asn-Leu-Leu-Lys-Ala-Val-Ser-Lys-Glu-(OH)NH2 (SEQ ID NO:453)
   b292) RNH-Val-Pro-Leu-Ser-Xaa₃-Thr-Val-Arg-Xaa₁-Thr-Xaa₂-Ile-Ser-Ile-Ser-Gln-Xaa₄-**[linker]**-Lys-Ala-Ile-Lys-Asn-Leu-Leu-Lys-Ala-Val-Ser-Lys-Glu-(OH)NH₂ (SEQ ID NO:454)
   b293) RNH-Val-Pro-Leu-Ser-Arg-Xaa₃-Val-Arg-Xaa₁-Thr-Xaa₂-Ile-Ser-Ile-Ser-Gln-Xaa₄-**[linker]**-Lys-Ala-Ile-Lys-Asn-Leu-Leu-Lys-Ala-Val-Ser-Lys-Glu-(OH)NH₂ (SEQ ID NO:455)
   b294) RNH-Val-Pro-Leu-Ser-Arg-Thr-Xaa₃-Arg-Xaa₁-Thr-Xaa₂-Ile-Ser-Ile-Ser-Gln-Xaa₄-**[linker]**-Lys-Ala-Ile-Lys-Asn-Leu-Leu-Lys-Ala-Val-Ser-Lys-Glu-(OH)NH₂ (SEQ ID NO:456)
   b295) RNH-Val-Pro-Leu-Ser-Arg-Thr-Val-Xaa₃-Xaa₁-Thr-Xaa₂-Ile-Ser-Ile-Ser-Gln-Xaa₄-**[linker]**-Lys-Ala-Ile-Lys-Asn-Leu-Leu-Lys-Ala-Val-Ser-Lys-Glu-(OH)NH₂ (SEQ ID NO:457)

Preferred embodiments of cyclic IP-10 analogs of the present invention corresponding to a portion the N-terminal region joined with a linker to a cyclic portion of the C-terminal region of IP-10 having the following structures (underlined residues are cyclized):
IP-10-(1-14)-[linker]-IP-10-(65-77)-cyclic (Glu71 -Lys74) acid or amide
   b296) RNH-Val-Pro-Leu-Ser-Arg-Thr-Val-Arg-Cys-Thr-Cys-Ile-Ser-Ile-[linker]-Leu-Lys-Ala-Val-Ser-Lys-Glu-Met-Ser-Lys-Arg-Ser-Pro-(OH)NH₂ (SEQ ID NO:458)
   b297) RNH-Xaa₃-Pro-Leu-Ser-Arg-Thr-Val-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:459)
   b298) RNH-Val-Xaa₃-Leu-Ser-Arg-Thr-Val-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:460)
   b299) RNH-Val-Pro-Xaa₃-Ser-Arg-Thr-Val-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:461)
   b300) RNH-Val-Pro-Leu-Xaa₃-Arg-Thr-Val-Arg-Xaa₁-Thr-Xaa₂-Ile-Ser-Ile-[**linker**]-Leu-Lys-Ala-Val-Ser-Lys-Glu-Met-Ser-Lys-Arg-Ser-Pro-(OH)NH₂ (SEQ ID NO:462)
   b301) RNH-Val-Pro-Leu-Ser-Xaa₃-Thr-Val-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:463)
   b302) RNH-Val-Pro-Leu-Ser-Arg-Xaa₃-Val-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:464)
   b303) RNH-Val-Pro-Leu-Ser-Arg-Thr-Xaa₃-Arg-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:465)
   b304) RNH-Val-Pro-Leu-Ser-Arg-Thr-Val-Xaa₃-Xaa₁-Thr-Xaa₂-Ile-Ser-Ile-**[linker]**-Leu-Lys-Ala-Val-Ser-Lys-Glu-Met-Ser-Lys-Arg-Ser-Pro-(OH)NH₂ (SEQ ID NO:466)
   b305) RNH-Val-Pro-Leu-Ser-Arg-Thr-Val-Arg-Cys-Thr-Cys-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:467)
   b306) RNH-Xaa₃-Pro-Leu-Ser-Arg-Thr-Val-Arg-Xaa₁-Thr-Xaa₂-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:468)
   b307) RNH-Val-Xaa₃-Leu-Ser-Arg-Thr-Val-Arg-Xaa₁-Thr-Xaa₂-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:469)
   b308) RNH-Val-Pro-Xaa₃-Ser-Arg-Thr-Val-Arg-Xaal-Thr-Xaa₂-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:470)
   b309) RNH-Val-Pro-Leu-Xaa₃-Arg-Thr-Val-Arg-Xaa₁-Thr-Xaa₂-Xaa₄-Ser-Ile-[**linker**]-Leu-Lys-Ala-Val-Ser-Lys-Glu-Met-Ser-Lys-Arg-Ser-Pro-(OH)NH₂ (SEQ ID NO:471)
   b310) RNH-Val-Pro-Leu-Ser-Xaa₃-Thr-Val-Arg-Xaa₁-Thr-Xaa₂-Xaa₄-Ser-Ile-[**linker**]-Leu-Lys-Ala-Val-Ser-Lys-Glu-Met-Ser-Lys-Arg-Ser-Pro-(OH)NH₂ (SEQ ID NO:472)
   b311) RNH-Val-Pro-Leu-Ser-Arg-Xaa₃-Val-Arg-Xaa₁-Thr-Xaa₂-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:473)
   b312) RNH-Val-Pro-Leu-Ser-Arg-Thr-Xaa₃-Arg-Xaa₁-Thr-Xaa₂-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:474)
   b313) RNH-Val-Pro-Leu-Ser-Arg-Thr-Val-Xaa₃-Xaa₁-Thr-Xaa₂-Xaa₄-Ser-Ile-**[linker]**-Leu-Lys-Ala-Val-Ser-Lys-Glu-Met-Ser-Lys-Arg-Ser-Pro-(OH)NH₂ (SEQ ID NO:475)
   b314) RNH-Val-Pro-Leu-Ser-Arg-Thr-Val-Arg-Cys-Thr-Cys-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:476)
   b315) RNH-Xaa₃-Pro-Leu-Ser-Arg-Thr-Val-ArgXaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:477)
   b316) RNH-Val-Xaa₃-Leu-Ser-Arg-Thr-Val-ArgXaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:478)
   b317) RNH-Val-Pro-Xaa₃-Ser-Arg-Thr-Val-ArgXaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:479)
   b318) RNH-Val-Pro-Leu-Xaa₃-Arg-Thr-Val-ArgXaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:480)
   b319) RNH-Val-Pro-Leu-Ser-Xaa₃-Thr-Val-Arg-Xaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:481)
   b320) RNH-Val-Pro-Leu-Ser-Arg-Xaa₃-Val-Arg-Xaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:482)
   b321) RNH-Val-Pro-Leu-Ser-Arg-Thr-Xaa₃-Arg-Xaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:483)
   b322) RNH-Val-Pro-Leu-Ser-Arg-Thr-Val-Xaa₃-Xaa₁-Thr-Xaa₂-Ile-Xaa₄-Ile-**[linker]**-Leu-Lys-Ala-Val-Ser-Lys-Glu-Met-Ser-Lys-Arg-Ser-Pro-(OH)NH₂ (SEQ ID NO:484)
   b323) RNH-Val-Pro-Leu-Ser-Arg-Thr-Val-Arg-Cys-Thr-Cys-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:485)
   b324) RNH-Xaa₃-Pro-Leu-Ser-Arg-Thr-Val-ArgXaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:486)
   b325) RNH-Val-Xaa₃-Leu-Ser-Arg-Thr-Val-ArgXaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:487)
   b326) RNH-Val-Pro-Xaa₃-Ser-Arg-Thr-Val-ArgXaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:488)
   b327) RNH-Val-Pro-Leu-Xaa₃-Arg-Thr-Val-ArgXaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:489)
   b328) RNH-Val-Pro-Leu-Ser-Xaa₃-Thr-Val-Arg-Xaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:490)
   b329) RNH-Val-Pro-Leu-Ser-Arg-Xaa₃-Val-Arg-Xaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:491)
   b330) RNH-Val-Pro-Leu-Ser-Arg-Thr-Xaa₃-Arg-Xaa₁-Thr-Xaa₂-Ile-Ser-Xaa₄-**[linker]**-Leu-Lys-Ala-Val-Ser-Lys-Glu-Met-Ser-Lys-Arg-Ser-Pro-(OH)NH2 (SEQ ID NO:492)
   b331) RNH-Val-Pro-Leu-Ser-Arg-Thr-Val-Xaa₃-Xaal -Thr-Xaa₂-Ile-Ser-Xaa₄-**[linker]**-Leu-Lys-Ala-Val-Ser-Lys-Glu-Met-Ser-Lys-Arg-Ser-Pro-(OH)NH₂ (SEQ ID NO:493)
IP-10-(1-17)-[linker]-IP-10-(65-77)-cyclic (Glu71-Lys74) acid or amide
   b332) RNH-Val-Pro-Leu-Ser-Arg-Thr-Val-Arg-Cys-Thr-Cys-Ile-Ser-Ile-Ser-Asn-Gln-**[linker]** -Leu-Lys-Ala-Val-Ser-Lys-Glu-Met-Ser-Lys-Arg-Ser-Pro-(OH)NH₂ (SEQ ID NO:494)
   b333) RNH-Xaa₃-Pro-Leu-Ser-Arg-Thr-Val-Arg-Xaa₁-Thr-Xaa₂-Ile-Ser-Ile-Ser-Asn-Gln-**[linker]-**Leu-Lys-Ala-Val-Ser-Lys-Glu-Met-Ser-Lys-Arg-Ser- Pro-(OH)NH₂ (SEQ ID NO:495)
   b334) RNH-Val-Xaa₃-Leu-Ser-Arg-Thr-Val-Arg-Xaa₁-Thr-Xaa₂-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:496)
   b335) RNH-Val-Pro-Xaa₃-Ser-Arg-Thr-Val-Arg-Xaa₁-Thr-Xaa₂-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:497)
   b336) RNH-Val-Pro-Leu-Xaa₃-Arg-Thr-Val-Arg-Xaa₁-Thr-Xaa₂-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:498)
   b337) RNH-Val-Pro-Leu-Ser-Xaa₃-Thr-Val-Arg-Xaa₁-Thr-Xaa₂-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:499)
   b338) RNH-Val-Pro-Leu-Ser-Arg-Xaa₃-Val-Arg-Xaa₁-Thr-Xaa₂-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:500)
   b339) RNH-Val-Pro-Leu-Ser-Arg-Thr-Xaa₃-Arg-Xaa₁-Thr-Xaa₂-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:501)
   b340) RNH-Val-Pro-Leu-Ser-Arg-Thr-Val-Xaa₃-Xaa₁-Thr-Xaa₂-Ile-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:502)
   b341) RNH-Val-Pro-Leu-Ser-Arg-Thr-Val-Arg-Cys-Thr-Cys-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:503)
   b342) RNH-Xaa₃-Pro-Leu-Ser-Arg-Thr-Val-Arg-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:504)
   b343) RNH-Val-Xaa₃-Leu-Ser-Arg-Thr-Val-Arg-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:505)
   b344) RNH-Val-Pro-Xaa₃-Ser-Arg-Thr-Val-Arg-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:506)
   b345) RNH-Val-Pro-Leu-Xaa₃-Arg-Thr-Val-Arg-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:507)
   b346) RNH-Val-Pro-Leu-Ser-Xaa₃-Thr-Val-Arg-Xaa₁-Thr-Xaa₂-Xaa₄-Ser-Ile-Ser-Asn-Gln-**[linker]-**Leu-Lys-Ala-Val-Ser-Lys-Glu-Met-Ser-Lys-Arg-Ser-Pro-(OH)NH₂ (SEQ ID NO:508)
   b347) RNH-Val-Pro-Leu-Ser-Arg-Xaa₃-Val-Arg-Xaa₁-Thr-Xaa₂-Xaa₄-Ser-Ile-Ser-Asn-Gln-**[linker]-**Leu-Lys-Ala-Val-Ser-Lys-Glu-Met-Ser-Lys-Arg-Ser-Pro-(OH)NH₂ (SEQ ID NO:509)
   b348) RNH-Val-Pro-Leu-Ser-Arg-Thr-Xaa₃-Arg-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:510)
   b349) RNH-Val-Pro-Leu-Ser-Arg-Thr-Val-Xaa₃-Xaa₁-Thr-Xaa₂-Xaa₄-Ser-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:511)
   b350) RNH-Val-Pro-Leu-Ser-Arg-Thr-Val-Arg-Cys-Thr-Cys-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:512)
   b351) RNH-Xaa₃-Pro-Leu-Ser-Arg-Thr-Val-Arg-Xaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:513)
   b352) RNH-Val-Xaa₃-Leu-Ser-Arg-Thr-Val-ArgXaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:514)
   b353) RNH-Val-Pro-Xaa3-Ser-Arg-Thr-Val-ArgXaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:515)
   b354) RNH-Val-Pro-Leu-Xaa₃-Arg-Thr-Val-ArgXaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:516)
   b355) RNH-Val-Pro-Leu-Ser-Xaa₃-Thr-Val-Arg-Xaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:517)
   b356) RNH-Val-Pro-Leu-Ser-Arg-Xaa₃-Val-Arg-Xaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:518)
   b357) RNH-Val-Pro-Leu-Ser-Arg-Thr-Xaa₃-Arg-Xaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:519)
   b358) RNH-Val-Pro-Leu-Ser-Arg-Thr-Val-Xaa₃-Xaa₁-Thr-Xaa₂-Ile-Xaa₄-Ile-Ser-Asn-Gln-**[linker]**-Leu-Lys-Ala-Val-Ser-Lys-Glu-Met-Ser-Lys-Arg-Ser-Pro-(OH)NH₂ (SEQ ID NO:520)
   b359) RNH-Val-Pro-Leu-Ser-Arg-Thr-Val-Arg-Cys-Thr-Cys-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:521)
   b360) RNH-Xaa₃-Pro-Leu-Ser-Arg-Thr-Val-Arg-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:522)
   b361) RNH-Val-Xaa₃-Leu-Ser-Arg-Thr-Val-Arg-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:523)
   b362) RNH-Val-Pro-Xaa₃-Ser-Arg-Thr-Val-Arg-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:524)
   b363) RNH-Val-Pro-Leu-Xaa₃-Arg-Thr-Val-ArgXaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:525)
   b364) RNH-Val-Pro-Leu-Ser-Xaa₃-Thr-Val-Arg-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:526)
   b365) RNH-Val-Pro-Leu-Ser-Arg-Xaa₃-Val-Arg-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:527)
   b366) RNH-Val-Pro-Leu-Ser-Arg-Thr-Xaa₃-Arg-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:528)
   b367) RNH-Val-Pro-Leu-Ser-Arg-Thr-Val-Xaa₃-Xaa₁-Thr-Xaa₂-Ile-Ser-Xaa₄-Ser-Asn-Gln-**[linker]**-Leu-Lys-Ala-Val-Ser-Lys-Glu-Met-Ser-Lys-Arg-Ser-Pro-(OH)NH₂ (SEQ ID NO:529)
   b368) RNH-Val-Pro-Leu-Ser-Arg-Thr-Val-Arg-Cys-Thr-Cys-Ile-Ser-Ile-Ser-Xaa₄-Gln-**[linker]-**Leu-Lys-Ala-Val-Ser-Lys-Glu-Met-Ser-Lys-Arg-Ser-Pro-(OH)NH₂ (SEQ ID NO:530)
   b369) RNH-Xaa₃-Pro-Leu-Ser-Arg-Thr-Val-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:531)
   b370) RNH-Val-Xaa₃-Leu-Ser-Arg-Thr-Val-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:532)
   b371) RNH-Val-Pro-Xaa₃-Ser-Arg-Thr-Val-ArgXaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:533)
   b372) RNH-Val-Pro-Leu-Xaa₃-Arg-Thr-Val-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:534)
   b373) RNH-Val-Pro-Leu-Ser-Xaa₃-Thr-Val-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:535)
   b374) RNH-Val-Pro-Leu-Ser-Arg-Xaa₃-Val-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:536)
   b375) RNH-Val-Pro-Leu-Ser-Arg-Thr-Xaa₃-Arg-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:537)
   b376) RNH-Val-Pro-Leu-Ser-Arg-Thr-Val-Xaa₃-Xaa₁-Thr-Xaa₂-Ile-Ser-Ile-Ser-Xaa₄-Gln-**[linker]**-Leu-Lys-Ala-Val-Ser-Lys-Glu-Met-Ser-Lys-Arg-Ser-Pro-(OH)NH₂ (SEQ ID NO:538)
   b377) RNH-Val-Pro-Leu-Ser-Arg-Thr-Val-Arg-Cys-Thr-Cys-Ile-Ser-Ile-Ser--Gln-Xaa₄-**[linker]**-Leu-Lys-Ala-Val-Ser-Lys-Glu-Met-Ser-Lys-Arg-Ser-Pro-(OH)NH₂ (SEQ ID NO:539)
   b378) RNH-Xaa₃-Pro-Leu-Ser-Arg-Thr-Val-Arg-Xaa₁-Thr-Xaa₂-Ile-Ser-Ile-Ser-Gln-Xaa₄-**[linker]**-Leu-Lys-Ala-Val-Ser-Lys-Glu-Met-Ser-Lys-Arg-Ser-Pro-(OH)NH₂ (SEQ ID NO:540)
   b379) RNH-Val-Xaa₃-Leu-Ser-Arg-Thr-Val-Arg-Xaa₁-Thr-Xaa₂-Ile-Ser-Ile-Ser-GlnXaa₄-**[linker]**-Leu-Lys-Ala-Val-Ser-Lys-Glu-Met-Ser-Lys-Arg-Ser-Pro-(OH)NH₂ (SEQ ID NO:541)
   b380) RNH-Val-Pro-Xaa₃-Ser-Arg-Thr-Val-Arg-Xaa₁-Thr-Xaa₂-Ile-Ser-Ile-Ser-GlnXaa₄-**[linker]**-Leu-Lys-Ala-Val-Ser-Lys-Glu-Met-Ser-Lys-Arg-Ser-Pro-(OH)NH₂ (SEQ ID NO:542)
   b381) RNH-Val-Pro-Leu-Xaa₃-Arg-Thr-Val-Arg-Xaa₁-Thr-Xaa₂-Ile-Ser-Ile-Ser-GInXaa₄-**[linker]**-Leu-Lys-Ala-Val-Ser-Lys-Glu-Met-Ser-Lys-Arg-Ser-Pro-(OH)NH₂ (SEQ ID NO:543)
   b382) RNH-Val-Pro-Leu-Ser-Xaa₃-Thr-Val-Arg-Xaa₁-Thr-Xaa₂-Ile-Ser-Ile-Ser-GlnXaa₄-**[linker]**-Leu-Lys-Ala-Val-Ser-Lys-Glu-Met-Ser-Lys-Arg- Ser-Pro-(OH)NH₂ (SEQ ID NO:544)
   b383) RNH-Val-Pro-Leu-Ser-Arg-Xaa₃-Val-Arg-Xaa₁-Thr-Xaa₂-Ile-Ser-Ile-Ser-Gln-Xaa₄-**[linker]**-Leu-Lys-Ala-Val-Ser-Lys-Glu-Met-Ser-Lys-Arg-Ser-Pro-(OH)NH₂ (SEQ ID NO:545)
   b384) RNH-Val-Pro-Leu-Ser-Arg-Thr-Xaa₃-Arg-Xaa₁-Thr-Xaa₂-Ile-Ser-Ile-Ser-Gln-Xaa₄-**[linker]**-Leu-Lys-Ala-Val-Ser-Lys-Glu-Met-Ser-Lys-Arg-Ser-Pro-(OH)NH₂ (SEQ ID NO:546)
   b385) RNH-Val-Pro-Leu-Ser-Arg-Thr-Val-Xaa₃-Xaa₁-Thr-Xaa₂-Ile-Ser-Ile-Ser-Gln-Xaa₄-**[linker]**-Leu-Lys-Ala-Vat-Ser-Lys-Glu-Met-Ser-Lys-Arg-Ser-Pro-(OH)NH₂ (SEQ ID NO:548)
IP-10-(1-14)-[linker]-IP-10-(54-66)-cyclic(Glu57-Lys62) acid or amide
   b386) RNH-Val-Pro-Leu-Ser-Arg-Thr-Val-Arg-Cys-Thr-Cys-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:549)
   b387) RNH-Xaa₃-Pro-Leu-Ser-Arg-Thr-Val-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:550)
   b388) RNH-Val-Xaa₃-Leu-Ser-Arg-Thr-Val-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:551)
   b389) RNH-Val-Pro-Xaa₃-Ser-Arg-Thr-Val-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:552)
   b390) RNH-Val-Pro-Leu-Xaa₃-Arg-Thr-Val-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:553)
   b391) RNH-Val-Pro-Leu-Ser-Xaa₃-Thr-Val-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:554)
   b392) RNH-Val-Pro-Leu-Ser-Arg-Xaa₃-Val-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:555)
   b393) RNH-Val-Pro-Leu-Ser-Arg-Thr-Xaa₃-Arg-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:556)
   b394) RNH-Val-Pro-Leu-Ser-Arg-Thr-Val-Xaa₃-Xaa₁-Thr-Xaa₂-Ile-Ser-Ile-**[linker]**-Leu-Asn-Pro-Glu-Ser-Lys-Ala-Ile-Lys-Asn-Leu-Leu-Lys-(OH)NH₂ (SEQ ID NO:557)
   b395) RNH-Val-Pro-Leu-Ser-Arg-Thr-Val-Arg-Cys-Thr-Cys-Xaa₄Ser-Ile-**[linker]**-Leu-Asn-Pro-Glu-Ser-Lys-Ala-Ile-Lys-Asn-Leu-Leu-Lys-(OH)NH₂ (SEQ ID NO:558)
   b396) RNH-Xaa₃-Pro-Leu-Ser-Arg-Thr-Val-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:559)
   b397) RNH-Val-Xaa₃-Leu-Ser-Arg-Thr-Val-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:560)
   b398) RNH-Val-Pro-Xaa₃-Ser-Arg-Thr-Val-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:561)
   b399) RNH-Val-Pro-Leu-Xaa₃-Arg-Thr-Val-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:562)
   b400) RNH-Val-Pro-Leu-Ser-Xaa₃-Thr-Val-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:563)
   b401) RNH-Val-Pro-Leu-Ser-Arg-Xaa₃-Val-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:564)
   b402) RNH-Val-Pro-Leu-Ser-Arg-Thr-Xaa₃-Arg-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:565)
   b403) RNH-Val-Pro-Leu-Ser-Arg-Thr-Val-Xaa₃-Xaa₁-Thr-Xaa₂-Xaa₄-Ser-Ile-**[linker]**-Leu-Asn-Pro-Glu-Ser-Lys-Ala-Ile-Lys-Asn-Leu-Leu-Lys-(OH)NH₂ (SEQ ID NO:566)
   b404) RNH-Val-Pro-Leu-Ser-Arg-Tk-Val-Arg-Cys-Thr-Cys-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:567)
   b405) RNH-Xaa₃-Pro-Leu-Ser-Arg-Thr-Val-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:568)
   b406) RNH-Val-Xaa₃-Leu-Ser-Arg-Thr-Val-Arg-Xaa₁-Thr-Xaa₂-Ile-Xaa₄-Ile-[**linker**]-Leu-Asn-Pro-Glu-Ser-Lys-Ala-Ile-Lys-Asn-Leu-Leu-Lys-(OH)NH₂ (SEQ ID NO:569)
   b407) RNH-Val-Pro-Xaa₃-Ser-Arg-Thr-Val-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:570)
   b408) RNH-Val-Pro-Leu-Xaa₃-Arg-Thr-Val-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:571)
   b409) RNH-Val-Pro-Leu-Ser-Xaa₃-Thr-Val-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:572)
   b410) RNH-Val-Pro-Leu-Ser-Arg-Xaa₃-Val-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:573)
   b411) RNH-Val-Pro-Leu-Ser-Arg-Thr-Xaa₃-Arg-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:574)
   b412) RNH-Val-Pro-Leu-Ser-Arg-Thr-Val-Xaa₃-Xaa₁-Thr-Xaa₂-Ile-Xaa₄-Ile-**[linker]**-Leu-Asn-Pro-Glu-Ser-Lys-Ala-Ile-Lys-Asn-Leu-Leu-Lys-(OH)NH₂ (SEQ ID NO:575)
   b413) RNH-Val-Pro-Leu-Ser-Arg-Thr-Val-Arg-Cys-Thr-Cys-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:576)
   b414) RNH-Xaa₃-Pro-Leu-Ser-Arg-Thr-Val-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:577)
   b415) RNH-Val-Xaa₃-Leu-Ser-Arg-Thr-Val-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:578)
   b416) RNH-Val-Pro-Xaa₃-Ser-Arg-Thr-Val-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:579)
   b417) RNH-Val-Pro-Leu-Xaa₃-Arg-Thr-Val-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:580)
   b418) RNH-Val-Pro-Leu-Ser-Xaa₃-Thr-Val-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:581)
   b419) RNH-Val-Pro-Leu-Ser-Arg-Xaa₃-Val-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:582)
   b420) RNH-Val-Pro-Leu-Ser-Arg-Thr-Xaa₃-Arg-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:583)
   b421) RNH-Val-Pro-Leu-Ser-Arg-Thr-Val-Xaa₃-Xaa₁-Thr-Xaa₂-Ile-Ser-Xaa₄-**[linker]**-Leu-Asn-Pro-Glu-Ser-Lys-Ala-Ile-Lys-Asn-Leu-Leu-Lys-(OH)NH₂ (SEQ ID NO:584)
IP-10-(1-17)-[linker]-IP-10-(54-66)-cyclic(Glu57-Lys62) acid or amide
   b422) RNH-Val-Pro-Leu-Ser-Arg-Thr-Val-Arg-Cys-Thr-Cys-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:585)
   b423) RNH-Xaa₃-Pro-Leu-Ser-Arg-Thr-Val-Arg-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:586)
   b424) RNH-Val-Xaa₃-Leu-Ser-Arg-Thr-Val-Arg-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:587)
   b425) RNH-Val-Pro-Xaa₃-Ser-Arg-Thr-Val-Arg-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:588)
   b426) RNH-Val-Pro-Leu-Xaa₃-Arg-Thr-Val-Arg-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:589)
   b427) RNH-Val-Pro-Leu-Ser-Xaa₃-Thr-Val-Arg-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:590)
   b428) RNH-Val-Pro-Leu-Ser-Arg-Xaa₃-Val-Arg-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:591)
   b429) RNH-Val-Pro-Leu-Ser-Arg-Thr-Xaa₃-Arg-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:592)
   b430) RNH-Val-Pro-Leu-Ser-Arg-Thr-Val-Xaa₃-Xaa₁-Thr-Xaa₂-Ile-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:593)
   b431) RNH-Val-Pro-Leu-Ser-Arg-Thr-Val-Arg-Cys-Thr-Cys-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:594)
   b432) RNH-Xaa₃-Pro-Leu-Ser-Arg-Thr-Val-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:595)
   b433) RNH-Val-Xaa₃-Leu-Ser-Arg-Thr-Val-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:596)
   b434) RNH-Val-Pro-Xaa₃-Ser-Arg-Thr-Val-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:597)
   b435) RNH-Val-Pro-Leu-Xaa₃-Arg-Thr-Val-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:598)
   b436) RNH-Val-Pro-Leu-Ser-Xaa₃-Thr-Val-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:599)
   b437) RNH-Val-Pro-Leu-Ser-Arg-Xaa₃-Val-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:600)
   b438) RNH-Val-Pro-Leu-Ser-Arg-Thr-Xaa₃-Arg-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:601)
   b439) RNH-Val-Pro-Leu-Ser-Arg-Thr-Val-Xaa₃-Xaa₁-Thr-Xaa₂-Xaa₄-Ser-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:602)
   b440) RNH-Val-Pro-Leu-Ser-Arg-Thr-Val-Arg-Cys-Thr-Cys-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:603)
   b441) RNH-Xaa₃-Pro-Leu-Ser-Arg-Thr-Val-Arg-Xaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:604)
   b442) RNH-Val-Xaa₃-Leu-Ser-Arg-Thr-Val-Arg-Xaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:605)
   b443) RNH-Val-Pro-Xaa₃-Ser-Arg-Thr-Val-Arg-Xaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:606)
   b444) RNH-Val-Pro-Leu-Xaa₃-Arg-Thr-Val-Arg-Xaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:607)
   b445) RNH-Val-Pro-Leu-Ser-Xaa₃-Thr-Val-Arg-Xaa₁-Thr-Xaa₂-Ile-Xa₁₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:608)
   b446) RNH-Val-Pro-Leu-Ser-Arg-Xaa₃-Val-Arg-Xaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:609)
   b447) RNH-Val-Pro-Leu-Ser-Arg-Thr-Xaa₃-Arg-Xaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:610)
   b448) RNH-Val-Pro-Leu-Ser-Arg-Thr-Val-Xaa₃-Xaa₁-Thr-Xaa₂-Ile-Xaa₄-Ile-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:611)
   b449) RNH-Val-Pro-Leu-Ser-Arg-Thr-Val-Arg-Cys-Thr-Cys-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:612)
   b450) RNH-Xaa₃-Pro-Leu-Ser-Arg-Thr-Val-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:613)
   b451) RNH-Val-Xaa₃-Leu-Ser-Arg-Thr-Val-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:614)
   b452) RNH-Val-Pro-Xaa₃-Ser-Arg-Thr-Val-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:615)
   b453) RNH-Val-Pro-Leu-Xaa₃-Arg-Thr-Val-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:616)
   b454) RNH-Val-Pro-Leu-Ser-Xaa₃-Thr-Val-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:617)
   b455) RNH-Val-Pro-Leu-Ser-Arg-Xaa₃-Val-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:618)
   b456) RNH-Val-Pro-Leu-Ser-Arg-Thr-Xaa₃-Arg-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:619)
   b457) RNH-Val-Pro-Leu-Ser-Arg-Thr-Val-Xaa₃-Xaa₁-Thr-Xaa₂-Ile-Ser-Xaa₄-Ser-Asn-Gln-**[linker]**-Leu-Asn-Pro-Glu-Ser-Lys-Ala-Ile-Lys-Asn-Leu-Leu-Lys-(OH)NH₂ (SEQ ID NO:620)
   b458) RNH-Val-Pro-Leu-Ser-Arg-Thr-Val-Arg-Cys-Thr-Cys-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:621)
   b459) RNH-Xaa₃-Pro-Leu-Ser-Arg-Thr-Val-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:622)
   b460) RNH-Vat-Xaa₃-Leu-Ser-Arg-Thr-Val-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:623)
   b461) RNH-Val-Pro-Xaa₃-Ser-Arg-Thr-Val-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:624)
   b462) RNH-Val-Pro-Leu-Xaa₃-Arg-Thr-Val-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:625)
   b463) RNH-Val-Pro-Leu-Ser-Xaa₃-Thr-Val-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:626)
   b464) RNH-Val-Pro-Leu-Ser-Arg-Xaa₃-Val-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:627)
   b465) RNH-Val-Pro-Leu-Ser-Arg-Thr-Xaa₃-Arg-Xaa₁-Thr-Xaa₂-Ile-Ser-Ile-Ser-Xaa₄-Gln-**[linker]**-Leu-Asn-Pro-Glu-Ser-Lys-Ala-Ile-Lys-Asn-Leu-Leu-Lys-(OH)NH₂ (SEQ ID NO:628)
   b466) RNH-Val-Pro-Leu-Ser-Arg-Thr-Val-Xaa₃-Xaa₁-Thr-Xaa₂-Ile-Ser-Ile-Ser-Xaa₄-Gln-**[linker]**-Leu-Asn-Pro-Glu-Ser-Lys-Ala-He-Lys-Asn-Leu-Leu-Lys-(OH)NH₂ (SEQ ID NO:629)
   b467) RNH-Val-Pro-Leu-Ser-Arg-Thr-Val-Arg-Cys-Thr-Cys-Ile-Ser-Ile-Ser-Gln-Xaa₄-[**linker**]-Leu-Asn-Pro-Glu-Ser-Lvs-Ala-Ile-Lys-Asn-Leu-Leu-Lys-(OH)NH₂ (SEQ ID NO:630)
   b468) RNH-Xaa₃-Pro-Leu-Ser-Arg-Thr-Val-Arg-Xaa₁-Thr-Xaa₂-Ile-Ser-Ile-Ser-Gln-Xaa₄-**[linker]**-Leu-Asn-Pro-Glu-Ser-Lys-Ala-Ile-Lys-Asn-Leu-Leu-Lys-(OH)NH₂ (SEQID NO:631)
   b469) RNH-Val-Xaa₃-Leu-Ser-Arg-Thr-Val-Arg-Xaa₁-Thr-Xaa₂-Ile-Ser-Ile-Ser-GlnXaa₄-**[linker]**-Leu-Asn-Pro-Glu-Ser-Lys-Ala-Ile-Lys-Asn-Leu-Leu-Lys-(OH)NH₂ (SEQ ID NO:632)
   b470) RNH-Val-Pro-Xaa₃-Ser-Arg-Thr-Val-Arg-Xaa₁-Thr-Xaa₂-Ile-Ser-Ile-Ser-GlnXaa₄-**[linker]**-Leu-Asn-Pro-Glu-Ser-Lys-Ala-Ile-Lys-Asn-Leu-Leu-Lys-(OH)NH₂ (SEQ ID NO:633)
   b471) RNH-Val-Pro-Leu-Xaa₃-Arg-Thr-Val-Arg-Xaa₁-Thr-Xaa₂-Ile-Ser-Ile-Ser-GlnXaa₄-**[linker]**-Leu-Asn-Pro-Glu-Ser-Lys-Ala-Ile-Lys-Asn-Leu-Leu-Lys-(OH)NH₂ (SEQ ID NO:634)
   b472) RNH-Val-Pro-Leu-Ser-Xaa₃-Thr-Val-Arg-Xaa₁-Thr-Xaa₂-Ile-Ser-Ile-Ser-GlnXaa₄-**[linker]**-Leu-Asn-Pro-Glu-Ser-Lys-Ala-Ile-Lys-Asn-Leu-Leu-Lys-(OH)NH₂ (SEQ ID NO:635)
   b473) RNH-Val-Pro-Leu-Ser-Arg-Xaa₃-Val-Arg-Xaa₁-Thr-Xaa₂-Ile-Ser-Ile-Ser-Gln-Xaa₄-**[linker]**-Leu-Asn-Pro-Glu-Ser-Lys-Ala-Ile-Lys-Asn-Leu-Leu-Lys-(OH)NH₂ (SEQ ID NO:636)
   b474) RNH-Val-Pro-Leu-Ser-Arg-Thr-Xaa₃-Arg-Xaa₁-Thr-Xaa₂-Ile-Ser-Ile-Ser-Gln-Xaa₄-**[linker]**-Leu-Asn-Pro-Glu-Ser-Lys-Ala-Ile-Lys-Asn-Leu-Leu-Lys-(OH)NH₂ (SEQ ID NO:637)
   b475) RNH-Val-Pro-Leu-Ser-Arg-Thr-Val-Xaa₃-Xaa₁-Thr-Xaa₂-Ile-Ser-Ile-Ser-Gln-Xaa₄-**[linker]**-Leu-Asn-Pro-Glu-Ser-Lys-Ala-Ile-Lys-Asn-Leu-Leu-Lys-(OH)NH₂ (SEQ ID NO:638)

In some embodiments IP-10 analogs were cyclized by etherification between Lys66 and Ser69 (underlined residues are cyclized).
IP-10-(1-14)-[linker]-IP-10-(59-71)-cyclic(Lys66-Ser69) acid or amide
   b476) RNH-Val-Pro-Leu-Ser-Arg-Thr-Val-Arg-Cys-Thr-Cys-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:639)
   b477) RNH-Xaa₃-Pro-Leu-Ser-Arg-Thr-Val-Arg-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:640)
   b478) RNH-Val-Xaa₃-Leu-Ser-Arg-Thr-Val-Arg-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:641)
   b479) RNH-Val-Pro-Xaa₃-Ser-Arg-Thr-Val-Arg-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:642)
   b480) RNH-Val-Pro-Leu-Xaa₃-Arg-Thr-Val-Arg-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:643)
   b481) RNH-Val-Pro-Leu-Ser-Xaa₃-Thr-Val-Arg-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:644)
   b482) RNH-Val-Pro-Leu-Ser-Arg-Xaa₃-Val-Arg-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:645)
   b483) RNH-Val-Pro-Leu-Ser-Arg-Thr-Xaa₃-Arg-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:646)
   b484) RNH-Val-Pro-Leu-Ser-Arg-Thr-Val-Xaa₃-Xaa₁-Thr-Xaa₂-Ile-Ser-Ile-**[linker]**-Lys-Ala-Ile-Lys-Asn-Leu-Leu-Lys-Ala-Val-Ser-Lys-Glu-(OH)NH₂ (SEQ ID NO:647)
   b485) RNH-Val-Pro-Leu-Ser-Arg-Thr-Val-Arg-Cys-Thr-Cys-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:648)
   b486) RNH-Xaa₃-Pro-Leu-Ser-Arg-Thr-Val-Arg-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:649)
   b487) RNH-Val-Xaa₃-Leu-Ser-Arg-Thr-Val-Arg-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:650)
   b488) RNH-Val-Pro-Xaa₃-Ser-Arg-Thr-Val-Arg-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:651)
   b489) RNH-Val-Pro-Leu-Xaa₃-Arg-Thr-Val-Arg-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:652)
   b490) RNH-Val-Pro-Leu-Ser-Xaa₃-Thr-Val-Arg-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:653)
   b491) RNH-Val-Pro-Leu-Ser-Arg-Xaa₃-Val-Arg-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:654)
   b492) RNH-Val-Pro-Leu-Ser-Arg-Thr-Xaa₃-Arg-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:655)
   b493) RNH-Val-Pro-Leu-Ser-Arg-Thr-Val-Xaa₃-Xaa₁-Thr-Xaa₂-Xaa₄-Ser-Ile-**[linker]**-Lys-Ala-Ile-Lys-Asn-Leu-Leu-Lys-Ala-Val-Ser-Lys-Glu-(OH)NH₂ (SEQ ID NO:656)
   b494) RNH-Val-Pro-Leu-Ser-Arg-Thr-Val-Arg-Cys-Thr-Cys-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:657)
   b495) RNH-Xaa₃-Pro-Leu-Ser-Arg-Thr-Val-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:658)
   b496) RNH-Val-Xaa₃-Leu-Ser-Arg-Thr-Val-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:659)
   b497) RNH-Val-Pro-Xaa₃-Ser-Arg-Thr-Val-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:660)
   b498) RNH-Val-Pro-Leu-Xaa₃-Arg-Thr-Val-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:661)
   b499) RNH-Val-Pro-Leu-Ser-Xaa₃-Thr-Val-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:662)
   b500) RNH-Val-Pro-Leu-Ser-Arg-Xaa₃-Val-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:663)
   b501) RNH-Val-Pro-Leu-Ser-Arg-Thr-Xaa₃-Arg-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:664)
   b502) RNH-Val-Pro-Leu-Ser-Arg-Thr-Val-Xaa₃-Xaa₁-Thr-Xaa₂-Ile-Xaa₄-Ile-**[linker]**-Lys-Ala-Ile-Lys-Asn-Leu-Leu-Lys-Ala-Val-Ser-Lys-Glu-(OH)NH₂ (SEQ ID NO:665)
   b503) RNH-Val-Pro-Leu-Ser-Arg-Thr-Val-Arg-Cys-Thr-Cys-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:666)
   b504) RNH-Xaa₃-Pro-Leu-Ser-Arg-Thr-Val-Arg-Xaa)-Thr-Xaa₂-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:667)
   b505) RNH-Val-Xaa₃-Leu-Ser-Arg-Thr-Val-Arg-Xaa₁-Thr-Xaa₂-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:668)
   b506) RNH-Val-Pro-Xaa₃-Ser-Arg-Thr-Val-Arg-Xaa₁-Thr-Xaa₂-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:669)
   b507) RNH-Val-Pro-Leu-Xaa₃-Arg-Thr-Val-Arg-Xaa₁-Thr-Xaa₂-Ile-Ser-Xa1₄-**[linker]**-Lys-Ala-Ile-Lys-Asn-Leu-Leu-Lys-Ala-Val-Ser-Lys-Glu-(OH)NH₂ (SEQ ID NO:670)
   b508) RNH-Val-Pro-Leu-Ser-Xaa₃-Thr-Val-Arg-Xaa₁-Thr-Xaa₂-Ile-Ser-Xaa₄-**[linker]**-Lys-Ala-Ile-Lys-Asn-Le1u-Leu-Lys-Ala-Val-Ser-Lys-Glu-(OH)NH₂ (SEQ ID NO:671)
   b509) RNH-Val-Pro-Leu-Ser-Arg-Xaa₃-Val-Arg-Xaa₁-Thr-Xaa₂-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:672)
   b510) RNH-Val-Pro-Leu-Ser-Arg-Thr-Xaa₃-Arg-Xaa₁-Thr-Xaa₂-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:673)
   b511) RNH-Val-Pro-Leu-Ser-Arg-Thr-Val-Xaa₃-Xaa₁-Thr-Xaa₂-Ile-Ser-Xaa₄-**[linker]**-Lys-Ala-Ile-Lys-Asn-Leu-Leu-Lys-Ala-Val-Ser-Lys-Glu-(OH)NH₂ (SEQ ID NO:674)
IP-10-(1-17)-[linker]-IP-10-(59-71)-cyclic(Lys66-Ser69) acid or amide
   b512) RNH-Val-Pro-Leu-Ser-Arg-Thr-Val-Arg-Cys-Thr-Cys-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:675)
   b513) RNH-Xaa₃-Pro-Leu-Ser-Arg-Thr-Val-Arg-Xaa₁-Thr-Xaa₂-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:676)
   b514) RNH-Val-Xaa₃-Leu-Ser-Arg-Thr-Val-Arg-Xaa₁-Thr-Xaa₂-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:677)
   b515) RNH-Val-Pro-Xaa₃-Ser-Arg-Thr-Val-Arg-Xaa₁-Thr-Xaa₂-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:678)
   b516) RNH-Val-Pro-Leu-Xaa₃-Arg-Thr-Val-Arg-Xaa₁-Thr-Xaa₂-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:679)
   b517) RNH-Val-Pro-Leu-Ser-Xaa₃-Thr-Val-Arg-Xaa₁-Thr-Xaa₂-Ile-Ser-Ile-Ser-Asn-Gln-**[linker]** -Lys-Ala-Ile-Lys-Asn-Leu-Leu-Lys-Ala-Val-Ser-Lys-Glu-(OH)NH₂ (SEQ ID NO:680)
   b518) RNH-Val-Pro-Leu-Ser-Arg-Xaa₃-Val-Arg-Xaa₁-Thr-Xaa₂-Ile-Ser-Ile-Ser-Asn-Gln-**[linker]** -Lys-Ala-Ile-Lys-Asn-Leu-Leu-Lys-Ala-Val-Ser-Lys-Glu-(OH)NH₂ (SEQ ID NO: 681)
   b519) RNH-Val-Pro-Leu-Ser-Arg-Thr-Xaa₃-Arg-Xaa₁-Thr-Xaa₂-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:682)
   b520) RNH-Val-Pro-Leu-Ser-Arg-Thr-Val-Xaa₃-Xaa₁-Thr-Xaa₂-Ile-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:683)
   b521) RNH-Val-Pro-Leu-Ser-Arg-Thr-Val-Arg-Cys-Thr-Cys-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:684)
   b522) RNH-Xaa₃-Pro-Leu-Ser-Arg-Thr-Val-Arg-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:685)
   b523) RNH-Val-Xaa₃-Leu-Ser-Arg-Thr-Val-Arg-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:686)
   b524) RNH-Val-Pro-Xaa₃-Ser-Arg-Thr-Val-Arg-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:687)
   b525) RNH-Val-Pro-Leu-Xaa₃-Arg-Thr-Val-Arg-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:688)
   b526) RNH-Val-Pro-Leu-Ser-Xaa₃-Thr-Val-Arg-Xaa₁-Thr-Xaa₂-Xaa_{d}-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:689)
   b527) RNH-Val-Pro-Leu-Ser-Arg-Xaa₃-Val-Arg-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:690)
   b528) RNH-Val-Pro-Leu-Ser-Arg-Thr-Xaa₃-Arg-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:691)
   b529) RNH-Val-Pro-Leu-Ser-Arg-Thr-Val-Xaa₃-Xaa₁-Thr-Xaa₂-Xaa₄-Ser-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:692)
   b530) RNH-Val-Pro-Leu-Ser-Arg-Thr-Val-Arg-Cys-Thr-Cys-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:693)
   b531) RNH-Xaa₃-Pro-Leu-Ser-Arg-Thr-Val-Arg-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:694)
   b532) RNH-Val-Xaa₃-Leu-Ser-Arg-Thr-Val-Arg-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:695)
   b533) RNH-Val-Pro-Xaa₃-Ser-Arg-Thr-Val-Arg-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:696)
   b534) RNH-Val-Pro-Leu-Xaa₃-Arg-Thr-Val-Arg-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:697)
   b535) RNH-Val-Pro-Leu-Ser-Xaa₃-Thr-Val-Arg-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:698)
   b536) RNH-Val-Pro-Leu-Ser-Arg-Xaa₃-Val-Arg-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:699)
   b537) RNH-Val-Pro-Leu-Ser-Arg-Thr-Xaa₃-Arg-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:700)
   b538) RNH-Val-Pro-Leu-Ser-Arg-Thr-Val-Xaa₃-Xaa₁-Thr-Xaa₂-Ile-Xaa₄-Ile-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:701)
   b539) RNH-Val-Pro-Leu-Ser-Arg-Thr-Val-Arg-Cys-Thr-Cys-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:702)
   b540) RNH-Xaa₃-Pro-Leu-Ser-Arg-Thr-Val-Arg-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:703)
   b541) RNH-Val-Xaa₃-Leu-Ser-Arg-Thr-Val-Arg-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:704)
   b542) RNH-Val-Pro-Xaa₃-Ser-Arg-Thr-Val-Arg-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:705)
   b543) RNH-Val-Pro-Leu-Xaa₃-Arg-Thr-Val-Arg-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:706)
   b544) RNH-Val-Pro-Leu-Ser-Xaa₃-Thr-Val-Arg-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:707)
   b545) RNH-Val-Pro-Leu-Ser-Arg-Xaa₃-Val-Arg-Xaa₁-Thr-Xaa₂-Ile-Ser-Xaa₄-Ser-Asn-Gln-**[linker]**-Lys-Ala-Ile-Lys-Asn-Leu-Leu-Lxs-Ala-Val-Ser-Lys-Glu-(OH)NH₂ (SEQ ID NO:708)
   b546) RNH-Val-Pro-Leu-Ser-Arg-Thr-Xaa₃-Arg-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:709)
   b548) RNH-Val-Pro-Leu-Ser-Arg-Thr-Val-Xaa₃-Xaa₁-Thr-Xaa₂-Ile-Ser-Xaa₄-Ser-Asn-Gln-**[linker]**-Lys-Ala-Ile-Lys-Asn-Leu-Leu-Lys-Ala-Val-Ser-Lys-Glu-(OH)NH₂ (SEQ ID NO:710)
   b549) RNH-Val-Pro-Leu-Ser-Arg-Thr-Val-Arg-Cys-Thr-Cys-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:711)
   b550) RNH-Xaa₃-Pro-Leu-Ser-Arg-Thr-Val-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:712)
   b551) RNH-Val-Xaa₃-Leu-Ser-Arg-Thr-Val-Arg-Xaa₁-Thr-Xaa₂-Ile-Ser-Ile-Ser-Xaa₄-Gln-**[linker]-**Lys-Ala-Ile-Lys-Asn-Leu-Leu-Lys-Ala-Val-Ser-Lys-Glu-(OH)NH₂ (SEQ ID NO:713)
   b552) RNH-Val-Pro-Xaa₃-Ser-Arg-Thr-Val-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:714)
   b553) RNH-Val-Pro-Leu-Xaa₃-Arg-Thr-Val-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:715)
   b554) RNH-Val-Pro-Leu-Ser-Xaa₃-Thr-Val-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:716)
   b555) RNH-Val-Pro-Leu-Ser-Arg-Xaa₃-Val-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:717)
   b556) RNH-Val-Pro-Leu-Ser-Arg-Thr-Xaa₃-Arg-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:718)
   b557) RNH-Val-Pro-Leu-Ser-Arg-Thr-Val-Xaa₃-Xaa₁-Thr-Xaa₂-Ile-Ser-Ile-Ser-Xaa₄-Gln-**[linker]**-Lys-Ala-Ile-Lys-Asn-Leu-Leu-Lys-Ala-Val-Ser-Lys-Glu-(OH)NH₂ (SEQ ID NO:719)
   b558) RNH-Val-Pro-Leu-Ser-Arg-Thr-Val-Arg-Cys-Thr-Cys-Ile-Ser-Ile-Ser-Gln-Xaa₄-**[linker]**-Lys-Ala-Ile-Lys-Asn-Leu-Leu-Lys-Ala-Val-Ser-Lys-Glu-(OH)NH₂ (SEQ ID NO:720)
   b559) RNH-Xaa₃-Pro-Leu-Ser-Arg-Thr-Val-Arg-Xaa₁-Thr-Xaa₂-Ile-Ser-Ile-Ser-Gln-Xaa₄-**[linker]**-Lys-Ala-Ile-Lys-Asn-Leu-Leu-Lys-Ala-Val-Ser-Lys-Glu-(OH)NH₂ (SEQ ID NO:721)
   b560) RNH-Val-Xaa₃-Leu-Ser-Arg-Thr-Val-Arg-Xaa₁-Thr-Xaa₂-Ile-Ser-Ile-Ser-GlnXaa₄-**[linker]**-Lys-Ala-Ile-Lys-Asn-Leu-Leu-Lys-Ala-Val-Ser-Lys-Glu-(OH)NH₂ (SEQ ID NO:722)
   b561) RNH-Val-Pro-Xaa₃-Ser-Arg-Thr-Val-Arg-Xaa₁-Thr-Xaa₂-Ile-Ser-Ile-Ser-GlnXaa₄-**[linker]**-Lys-Ala-Ile-Lys-Asn-Leu-Leu-Lys-Ala-Val-Ser-Lys-Glu-(OH)NH₂ (SEQ ID NO:723)
   b562) RNH-Val-Pro-Leu-Xaa₃-Arg-Thr-Val-Arg-Xaa₁-Thr-Xaa₂-Ile-Ser-Ile-Ser-GlnXaa₄-**[linker]**-Lys-Ala-Ile-Lys-Asn-Leu-Leu-Lys-Ala-Val-Ser-Lys-Glu-(OH)NH₂ (SEQ ID NO:724)
   b563) RNH-Val-Pro-Leu-Ser-Xaa₃-Thr-Val-Arg-Xaa₁-Thr-Xaa₂-Ile-Ser-Ile-Ser-GlnXaa₄-**[linker]** -Lys-Ala-Ile-Lys-Asn-Leu-Leu-Lys-Ala-Val-Ser-Lys-Glu-(OH)NH₂ (SEQ ID NO:725)
   b564) RNH-Val-Pro-Leu-Ser-Arg-Xaa₃-Val-Arg-Xaa₁-Thr-Xaa₂-Ile-Ser-Ile-Ser-Gln-Xaa₄-**[linker]**-Lys-Ala-Ile-Lys-Asn-Leu-Leu-Lys-Ala-Val-Ser-Lys-Glu-(OH)NH₂ (SEQ ID NO:726)
   b565) RNH-Val-Pro-Leu-Ser-Arg-Thr-Xaa₃-Arg-Xaa₁-Thr-Xaa₂-Ile-Ser-Ile-Ser-Gln-Xaa₄-**[linker]**-Lys-Ala-Ile-Lys-Asn-Leu-Leu-Lys-Ala-Val-Ser-Lys-Glu-(OH)NH₂ (SEQ ID NO:727)
   b566) RNH-Val-Pro-Leu-Ser-Arg-Thr-Val-Xaa₃-Xaa₁-Thr-Xaa₂-Ile-Ser-Ile-Ser-Gln-Xaa₄-**[linker]**-Lys-Ala-Ile-Lys-Asn-Leu-Leu-Lys-Ala-Val-Ser-Lys-Glu-(OH)NH₂ (SEQ ID NO:728)

In the above structures:
**R** can be independently selected from substituted, unsubstituted, hetero-, straight-chained, branched, cyclic, saturated or unsaturated aliphatic radicals; or a substituted, unsubstituted, or hetero- aromatic radicals; PEG and derivatives thereof; and any other modifying group;
**Xaa₁** is selected from the group consisting of any L- or D-natural amino acid and any non-natural amino acid.
**Xaa₂** is selected from the group consisting of any L- or D-natural amino acid and any non-natural amino acid.
**Xaa₃** is selected from the group consisting of L-Pro, D-Pro, **P*, Btd** and any L- or D-natural and non-natural amino acid.
**Xaa₄** is selected from the group consisting of **P***, **Btd** and any L- or D-natural amino acid and any non-natural amino acid.
**P*** is: 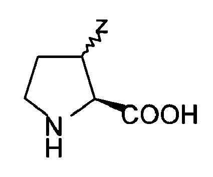 with Z= hydrogen, alkyl, alkenyl, alkynyl, alkylcarbonyl, arylcarbonyl, aryl, aryl-hydroxy, and more

A wide variety of amino acid substitutions may be made in polypeptide sequences, such as lysine to glutamic acid, lysine to aspartic acid, Orn to Glu, Orn to Asp. Moieties other than naturally occurring amino acids may also be substituted, such as Btd:
**Btd*** is: 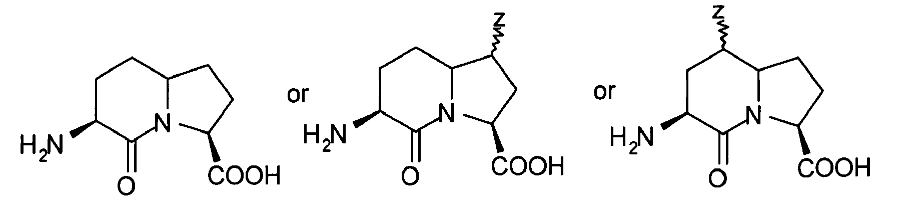
Z= hydrogen, alkyl, alkenyl, alkylcarbonyl, arylcarbonyl, aryl, aryl-hydroxy, and more

In some embodiments, **R** can be selected from the group consisting of hydrogen, alkyl, alkenyl, alkynyl, alkylcarbonyl, arylcarbonyl, aryl, PEG (polyethyleneglycol) and any other modifying group described herein. In some embodiments, **R** can comprise the peptide sequence Glu-Leu-Arg and a linker. In some embodiments, **R** can consist of only the peptide sequence Glu-Leu-Arg.

The **linker** is optional in some embodiments and can comprise, for example, any natural or non-natural amino acid. The amino acids can be represented by a formula: 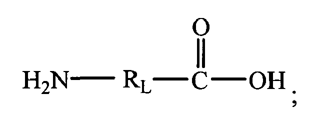 wherein R_{L} may be a substituted, unsubstituted, hetero-, straight-chained, branched, cyclic, saturated or unsaturated aliphatic radical; or a substituted, unsubstituted, or hetero- aromatic radical. In some embodiments, R_{L} can be substituted, unsubstituted, or hetero- forms of methyl, iso-propyl, sec-butyl, iso-butyl, benzyl, or a combination thereof.

In embodiments where R_{L} is substituted, examples of substitutents include, but are not limited to, hydroxyl, carboxyl, amino, imino groups and combinations thereof. In embodiments where R_{L} is heteroaliphatic, examples of heteroatoms include, but are not limited to, sulfur, phosphorous, oxygen, nitrogen and combinations thereof. In some embodiments, R_{L} can comprise substituted or unsubstituted poly(alkylene glycols), which include, but are not limited to, PEG, and PEG derivatives such as poly(ethylene oxide), poly(propylene glycol), poly(tetramethylene glycol), poly(ethylene oxide-co-propylene oxide), and copolymers and combinations thereof.

In some embodiments, R_{L} can be a substituted or unsubstituted alkylene comprising Cₙ carbons in the alkylene backbone, wherein n is an integer ranging from 1 to about 20; from about 2 to about 16; from about 3 to about 12; from about 4 to about 10; from about 3 to about 8, and any range therein. In these embodiments, R_{L} can be, for example, 11-amino-undecanoic acid.

In some embodiments, the linker comprises any combination of natural or non-natural amino acids, (Xaa), wherein the number of amino acids ranges from 1 to about 20; from about 2 to about 10; from about 3 to about 10, from about 1 to about 5, from about 1 to about 4, or any range therein. In some embodiments, the linker can comprise any combination of up to four natural or non-natural amino acids such as, for example, -(Gly)₄-(SEQ ID NO:1640).

In some embodiments, there is no linker. In some embodiments, the mimetics are comprised of portions of the human CXC chemokine IP-10 that are connected directly to each other through amide bonds. In some embodiments, the mimetics are comprised of the human CXC chemokine IP-10 that are connected by disulfide bonds such as, the disulfide bonds that can form between Cys residues.

Any additional amino acids identified in square brackets in a header indicates that the peptide described by the header is at least an analog of a native fragment, and the position of the amino acid substitutions are noted by the standard abbreviation for the amino acid substituent with a superscript number indicating the residue positions at which the modification has occurred.

### Methods of Administration

The terms "administer, "administration," "administering," and the like, can refer to a method of incorporating a compound into a subject to treat, prevent, or ameliorate the symptoms of, an abnormal condition in the subject. When a compound of the invention is provided in combination with one or more additional active agents, the administration can include administering the compound as a prodrug or codrug and can include sequential or concurrent administration with the other agent(s). One of skill will appreciate that any method of administration can be used in the practice of the present invention including, but not limited to, oral, injection, parenteral, dermal, and aerosol applications.

An "effective amount" of a compound of the invention includes a therapeutically effective amount or a prophylatically effective amount. A "therapeutically effective amount" refers to an amount effective, at dosages and for periods of time selected by one of skill, to achieve the desired therapeutic result. The term "therapeutically effective amount" may also refer to that amount of active compound, prodrug or pharmaceutical agent that elicits a biological or medicinal response in a tissue, system, animal or human that is being sought by a researcher, veterinarian, medical doctor or other clinician in order to provide a therapeutic effect.

In some embodiments, a therapeutic effect relieves or prevents, to some extent, one or more of the symptoms of an abnormal condition. In some embodiments, a therapeutic effect can include, but is not limited to, one or more of the following: (a) an increase or decrease in the number of lymphocytic cells present at a specified location; (b) an increase or decrease in the ability of lymphocytic cells to migrate; (c) an increase or decrease in the response of lymphocytic cells to a stimulus; (d) an increase or decrease in the proliferation, growth, and/or differentiation of cells; (e) inhibition (i.e., slowing or stopping) or acceleration of cell death; (f) relieving, to some extent, one or more of the symptoms associated with an abnormal condition; (g) enhancing or inhibiting the function of the affected population of cells; (h) activating an enzyme activity present in cells associated with the abnormal condition; and (i) inhibiting an enzyme activity present in cells associated with the abnormal condition.

A "prophylactically effective amount" refers to an amount administered that is effective, at dosages and for periods of time determined readily by one of skill, at achieving a desired prophylactic result such as, for example, preventing or inhibiting a cytotoxic effect of a cytotoxic agent. Often, a prophylactic dose is used in organisms prior to, or at an early stage of, a disease, and in these embodiments, a prophylactically effective amount may be less than a therapeutically effective amount in some embodiments. The term "preventing" can refer, in some embodiments, to decreasing the probability that an organism contracts or develops an abnormal condition.

In some embodiments, the therapeutically or prophylactically effective amounts of chemokine analogs may range from about 0.1 nM-0.1 M, 0.1 nM-0.05 M, 0.05 nM-15 µM or 0.01 nM-10 µM, or any range therein. One of skill will appreciate that dosage values may vary with the severity of the condition to be alleviated. For any particular subject, specific dosage regimens may be adjusted over time according to the individual need and the professional judgement of the person administering or supervising the administration of the compositions. Accordingly, one of skill will also appreciate that the dosage ranges set forth herein are exemplary only and do not limit the dosage ranges that may be selected by medical practitioners.

The amount of active compound in the composition may vary according to factors such as the disease state, age, sex, and weight of the individual. Dosage regimens may be adjusted to provide the optimum therapeutic response. For example, a single bolus may be administered, several divided doses may be administered over time or the dose may be proportionally reduced or increased as indicated by the exigencies of the therapeutic situation. It may be advantageous to formulate parenteral compositions in dosage unit form for ease of administration and uniformity of dosage.

The term "dosage unit form" as used herein can refer to physically discrete units suited as unitary dosages for subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic effect to be achieved, and (b) the limitations inherent in the art of compounding such an active compound for the treatment of sensitivity in individuals.

The terms "pharmaceutically acceptable carrier" or "excipient" can include any and all solvents, dispersion media, coatings, antibacterial and antifungal agents, isotonic and absorption delaying agents, and the like that are physiologically compatible. In one embodiment, the carrier is suitable for parenteral administration. Alternatively, the carrier can be suitable for intravenous, intraperitoneal, intramuscular, sublingual or oral administration. Pharmaceutically acceptable carriers include sterile aqueous solutions or dispersions and sterile powders for the extemporaneous preparation of sterile injectable solutions or dispersion. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions. Pharmaceutically acceptable carrier "may comprise pharmaceutically acceptable salts."

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, monostearate salts and gelatin.

Moreover, the chemokine analogs may be administered in a time release formulation, for example in a composition which includes a slow release polymer. The active compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are patented or generally known to those skilled in the art.

Additionally, suspensions may be prepared for injection. Suitable lipophilic solvents or vehicles include fatty oils such as sesame oil; or synthetic fatty acid esters, such as ethyl oleate or triglycerides; or liposomes. Suspensions to be used for injection may also contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

Pharmaceutical formulations for parenteral administration may include liposomes. Liposomes and emulsions are well known examples of delivery vehicles or carriers that are especially useful for hydrophobic drugs. Depending on biological stability of the therapeutic reagent, additional strategies for protein stabilization may be employed. Furthermore, one may administer the drug in a targeted drug delivery system, for example, in a liposome coated with target-specific antibody. The liposomes will bind to the target protein and be taken up selectively by the cell expressing the target protein.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof. In accordance with an alternative aspect of the invention, a chemokine analog may be formulated with one or more additional compounds that enhance the solubility of the chemokine analog such as, for example, through pegylation.

If the compounds of the invention are to be administered by inhalation, they may be conveniently delivered in the form of an aerosol spray presentation from pressurized packs or a nebuliser, together with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of gelatin, for example, for use in an inhaler may be formulated containing a powder mix of the compound and a suitable powder base such as starch or lactose.

The following examples illustrate, but do not limit, the present invention.

### EXAMPLE 1

Peptides of the invention may be synthesized chemically using the Fmoc/tBu strategy on a continuous flow peptide synthesizer, as for example has been carried out using the following protocols:

### Reagents (solvents, support, chemicals)

Main Solvent: N,N-Dimethylformamide (DMF): certified ACS spectroanalyzed from Fisher (D131-4) M. W = 73.10. The DMF is treated with activated molecular sieves, type 4A (from BDH: B54005) for at least two weeks then tested with FDNB (2,4-Dinitrofluorobenzene from Eastman).

Procedure: Mix equal volumes of FDNB solution (1mg/ml in 95% EtOH) and DMF; Let stand 30 minutes; read the absorbance at 381 nm over a FDNB blank (0.5ml FDNB + 0.5ml 95% EtOH). If the absorbance ~ 0.2, the DMF is suitable to be used for the synthesis.

Deblocking Agent: 20% Piperidine (from Aldrich Chemical company, catalog No: 10,409-4) in DMF containing 0.5 % Triton X100 v/v (from Sigma, catalg No: T-9284).

Activating Agents: 2-(H-benzotriazol-lyl) 1,1,3,3-tetramethyluronium tetrafluoroborate (TBTU: M.W.=321.09. from Quantum Richilieu, catalog No: R0139)/Hydroxybenzotriazole (HOBt M.W.=135.1 from Quantum Richilieu, catalog No.: R0166-100) respectively, 0.52 M in DMF and 4-Methylmorpholine (NMM; M.W.=101.15, d=0.926 from Aldrich, catalog No.: M5,655-7): 0.9 M in DMF or in the case of sensitive amino acids to racemization like Cys, we use 2,4,6-Collidine, 99% (M.W.=121.18,d=0.917, from Aldrich, catalog No: 14,238-7): 0.78M in DMF/DCM, 1/1 v/v.

Support: TentaGel R RAM (90 µm), RinK-type Fmoc (from Peptides International, catalog No.: RTS -9995-PI): 0.21 mmol/g, 0.5g for 0.1 mmol of peptide.

Fmoc-L-amino derivative, side-chains protected with: Boc; tBu; Trt groups: with 4 fold excess (from Peptides International, Bachem, Novabiochem, Chem-Impex Inc). Glu24 and Lys24 are Allyl-protected (from Millipore/Perseptive Biosystems).

### Initial Amino Loading and Peptide Synthesis Procedure

The first amino acid Asn31 and the remaining residues are double coupled at room temp. or at 45°C automatically with 4-fold excess in each coupling. The synthesis is interrupted after residue Leu19. The peptide-bound support is removed from the synthesizer column and placed in a react-vial containing a small magnetic bar for gentle stirring.

### Removal of The Allyl Groups

A solution of tetrakis(triphenylphosphine)Palladium(0) Pd(PPh3)4 (from Sigma-Aldrich, catalog No: 21,666-6); M.W.=1155.58 x 0.1 mmol peptide x 3 fold = 347mg dissolved in 5% Acetic Acid; 2.5% NMM in CHC13 to 0.14 M, under argon. The solution is added to the support-bound peptide previously removed from the coulmn in a reactvial containing a small mangnetic bar for gentle stirring. The mixture is flushed with argon, sealed and stirred at room temperature for 6 hours. The support-bound peptide is transferred to a filter funnel, washed with 30 ml of a solution made of 0.5% Sodium Diethyldithiocarbonate/ in DMF, then DCM; DCM/DMF (1 : 1) and DMF. A positive Kaiser test indicates the deprotection of the amino side chaine of the Lys20.

### Lactam Formation:

Activating agent: 7-Azabenztriazol-1-yloxytris (pyrrolindino) phosphonium-hexafluorophosphate (PyAOP: M.W.=521.7 from PerSeptive Biosystems GmbH, catalog No: GEN076531), 1.4-fold: 0.105mmol x 1.4 x 521.7 = 76.6mg and NMM 1.5-fold: 0.105 x 1.4 x 1.5 = 0.23 mmol v = 0.23/0.9 M NMM solution = 263 µl).

The cyclisation may be carried out in an amino acid vial at room temperature overnight (~16 hours) with gentle agitation. The completion of cyclization may be indicated by a negative kaiser test. The support-bound peptide may be poured into the column, washed with DMF and the synthesis continues to completion, with a cyclic amide bridge thereby introduced into the peptide.

Final Product Removal From The Support: the support-bound peptide is removed from the synthesizer in to a medium filter funnel, washed with DCM to replace the non-volatile DMF and thoroughly dried under high vacuum for at least two hours, or preferably, overnight. Support: 0.5g resin-peptide.

Cleavage Mixture (reagent K): 100 ml of a trifluoroacetic acid (TFA)/Phenol/Water/Thio-Anisol/EDT (82/5/5/5/2.5)(v/v) mixture is prepared. The support-bound peptide (0.5 g) is poured into 7.5 ml of reagent K with gentle agitation on a rocker, allowed to react for 4 hours at room temperature, filtered, and washed with neat TFA. The 7.5 ml of reagent K contains the following:

| | |
|---|---|
| TFA | 6.15ml (Halocarbon) |
| Phenol | 0.375ml (Aldrich) |
| Water | 0.375ml (MillQ) |
| Thio-Anisol | 0.375ml (Aldrich) |
| EDT | 0.187ml (Aldrich) |
| Total | 7.5ml |

The cleavage may be performed at room temperature for 4 hours with gentle agitation on a rocker.

### Precipitation of the Peptide

The cleaved peptide solution is filtered through a filter funnel in a 50 ml round bottom flask. The support is rinsed twice with 4 ml TFA. The TFA solution is concentrated on a rotavap and added drop wise into a cold diethyl ether previously treated with activated neutral aluminum oxide to make it free of peroxide. Approximately 10-fold excess of ether are used. The beads are stored until the yield is determined and peptide characterized. The precipitate is collected at room temperature in screw capped 50 ml polypropylene vial by centrifugation at 2K rpm using a top bench centrifuge (4 minutes run time). The pellet is washed 3x with cold ether, centrifuged and dried with a flow of argon. The precipitate is dissolved in 20 % acetonitrile, 0.1 % TFA and lyophilized.

### Crude Product Characterization:

The product is characterized by analytical HPLC.
Experimental conditions: Column: Vydac 218TP54: C18 reversed-phase 5 µm, 4.6 mm ID x 150 mm L.
Eluants: 0.1 % TFA/H₂O (solvant A); 0.1% TFA/acetonitrile (solvent B).
Elution Conditions: 20-50% B (40 min); 60-90% B (5 min); 90-20% B (5 min); 20% B (10 min). At 1.0 ml/min and A214 nm = 0.5 absorbance unit full scale.

### Sample Preparation:

An aliquot of the product is weighed and dissolved in 20% acetonitrile 0.1% TFA at a concentration of 2 mg/ml. The solution is microfuged and 20µl is applied onto the column. The main peak or the major peaks are collected, SpeedVac dried and molecular weight determined by mass spectrometry.

### EXAMPLE 2

The following sequences were prepared for testing their ability to bind to an IP-10 receptor and their efficacy in mediating intracellular calcium mobilization ([Ca²⁺]ᵢ) at a variety of concentrations:

| Acetylated-IP-10-(1-16)-[linker]-IP-10-(66-78) |
|---|
| Ac-Val-Pro-Leu-Ser-Arg-Thr-Val-Arg-Cys-Thr-Cys-Ile-Ser-Ile-Ser15- Asn-UDA-Leu66-Lys-Ala-Val-Ser-Lys-Glu-Met-Ser-Lys-Arg-Ser-Pro (SEQ ID NO:1641) |

| Acetylated-[Ala⁹,Phe¹¹]-IP-10-(1-16)-[linker]-IP-10-(66-78) |
|---|
| Ac-Val-Pro-Leu-Ser-Arg-Thr-Val-Arg-Ala9-Thr-Phe11-Ile-Ser-Ile-Ser15- Asn-UDA-Leu66-Lys-Ala-Val-Ser-Lys-Glu-Met-Ser-Lys-Arg-Ser-Pro (SEQ ID NO:1642) |

| [Pro⁷]-IP-10-(1-15)-[linker]-IP-10-(58-71) |
|---|
| Val-Pro-Leu-Ser-Arg-Thr-Pro⁷-Arg-Cys-Thr-Cys-Ile-Ser-Ile-Ser¹⁵- UDA-Glu⁵⁸-Ser-Lys-Ala-Ile-Lys-Asn-Leu-Leu-Lys-Ala-Val-Ser-Lys (SEQ ID NO:1643) |

| [Ser⁹,Ser¹¹,Glu⁶³]-IP-10-(1-15)-[linker]-IP-10-(58-71) |
|---|
| Val-Pro-Leu-Ser-Arg-Thr-Val-Arg-Ser⁹-Thr-Ser¹¹-Ile-Ser-Ile-Ser¹⁵- UDA-Glu⁵⁸-Ser-Lys-Ala-Ile-Glu⁶³-Asn-Leu-Leu-Lys-Ala-Val-Ser-Lys (SEQ ID NO:1644) |

| [Ser⁹,Ser¹¹,Glu⁶⁷]-IP-10-(1-15)-[linker]-IP-10-(58-71) |
|---|
| Val-Pro-Leu-Ser-Arg-Thr-Val-Arg-Ser9-Thr-Ser11-Ile-Ser-Ile-Ser15- UDA-Glu58-Ser-Lys-Ala-Ile-Lys-Asn-Leu-Leu-Glu67-Ala-Val-Ser-Lys (SEQ ID NO:1645) |

wherein, UDA is 11-amino undecanoic acid.

Binding and Calcium Mobilization: Suspensions of CXCR-3/300-19 cells were used to assess binding and intracellular calcium mobilization induced by IP-10 analogs. These are mouse pre-B lymphocytes transfected with the CXCR3 receptor, (*Moser, et al).* The cells were washed in RPMI media and resuspended in RPMI media supplemented with 10% FCS, then plated at 1.2 X 10⁵ cells per well of 96-well black wall/clear bottom plates coated with poly-D-lysine (Becton Dickinson) and loaded with 100 uL fluorescent calcium indicator FLIPR Calcium 3 assay kit component A (Molecular Probes) for 1hr at 37°C. The cells on the plates were then spun at 1000 rpm for 15 minutes at room temperature.

Each of the sequences successfully bound to the cellular receptors. The intracellular calcium mobilization in response to 25 uL (0-100000 nM final concentrations) of the appropriate and various concentrations of analogue was measured at 37°C by monitoring fluorescence as a function of time in all the wells using the Flexstation Fluorometric Imaging Plate Reader (Molecular Devices). All analogues were run simultaneously with rhIP-10 (R&D Systems) as the standard. Table 1 provides the dosage effect of the binding of each of the IP-10 analogs on the calcium mobilization activity of the cells.

**Table 1.**

| | | | | | |
|---|---|---|---|---|---|
| **Peptide Analogs** | **Dosage (µM)** | | | | |
| **(SEQ ID NO:)** | **1.2** | **3.7** | **11.1** | **33.3** | **100** |
| **1641** | 12.6 | 12.7 | 14.1 | 20 | 52.9 |
| **1642** | 15.2 | 13.8 | 15.4 | 20 | 3.1 |
| **1643** | 16 | 14 | 14.4 | 18.6 | 55.1 |
| **1644** | 18.8 | 18.5 | 17.2 | 18.9 | 46.6 |
| **1645** | 17.4 | 15.4 | 12.5 | 13.3 | 3.4 |

FIG. 1 illustates the induction of [Ca²⁺]i mobilization by select IP-10 analogs at a concentration of 100 µM according to some embodiments of the present invention. The results are representative of three independent experiments. SEQ ID NOs: 1641-1645 all bound to the receptor and affected calcium mobilization. SEQ ID NOs: 1641, 1643, and 1644, however, increased calcium mobilization by 300 to over 500%. The results are compared to a recombinant human IP-10 chemokine, as described above.

The acetylated-IP-10-(1-16)-[linker]-IP-10-(66-78) analog represented by SEQ ID NO:1641 increased intracellular calcium mobilization by nearly 500%, but an [Ala⁹,Phe¹¹] amino acid substitution in the same type of analog decreased the effect on calcium mobilization dramatically as shown by the effects of SEQ ID NO: 1642.

In an IP-10-(1-15)-[linker]-IP-10-(58-71) analog, a [Pro⁷] amino acid substitution in SEQ ID NO:1643 resulted in an increase in intracellular calcium mobilization when compared to the results of SEQ ID NO:1641. A [Ser⁹,Ser¹¹,Glu⁶³] amino acid substitution of the same type of analog still provide a very substantial increase in intracellular calcium mobilization of over 300% using SEQ ID NO:1644. Interestingly, however, a [Glu⁶⁷] amino acid substitution decreased the effect on calcium mobilization dramatically as shown by the effect of SEQ ID NO:1645.

The results provided by this example show that IP-10 analogs having a total of about 30 amino acids and conserving N-terminal residues 1-15 and C-terminal residues 66-71 of the IP-10 chemokine are effective at binding and can increase the cellular activity induced by the binding to different degrees, depending on the dosage of the analog administered and the presence of amino acid substitutions. In particular, the results suggest that the Cys⁹ and Cys¹¹ residues can be substituted with Ser⁹ and Ser¹¹ in the conserved N-terminal 1-15 region with little effect, and Lys⁶³ can be substituted by Glu⁶³ with little effect, but substantial differences in results occur where Lys⁶⁷ is substituted with Glu⁶⁷, which is in the range of the conserved C-terminal region of 66-71.

Accordingly, an IP-10 analog that is supported by these results would range from about 21 to about 34 amino acids in length and comprise:
an N-terminal region comprising and conserving the IP-10 chemokine residues 1-15
   R-Val-Pro-Leu-Ser-Arg-Thr-Val-Arg-Cys-Thr-Cys-Ile-Ser-Ile-Ser¹⁵-(OH)NH₂ (SEQ ID NO:1646), and conservatively modified variants thereof,
a C-terminal region comprising and conserving the IP-10 chemokine residues 66-71, and conservatively modified variants thereof,
   Leu⁶⁶-Lys-Ala-Val-Ser-Lys⁷¹-(OH)NH₂ (SEQ ID NO:1647), and conservatively modified variants thereof,
and an optional linker having up to 4 amino acids, wherein the linker is preferably 11-aminoundecanoic acid.

### EXAMPLE 3

An IP-10 can be modified with a polysaccharide such as heparin. The glycosaminoglycan can be connected to an amine functional group as an aldehyde-terminated heparin. An example of an aldehyde-terminated heparin is represented by the following formula: 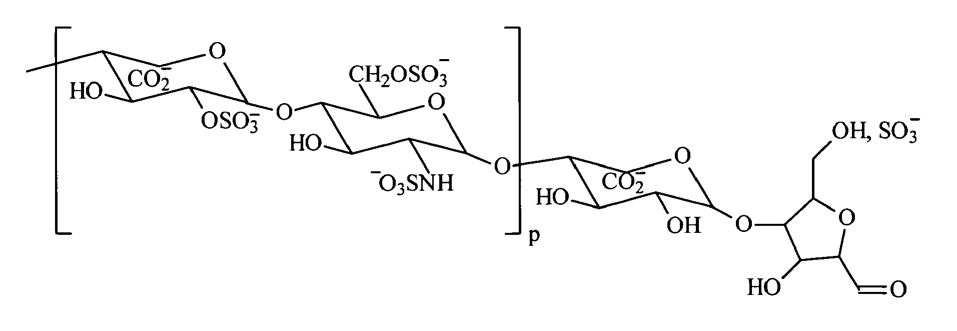 wherein p is an integer not equal to 0.

The aldehyde-terminated heparin can be combined with the amine functional group in a DMF/water solvent and subsequently reduced with NaCNBH₃ to produce the following structure:

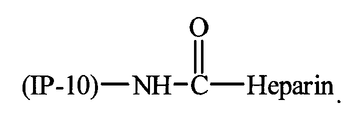

### EXAMPLE 4

An IP-10 mimetic can be modified with a polyalkylene glycol using a variety of techniques. There are a variety of available PEG sizes and derivatives that are commercially designed for specific applications such as, for example, attachment to a variety of different chemical functionalities including, but not limited to, amines, thiols, hydroxyls, sulfhydryls, and carboxyls.

In one example, an amine group of an IP-10 can be combined with a carboxyl-terminated PEG (Nektar Corp.) in the presence of, for example, EDC or DCC to form the following structure: 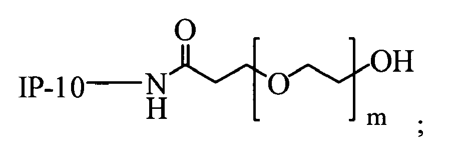 wherein m is an integer not equal to 0.

In another example, either a succinimidyl derivative of mPEG (Nektar Corp.) or an isocyanate-terminated mPEG (Nektar Corp.) can be combined with an IP-10 mimetic under conditions known to those of skill in the art. In another example, the carboxyl group of an IP-10 mimetic can be activated with, for example, EDC or DCC and combined with an amino-terminated mPEG (Nektar Corp.) In another example, an amine group of an IP-10 mimetic can be combined with a methacrylate-terminated mPEG (Nektar Corp.) in the presence of an initiator capable of undergoing thermal or photolytic free radical decomposition. Examples of suitable initiators include benzyl-N,N-diethyldithiocarbamate or *p*-xylene-N,N-diethyldithiocarbamate.

The invention is not limited in its application to the details of structures and the arrangements of components set forth herein. It should be understood that although the present invention has been disclosed using select embodiments and optional features, modifications and variations of the inventions can be readily made by those skilled in the art, and that such modifications and variations are considered to be within the scope of the inventions.

The inventions have been described broadly and generically herein, and Applicant includes an inherent proviso, or negative limitation, that will allow removing any species subject matter from genus teachings, regardless or whether or not the material to be removed was specifically recited or stated as being removable. Some changes can be made in the form and detail of the teachings by one of skill without departing from the spirit and the scope of the invention. All patents and publications described herein are hereby incorporated by reference to the same extent as if each individual patent or publication was specifically and individually indicated to be incorporated by reference herein in its entirety.

## Claims

1. A composition comprising an IP-10 chemokine analog having a length ranging from about 21 to about 34 amino acids and comprising:
an N-terminal region having a first conserved sequence consisting of the IP-10 chemokine residues 1-15,
Val¹-Pro-Leu-Ser-Arg-Thr-Val-Arg-Cys-Thr-Cys-Ile-Ser-Ile-Ser¹⁵ (SEQ ID NO:1646), and conservatively modified variants thereof;
a C-terminal region having a second conserved sequence consisting of the IP-10 chemokine residues 66-71,
Leu⁶⁶-Lys-Ala-Val-Ser-Lys⁷¹ (SEQ ID NO:1647), and conservatively modified variants thereof; and,
a linker consisting of up to 4 amino acids; wherein,
if the N-terminal region is composed of more than 16 residues, then at least Lys⁶³ or Lys⁶⁷ of the C-terminal region is substituted with Glu;
the N-terminus of the IP-10 analog consists of a hydrogen or is modified using an N-terminal modifier comprising a component selected from a group consisting of a poly(ethylene glycol) or derivative thereof, a glycosaminoglycan, a diagnostic label, a radioactive group, an acyl group, an acetyl group, a peptide, and a modifier capable of reducing the ability of the IP-10 analog to act as a substrate for aminopeptidases; and,
the linker is selected from a group consisting of (a) up to four natural amino acids, and (b) any non-natural amino acid having the following structure: 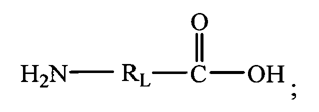
wherein, R_{L} is selected from a group consisting of saturated and unsaturated aliphatics and heteroaliphatics consisting of 20 or fewer carbon atoms that are optionally substituted with a hydroxyl, carboxyl, amino, amido, or imino group; or an aromatic group having from 5 to 7 members in the ring; and -(CH₂)ₙ-, wherein n is an integer ranging from 1 to 20.

2. The composition of claim 1 having a length of about 26 to 32 amino acid residues and comprising:
an N-terminal region consisting essentially of SEQ ID NO:1646 and conservatively modified variants thereof in residues 1-15, and
a C-terminal region comprising SEQ ID NO:1647 and conservatively modified variants thereof.

3. The composition of claim 1, wherein the linker is 11-aminoundecanoic acid.

4. The analog of claim 1 comprising an amino acid sequence selected from a group consisting of SEQ ID N0:1641, SEQ ID NO:1642, SEQ ID NO:1643, SEQ ID NO:1644, and SEQ ID NO: 1645.

5. The composition of claim 1 comprising an amino acid sequence selected from a group consisting of SEQ ID NOs:296 - 349 and 404 - 457, variants b134 - b187 and b242 - b295, inclusive; wherein, Xaa₁, Xaa₂, Xaa₃, and Xaa₄ are each independently selected from a group consisting of any natural amino acid or any non-natural amino acid having the following structure: 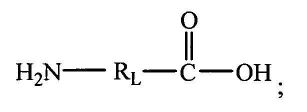 wherein, R_{L} is selected from a group consisting of saturated and unsaturated aliphatics and heteroaliphatics consisting of 20 or fewer carbon atoms that are optionally substituted with a hydroxyl, carboxyl, amino, amido, or imino group; or an aromatic group having from 5 to 7 members in the ring; and -(CH₂)ₙ-, wherein n is an integer ranging from 1 to 20.

6. The composition of claim 5, wherein the linker is 11-aminoundecanoic acid.

7. The composition of claim 1 comprising an amino acid sequence selected from a group consisting of SEQ ID NOs:494 - 548 and 675 - 728, variants b332 - b385 and b512 - b566, inclusive; wherein, Xaa₁, Xaa₂, Xaa₃, and Xaa₄ are each independently selected from a group consisting of any natural amino acid or any non-natural amino acid having the following structure: 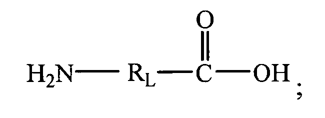 wherein, R_{L} is selected from a group consisting of saturated and unsaturated aliphatics and heteroaliphatics consisting of 20 or fewer carbon atoms that are optionally substituted with a hydroxyl, carboxyl, amino, amido, or imino group; or an aromatic group having from 5 to 7 members in the ring; and -(CH₂)ₙ-, wherein n is an integer ranging from 1 to 20.

8. The composition of claim 5, wherein the linker is 11-aminoundecanoic acid.

9. A method of increasing the IP-10-mediated activity of a cell having a receptor capable of binding to an IP-10 analog, comprising:
binding the receptor to an IP-10 analog having a length ranging from about 21 to about 34 amino acids and comprising:
an N-terminal region having a first conserved sequence consisting of the IP-10 chemokine residues 1-15,
Val¹-Pro-Leu-Ser-Arg-Thr-Val-Arg-Cys-Thr-Cys-Ile-Ser-Ile-Ser¹⁵ (SEQ ID NO:1646), and conservatively modified variants thereof;
a C-terminal region having a second conserved sequence consisting of the IP-10 chemokine residues 66-71,
Leu⁶⁶-Lys-Ala-Val-Ser-Lys⁷¹ (SEQ ID NO:1647), and conservatively modified variants thereof; and,
a linker consisting of up to 4 amino acids; wherein,
if the N-terminal region is composed of more than 16 residues, then at least Lys⁶³ or Lys ⁶⁷ of the C-terminal region is substituted with Glu;
the N-terminus of the IP-10 analog consists of a hydrogen or is modified using an N-terminal modifier comprising a component selected from a group consisting of a poly(ethylene glycol) or derivative thereof, a glycosaminoglycan, a diagnostic label, a radioactive group, an acyl group, an acetyl group, a peptide, and a modifier capable of reducing the ability of the IP-10 analog to act as a substrate for aminopeptidases;
and the linker is selected from a group consisting of (a) up to four natural amino acids, and (b) any non-natural amino acid having the following structure: 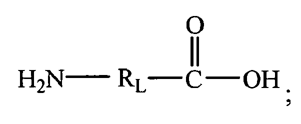
wherein, R_{L} is selected from a group consisting of saturated and unsaturated aliphatics and heteroaliphatics consisting of 20 or fewer carbon atoms that are optionally substituted with a hydroxyl, carboxyl, amino, amido, or imino group; or an aromatic group having from 5 to 7 members in the ring; and -(CH₂)ₙ-, wherein n is an integer ranging from 1 to 20.

10. The method of claim 9, wherein the IP-10 analog has a length of about 26 to 32 amino acid residues and comprises:
an N-terminal region consisting essentially of SEQ ID NO:1646 and conservatively modified variants thereof in residues 1-15, and
a C-terminal region comprising SEQ ID NO:1647 and conservatively modified variants thereof.

11. The method of claim 9, wherein the linker is 11-aminoundecanoic acid.

12. The method of claim 9, wherein the IP-10 analog comprises an amino acid sequence selected from a group consisting of SEQ ID NO:1641, SEQ ID NO:1642, SEQ ID NO: 1643, SEQ ID NO:1644, and SEQ ID NO: 1645.

13. The method of claim 9 comprising an amino acid sequence selected from a group consisting of SEQ ID NOs:296 - 349 and 404 - 457, variants b134 - b187 and b242 - b295, inclusive; wherein, Xaa₁, Xaa₂, Xaa₃, and Xaa₄ are each independently selected from a group consisting of any natural amino acid or any non-natural amino acid having the following structure: 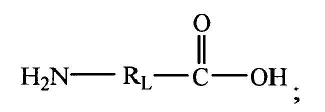 wherein, R_{L} is selected from a group consisting of saturated and unsaturated aliphatics and heteroaliphatics consisting of 20 or fewer carbon atoms that are optionally substituted with a hydroxyl, carboxyl, amino, amido, or imino group; or an aromatic group having from 5 to 7 members in the ring; and -(CH₂)ₙ-, wherein n is an integer ranging from 1 to 20.

14. The method of claim 13, wherein the linker is 11-aminoundecanoic acid.

15. The method of claim 9 comprising an amino acid sequence selected from a group consisting of SEQ ID NOs:494 - 548 and 675 - 728, variants b332 - b385 and b512 - b566, inclusive; wherein, Xaa₁, Xaa₂, Xaa₃, and Xaa₄ are each independently selected from a group consisting of any natural amino acid or any non-natural amino acid having the following structure: 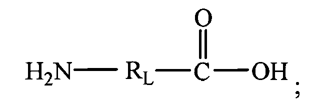 wherein, R_{L} is selected from a group consisting of saturated and unsaturated aliphatics and heteroaliphatics consisting of 20 or fewer carbon atoms that are optionally substituted with a hydroxyl, carboxyl, amino, amido, or imino group; or an aromatic group having from 5 to 7 members in the ring; and -(CH₂)ₙ-, wherein n is an integer ranging from 1 to 20.

16. The method of claim 15, wherein the linker is 11-aminoundecanoic acid.

17. An antibody produced using the IP-10 analog of claim 1 as an antigen.

18. The antibody of claim 17, wherein the antibody is monoclonal.

19. The antibody of claim 17, wherein the antibody is humanized.

20. The antibody of claim 17, wherein the antigen is the IP-10 analog of claim 4.
